# EUROPEAN PATENT SPECIFICATION

(11) **EP 1 786 899 B1**
(45) Date of publication and mention of the grant of the patent: **27.06.2012**
(21) Application number: 05783597.7
(22) Date of filing: 09.09.2005
(51) Int. Cl.: C12N 15/53, C12N 9/02, C12N 1/20, C12P 13/14, C12R 1/15

(54) **L-GLUTAMIC ACID-PRODUCING MICROORGANISM AND A METHOD FOR PRODUCING L-GLUTAMIC ACID**
L-GLUTAMINSÄURE PRODUZIERENDE MIKROORGANISMEN UND METHODE ZUR HERSTELLUNG VON L-GLUTAMINSÄURE
MICRO-ORGANISME PRODUCTEUR D'ACIDE L-GLUTAMIQUE ET PROCEDE DE PRODUCTION DE L'ACIDE L-GLUTAMIQUE

(30) Priority: 10.09.2004 JP 2004264458
(43) Date of publication of application: 23.05.2007
(73) Proprietor: Ajinomoto Co., Inc., Tokyo 104-8315 (JP)
(72) Inventor: HIRANO, Seiko, c/o Ajinomoto Co. Inc., Kanagawa 210-8681 (JP); YAMAGUCHI, Mikiko, c/o Ajinomoto Co. Inc., Kanagawa 210-8681 (JP); NAKAMURA, Jun, c/o Ajinomoto Co. Inc., Kanagawa 210-8681 (JP); ITO, Hisao, c/o Ajinomoto Co. Inc., Kanagawa 210-8681 (JP)
(74) Representative: HOFFMANN EITLE
(86) International application number: PCT/JP2005/017098
(87) International publication number: WO 2006/028298

(56) References cited:
- EP-A- 0 771 879
- KIMURA E: "METABOLIC ENGINEERING OF GLUTAMATE PRODUCTION" ADVANCES IN BIOCHEMICAL ENGINEERING, BIOTECHNOLOGY, SPRINGER, BERLIN, DE, vol. 79, 2003, pages 37-57, XP008048189 ISSN: 0724-6145
- USUDA Y ET AL: "MOLECULAR CLONING OF THE CORYNEBACTERIUM GLUTAMICUM ('BREVIBACTERIUM LACTOFERMENTUM' AJ12036) ODHA GENE ENCODING A NOVEL TYPE OF 2-OXOGLUTARATE DEHYDROGENASE" MICROBIOLOGY, SOCIETY FOR GENERAL MICROBIOLOGY, READING, GB, vol. 142, no. PART 12, December 1996 (1996-12), pages 3347-3354, XP001037496 ISSN: 1350-0872

### SEQUENCE LISTING

<110> Ajinomoto Co., Inc.
<120> C453-C5043
<130> L-glutamic acid-producing microorganism and a method for producing L-glutamic acid
<150> JP2004-264458
   <151> 2004-09-10
<160> 51
<170> PatentIn version 3.1
<210> 1
   <211> 40
   <212> DNA
   <213> Artificial
<220>
   <223> primer
<400> 1
   gccgggatcc tccggtgaat tcctgcgtac catgtctcgc 40
<210> 2
   <211> 40
   <212> DNA
   <213> Artificial
<220>
   <223> primer
<400> 2
   gccgggatcc ctgtgtgatt cacactgcat aaggccctct 40
<210> 3
   <211> 24
   <212> DNA
   <213> Artificial
<220>
   <223> primer
<400> 3 24
   gcttccggct cgtatgttgt gtgg 24
<210> 4
   <211> 20
   <212> DNA
   <213> Artificial
<220>
   <223> primer
<400> 4 20
   gatcgtgacc gcacagattc 20
<210> 5
   <211> 40
   <212> DNA
   <213> Artificial
<220>
   <223> primer
<400> 5 40
   gccgggatcc ccatcgccgc catccctgat ggtttcaatc 40
<210> 6
   <211> 40
   <212> DNA
   <213> Artificial
<220>
   <223> primer
<400> 6 40
   gccgggatcc ggccctggcc tgcggcggtg tcgatggcgg 40
<210> 7
   <211> 33
   <212> DNA
   <213> Artificial
<220>
   <223> primer
<400> 7 33
   gctaagcgcg tcgacgtcgg aggcaccatc cac 33
<210> 8
   <211> 42
   <212> DNA
   <213> Artificial
<220>
   <223> primer
<400> 8
   ctcctaccat cgacggcagg ccccaaccct gatcgtgcct ga 42
<210> 9
   <211> 4394
   <212> DNA
   <213> Corynebacterium glutamicum
<220>
   <221> CDS
   <222> (443)..(4213)
   <223>
<400> 9
   gtcgacaagc aaaatcgaag cggcagcacg ccgcgtcgga gccttaaacg ccatcgccgc      60
   catccctgat ggtttcaatc atcaagtcgg tgaacgcggg cgcaacctgt catccggaca     120
   gcgccaactg atcgcgctgg cgcgcgccga actcatcgag ccttccatca tgcttctcga     180
   cgaagccacc tccaccctcg accccgccac cgaagccgtt atcctcaacg cctccgatcg     240
   agtcactaag ggacgcacca gcatcatcgt cgcgcaccgc ttggcaaccg ctaaaagggc     300
   cgaccgtatt cttgttgttg aacaaggacg tatcattgag gacggatctc acgacgcgtt     360
   gttgtctgct aacggcacct acgcccgcat gtggcattta atggcctgac acgttatttt     420
   taggagaact gtcaacaaat ta atg cta caa ctg ggg ctt agg cat aat cag      472
                            Met Leu Gln Leu Gly Leu Arg His Asn Gln
                            1               5                   10
   cca acg acc aac gtt aca gtg gat aaa ata aag ctc aat aaa ccc tca      520
   Pro Thr Thr Asn Val Thr Val Asp Lys Ile Lys Leu Asn Lys Pro Ser
                   15                  20                  25
   aga agc aag gaa aag agg cga gta cct gcc gtg agc agc gct agt act      568
   Arg Ser Lys Glu Lys Arg Arg Val Pro Ala Val Ser Ser Ala Ser Thr
               30                  35                  40
   ttc ggc cag aat gcg tgg ctg gta gac gag atg ttc cag cag ttc cag      616
   Phe Gly Gln Asn Ala Trp Leu Val Asp Glu Met Phe Gln Gln Phe Gln
           45                  50                  55
   aag gac ccc aag tcc gtg gac aag gaa tgg aga gaa ctc ttt gag gcg      664
   Lys Asp Pro Lys Ser Val Asp Lys Glu Trp Arg Glu Leu Phe Glu Ala
   60                  65                  70
   cag ggg gga cca aat gct acc ccc gct aca aca gaa gca cag cct tca     712
   Gln Gly Gly Pro Asn Ala Thr Pro Ala Thr Thr Glu Ala Gln Pro Ser
   75                  80                  85                  90
   gcg ccc aag gag tct gcg aaa cca gca cca aag gct gcc cct gca gcc     760
   Ala Pro Lys Glu Ser Ala Lys Pro Ala Pro Lys Ala Ala Pro Ala Ala
                   95                  100                 105
   aag gca gca ccg cgc gta gaa acc aag ccg gcc gcc aag acc gcc cct     808
   Lys Ala Ala Pro Arg Val Glu Thr Lys Pro Ala Ala Lys Thr Ala Pro
               110                 115                 120
   aag gcc aag gag tcc tca gtg cca cag caa cct aag ctt ccg gag cca     856
   Lys Ala Lys Glu Ser Ser Val Pro Gln Gln Pro Lys Leu Pro Glu Pro
           125                 130                 135
   gga caa acc cca atc agg ggt att ttc aag tcc atc gcg aag aac atg     904
   Gly Gln Thr Pro Ile Arg Gly Ile Phe Lys Ser Ile Ala Lys Asn Met
       140                 145                 150
   gat atc tcc ctg gaa atc cca acc gca acc tcg gtt cgc gat atg cca     952
   Asp Ile Ser Leu Glu Ile Pro Thr Ala Thr Ser Val Arg Asp Met Pro
   155                 160                 165                 170
   gct cgc ctc atg ttc gaa aac cgc gcg atg gtc aac gat cag ctc aag     1000
   Ala Arg Leu Met Phe Glu Asn Arg Ala Met Val Asn Asp Gln Leu Lys
                   175                 180                 185
   cgc acc cgc ggt ggc aag atc tcc ttc acc cac atc att ggc tac gcc     1048
   Arg Thr Arg Gly Gly Lys Ile Ser Phe Thr His Ile Ile Gly Tyr Ala
               190                 195                 200
   atg gtg aag gca gtc atg gct cac ccg gac atg aac aac tcc tac gac     1096
   Met Val Lys Ala Val Met Ala His Pro Asp Met Asn Asn Ser Tyr Asp
           205                 210                 215
   gtc atc gac ggc aag cca acc ctg atc gtg cct gag cac atc aac ctg     1144
   Val Ile Asp Gly Lys Pro Thr Leu Ile Val Pro Glu His Ile Asn Leu
       220                 225                 230
   ggc ctt gcc atc gac ctt cct cag aag gac ggc tcc cgc gca ctt gtc     1192
   Gly Leu Ala Ile Asp Leu Pro Gln Lys Asp Gly Ser Arg Ala Leu Val
   235                 240                 245                 250
   gta gca gcc atc aag gaa acc gag aag atg aac ttc tcc gag ttc ctc     1240
   Val Ala Ala Ile Lys Glu Thr Glu Lys Met Asn Phe Ser Glu Phe Leu
                   255                 260                 265
   gca gca tac gaa gac atc gtg aca cgc tcc cgc aag ggc aag ctc acc     1288
   Ala Ala Tyr Glu Asp Ile Val Thr Arg Ser Arg Lys Gly Lys Leu Thr
               270                 275                 280
   atg gat gac tac cag ggc gtt acc gtt tcc ttg acc aac cca ggt ggc     1336
   Met Asp Asp Tyr Gln Gly Val Thr Val Ser Leu Thr Asn Pro Gly Gly
           285                 290                 295
   atc ggt acc cgc cac tct gtc cca cgt ctg acc aag ggc cag ggc acc     1384
   Ile Gly Thr Arg His Ser Val Pro Arg Leu Thr Lys Gly Gln Gly Thr
       300                 305                 310
   atc atc ggt gtc ggt tcc atg gat tac cca gca gag ttc cag ggc gct     1432
   Ile Ile Gly Val Gly Ser Met Asp Tyr Pro Ala Glu Phe Gln Gly Ala
   315                 320                 325                 330
   tcc gaa gac cgc ctt gca gag ctc ggc gtt gga aag ctt gtc acc atc      1480
   Ser Glu Asp Arg Leu Ala Glu Leu Gly Val Gly Lys Leu Val Thr Ile
                   335                 340                 345
   acc tcc acc tac gat cac cgc gtg atc cag ggt gct gtg tcc ggt gaa      1528
   Thr Ser Thr Tyr Asp His Arg Val Ile Gln Gly Ala Val Ser Gly Glu
               350                 355                 360
   ttc ctg cgt acc atg tct cgc ctg ctc acc gat gat tcc ttc tgg gat      1576
   Phe Leu Arg Thr Met Ser Arg Leu Leu Thr Asp Asp Ser Phe Trp Asp
           365                 370                 375
   gag atc ttc gac gca atg aac gtt cct tac acc cca atg cgt tgg gca      1624
   Glu Ile Phe Asp Ala Met Asn Val Pro Tyr Thr Pro Met Arg Trp Ala
       380                 385                 390
   cag gac gtt cca aac acc ggt gtt gat aag aac acc cgc gtc atg cag      1672
   Gln Asp Val Pro Asn Thr Gly Val Asp Lys Asn Thr Arg Val Met Gln
   395                 400                 405                 410
   ctc att gag gca tac cgc tcc cgt gga cac ctc atc gct gac acc aac      1720
   Leu Ile Glu Ala Tyr Arg Ser Arg Gly His Leu Ile Ala Asp Thr Asn
                   415                 420                 425
   cca ctt tca tgg gtt cag cct ggc atg cca gtt cca gac cac cgc gac      1768
   Pro Leu Ser Trp Val Gln Pro Gly Met Pro Val Pro Asp His Arg Asp
               430                 435                 440
   ctc gac atc gag acc cac agc ctg acc atc tgg gat ctg gac cgt acc      1816
   Leu Asp Ile Glu Thr His Ser Leu Thr Ile Trp Asp Leu Asp Arg Thr
           445                 450                 455
   ttc agc gtc ggt ggc ttc ggc ggc aag gag acc atg acc ctg cgc gag      1864
   Phe Ser Val Gly Gly Phe Gly Gly Lys Glu Thr Met Thr Leu Arg Glu
       460                 465                 470
   gta ctg tcc cgc ctg cgc gct gcc tac acc ttg aag gtc ggc tcc gaa      1912
   Val Leu Ser Arg Leu Arg Ala Ala Tyr Thr Leu Lys Val Gly Ser Glu
   475                 480                 485                 490
   tac acc cac atc ctg gac cgc gac gag cgc acc tgg ctg cag gac cgc      1960
   Tyr Thr His Ile Leu Asp Arg Asp Glu Arg Thr Trp Leu Gln Asp Arg
                   495                 500                 505
   ctc gaa gcc gga atg cca aag cca acc cag gca gag cag aag tac atc      2008
   Leu Glu Ala Gly Met Pro Lys Pro Thr Gln Ala Glu Gln Lys Tyr Ile
               510                 515                 520
   ctg cag aag ctg aac gcc gca gag gct ttc gag aac ttc ctg cag acc      2056
   Leu Gln Lys Leu Asn Ala Ala Glu Ala Phe Glu Asn Phe Leu Gln Thr
           525                 530                 535
   aag tac gtc ggc cag aag cgc ttc tcc ctc gaa ggt gca gaa gct ctc      2104
   Lys Tyr Val Gly Gln Lys Arg Phe Ser Leu Glu Gly Ala Glu Ala Leu
       540                 545                 550
   atc cca ctg atg gac tcc gcc atc gac acc gcc gca ggc cag ggc ctc      2152
   Ile Pro Leu Met Asp Ser Ala Ile Asp Thr Ala Ala Gly Gln Gly Leu
   555                 560                 565                 570
   gac gaa gtt gtc atc ggt atg cca cac cgt ggt cgc ctc aac gtg ctg      2200
   Asp Glu Val Val Ile Gly Met Pro His Arg Gly Arg Leu Asn Val Leu
   575 580 585
   ttc aac atc gtg ggc aag cca ctg gca tcc atc ttc aac gag ttt gaa 2248
   Phe Asn Ile Val Gly Lys Pro Leu Ala Ser Ile Phe Asn Glu Phe Glu
   590 595 600
   ggc caa atg gag cag ggc cag atc ggt ggc tcc ggt gac gtg aag tac 2296
   Gly Gln Met Glu Gln Gly Gln Ile Gly Gly Ser Gly Asp Val Lys Tyr
   605 610 615
   cac ctc ggt tcc gaa ggc cag cac ctg cag atg ttc ggc gac ggc gag 2344
   His Leu Gly Ser Glu Gly Gln His Leu Gln Met Phe Gly Asp Gly Glu
   620 625 630
   atc aag gtc tcc ctg act gct aac ccg tcc cac ctg gaa gct gtt aac 2392
   Ile Lys Val Ser Leu Thr Ala Asn Pro Ser His Leu Glu Ala Val Asn
   635 640 645 650
   cca gtg atg gaa ggt atc gtc cgc gca aag cag gac tac ctg gac aag 2440
   Pro Val Met Glu Gly Ile Val Arg Ala Lys Gln Asp Tyr Leu Asp Lys
   655 660 665
   ggc gta gac ggc aag act gtt gtg cca ctg ctg ctc cac ggt gac gct 2488
   Gly Val Asp Gly Lys Thr Val Val Pro Leu Leu Leu His Gly Asp Ala
   670 675 680
   gca ttc gca ggc ctg ggc atc gtg cca gaa acc atc aac ctg gct aag 2536
   Ala Phe Ala Gly Leu Gly Ile Val Pro Glu Thr Ile Asn Leu Ala Lys
   685 690 695
   ctg cgt ggc tac gac gtc gga ggc acc atc cac atc gtg gtg aac aac 2584
   Leu Arg Gly Tyr Asp Val Gly Gly Thr Ile His Ile Val Val Asn Asn
   700 705 710
   cag atc ggc ttc acc acc acc cca gac tcc agc cgc tcc atg cac tac 2632
   Gln Ile Gly Phe Thr Thr Thr Pro Asp Ser Ser Arg Ser Met His Tyr
   715 720 725 730
   gca acc gac tac gcc aag gca ttc ggc tgc cca gtc ttc cac gtc aat 2680
   Ala Thr Asp Tyr Ala Lys Ala Phe Gly Cys Pro Val Phe His Val Asn
   735 740 745
   ggt gat gac cca gag gca gtt gtc tgg gtt ggc cag ctg gca acc gag 2728
   Gly Asp Asp Pro Glu Ala Val Val Trp Val Gly Gln Leu Ala Thr Glu
   750 755 760
   tac cgt cgt cgc ttc ggc aag gac gtc ttc atc gac ctc gtt tgc tac 2776
   Tyr Arg Arg Arg Phe Gly Lys Asp Val Phe Ile Asp Leu Val Cys Tyr
   765 770 775
   cgc ctc cgc ggc cac aac gaa gct gat gat cct tcc atg acc cag cca 2824
   Arg Leu Arg Gly His Asn Glu Ala Asp Asp Pro Ser Met Thr Gln Pro
   780 785 790
   aag atg tat gag ctc atc acc ggc cgc gag acc gtt cgt gct cag tac 2872
   Lys Met Tyr Glu Leu Ile Thr Gly Arg Glu Thr Val Arg Ala Gln Tyr
   795 800 805 810
   acc gaa gac ctg ctc gga cgt gga gac ctc tcc aac gaa gat gca gaa 2920
   Thr Glu Asp Leu Leu Gly Arg Gly Asp Leu Ser Asn Glu Asp Ala Glu
   815 820 825
   gca gtc gtc cgc gac ttc cac gac cag atg gaa tct gtg ttc aac gaa 2968
   Ala Val Val Arg Asp Phe His Asp Gln Met Glu Ser Val Phe Asn Glu
   830 835 840
   gtc aag gaa ggc ggc aag aag cag gct gag gca cag acc ggc atc acc 3016
   Val Lys Glu Gly Gly Lys Lys Gln Ala Glu Ala Gln Thr Gly Ile Thr
   845 850 855
   ggc tcc cag aag ctt cca cac ggc ctt gag acc aac atc tcc cgt gaa 3064
   Gly Ser Gln Lys Leu Pro His Gly Leu Glu Thr Asn Ile Ser Arg Glu
   860 865 870
   gag ctc ctg gaa ctg gga cag gct ttc gcc aac acc cca gaa ggc ttc 3112
   Glu Leu Leu Glu Leu Gly Gln Ala Phe Ala Asn Thr Pro Glu Gly Phe
   875 880 885 890
   aac tac cac cca cgt gtg gct cca gtt gct aag aag cgc gtc tcc tct 3160
   Asn Tyr His Pro Arg Val Ala Pro Val Ala Lys Lys Arg Val Ser Ser
   895 900 905
   gtc acc gaa ggt ggc atc gac tgg gca tgg ggc gag ctc ctc gcc ttc 3208
   Val Thr Glu Gly Gly Ile Asp Trp Ala Trp Gly Glu Leu Leu Ala Phe
   910 915 920
   ggt tcc ctg gct aac tcc ggc cgc ttg gtt cgc ctt gca ggt gaa gat 3256
   Gly Ser Leu Ala Asn Ser Gly Arg Leu Val Arg Leu Ala Gly Glu Asp
   925 930 935
   tcc cgc cgc ggt acc ttc acc cag cgc cac gca gtt gcc atc gac cca 3304
   Ser Arg Arg Gly Thr Phe Thr Gln Arg His Ala Val Ala Ile Asp Pro
   940 945 950
   gcg acc gct gaa gag ttc aac cca ctc cac gag ctt gca cag tcc aag 3352
   Ala Thr Ala Glu Glu Phe Asn Pro Leu His Glu Leu Ala Gln Ser Lys
   955 960 965 970
   ggc aac aac ggt aag ttc ctg gtc tac aac tcc gca ctg acc gag tac 3400
   Gly Asn Asn Gly Lys Phe Leu Val Tyr Asn Ser Ala Leu Thr Glu Tyr
   975 980 985
   gca ggc atg ggc ttc gag tac ggc tac tcc gta gga aac gaa gac tcc 3448
   Ala Gly Met Gly Phe Glu Tyr Gly Tyr Ser Val Gly Asn Glu Asp Ser
   990 995 1000
   gtc gtt gca tgg gaa gca cag ttc ggc gac ttc gcc aac ggc gct 3493
   Val Val Ala Trp Glu Ala Gln Phe Gly Asp Phe Ala Asn Gly Ala
   1005 1010 1015
   cag acc atc atc gat gag tac gtc tcc tca ggc gaa gct aag tgg 3538
   Gln Thr Ile Ile Asp Glu Tyr Val Ser Ser Gly Glu Ala Lys Trp
   1020 1025 1030
   ggc cag acc tcc aag ctg atc ctt ctg ctg cct cac ggc tac gaa 3583
   Gly Gln Thr Ser Lys Leu Ile Leu Leu Leu Pro His Gly Tyr Glu
   1035 1040 1045
   ggc cag ggc cca gac cac tct tcc gca cgt atc gag cgc ttc ctg 3628
   Gly Gln Gly Pro Asp His Ser Ser Ala Arg Ile Glu Arg Phe Leu
   1050 1055 1060
   cag ctg tgc gct gag ggt tcc atg act gtt gct cag cca tcc acc 3673
   Gln Leu Cys Ala Glu Gly Ser Met Thr Val Ala Gln Pro Ser Thr
   1065 1070 1075
   cca gca aac cac ttc cac ctg ctg cgt cgt cac gct ctg tcc gac 3718
   Pro Ala Asn His Phe His Leu Leu Arg Arg His Ala Leu Ser Asp
           1080                1085                1090
   ctg aag cgt cca ctg gtt atc ttc acc ccg aag tcc atg ctg cgt      3763
   Leu Lys Arg Pro Leu Val Ile Phe Thr Pro Lys Ser Met Leu Arg
           1095                1100                1105
   aac aag gct gct gcc tcc gca cca gaa gac ttc act gag gtc acc      3808
   Asn Lys Ala Ala Ala Ser Ala Pro Glu Asp Phe Thr Glu Val Thr
           1110                1115                1120
   aag ttc caa tcc gtg atc gac gat cca aac gtt gca gat gca gcc      3853
   Lys Phe Gln Ser Val Ile Asp Asp Pro Asn Val Ala Asp Ala Ala
           1125                1130                1135
   aag gtg aag aag gtc atg ctg gtc tcc ggc aag ctg tac tac gaa      3898
   Lys Val Lys Lys Val Met Leu Val Ser Gly Lys Leu Tyr Tyr Glu
           1140                1145                1150
   ttg gca aag cgc aag gag aag gac gga cgc gac gac atc gcg atc      3943
   Leu Ala Lys Arg Lys Glu Lys Asp Gly Arg Asp Asp Ile Ala Ile
           1155                1160                1165
   gtt cgt atc gaa atg ctc cac cca att ccg ttc aac cgc atc tcc      3988
   Val Arg Ile Glu Met Leu His Pro Ile Pro Phe Asn Arg Ile Ser
           1170                1175                1180
   gag gct ctt gcc ggc tac cct aac gct gag gaa gtc ctc ttc gtt      4033
   Glu Ala Leu Ala Gly Tyr Pro Asn Ala Glu Glu Val Leu Phe Val
           1185                1190                1195
   cag gat gag cca gca aac cag ggc cca tgg ccg ttc tac cag gag      4078
   Gln Asp Glu Pro Ala Asn Gln Gly Pro Trp Pro Phe Tyr Gln Glu
           1200                1205                1210
   cac ctc cca gag ctg atc ccg aac atg cca aag atg cgc cgc gtt      4123
   His Leu Pro Glu Leu Ile Pro Asn Met Pro Lys Met Arg Arg Val
           1215                1220                1225
   tcc cgc cgc gct cag tcc tcc acc gca act ggt gtt gct aag gtg      4168
   Ser Arg Arg Ala Gln Ser Ser Thr Ala Thr Gly Val Ala Lys Val
           1230                1235                1240
   cac cag ctg gag gag aag cag ctt atc gac gag gct ttc gag gct      4213
   His Gln Leu Glu Glu Lys Gln Leu Ile Asp Glu Ala Phe Glu Ala
           1245                1250                1255
   taagtcttta tagtcctgca ctagcctaga gggccttatg cagtgtgaat cacacagcat   4273
   aaggcccttt ttgctgccgt ggttgcctaa ggtggaaggc atgaaacgaa tctgtgcggt   4333
   cacgatctct tcagtacttt tgctaagtgg ctgctcctcc acttccacca cgcagctcga   4393
   g                                                                   4394
<210> 10
   <211> 1257
   <212> PRT
   <213> Corynebacterium glutamicum
<400> 10
   Met Leu Gln Leu Gly Leu Arg His Asn Gln Pro Thr Thr Asn Val Thr
   1               5                   10                  15
   Val Asp Lys Ile Lys Leu Asn Lys Pro Ser Arg Ser Lys Glu Lys Arg
   20 25 30
   Arg Val Pro Ala Val Ser Ser Ala Ser Thr Phe Gly Gln Asn Ala Trp
   35 40 45
   Leu Val Asp Glu Met Phe Gln Gln Phe Gln Lys Asp Pro Lys Ser Val
   50 55 60
   Asp Lys Glu Trp Arg Glu Leu Phe Glu Ala Gln Gly Gly Pro Asn Ala
   65 70 75 80
   Thr Pro Ala Thr Thr Glu Ala Gln Pro Ser Ala Pro Lys Glu Ser Ala
   85 90 95
   Lys Pro Ala Pro Lys Ala Ala Pro Ala Ala Lys Ala Ala Pro Arg Val
   100 105 110
   Glu Thr Lys Pro Ala Ala Lys Thr Ala Pro Lys Ala Lys Glu Ser Ser
   115 120 125
   Val Pro Gln Gln Pro Lys Leu Pro Glu Pro Gly Gln Thr Pro Ile Arg
   130 135 140
   Gly Ile Phe Lys Ser Ile Ala Lys Asn Met Asp Ile Ser Leu Glu Ile
   145 150 155 160
   Pro Thr Ala Thr Ser Val Arg Asp Met Pro Ala Arg Leu Met Phe Glu
   165 170 175
   Asn Arg Ala Met Val Asn Asp Gln Leu Lys Arg Thr Arg Gly Gly Lys
   180 185 190
   Ile Ser Phe Thr His Ile Ile Gly Tyr Ala Met Val Lys Ala Val Met
   195 200 205
   Ala His Pro Asp Met Asn Asn Ser Tyr Asp Val Ile Asp Gly Lys Pro
   210 215 220
   Thr Leu Ile Val Pro Glu His Ile Asn Leu Gly Leu Ala Ile Asp Leu
   225 230 235 240
   Pro Gln Lys Asp Gly Ser Arg Ala Leu Val Val Ala Ala Ile Lys Glu
   245 250 255
   Thr Glu Lys Met Asn Phe Ser Glu Phe Leu Ala Ala Tyr Glu Asp Ile
   260 265 270
   Val Thr Arg Ser Arg Lys Gly Lys Leu Thr Met Asp Asp Tyr Gln Gly
   275 280 285
   Val Thr Val Ser Leu Thr Asn Pro Gly Gly Ile Gly Thr Arg His Ser
   290 295 300
   Val Pro Arg Leu Thr Lys Gly Gln Gly Thr Ile Ile Gly Val Gly Ser
   305 310 315 320
   Met Asp Tyr Pro Ala Glu Phe Gln Gly Ala Ser Glu Asp Arg Leu Ala
   325 330 335
   Glu Leu Gly Val Gly Lys Leu Val Thr Ile Thr Ser Thr Tyr Asp His
   340 345 350
   Arg Val Ile Gln Gly Ala Val Ser Gly Glu Phe Leu Arg Thr Met Ser
   355 360 365
   Arg Leu Leu Thr Asp Asp Ser Phe Trp Asp Glu Ile Phe Asp Ala Met
   370 375 380
   Asn Val Pro Tyr Thr Pro Met Arg Trp Ala Gln Asp Val Pro Asn Thr
   385 390 395 400
   Gly Val Asp Lys Asn Thr Arg Val Met Gln Leu Ile Glu Ala Tyr Arg
   405 410 415
   Ser Arg Gly His Leu Ile Ala Asp Thr Asn Pro Leu Ser Trp Val Gln
   420 425 430
   Pro Gly Met Pro Val Pro Asp His Arg Asp Leu Asp Ile Glu Thr His
   435 440 445
   Ser Leu Thr Ile Trp Asp Leu Asp Arg Thr Phe Ser Val Gly Gly Phe
   450 455 460
   Gly Gly Lys Glu Thr Met Thr Leu Arg Glu Val Leu Ser Arg Leu Arg
   465 470 475 480
   Ala Ala Tyr Thr Leu Lys Val Gly Ser Glu Tyr Thr His Ile Leu Asp
   485 490 495
   Arg Asp Glu Arg Thr Trp Leu Gln Asp Arg Leu Glu Ala Gly Met Pro
   500 505 510
   Lys Pro Thr Gln Ala Glu Gln Lys Tyr Ile Leu Gln Lys Leu Asn Ala
   515 520 525
   Ala Glu Ala Phe Glu Asn Phe Leu Gln Thr Lys Tyr Val Gly Gln Lys
   530 535 540
   Arg Phe Ser Leu Glu Gly Ala Glu Ala Leu Ile Pro Leu Met Asp Ser
   545 550 555 560
   Ala Ile Asp Thr Ala Ala Gly Gln Gly Leu Asp Glu Val Val Ile Gly
   565 570 575
   Met Pro His Arg Gly Arg Leu Asn Val Leu Phe Asn Ile Val Gly Lys
   580 585 590
   Pro Leu Ala Ser Ile Phe Asn Glu Phe Glu Gly Gln Met Glu Gln Gly
   595 600 605
   Gln Ile Gly Gly Ser Gly Asp Val Lys Tyr His Leu Gly Ser Glu Gly
   610 615 620
   Gln His Leu Gln Met Phe Gly Asp Gly Glu Ile Lys Val Ser Leu Thr
   625 630 635 640
   Ala Asn Pro Ser His Leu Glu Ala Val Asn Pro Val Met Glu Gly Ile
   645 650 655
   Val Arg Ala Lys Gln Asp Tyr Leu Asp Lys Gly Val Asp Gly Lys Thr
   660 665 670
   Val Val Pro Leu Leu Leu His Gly Asp Ala Ala Phe Ala Gly Leu Gly
   675 680 685
   Ile Val Pro Glu Thr Ile Asn Leu Ala Lys Leu Arg Gly Tyr Asp Val
   690 695 700
   Gly Gly Thr Ile His Ile Val Val Asn Asn Gln Ile Gly Phe Thr Thr
   705 710 715 720
   Thr Pro Asp Ser Ser Arg Ser Met His Tyr Ala Thr Asp Tyr Ala Lys
   725 730 735
   Ala Phe Gly Cys Pro Val Phe His Val Asn Gly Asp Asp Pro Glu Ala
   740 745 750
   Val Val Trp Val Gly Gln Leu Ala Thr Glu Tyr Arg Arg Arg Phe Gly
   755 760 765
   Lys Asp Val Phe Ile Asp Leu Val Cys Tyr Arg Leu Arg Gly His Asn
   770 775 780
   Glu Ala Asp Asp Pro Ser Met Thr Gln Pro Lys Met Tyr Glu Leu Ile
   785 790 795 800
   Thr Gly Arg Glu Thr Val Arg Ala Gln Tyr Thr Glu Asp Leu Leu Gly
   805 810 815
   Arg Gly Asp Leu Ser Asn Glu Asp Ala Glu Ala Val Val Arg Asp Phe
   820 825 830
   His Asp Gln Met Glu Ser Val Phe Asn Glu Val Lys Glu Gly Gly Lys
   835 840 845
   Lys Gln Ala Glu Ala Gln Thr Gly Ile Thr Gly Ser Gln Lys Leu Pro
   850 855 860
   His Gly Leu Glu Thr Asn Ile Ser Arg Glu Glu Leu Leu Glu Leu Gly
   865 870 875 880
   Gln Ala Phe Ala Asn Thr Pro Glu Gly Phe Asn Tyr His Pro Arg Val
   885 890 895
   Ala Pro Val Ala Lys Lys Arg Val Ser Ser Val Thr Glu Gly Gly Ile
   900 905 910
   Asp Trp Ala Trp Gly Glu Leu Leu Ala Phe Gly Ser Leu Ala Asn Ser
   915 920 925
   Gly Arg Leu Val Arg Leu Ala Gly Glu Asp Ser Arg Arg Gly Thr Phe
   930 935 940
   Thr Gln Arg His Ala Val Ala Ile Asp Pro Ala Thr Ala Glu Glu Phe
   945 950 955 960
   Asn Pro Leu His Glu Leu Ala Gln Ser Lys Gly Asn Asn Gly Lys Phe
   965 970 975
   Leu Val Tyr Asn Ser Ala Leu Thr Glu Tyr Ala Gly Met Gly Phe Glu
   980 985 990
   Tyr Gly Tyr Ser Val Gly Asn Glu Asp Ser Val Val Ala Trp Glu Ala
   995 1000 1005
   Gln Phe Gly Asp Phe Ala Asn Gly Ala Gln Thr Ile Ile Asp Glu
   1010 1015 1020
   Tyr Val Ser Ser Gly Glu Ala Lys Trp Gly Gln Thr Ser Lys Leu
   1025 1030 1035
   Ile Leu Leu Leu Pro His Gly Tyr Glu Gly Gln Gly Pro Asp His
   1040 1045 1050
   Ser Ser Ala Arg Ile Glu Arg Phe Leu Gln Leu Cys Ala Glu Gly
   1055 1060 1065
   Ser Met Thr Val Ala Gln Pro Ser Thr Pro Ala Asn His Phe His
   1070 1075 1080
   Leu Leu Arg Arg His Ala Leu Ser Asp Leu Lys Arg Pro Leu Val
   1085 1090 1095
   Ile Phe Thr Pro Lys Ser Met Leu Arg Asn Lys Ala Ala Ala Ser
   1100 1105 1110
   Ala Pro Glu Asp Phe Thr Glu Val Thr Lys Phe Gln Ser Val Ile
   1115 1120 1125
   Asp Asp Pro Asn Val Ala Asp Ala Ala Lys Val Lys Lys Val Met
   1130 1135 1140
   Leu Val Ser Gly Lys Leu Tyr Tyr Glu Leu Ala Lys Arg Lys Glu
   1145 1150 1155
   Lys Asp Gly Arg Asp Asp Ile Ala Ile Val Arg Ile Glu Met Leu
   1160 1165 1170
   His Pro Ile Pro Phe Asn Arg Ile Ser Glu Ala Leu Ala Gly Tyr
   1175 1180 1185
   Pro Asn Ala Glu Glu Val Leu Phe Val Gln Asp Glu Pro Ala Asn
   1190 1195 1200
   Gln Gly Pro Trp Pro Phe Tyr Gln Glu His Leu Pro Glu Leu Ile
   1205 1210 1215
   Pro Asn Met Pro Lys Met Arg Arg Val Ser Arg Arg Ala Gln Ser
   1220 1225 1230
   Ser Thr Ala Thr Gly Val Ala Lys Val His Gln Leu Glu Glu Lys
   1235 1240 1245
   Gln Leu Ile Asp Glu Ala Phe Glu Ala
   1250 1255
<210> 11
   <211> 4391
   <212> DNA
   <213> Corynebacterium glutamicum
<220>
   <221> CDS
   <222> (443)..(4213)
   <223>
<400> 11
   gtcgacaagc aaaatcgaag cggcagcacg ccgcgtcgga gccttaaacg ccatcgccgc 60
   catccctgat ggtttcaatc atcaagtcgg tgaacgcggg cgcaacctgt catccggaca 120
   gcgccaactg atcgcgctgg cgcgcgccga actcatcgag ccttccatca tgcttctcga 180
   cgaagccacc tccaccctcg accccgccac cgaagccgtt atcctcaacg cctccgatcg 240
   agtcactaag ggacgcacca gcatcatcgt cgcgcaccgc ttggcaaccg ctaaaagggc 300
   cgaccgtatt cttgttgttg aacaaggacg tatcattgag gacggatctc acgacgcgtt 360
   gttgtctgct aacggcacct acgcccgcat gtggcattta atggcctgac acgttatttt 420
   taggagaact gtcaacaaat ta atg cta caa ctg ggg ctt agg cat aat cag 472
   Met Leu Gln Leu Gly Leu Arg His Asn Gln
   1 5 10
   cca acg acc aac gtt aca gtg gat aaa ata aag ctc aat aaa ccc tca 520
   Pro Thr Thr Asn Val Thr Val Asp Lys Ile Lys Leu Asn Lys Pro Ser
   15 20 25
   aga agc aag gaa aag agg cga gta cct gcc gtg agc agc gct agt act 568
   Arg Ser Lys Glu Lys Arg Arg Val Pro Ala Val Ser Ser Ala Ser Thr
   30 35 40
   ttc ggc cag aat gcg tgg ctg gta gac gag atg ttc cag cag ttc cag 616
   Phe Gly Gln Asn Ala Trp Leu Val Asp Glu Met Phe Gln Gln Phe Gln
   45 50 55
   aag gac ccc aag tcc gtg gac aag gaa tgg aga gaa ctc ttt gag gcg 664
   Lys Asp Pro Lys Ser Val Asp Lys Glu Trp Arg Glu Leu Phe Glu Ala
           60                  65                  70
   cag ggg gga cca aat gct acc ccc gct aca aca gaa gca cag cct tca      712
   Gln Gly Gly Pro Asn Ala Thr Pro Ala Thr Thr Glu Ala Gln Pro Ser
   75                  80                  85                  90
   gcg ccc aag gag tct gcg aaa cca gca cca aag gct gcc cct gca gcc      760
   Ala Pro Lys Glu Ser Ala Lys Pro Ala Pro Lys Ala Ala Pro Ala Ala
                   95                  100                 105
   aag gca gca ccg cgc gta gaa acc aag ccg gcc gcc aag acc gcc cct      808
   Lys Ala Ala Pro Arg Val Glu Thr Lys Pro Ala Ala Lys Thr Ala Pro
               110                 115                 120
   aag gcc aag gag tcc tca gtg cca cag caa cct aag ctt ccg gag cca      856
   Lys Ala Lys Glu Ser Ser Val Pro Gln Gln Pro Lys Leu Pro Glu Pro
           125                 130                 135
   gga caa acc cca atc agg ggt att ttc aag tcc atc gcg aag aac atg      904
   Gly Gln Thr Pro Ile Arg Gly Ile Phe Lys Ser Ile Ala Lys Asn Met
       140                 145                 150
   gat atc tcc ctg gaa atc cca acc gca acc tcg gtt cgc gat atg cca      952
   Asp Ile Ser Leu Glu Ile Pro Thr Ala Thr Ser Val Arg Asp Met Pro
   155                 160                 165                 170
   gct cgc ctc atg ttc gaa aac cgc gcg atg gtc aac gat cag ctc aag     1000
   Ala Arg Leu Met Phe Glu Asn Arg Ala Met Val Asn Asp Gln Leu Lys
                   175                 180                 185
   cgc acc cgc ggt ggc aag atc tcc ttc acc cac atc att ggc tac gcc     1048
   Arg Thr Arg Gly Gly Lys Ile Ser Phe Thr His Ile Ile Gly Tyr Ala
               190                 195                 200
   atg gtg aag gca gtc atg gct cac ccg gac atg aac aac tcc tac cat     1096
   Met Val Lys Ala Val Met Ala His Pro Asp Met Asn Asn Ser Tyr His
           205                 210                 215
   cga cgg cag gcc cca acc ctg atc gtg cct gag cac atc aac ctg ggc     1144
   Arg Arg Gln Ala Pro Thr Leu Ile Val Pro Glu His Ile Asn Leu Gly
       220                 225                 230
   ctt gcc atc gac ctt cct cag aag gac ggc tcc cgc gca ctt gtc gta     1192
   Leu Ala Ile Asp Leu Pro Gln Lys Asp Gly Ser Arg Ala Leu Val Val
   235                 240                 245                 250
   gca gcc atc aag gaa acc gag aag atg aac ttc tcc gag ttc ctc gca     1240
   Ala Ala Ile Lys Glu Thr Glu Lys Met Asn Phe Ser Glu Phe Leu Ala
                   255                 260                 265
   gca tac gaa gac atc gtg aca cgc tcc cgc aag ggc aag ctc acc atg     1288
   Ala Tyr Glu Asp Ile Val Thr Arg Ser Arg Lys Gly Lys Leu Thr Met
               270                 275                 280
   gat gac tac cag ggc gtt acc gtt tcc ttg acc aac cca ggt ggc atc     1336
   Asp Asp Tyr Gln Gly Val Thr Val Ser Leu Thr Asn Pro Gly Gly Ile
           285                 290                 295
   ggt acc cgc cac tct gtc cca cgt ctg acc aag ggc cag ggc acc atc     1384
   Gly Thr Arg His Ser Val Pro Arg Leu Thr Lys Gly Gln Gly Thr Ile
       300                 305                 310
   atc ggt gtc ggt tcc atg gat tac cca gca gag ttc cag ggc gct tcc     1432
   Ile Gly Val Gly Ser Met Asp Tyr Pro Ala Glu Phe Gln Gly Ala Ser
   315 320 325 330
   gaa gac cgc ctt gca gag ctc ggc gtt gga aag ctt gtc acc atc acc 1480
   Glu Asp Arg Leu Ala Glu Leu Gly Val Gly Lys Leu Val Thr Ile Thr
   335 340 345
   tcc acc tac gat cac cgc gtg atc cag ggt gct gtg tcc ggt gaa ttc 1528
   Ser Thr Tyr Asp His Arg Val Ile Gln Gly Ala Val Ser Gly Glu Phe
   350 355 360
   ctg cgt acc atg tct cgc ctg ctc acc gat gat tcc ttc tgg gat gag 1576
   Leu Arg Thr Met Ser Arg Leu Leu Thr Asp Asp Ser Phe Trp Asp Glu
   365 370 375
   atc ttc gac gca atg aac gtt cct tac acc cca atg cgt tgg gca cag 1624
   Ile Phe Asp Ala Met Asn Val Pro Tyr Thr Pro Met Arg Trp Ala Gln
   380 385 390
   gac gtt cca aac acc ggt gtt gat aag aac acc cgc gtc atg cag ctc 1672
   Asp Val Pro Asn Thr Gly Val Asp Lys Asn Thr Arg Val Met Gln Leu
   395 400 405 410
   att gag gca tac cgc tcc cgt gga cac ctc atc gct gac acc aac cca 1720
   Ile Glu Ala Tyr Arg Ser Arg Gly His Leu Ile Ala Asp Thr Asn Pro
   415 420 425
   ctt tca tgg gtt cag cct ggc atg cca gtt cca gac cac cgc gac ctc 1768
   Leu Ser Trp Val Gln Pro Gly Met Pro Val Pro Asp His Arg Asp Leu
   430 435 440
   gac atc gag acc cac agc ctg acc atc tgg gat ctg gac cgt acc ttc 1816
   Asp Ile Glu Thr His Ser Leu Thr Ile Trp Asp Leu Asp Arg Thr Phe
   445 450 455
   agc gtc ggt ggc ttc ggc ggc aag gag acc atg acc ctg cgc gag gta 1864
   Ser Val Gly Gly Phe Gly Gly Lys Glu Thr Met Thr Leu Arg Glu Val
   460 465 470
   ctg tcc cgc ctg cgc gct gcc tac acc ttg aag gtc ggc tcc gaa tac 1912
   Leu Ser Arg Leu Arg Ala Ala Tyr Thr Leu Lys Val Gly Ser Glu Tyr
   475 480 485 490
   acc cac atc ctg gac cgc gac gag cgc acc tgg ctg cag gac cgc ctc 1960
   Thr His Ile Leu Asp Arg Asp Glu Arg Thr Trp Leu Gln Asp Arg Leu
   495 500 505
   gaa gcc gga atg cca aag cca acc cag gca gag cag aag tac atc ctg 2008
   Glu Ala Gly Met Pro Lys Pro Thr Gln Ala Glu Gln Lys Tyr Ile Leu
   510 515 520
   cag aag ctg aac gcc gca gag gct ttc gag aac ttc ctg cag acc aag 2056
   Gln Lys Leu Asn Ala Ala Glu Ala Phe Glu Asn Phe Leu Gln Thr Lys
   525 530 535
   tac gtc ggc cag aag cgc ttc tcc ctc gaa ggt gca gaa gct ctc atc 2104
   Tyr Val Gly Gln Lys Arg Phe Ser Leu Glu Gly Ala Glu Ala Leu Ile
   540 545 550
   cca ctg atg gac tcc gcc atc gac acc gcc gca ggc cag ggc ctc gac 2152
   Pro Leu Met Asp Ser Ala Ile Asp Thr Ala Ala Gly Gln Gly Leu Asp
   555 560 565 570
   gaa gtt gtc atc ggt atg cca cac cgt ggt cgc ctc aac gtg ctg ttc 2200
   Glu Val Val Ile Gly Met Pro His Arg Gly Arg Leu Asn Val Leu Phe
                       575                 580                 585
   aac atc gtg ggc aag cca ctg gca tcc atc ttc aac gag ttt gaa ggc    2248
   Asn Ile Val Gly Lys Pro Leu Ala Ser Ile Phe Asn Glu Phe Glu Gly
                   590                 595                 600
   caa atg gag cag ggc cag atc ggt ggc tcc ggt gac gtg aag tac cac    2296
   Gln Met Glu Gln Gly Gln Ile Gly Gly Ser Gly Asp Val Lys Tyr His
               605                 610                 615
   ctc ggt tcc gaa ggc cag cac ctg cag atg ttc ggc gac ggc gag atc    2344
   Leu Gly Ser Glu Gly Gln His Leu Gln Met Phe Gly Asp Gly Glu Ile
           620                 625                 630
   aag gtc tcc ctg act gct aac ccg tcc cac ctg gaa gct gtt aac cca    2392
   Lys Val Ser Leu Thr Ala Asn Pro Ser His Leu Glu Ala Val Asn Pro
   635                 640                 645                 650
   gtg atg gaa ggt atc gtc cgc gca aag cag gac tac ctg gac aag ggc    2440
   Val Met Glu Gly Ile Val Arg Ala Lys Gln Asp Tyr Leu Asp Lys Gly
                       655                 660                 665
   gta gac ggc aag act gtt gtg cca ctg ctg ctc cac ggt gac gct gca    2488
   Val Asp Gly Lys Thr Val Val Pro Leu Leu Leu His Gly Asp Ala Ala
                   670                 675                 680
   ttc gca ggc ctg ggc atc gtg cca gaa acc atc aac ctg gct aag ctg    2536
   Phe Ala Gly Leu Gly Ile Val Pro Glu Thr Ile Asn Leu Ala Lys Leu
               685                 690                 695
   cgt ggc tac gac gtc gga ggc acc atc cac atc gtg gtg aac aac cag    2584
   Arg Gly Tyr Asp Val Gly Gly Thr Ile His Ile Val Val Asn Asn Gln
           700                 705                 710
   atc ggc ttc acc acc acc cca gac tcc agc cgc tcc atg cac tac gca    2632
   Ile Gly Phe Thr Thr Thr Pro Asp Ser Ser Arg Ser Met His Tyr Ala
   715                 720                 725                 730
   acc gac tac gcc aag gca ttc ggc tgc cca gtc ttc cac gtc aat ggt    2680
   Thr Asp Tyr Ala Lys Ala Phe Gly Cys Pro Val Phe His Val Asn Gly
                       735                 740                 745
   gat gac cca gag gca gtt gtc tgg gtt ggc cag ctg gca acc gag tac    2728
   Asp Asp Pro Glu Ala Val Val Trp Val Gly Gln Leu Ala Thr Glu Tyr
                   750                 755                 760
   cgt cgt cgc ttc ggc aag gac gtc ttc atc gac ctc gtt tgc tac cgc    2776
   Arg Arg Arg Phe Gly Lys Asp Val Phe Ile Asp Leu Val Cys Tyr Arg
               765                 770                 775
   ctc cgc ggc cac aac gaa gct gat gat cct tcc atg acc cag cca aag    2824
   Leu Arg Gly His Asn Glu Ala Asp Asp Pro Ser Met Thr Gln Pro Lys
           780                 785                 790
   atg tat gag ctc atc acc ggc cgc gag acc gtt cgt gct cag tac acc    2872
   Met Tyr Glu Leu Ile Thr Gly Arg Glu Thr Val Arg Ala Gln Tyr Thr
   795                 800                 805                 810
   gaa gac ctg ctc gga cgt gga gac ctc tcc aac gaa gat gca gaa gca    2920
   Glu Asp Leu Leu Gly Arg Gly Asp Leu Ser Asn Glu Asp Ala Glu Ala
                       815                 820                 825
   gtc gtc cgc gac ttc cac gac cag atg gaa tct gtg ttc aac gaa gtc    2968
   Val Val Arg Asp Phe His Asp Gln Met Glu Ser Val Phe Asn Glu Val
   830 835 840
   aag gaa ggc ggc aag aag cag gct gag gca cag acc ggc atc acc ggc 3016
   Lys Glu Gly Gly Lys Lys Gln Ala Glu Ala Gln Thr Gly Ile Thr Gly
   845 850 855
   tcc cag aag ctt cca cac ggc ctt gag acc aac atc tcc cgt gaa gag 3064
   Ser Gln Lys Leu Pro His Gly Leu Glu Thr Asn Ile Ser Arg Glu Glu
   860 865 870
   ctc ctg gaa ctg gga cag gct ttc gcc aac acc cca gaa ggc ttc aac 3112
   Leu Leu Glu Leu Gly Gln Ala Phe Ala Asn Thr Pro Glu Gly Phe Asn
   875 880 885 890
   tac cac cca cgt gtg gct cca gtt gct aag aag cgc gtc tcc tct gtc 3160
   Tyr His Pro Arg Val Ala Pro Val Ala Lys Lys Arg Val Ser Ser Val
   895 900 905
   acc gaa ggt ggc atc gac tgg gca tgg ggc gag ctc ctc gcc ttc ggt 3208
   Thr Glu Gly Gly Ile Asp Trp Ala Trp Gly Glu Leu Leu Ala Phe Gly
   910 915 920
   tcc ctg gct aac tcc ggc cgc ttg gtt cgc ctt gca ggt gaa gat tcc 3256
   Ser Leu Ala Asn Ser Gly Arg Leu Val Arg Leu Ala Gly Glu Asp Ser
   925 930 935
   cgc cgc ggt acc ttc acc cag cgc cac gca gtt gcc atc gac cca gcg 3304
   Arg Arg Gly Thr Phe Thr Gln Arg His Ala Val Ala Ile Asp Pro Ala
   940 945 950
   acc gct gaa gag ttc aac cca ctc cac gag ctt gca cag tcc aag ggc 3352
   Thr Ala Glu Glu Phe Asn Pro Leu His Glu Leu Ala Gln Ser Lys Gly
   955 960 965 970
   aac aac ggt aag ttc ctg gtc tac aac tcc gca ctg acc gag tac gca 3400
   Asn Asn Gly Lys Phe Leu Val Tyr Asn Ser Ala Leu Thr Glu Tyr Ala
   975 980 985
   ggc atg ggc ttc gag tac ggc tac tcc gta gga aac gaa gac tcc gtc 3448
   Gly Met Gly Phe Glu Tyr Gly Tyr Ser Val Gly Asn Glu Asp Ser Val
   990 995 1000
   gtt gca tgg gaa gca cag ttc ggc gac ttc gcc aac ggc gct cag 3493
   Val Ala Trp Glu Ala Gln Phe Gly Asp Phe Ala Asn Gly Ala Gln
   1005 1010 1015
   acc atc atc gat gag tac gtc tcc tca ggc gaa gct aag tgg ggc 3538
   Thr Ile Ile Asp Glu Tyr Val Ser Ser Gly Glu Ala Lys Trp Gly
   1020 1025 1030
   cag acc tcc aag ctg atc ctt ctg ctg cct cac ggc tac gaa ggc 3583
   Gln Thr Ser Lys Leu Ile Leu Leu Leu Pro His Gly Tyr Glu Gly
   1035 1040 1045
   cag ggc cca gac cac tct tcc gca cgt atc gag cgc ttc ctg cag 3628
   Gln Gly Pro Asp His Ser Ser Ala Arg Ile Glu Arg Phe Leu Gln
   1050 1055 1060
   ctg tgc gct gag ggt tcc atg act gtt gct cag cca tcc acc cca 3673
   Leu Cys Ala Glu Gly Ser Met Thr Val Ala Gln Pro Ser Thr Pro
   1065 1070 1075
   gca aac cac ttc cac ctg ctg cgt cgt cac gct ctg tcc gac ctg 3718
   Ala Asn His Phe His Leu Leu Arg Arg His Ala Leu Ser Asp Leu
                   1080                      1085                      1090
   aag cgt cca  ctg gtt atc ttc acc  ccg aag tcc atg ctg  cgt aac      3763
   Lys Arg Pro  Leu Val Ile Phe Thr  Pro Lys Ser Met Leu  Arg Asn
                   1095                      1100                      1105
   aag gct gct  gcc tcc gca cca gaa  gac ttc act gag gtc  acc aag      3808
   Lys Ala Ala  Ala Ser Ala Pro Glu  Asp Phe Thr Glu Val  Thr Lys
                   1110                      1115                      1120
   ttc caa tcc  gtg atc gac gat cca  aac gtt gca gat gca  gcc aag      3853
   Phe Gln Ser  Val Ile Asp Asp Pro  Asn Val Ala Asp Ala  Ala Lys
                   1125                      1130                      1135
   gtg aag aag  gtc atg ctg gtc tcc  ggc aag ctg tac tac  gaa ttg      3898
   Val Lys Lys  Val Met Leu Val Ser  Gly Lys Leu Tyr Tyr  Glu Leu
                   1140                      1145                      1150
   gca aag cgc  aag gag aag gac gga  cgc gac gac atc gcg  atc gtt      3943
   Ala Lys Arg  Lys Glu Lys Asp Gly  Arg Asp Asp Ile Ala  Ile Val
                   1155                      1160                      1165
   cgt atc gaa  atg ctc cac cca att  ccg ttc aac cgc atc  tcc gag      3988
   Arg Ile Glu  Met Leu His Pro Ile  Pro Phe Asn Arg Ile  Ser Glu
                   1170                      1175                      1180
   gct ctt gcc  ggc tac cct aac gct  gag gaa gtc ctc ttc  gtt cag      4033
   Ala Leu Ala  Gly Tyr Pro Asn Ala  Glu Glu Val Leu Phe  Val Gln
                   1185                      1190                      1195
   gat gag cca  gca aac cag ggc cca  tgg ccg ttc tac cag  gag cac      4078
   Asp Glu Pro  Ala Asn Gln Gly Pro  Trp Pro Phe Tyr Gln  Glu His
                   1200                      1205                      1210
   ctc cca gag  ctg atc ccg aac atg  cca aag atg cgc cgc  gtt tcc      4123
   Leu Pro Glu  Leu Ile Pro Asn Met  Pro Lys Met Arg Arg  Val Ser
                   1215                      1220                      1225
   cgc cgc gct  cag tcc tcc acc gca  act ggt gtt gct aag  gtg cac      4168
   Arg Arg Ala  Gln Ser Ser Thr Ala  Thr Gly Val Ala Lys  Val His
                   1230                      1235                      1240
   cag ctg gag  gag aag cag ctt atc  gac gag gct ttc gag  gct taa      4213
   Gln Leu Glu  Glu Lys Gln Leu Ile  Asp Glu Ala Phe Glu  Ala
                   1245                      1250                      1255
   gtctttatag tcctgcacta gcctagaggg ccttatgcag tgtgaatcac acagcataag   4273
   gccctttttg ctgccgtggt tgcctaaggt ggaaggcatg aaacgaatct gtgcggtcac    4333
   gatctcttca gtacttttgc taagtggctg ctcctccact tccaccacgc agctcgag     4391
<210> 12
   <211> 1256
   <212> PRT
   <213> Corynebacterium glutamicum
<400> 12
   Met Leu Gln Leu Gly Leu Arg His Asn Gln Pro Thr Thr Asn Val Thr
   1 5 10 15
   Val Asp Lys Ile Lys Leu Asn Lys Pro Ser Arg Ser Lys Glu Lys Arg
   20 25 30
   Arg Val Pro Ala Val Ser Ser Ala Ser Thr Phe Gly Gln Asn Ala Trp
   35 40 45
   Leu Val Asp Glu Met Phe Gln Gln Phe Gln Lys Asp Pro Lys Ser Val
   50 55 60
   Asp Lys Glu Trp Arg Glu Leu Phe Glu Ala Gln Gly Gly Pro Asn Ala
   65 70 75 80
   Thr Pro Ala Thr Thr Glu Ala Gln Pro Ser Ala Pro Lys Glu Ser Ala
   85 90 95
   Lys Pro Ala Pro Lys Ala Ala Pro Ala Ala Lys Ala Ala Pro Arg Val
   100 105 110
   Glu Thr Lys Pro Ala Ala Lys Thr Ala Pro Lys Ala Lys Glu Ser Ser
   115 120 125
   Val Pro Gln Gln Pro Lys Leu Pro Glu Pro Gly Gln Thr Pro Ile Arg
   130 135 140
   Gly Ile Phe Lys Ser Ile Ala Lys Asn Met Asp Ile Ser Leu Glu Ile
   145 150 155 160
   Pro Thr Ala Thr Ser Val Arg Asp Met Pro Ala Arg Leu Met Phe Glu
   165 170 175
   Asn Arg Ala Met Val Asn Asp Gln Leu Lys Arg Thr Arg Gly Gly Lys
   180 185 190
   Ile Ser Phe Thr His Ile Ile Gly Tyr Ala Met Val Lys Ala Val Met
   195 200 205
   Ala His Pro Asp Met Asn Asn Ser Tyr His Arg Arg Gln Ala Pro Thr
   210 215 220
   Leu Ile Val Pro Glu His Ile Asn Leu Gly Leu Ala Ile Asp Leu Pro
   225 230 235 240
   Gln Lys Asp Gly Ser Arg Ala Leu Val Val Ala Ala Ile Lys Glu Thr
   245 250 255
   Glu Lys Met Asn Phe Ser Glu Phe Leu Ala Ala Tyr Glu Asp Ile Val
   260 265 270
   Thr Arg Ser Arg Lys Gly Lys Leu Thr Met Asp Asp Tyr Gln Gly Val
   275 280 285
   Thr Val Ser Leu Thr Asn Pro Gly Gly Ile Gly Thr Arg His Ser Val
   290 295 300
   Pro Arg Leu Thr Lys Gly Gln Gly Thr Ile Ile Gly Val Gly Ser Met
   305 310 315 320
   Asp Tyr Pro Ala Glu Phe Gln Gly Ala Ser Glu Asp Arg Leu Ala Glu
   325 330 335
   Leu Gly Val Gly Lys Leu Val Thr Ile Thr Ser Thr Tyr Asp His Arg
   340 345 350
   Val Ile Gln Gly Ala Val Ser Gly Glu Phe Leu Arg Thr Met Ser Arg
   355 360 365
   Leu Leu Thr Asp Asp Ser Phe Trp Asp Glu Ile Phe Asp Ala Met Asn
   370 375 380
   Val Pro Tyr Thr Pro Met Arg Trp Ala Gln Asp Val Pro Asn Thr Gly
   385 390 395 400
   Val Asp Lys Asn Thr Arg Val Met Gln Leu Ile Glu Ala Tyr Arg Ser
   405 410 415
   Arg Gly His Leu Ile Ala Asp Thr Asn Pro Leu Ser Trp Val Gln Pro
   420 425 430
   Gly Met Pro Val Pro Asp His Arg Asp Leu Asp Ile Glu Thr His Ser
   435 440 445
   Leu Thr Ile Trp Asp Leu Asp Arg Thr Phe Ser Val Gly Gly Phe Gly
   450 455 460
   Gly Lys Glu Thr Met Thr Leu Arg Glu Val Leu Ser Arg Leu Arg Ala
   465 470 475 480
   Ala Tyr Thr Leu Lys Val Gly Ser Glu Tyr Thr His Ile Leu Asp Arg
   485 490 495
   Asp Glu Arg Thr Trp Leu Gln Asp Arg Leu Glu Ala Gly Met Pro Lys
   500 505 510
   Pro Thr Gln Ala Glu Gln Lys Tyr Ile Leu Gln Lys Leu Asn Ala Ala
   515 520 525
   Glu Ala Phe Glu Asn Phe Leu Gln Thr Lys Tyr Val Gly Gln Lys Arg
   530 535 540
   Phe Ser Leu Glu Gly Ala Glu Ala Leu Ile Pro Leu Met Asp Ser Ala
   545 550 555 560
   Ile Asp Thr Ala Ala Gly Gln Gly Leu Asp Glu Val Val Ile Gly Met
   565 570 575
   Pro His Arg Gly Arg Leu Asn Val Leu Phe Asn Ile Val Gly Lys Pro
   580 585 590
   Leu Ala Ser Ile Phe Asn Glu Phe Glu Gly Gln Met Glu Gln Gly Gln
   595 600 605
   Ile Gly Gly Ser Gly Asp Val Lys Tyr His Leu Gly Ser Glu Gly Gln
   610 615 620
   His Leu Gln Met Phe Gly Asp Gly Glu Ile Lys Val Ser Leu Thr Ala
   625 630 635 640
   Asn Pro Ser His Leu Glu Ala Val Asn Pro Val Met Glu Gly Ile Val
   645 650 655
   Arg Ala Lys Gln Asp Tyr Leu Asp Lys Gly Val Asp Gly Lys Thr Val
   660 665 670
   Val Pro Leu Leu Leu His Gly Asp Ala Ala Phe Ala Gly Leu Gly Ile
   675 680 685
   Val Pro Glu Thr Ile Asn Leu Ala Lys Leu Arg Gly Tyr Asp Val Gly
   690 695 700
   Gly Thr Ile His Ile Val Val Asn Asn Gln Ile Gly Phe Thr Thr Thr
   705 710 715 720
   Pro Asp Ser Ser Arg Ser Met His Tyr Ala Thr Asp Tyr Ala Lys Ala
   725 730 735
   Phe Gly Cys Pro Val Phe His Val Asn Gly Asp Asp Pro Glu Ala Val
   740 745 750
   Val Trp Val Gly Gln Leu Ala Thr Glu Tyr Arg Arg Arg Phe Gly Lys
   755 760 765
   Asp Val Phe Ile Asp Leu Val Cys Tyr Arg Leu Arg Gly His Asn Glu
   770 775 780
   Ala Asp Asp Pro Ser Met Thr Gln Pro Lys Met Tyr Glu Leu Ile Thr
   785 790 795 800
   Gly Arg Glu Thr Val Arg Ala Gln Tyr Thr Glu Asp Leu Leu Gly Arg
   805 810 815
   Gly Asp Leu Ser Asn Glu Asp Ala Glu Ala Val Val Arg Asp Phe His
   820 825 830
   Asp Gln Met Glu Ser Val Phe Asn Glu Val Lys Glu Gly Gly Lys Lys
   835 840 845
   Gln Ala Glu Ala Gln Thr Gly Ile Thr Gly Ser Gln Lys Leu Pro His
   850 855 860
   Gly Leu Glu Thr Asn Ile Ser Arg Glu Glu Leu Leu Glu Leu Gly Gln
   865 870 875 880
   Ala Phe Ala Asn Thr Pro Glu Gly Phe Asn Tyr His Pro Arg Val Ala
   885 890 895
   Pro Val Ala Lys Lys Arg Val Ser Ser Val Thr Glu Gly Gly Ile Asp
   900 905 910
   Trp Ala Trp Gly Glu Leu Leu Ala Phe Gly Ser Leu Ala Asn Ser Gly
   915 920 925
   Arg Leu Val Arg Leu Ala Gly Glu Asp Ser Arg Arg Gly Thr Phe Thr
   930 935 940
   Gln Arg His Ala Val Ala Ile Asp Pro Ala Thr Ala Glu Glu Phe Asn
   945 950 955 960
   Pro Leu His Glu Leu Ala Gln Ser Lys Gly Asn Asn Gly Lys Phe Leu
   965 970 975
   Val Tyr Asn Ser Ala Leu Thr Glu Tyr Ala Gly Met Gly Phe Glu Tyr
   980 985 990
   Gly Tyr Ser Val Gly Asn Glu Asp Ser Val Val Ala Trp Glu Ala Gln
   995 1000 1005
   Phe Gly Asp Phe Ala Asn Gly Ala Gln Thr Ile Ile Asp Glu Tyr
   1010 1015 1020
   Val Ser Ser Gly Glu Ala Lys Trp Gly Gln Thr Ser Lys Leu Ile
   1025 1030 1035
   Leu Leu Leu Pro His Gly Tyr Glu Gly Gln Gly Pro Asp His Ser
   1040 1045 1050
   Ser Ala Arg Ile Glu Arg Phe Leu Gln Leu Cys Ala Glu Gly Ser
   1055 1060 1065
   Met Thr Val Ala Gln Pro Ser Thr Pro Ala Asn His Phe His Leu
   1070 1075 1080
   Leu Arg Arg His Ala Leu Ser Asp Leu Lys Arg Pro Leu Val Ile
   1085 1090 1095
   Phe Thr Pro Lys Ser Met Leu Arg Asn Lys Ala Ala Ala Ser Ala
   1100 1105 1110
   Pro Glu Asp Phe Thr Glu Val Thr Lys Phe Gln Ser Val Ile Asp
   1115 1120 1125
   Asp Pro Asn Val Ala Asp Ala Ala Lys Val Lys Lys Val Met Leu
   1130 1135 1140
   Val Ser Gly Lys Leu Tyr Tyr Glu Leu Ala Lys Arg Lys Glu Lys
   1145 1150 1155
   Asp Gly Arg Asp Asp Ile Ala Ile Val Arg Ile Glu Met Leu His
       1160                1165                1170
   Pro Ile  Pro Phe Asn Arg Ile  Ser Glu Ala Leu Ala  Gly Tyr Pro
       1175                1180                1185
   Asn Ala  Glu Glu Val Leu Phe  Val Gln Asp Glu Pro  Ala Asn Gln
       1190                1195                1200
   Gly Pro  Trp Pro Phe Tyr Gln  Glu His Leu Pro Glu  Leu Ile Pro
       1205                1210                1215
   Asn Met  Pro Lys Met Arg Arg  Val Ser Arg Arg Ala  Gln Ser Ser
       1220                1225                1230
   Thr Ala  Thr Gly Val Ala Lys  Val His Gln Leu Glu  Glu Lys Gln
       1235                1240                1245
   Leu Ile  Asp Glu Ala Phe Glu  Ala
       1250                1255
<210> 13
   <211> 4388
   <212> DNA
   <213> Corynebacterium glutamicum
<220>
   <221> CDS
   <222> (443)..(4213)
   <223>
<400> 13
   gtcgacaagc aaaatcgaag cggcagcacg ccgcgtcgga gccttaaacg ccatcgccgc     60
   catccctgat ggtttcaatc atcaagtcgg tgaacgcggg cgcaacctgt catccggaca    120
   gcgccaactg atcgcgctgg cgcgcgccga actcatcgag ccttccatca tgcttctcga    180
   cgaagccacc tccaccctcg accccgccac cgaagccgtt atcctcaacg cctccgatcg    240
   agtcactaag ggacgcacca gcatcatcgt cgcgcaccgc ttggcaaccg ctaaaagggc    300
   cgaccgtatt cttgttgttg aacaaggacg tatcattgag gacggatctc acgacgcgtt    360
   gttgtctgct aacggcacct acgcccgcat gtggcattta atggcctgac acgttatttt    420
   taggagaact gtcaacaaat ta atg cta caa ctg ggg ctt agg cat aat cag      472
                            Met Leu Gln Leu Gly Leu Arg His Asn Gln
                            1               5                   10
   cca acg acc aac gtt aca gtg gat aaa ata aag ctc aat aaa ccc tca      520
   Pro Thr Thr Asn Val Thr Val Asp Lys Ile Lys Leu Asn Lys Pro Ser
                   15                  20                  25
   aga agc aag gaa aag agg cga gta cct gcc gtg agc agc gct agt act      568
   Arg Ser Lys Glu Lys Arg Arg Val Pro Ala Val Ser Ser Ala Ser Thr
               30                  35                  40
   ttc ggc cag aat gcg tgg ctg gta gac gag atg ttc cag cag ttc cag      616
   Phe Gly Gln Asn Ala Trp Leu Val Asp Glu Met Phe Gln Gln Phe Gln
           45                  50                  55
   aag gac ccc aag tcc gtg gac aag gaa tgg aga gaa ctc ttt gag gcg      664
   Lys Asp Pro Lys Ser Val Asp Lys Glu Trp Arg Glu Leu Phe Glu Ala
       60                  65                  70
   cag ggg gga cca aat gct acc ccc gct aca aca gaa gca cag cct tca      712
   Gln Gly Gly Pro Asn Ala Thr Pro Ala Thr Thr Glu Ala Gln Pro Ser
   75                  80                  85                  90
   gcg ccc aag gag tct gcg aaa cca gca cca aag gct gcc cct gca gcc      760
   Ala Pro Lys Glu Ser Ala Lys Pro Ala Pro Lys Ala Ala Pro Ala Ala
                   95                  100                 105
   aag gca gca ccg cgc gta gaa acc aag ccg gcc gcc aag acc gcc cct      808
   Lys Ala Ala Pro Arg Val Glu Thr Lys Pro Ala Ala Lys Thr Ala Pro
               110                 115                 120
   aag gcc aag gag tcc tca gtg cca cag caa cct aag ctt ccg gag cca      856
   Lys Ala Lys Glu Ser Ser Val Pro Gln Gln Pro Lys Leu Pro Glu Pro
           125                 130                 135
   gga caa acc cca atc agg ggt att ttc aag tcc atc gcg aag aac atg      904
   Gly Gln Thr Pro Ile Arg Gly Ile Phe Lys Ser Ile Ala Lys Asn Met
       140                 145                 150
   gat atc tcc ctg gaa atc cca acc gca acc tcg gtt cgc gat atg cca      952
   Asp Ile Ser Leu Glu Ile Pro Thr Ala Thr Ser Val Arg Asp Met Pro
   155                 160                 165                 170
   gct cgc ctc atg ttc gaa aac cgc gcg atg gtc aac gat cag ctc aag      1000
   Ala Arg Leu Met Phe Glu Asn Arg Ala Met Val Asn Asp Gln Leu Lys
                   175                 180                 185
   cgc acc cgc ggt ggc aag atc tcc ttc acc cac atc att ggc tac gcc      1048
   Arg Thr Arg Gly Gly Lys Ile Ser Phe Thr His Ile Ile Gly Tyr Ala
               190                 195                 200
   atg gtg aag gca gtc atg gct cac ccg gac atg aac aac tcc tac gac      1096
   Met Val Lys Ala Val Met Ala His Pro Asp Met Asn Asn Ser Tyr Asp
           205                 210                 215
   gtc atc gac ggc aag cca acc ctg atc gtg cct gag cac atc aac ctg      1144
   Val Ile Asp Gly Lys Pro Thr Leu Ile Val Pro Glu His Ile Asn Leu
       220                 225                 230
   ggc ctt gcc atc gac ctt cct cag aag gac ggc tcc cgc gca ctt gtc      1192
   Gly Leu Ala Ile Asp Leu Pro Gln Lys Asp Gly Ser Arg Ala Leu Val
   235                 240                 245                 250
   gta gca gcc atc aag gaa acc gag aag atg aac ttc tcc gag ttc ctc      1240
   Val Ala Ala Ile Lys Glu Thr Glu Lys Met Asn Phe Ser Glu Phe Leu
                   255                 260                 265
   gca gca tac gaa gac atc gtg aca cgc tcc cgc aag ggc aag ctc acc      1288
   Ala Ala Tyr Glu Asp Ile Val Thr Arg Ser Arg Lys Gly Lys Leu Thr
               270                 275                 280
   atg gat gac tac cag ggc gtt acc gtt tcc ttg acc aac cca ggt ggc      1336
   Met Asp Asp Tyr Gln Gly Val Thr Val Ser Leu Thr Asn Pro Gly Gly
           285                 290                 295
   atc ggt acc cgc cac tct gtc cca cgt ctg acc aag ggc cag ggc acc      1384
   Ile Gly Thr Arg His Ser Val Pro Arg Leu Thr Lys Gly Gln Gly Thr
       300                 305                 310
   atc atc ggt gtc ggt tcc atg gat tac cca gca gag ttc cag ggc gct      1432
   Ile Ile Gly Val Gly Ser Met Asp Tyr Pro Ala Glu Phe Gln Gly Ala
   315                 320                 325                 330
   tcc gaa gac cgc ctt gca gag ctc ggc gtt gga aag ctt gtc acc atc 1480
   Ser Glu Asp Arg Leu Ala Glu Leu Gly Val Gly Lys Leu Val Thr Ile
   335 340 345
   acc tcc acc tac gat cac cgc gtg atc cag ggt gct gtg tcc ggt gaa 1528
   Thr Ser Thr Tyr Asp His Arg Val Ile Gln Gly Ala Val Ser Gly Glu
   350 355 360
   ttc ctg cgt acc atg tct cgc ctg ctc acc gat gat tcc ttc tgg gat 1576
   Phe Leu Arg Thr Met Ser Arg Leu Leu Thr Asp Asp Ser Phe Trp Asp
   365 370 375
   gag atc ttc gac gca atg aac gtt cct tac acc cca atg cgt tgg gca 1624
   Glu Ile Phe Asp Ala Met Asn Val Pro Tyr Thr Pro Met Arg Trp Ala
   380 385 390
   cag gac gtt cca aac acc ggt gtt gat aag aac acc cgc gtc atg cag 1672
   Gln Asp Val Pro Asn Thr Gly Val Asp Lys Asn Thr Arg Val Met Gln
   395 400 405 410
   ctc att gag gca tac cgc tcc cgt gga cac ctc atc gct gac acc aac 1720
   Leu Ile Glu Ala Tyr Arg Ser Arg Gly His Leu Ile Ala Asp Thr Asn
   415 420 425
   cca ctt tca tgg gtt cag cct ggc atg cca gtt cca gac cac cgc gac 1768
   Pro Leu Ser Trp Val Gln Pro Gly Met Pro Val Pro Asp His Arg Asp
   430 435 440
   ctc gac atc gag acc cac agc ctg acc atc tgg gat ctg gac cgt acc 1816
   Leu Asp Ile Glu Thr His Ser Leu Thr Ile Trp Asp Leu Asp Arg Thr
   445 450 455
   ttc agc gtc ggt ggc ttc ggc ggc aag gag acc atg acc ctg cgc gag 1864
   Phe Ser Val Gly Gly Phe Gly Gly Lys Glu Thr Met Thr Leu Arg Glu
   460 465 470
   gta ctg tcc cgc ctg cgc gct gcc tac acc ttg aag gtc ggc tcc gaa 1912
   Val Leu Ser Arg Leu Arg Ala Ala Tyr Thr Leu Lys Val Gly Ser Glu
   475 480 485 490
   tac acc cac atc ctg gac cgc gac gag cgc acc tgg ctg cag gac cgc 1960
   Tyr Thr His Ile Leu Asp Arg Asp Glu Arg Thr Trp Leu Gln Asp Arg
   495 500 505
   ctc gaa gcc gga atg cca aag cca acc cag gca gag cag aag tac atc 2008
   Leu Glu Ala Gly Met Pro Lys Pro Thr Gln Ala Glu Gln Lys Tyr Ile
   510 515 520
   ctg cag aag ctg aac gcc gca gag gct ttc gag aac ttc ctg cag acc 2056
   Leu Gln Lys Leu Asn Ala Ala Glu Ala Phe Glu Asn Phe Leu Gln Thr
   525 530 535
   aag tac gtc ggc cag aag cgc ttc tcc ctc gaa ggt gca gaa gct ctc 2104
   Lys Tyr Val Gly Gln Lys Arg Phe Ser Leu Glu Gly Ala Glu Ala Leu
   540 545 550
   atc cca ctg atg gac tcc gcc atc gac acc gcc gca ggc cag ggc ctc 2152
   Ile Pro Leu Met Asp Ser Ala Ile Asp Thr Ala Ala Gly Gln Gly Leu
   555 560 565 570
   gac gaa gtt gtc atc ggt atg cca cac cgt ggt cgc ctc aac gtg ctg 2200
   Asp Glu Val Val Ile Gly Met Pro His Arg Gly Arg Leu Asn Val Leu
   575 580 585
   ttc aac atc gtg ggc aag cca ctg gca tcc atc ttc aac gag ttt gaa 2248
   Phe Asn Ile Val Gly Lys Pro Leu Ala Ser Ile Phe Asn Glu Phe Glu
   590 595 600
   ggc caa atg gag cag ggc cag atc ggt ggc tcc ggt gac gtg aag tac 2296
   Gly Gln Met Glu Gln Gly Gln Ile Gly Gly Ser Gly Asp Val Lys Tyr
   605 610 615
   cac ctc ggt tcc gaa ggc cag cac ctg cag atg ttc ggc gac ggc gag 2344
   His Leu Gly Ser Glu Gly Gln His Leu Gln Met Phe Gly Asp Gly Glu
   620 625 630
   atc aag gtc tcc ctg act gct aac ccg tcc cac ctg gaa gct gtt aac 2392
   Ile Lys Val Ser Leu Thr Ala Asn Pro Ser His Leu Glu Ala Val Asn
   635 640 645 650
   cca gtg atg gaa ggt atc gtc cgc gca aag cag gac tac ctg gac aag 2440
   Pro Val Met Glu Gly Ile Val Arg Ala Lys Gln Asp Tyr Leu Asp Lys
   655 660 665
   ggc gta gac ggc aag act gtt gtg cca ctg ctg ctc cac ggt gac gct 2488
   Gly Val Asp Gly Lys Thr Val Val Pro Leu Leu Leu His Gly Asp Ala
   670 675 680
   gca ttc gca ggc ctg ggc atc gtg cca gaa acc atc aac ctg gct aag 2536
   Ala Phe Ala Gly Leu Gly Ile Val Pro Glu Thr Ile Asn Leu Ala Lys
   685 690 695
   cgc gtc gac gtc gga ggc acc atc cac atc gtg gtg aac aac cag atc 2584
   Arg Val Asp Val Gly Gly Thr Ile His Ile Val Val Asn Asn Gln Ile
   700 705 710
   ggc ttc acc acc acc cca gac tcc agc cgc tcc atg cac tac gca acc 2632
   Gly Phe Thr Thr Thr Pro Asp Ser Ser Arg Ser Met His Tyr Ala Thr
   715 720 725 730
   gac tac gcc aag gca ttc ggc tgc cca gtc ttc cac gtc aat ggt gat 2680
   Asp Tyr Ala Lys Ala Phe Gly Cys Pro Val Phe His Val Asn Gly Asp
   735 740 745
   gac cca gag gca gtt gtc tgg gtt ggc cag ctg gca acc gag tac cgt 2728
   Asp Pro Glu Ala Val Val Trp Val Gly Gln Leu Ala Thr Glu Tyr Arg
   750 755 760
   cgt cgc ttc ggc aag gac gtc ttc atc gac ctc gtt tgc tac cgc ctc 2776
   Arg Arg Phe Gly Lys Asp Val Phe Ile Asp Leu Val Cys Tyr Arg Leu
   765 770 775
   cgc ggc cac aac gaa gct gat gat cct tcc atg acc cag cca aag atg 2824
   Arg Gly His Asn Glu Ala Asp Asp Pro Ser Met Thr Gln Pro Lys Met
   780 785 790
   tat gag ctc atc acc ggc cgc gag acc gtt cgt gct cag tac acc gaa 2872
   Tyr Glu Leu Ile Thr Gly Arg Glu Thr Val Arg Ala Gln Tyr Thr Glu
   795 800 805 810
   gac ctg ctc gga cgt gga gac ctc tcc aac gaa gat gca gaa gca gtc 2920
   Asp Leu Leu Gly Arg Gly Asp Leu Ser Asn Glu Asp Ala Glu Ala Val
   815 820 825
   gtc cgc gac ttc cac gac cag atg gaa tct gtg ttc aac gaa gtc aag 2968
   Val Arg Asp Phe His Asp Gln Met Glu Ser Val Phe Asn Glu Val Lys
   830 835 840
   gaa ggc ggc aag aag cag gct gag gca cag acc ggc atc acc ggc tcc        3016
   Glu Gly Gly Lys Lys Gln Ala Glu Ala Gln Thr Gly Ile Thr Gly Ser
           845                 850                 855
   cag aag ctt cca cac ggc ctt gag acc aac atc tcc cgt gaa gag ctc        3064
   Gln Lys Leu Pro His Gly Leu Glu Thr Asn Ile Ser Arg Glu Glu Leu
       860                 865                 870
   ctg gaa ctg gga cag gct ttc gcc aac acc cca gaa ggc ttc aac tac        3112
   Leu Glu Leu Gly Gln Ala Phe Ala Asn Thr Pro Glu Gly Phe Asn Tyr
   875                 880                 885                 890
   cac cca cgt gtg gct cca gtt gct aag aag cgc gtc tcc tct gtc acc        3160
   His Pro Arg Val Ala Pro Val Ala Lys Lys Arg Val Ser Ser Val Thr
                   895                 900                 905
   gaa ggt ggc atc gac tgg gca tgg ggc gag ctc ctc gcc ttc ggt tcc        3208
   Glu Gly Gly Ile Asp Trp Ala Trp Gly Glu Leu Leu Ala Phe Gly Ser
               910                 915                 920
   ctg gct aac tcc ggc cgc ttg gtt cgc ctt gca ggt gaa gat tcc cgc        3256
   Leu Ala Asn Ser Gly Arg Leu Val Arg Leu Ala Gly Glu Asp Ser Arg
           925                 930                 935
   cgc ggt acc ttc acc cag cgc cac gca gtt gcc atc gac cca gcg acc        3304
   Arg Gly Thr Phe Thr Gln Arg His Ala Val Ala Ile Asp Pro Ala Thr
       940                 945                 950
   gct gaa gag ttc aac cca ctc cac gag ctt gca cag tcc aag ggc aac        3352
   Ala Glu Glu Phe Asn Pro Leu His Glu Leu Ala Gln Ser Lys Gly Asn
   955                 960                 965                 970
   aac ggt aag ttc ctg gtc tac aac tcc gca ctg acc gag tac gca ggc        3400
   Asn Gly Lys Phe Leu Val Tyr Asn Ser Ala Leu Thr Glu Tyr Ala Gly
                   975                 980                 985
   atg ggc ttc gag tac ggc tac tcc gta gga aac gaa gac tcc  gtc gtt       3448
   Met Gly Phe Glu Tyr Gly Tyr Ser Val Gly Asn Glu Asp Ser  Val Val
               990                 995                 1000
   gca tgg gaa  gca cag ttc ggc gac  ttc gcc aac ggc gct  cag acc         3493
   Ala Trp Glu  Ala Gln Phe Gly Asp  Phe Ala Asn Gly Ala  Gln Thr
           1005                 1010                 1015
   atc atc gat  gag tac gtc tcc tca  ggc gaa gct aag tgg  ggc cag         3538
   Ile Ile Asp  Glu Tyr Val Ser Ser  Gly Glu Ala Lys Trp  Gly Gln
           1020                 1025                 1030
   acc tcc aag  ctg atc ctt ctg ctg  cct cac ggc tac gaa  ggc cag         3583
   Thr Ser Lys  Leu Ile Leu Leu Leu  Pro His Gly Tyr Glu  Gly Gln
           1035                 1040                 1045
   ggc cca gac  cac tct tcc gca cgt  atc gag cgc ttc ctg  cag ctg         3628
   Gly Pro Asp  His Ser Ser Ala Arg  Ile Glu Arg Phe Leu  Gln Leu
           1050                 1055                 1060
   tgc gct gag  ggt tcc atg act gtt  gct cag cca tcc acc  cca gca         3673
   Cys Ala Glu  Gly Ser Met Thr Val  Ala Gln Pro Ser Thr  Pro Ala
           1065                 1070                 1075
   aac cac ttc  cac ctg ctg cgt cgt  cac gct ctg tcc gac  ctg aag         3718
   Asn His Phe  His Leu Leu Arg Arg  His Ala Leu Ser Asp  Leu Lys
           1080                 1085                 1090
   cgt cca ctg gtt atc ttc acc ccg aag tcc atg ctg cgt aac aag 3763
   Arg Pro Leu Val Ile Phe Thr Pro Lys Ser Met Leu Arg Asn Lys
   1095 1100 1105
   gct gct gcc tcc gca cca gaa gac ttc act gag gtc acc aag ttc 3808
   Ala Ala Ala Ser Ala Pro Glu Asp Phe Thr Glu Val Thr Lys Phe
   1110 1115 1120
   caa tcc gtg atc gac gat cca aac gtt gca gat gca gcc aag gtg 3853
   Gln Ser Val Ile Asp Asp Pro Asn Val Ala Asp Ala Ala Lys Val
   1125 1130 1135
   aag aag gtc atg ctg gtc tcc ggc aag ctg tac tac gaa ttg gca 3898
   Lys Lys Val Met Leu Val Ser Gly Lys Leu Tyr Tyr Glu Leu Ala
   1140 1145 1150
   aag cgc aag gag aag gac gga cgc gac gac atc gcg atc gtt cgt 3943
   Lys Arg Lys Glu Lys Asp Gly Arg Asp Asp Ile Ala Ile Val Arg
   1155 1160 1165
   atc gaa atg ctc cac cca att ccg ttc aac cgc atc tcc gag gct 3988
   Ile Glu Met Leu His Pro Ile Pro Phe Asn Arg Ile Ser Glu Ala
   1170 1175 1180
   ctt gcc ggc tac cct aac gct gag gaa gtc ctc ttc gtt cag gat 4033
   Leu Ala Gly Tyr Pro Asn Ala Glu Glu Val Leu Phe Val Gln Asp
   1185 1190 1195
   gag cca gca aac cag ggc cca tgg ccg ttc tac cag gag cac ctc 4078
   Glu Pro Ala Asn Gln Gly Pro Trp Pro Phe Tyr Gln Glu His Leu
   1200 1205 1210
   cca gag ctg atc ccg aac atg cca aag atg cgc cgc gtt tcc cgc 4123
   Pro Glu Leu Ile Pro Asn Met Pro Lys Met Arg Arg Val Ser Arg
   1215 1220 1225
   cgc gct cag tcc tcc acc gca act ggt gtt gct aag gtg cac cag 4168
   Arg Ala Gln Ser Ser Thr Ala Thr Gly Val Ala Lys Val His Gln
   1230 1235 1240
   ctg gag gag aag cag ctt atc gac gag gct ttc gag gct taa gtc 4213
   Leu Glu Glu Lys Gln Leu Ile Asp Glu Ala Phe Glu Ala Val
   1245 1250 1255
   tttatagtcc tgcactagcc tagagggcct tatgcagtgt gaatcacaca gcataaggcc 4273
   ctttttgctg ccgtggttgc ctaaggtgga aggcatgaaa cgaatctgtg cggtcacgat 4333
   ctcttcagta cttttgctaa gtggctgctc ctccacttcc accacgcagc tcgag 4388
<210> 14
   <211> 1255
   <212> PRT
   <213> Corynebacterium glutamicum
<400> 14
   Met Leu Gln Leu Gly Leu Arg His Asn Gln Pro Thr Thr Asn Val Thr
   1 5 10 15
   Val Asp Lys Ile Lys Leu Asn Lys Pro Ser Arg Ser Lys Glu Lys Arg
   20 25 30
   Arg Val Pro Ala Val Ser Ser Ala Ser Thr Phe Gly Gln Asn Ala Trp
   35 40 45
   Leu Val Asp Glu Met Phe Gln Gln Phe Gln Lys Asp Pro Lys Ser Val
   50 55 60
   Asp Lys Glu Trp Arg Glu Leu Phe Glu Ala Gln Gly Gly Pro Asn Ala
   65 70 75 80
   Thr Pro Ala Thr Thr Glu Ala Gln Pro Ser Ala Pro Lys Glu Ser Ala
   85 90 95
   Lys Pro Ala Pro Lys Ala Ala Pro Ala Ala Lys Ala Ala Pro Arg Val
   100 105 110
   Glu Thr Lys Pro Ala Ala Lys Thr Ala Pro Lys Ala Lys Glu Ser Ser
   115 120 125
   Val Pro Gln Gln Pro Lys Leu Pro Glu Pro Gly Gln Thr Pro Ile Arg
   130 135 140
   Gly Ile Phe Lys Ser Ile Ala Lys Asn Met Asp Ile Ser Leu Glu Ile
   145 150 155 160
   Pro Thr Ala Thr Ser Val Arg Asp Met Pro Ala Arg Leu Met Phe Glu
   165 170 175
   Asn Arg Ala Met Val Asn Asp Gln Leu Lys Arg Thr Arg Gly Gly Lys
   180 185 190
   Ile Ser Phe Thr His Ile Ile Gly Tyr Ala Met Val Lys Ala Val Met
   195 200 205
   Ala His Pro Asp Met Asn Asn Ser Tyr Asp Val Ile Asp Gly Lys Pro
   210 215 220
   Thr Leu Ile Val Pro Glu His Ile Asn Leu Gly Leu Ala Ile Asp Leu
   225 230 235 240
   Pro Gln Lys Asp Gly Ser Arg Ala Leu Val Val Ala Ala Ile Lys Glu
   245 250 255
   Thr Glu Lys Met Asn Phe Ser Glu Phe Leu Ala Ala Tyr Glu Asp Ile
   260 265 270
   Val Thr Arg Ser Arg Lys Gly Lys Leu Thr Met Asp Asp Tyr Gln Gly
   275 280 285
   Val Thr Val Ser Leu Thr Asn Pro Gly Gly Ile Gly Thr Arg His Ser
   290 295 300
   Val Pro Arg Leu Thr Lys Gly Gln Gly Thr Ile Ile Gly Val Gly Ser
   305 310 315 320
   Met Asp Tyr Pro Ala Glu Phe Gln Gly Ala Ser Glu Asp Arg Leu Ala
   325 330 335
   Glu Leu Gly Val Gly Lys Leu Val Thr Ile Thr Ser Thr Tyr Asp His
   340 345 350
   Arg Val Ile Gln Gly Ala Val Ser Gly Glu Phe Leu Arg Thr Met Ser
   355 360 365
   Arg Leu Leu Thr Asp Asp Ser Phe Trp Asp Glu Ile Phe Asp Ala Met
   370 375 380
   Asn Val Pro Tyr Thr Pro Met Arg Trp Ala Gln Asp Val Pro Asn Thr
   385 390 395 400
   Gly Val Asp Lys Asn Thr Arg Val Met Gln Leu Ile Glu Ala Tyr Arg
   405 410 415
   Ser Arg Gly His Leu Ile Ala Asp Thr Asn Pro Leu Ser Trp Val Gln
   420 425 430
   Pro Gly Met Pro Val Pro Asp His Arg Asp Leu Asp Ile Glu Thr His
   435 440 445
   Ser Leu Thr Ile Trp Asp Leu Asp Arg Thr Phe Ser Val Gly Gly Phe
   450 455 460
   Gly Gly Lys Glu Thr Met Thr Leu Arg Glu Val Leu Ser Arg Leu Arg
   465 470 475 480
   Ala Ala Tyr Thr Leu Lys Val Gly Ser Glu Tyr Thr His Ile Leu Asp
   485 490 495
   Arg Asp Glu Arg Thr Trp Leu Gln Asp Arg Leu Glu Ala Gly Met Pro
   500 505 510
   Lys Pro Thr Gln Ala Glu Gln Lys Tyr Ile Leu Gln Lys Leu Asn Ala
   515 520 525
   Ala Glu Ala Phe Glu Asn Phe Leu Gln Thr Lys Tyr Val Gly Gln Lys
   530 535 540
   Arg Phe Ser Leu Glu Gly Ala Glu Ala Leu Ile Pro Leu Met Asp Ser
   545 550 555 560
   Ala Ile Asp Thr Ala Ala Gly Gln Gly Leu Asp Glu Val Val Ile Gly
   565 570 575
   Met Pro His Arg Gly Arg Leu Asn Val Leu Phe Asn Ile Val Gly Lys
   580 585 590
   Pro Leu Ala Ser Ile Phe Asn Glu Phe Glu Gly Gln Met Glu Gln Gly
   595 600 605
   Gln Ile Gly Gly Ser Gly Asp Val Lys Tyr His Leu Gly Ser Glu Gly
   610 615 620
   Gln His Leu Gln Met Phe Gly Asp Gly Glu Ile Lys Val Ser Leu Thr
   625 630 635 640
   Ala Asn Pro Ser His Leu Glu Ala Val Asn Pro Val Met Glu Gly Ile
   645 650 655
   Val Arg Ala Lys Gln Asp Tyr Leu Asp Lys Gly Val Asp Gly Lys Thr
   660 665 670
   Val Val Pro Leu Leu Leu His Gly Asp Ala Ala Phe Ala Gly Leu Gly
   675 680 685
   Ile Val Pro Glu Thr Ile Asn Leu Ala Lys Arg Val Asp Val Gly Gly
   690 695 700
   Thr Ile His Ile Val Val Asn Asn Gln Ile Gly Phe Thr Thr Thr Pro
   705 710 715 720
   Asp Ser Ser Arg Ser Met His Tyr Ala Thr Asp Tyr Ala Lys Ala Phe
   725 730 735
   Gly Cys Pro Val Phe His Val Asn Gly Asp Asp Pro Glu Ala Val Val
   740 745 750
   Trp Val Gly Gln Leu Ala Thr Glu Tyr Arg Arg Arg Phe Gly Lys Asp
   755 760 765
   Val Phe Ile Asp Leu Val Cys Tyr Arg Leu Arg Gly His Asn Glu Ala
   770 775 780
   Asp Asp Pro Ser Met Thr Gln Pro Lys Met Tyr Glu Leu Ile Thr Gly
   785 790 795 800
   Arg Glu Thr Val Arg Ala Gln Tyr Thr Glu Asp Leu Leu Gly Arg Gly
   805 810 815
   Asp Leu Ser Asn Glu Asp Ala Glu Ala Val Val Arg Asp Phe His Asp
   820 825 830
   Gln Met Glu Ser Val Phe Asn Glu Val Lys Glu Gly Gly Lys Lys Gln
   835 840 845
   Ala Glu Ala Gln Thr Gly Ile Thr Gly Ser Gln Lys Leu Pro His Gly
   850 855 860
   Leu Glu Thr Asn Ile Ser Arg Glu Glu Leu Leu Glu Leu Gly Gln Ala
   865 870 875 880
   Phe Ala Asn Thr Pro Glu Gly Phe Asn Tyr His Pro Arg Val Ala Pro
   885 890 895
   Val Ala Lys Lys Arg Val Ser Ser Val Thr Glu Gly Gly Ile Asp Trp
   900 905 910
   Ala Trp Gly Glu Leu Leu Ala Phe Gly Ser Leu Ala Asn Ser Gly Arg
   915 920 925
   Leu Val Arg Leu Ala Gly Glu Asp Ser Arg Arg Gly Thr Phe Thr Gln
   930 935 940
   Arg His Ala Val Ala Ile Asp Pro Ala Thr Ala Glu Glu Phe Asn Pro
   945 950 955 960
   Leu His Glu Leu Ala Gln Ser Lys Gly Asn Asn Gly Lys Phe Leu Val
   965 970 975
   Tyr Asn Ser Ala Leu Thr Glu Tyr Ala Gly Met Gly Phe Glu Tyr Gly
   980 985 990
   Tyr Ser Val Gly Asn Glu Asp Ser Val Val Ala Trp Glu Ala Gln Phe
   995 1000 1005
   Gly Asp Phe Ala Asn Gly Ala Gln Thr Ile Ile Asp Glu Tyr Val
   1010 1015 1020
   Ser Ser Gly Glu Ala Lys Trp Gly Gln Thr Ser Lys Leu Ile Leu
   1025 1030 1035
   Leu Leu Pro His Gly Tyr Glu Gly Gln Gly Pro Asp His Ser Ser
   1040 1045 1050
   Ala Arg Ile Glu Arg Phe Leu Gln Leu Cys Ala Glu Gly Ser Met
   1055 1060 1065
   Thr Val Ala Gln Pro Ser Thr Pro Ala Asn His Phe His Leu Leu
   1070 1075 1080
   Arg Arg His Ala Leu Ser Asp Leu Lys Arg Pro Leu Val Ile Phe
   1085 1090 1095
   Thr Pro Lys Ser Met Leu Arg Asn Lys Ala Ala Ala Ser Ala Pro
   1100 1105 1110
   Glu Asp Phe Thr Glu Val Thr Lys Phe Gln Ser Val Ile Asp Asp
   1115 1120 1125
   Pro Asn Val Ala Asp Ala Ala Lys Val Lys Lys Val Met Leu Val
   1130 1135 1140
   Ser Gly Lys Leu Tyr Tyr Glu Leu Ala Lys Arg Lys Glu Lys Asp
   1145 1150 1155
   Gly Arg Asp Asp Ile Ala Ile Val Arg Ile Glu Met Leu His Pro
   1160 1165 1170
   Ile Pro Phe Asn Arg Ile Ser Glu Ala Leu Ala Gly Tyr Pro Asn
   1175 1180 1185
   Ala Glu Glu Val Leu Phe Val Gln Asp Glu Pro Ala Asn Gln Gly
   1190 1195 1200
   Pro Trp Pro Phe Tyr Gln Glu His Leu Pro Glu Leu Ile Pro Asn
   1205 1210 1215
   Met Pro Lys Met Arg Arg Val Ser Arg Arg Ala Gln Ser Ser Thr
   1220 1225 1230
   Ala Thr Gly Val Ala Lys Val His Gln Leu Glu Glu Lys Gln Leu
   1235 1240 1245
   Ile Asp Glu Ala Phe Glu Ala
   1250 1255
<210> 15
   <211> 4385
   <212> DNA
   <213> Corynebacterium glutamicum
<220>
   <221> CDS
   <222> (443).. (4213)
   <223>
<400> 15
   gtcgacaagc aaaatcgaag cggcagcacg ccgcgtcgga gccttaaacg ccatcgccgc 60
   catccctgat ggtttcaatc atcaagtcgg tgaacgcggg cgcaacctgt catccggaca 120
   gcgccaactg atcgcgctgg cgcgcgccga actcatcgag ccttccatca tgcttctcga 180
   cgaagccacc tccaccctcg accccgccac cgaagccgtt atcctcaacg cctccgatcg 240
   agtcactaag ggacgcacca gcatcatcgt cgcgcaccgc ttggcaaccg ctaaaagggc 300
   cgaccgtatt cttgttgttg aacaaggacg tatcattgag gacggatctc acgacgcgtt 360
   gttgtctgct aacggcacct acgcccgcat gtggcattta atggcctgac acgttatttt 420
   taggagaact gtcaacaaat ta atg cta caa ctg ggg ctt agg cat aat cag 472
   Met Leu Gln Leu Gly Leu Arg His Asn Gln
   1 5 10
   cca acg acc aac gtt aca gtg gat aaa ata aag ctc aat aaa ccc tca 520
   Pro Thr Thr Asn Val Thr Val Asp Lys Ile Lys Leu Asn Lys Pro Ser
   15 20 25
   aga agc aag gaa aag agg cga gta cct gcc gtg agc agc gct agt act 568
   Arg Ser Lys Glu Lys Arg Arg Val Pro Ala Val Ser Ser Ala Ser Thr
   30 35 40
   ttc ggc cag aat gcg tgg ctg gta gac gag atg ttc cag cag ttc cag 616
   Phe Gly Gln Asn Ala Trp Leu Val Asp Glu Met Phe Gln Gln Phe Gln
   45 50 55
   aag gac ccc aag tcc gtg gac aag gaa tgg aga gaa ctc ttt gag gcg 664
   Lys Asp Pro Lys Ser Val Asp Lys Glu Trp Arg Glu Leu Phe Glu Ala
   60 65 70
   cag ggg gga cca aat gct acc ccc gct aca aca gaa gca cag cct tca 712
   Gln Gly Gly Pro Asn Ala Thr Pro Ala Thr Thr Glu Ala Gln Pro Ser
   75 80 85 90
   gcg ccc aag gag tct gcg aaa cca gca cca aag gct gcc cct gca gcc 760
   Ala Pro Lys Glu Ser Ala Lys Pro Ala Pro Lys Ala Ala Pro Ala Ala
   95 100 105
   aag gca gca ccg cgc gta gaa acc aag ccg gcc gcc aag acc gcc cct 808
   Lys Ala Ala Pro Arg Val Glu Thr Lys Pro Ala Ala Lys Thr Ala Pro
   110 115 120
   aag gcc aag gag tcc tca gtg cca cag caa cct aag ctt ccg gag cca 856
   Lys Ala Lys Glu Ser Ser Val Pro Gln Gln Pro Lys Leu Pro Glu Pro
   125 130 135
   gga caa acc cca atc agg ggt att ttc aag tcc atc gcg aag aac atg 904
   Gly Gln Thr Pro Ile Arg Gly Ile Phe Lys Ser Ile Ala Lys Asn Met
   140 145 150
   gat atc tcc ctg gaa atc cca acc gca acc tcg gtt cgc gat atg cca 952
   Asp Ile Ser Leu Glu Ile Pro Thr Ala Thr Ser Val Arg Asp Met Pro
   155 160 165 170
   gct cgc ctc atg ttc gaa aac cgc gcg atg gtc aac gat cag ctc aag 1000
   Ala Arg Leu Met Phe Glu Asn Arg Ala Met Val Asn Asp Gln Leu Lys
   175 180 185
   cgc acc cgc ggt ggc aag atc tcc ttc acc cac atc att ggc tac gcc 1048
   Arg Thr Arg Gly Gly Lys Ile Ser Phe Thr His Ile Ile Gly Tyr Ala
   190 195 200
   atg gtg aag gca gtc atg gct cac ccg gac atg aac aac tcc tac cat 1096
   Met Val Lys Ala Val Met Ala His Pro Asp Met Asn Asn Ser Tyr His
   205 210 215
   cga cgg cag gcc cca acc ctg atc gtg cct gag cac atc aac ctg ggc 1144
   Arg Arg Gln Ala Pro Thr Leu Ile Val Pro Glu His Ile Asn Leu Gly
   220 225 230
   ctt gcc atc gac ctt cct cag aag gac ggc tcc cgc gca ctt gtc gta 1192
   Leu Ala Ile Asp Leu Pro Gln Lys Asp Gly Ser Arg Ala Leu Val Val
   235 240 245 250
   gca gcc atc aag gaa acc gag aag atg aac ttc tcc gag ttc ctc gca 1240
   Ala Ala Ile Lys Glu Thr Glu Lys Met Asn Phe Ser Glu Phe Leu Ala
   255 260 265
   gca tac gaa gac atc gtg aca cgc tcc cgc aag ggc aag ctc acc atg 1288
   Ala Tyr Glu Asp Ile Val Thr Arg Ser Arg Lys Gly Lys Leu Thr Met
   270 275 280
   gat gac tac cag ggc gtt acc gtt tcc ttg acc aac cca ggt ggc atc 1336
   Asp Asp Tyr Gln Gly Val Thr Val Ser Leu Thr Asn Pro Gly Gly Ile
   285 290 295
   ggt acc cgc cac tct gtc cca cgt ctg acc aag ggc cag ggc acc atc 1384
   Gly Thr Arg His Ser Val Pro Arg Leu Thr Lys Gly Gln Gly Thr Ile
   300 305 310
   atc ggt gtc ggt tcc atg gat tac cca gca gag ttc cag ggc gct tcc 1432
   Ile Gly Val Gly Ser Met Asp Tyr Pro Ala Glu Phe Gln Gly Ala Ser
   315 320 325 330
   gaa gac cgc ctt gca gag ctc ggc gtt gga aag ctt gtc acc atc acc 1480
   Glu Asp Arg Leu Ala Glu Leu Gly Val Gly Lys Leu Val Thr Ile Thr
   335 340 345
   tcc acc tac gat cac cgc gtg atc cag ggt gct gtg tcc ggt gaa ttc 1528
   Ser Thr Tyr Asp His Arg Val Ile Gln Gly Ala Val Ser Gly Glu Phe
   350 355 360
   ctg cgt acc atg tct cgc ctg ctc acc gat gat tcc ttc tgg gat gag 1576
   Leu Arg Thr Met Ser Arg Leu Leu Thr Asp Asp Ser Phe Trp Asp Glu
   365 370 375
   atc ttc gac gca atg aac gtt cct tac acc cca atg cgt tgg gca cag 1624
   Ile Phe Asp Ala Met Asn Val Pro Tyr Thr Pro Met Arg Trp Ala Gln
   380 385 390
   gac gtt cca aac acc ggt gtt gat aag aac acc cgc gtc atg cag ctc 1672
   Asp Val Pro Asn Thr Gly Val Asp Lys Asn Thr Arg Val Met Gln Leu
   395 400 405 410
   att gag gca tac cgc tcc cgt gga cac ctc atc gct gac acc aac cca 1720
   Ile Glu Ala Tyr Arg Ser Arg Gly His Leu Ile Ala Asp Thr Asn Pro
   415 420 425
   ctt tca tgg gtt cag cct ggc atg cca gtt cca gac cac cgc gac ctc 1768
   Leu Ser Trp Val Gln Pro Gly Met Pro Val Pro Asp His Arg Asp Leu
   430 435 440
   gac atc gag acc cac agc ctg acc atc tgg gat ctg gac cgt acc ttc 1816
   Asp Ile Glu Thr His Ser Leu Thr Ile Trp Asp Leu Asp Arg Thr Phe
   445 450 455
   agc gtc ggt ggc ttc ggc ggc aag gag acc atg acc ctg cgc gag gta 1864
   Ser Val Gly Gly Phe Gly Gly Lys Glu Thr Met Thr Leu Arg Glu Val
   460 465 470
   ctg tcc cgc ctg cgc gct gcc tac acc ttg aag gtc ggc tcc gaa tac 1912
   Leu Ser Arg Leu Arg Ala Ala Tyr Thr Leu Lys Val Gly Ser Glu Tyr
   475 480 485 490
   acc cac atc ctg gac cgc gac gag cgc acc tgg ctg cag gac cgc ctc 1960
   Thr His Ile Leu Asp Arg Asp Glu Arg Thr Trp Leu Gln Asp Arg Leu
   495 500 505
   gaa gcc gga atg cca aag cca acc cag gca gag cag aag tac atc ctg 2008
   Glu Ala Gly Met Pro Lys Pro Thr Gln Ala Glu Gln Lys Tyr Ile Leu
   510 515 520
   cag aag ctg aac gcc gca gag gct ttc gag aac ttc ctg cag acc aag 2056
   Gln Lys Leu Asn Ala Ala Glu Ala Phe Glu Asn Phe Leu Gln Thr Lys
   525 530 535
   tac gtc ggc cag aag cgc ttc tcc ctc gaa ggt gca gaa gct ctc atc 2104
   Tyr Val Gly Gln Lys Arg Phe Ser Leu Glu Gly Ala Glu Ala Leu Ile
   540 545 550
   cca ctg atg gac tcc gcc atc gac acc gcc gca ggc cag ggc ctc gac 2152
   Pro Leu Met Asp Ser Ala Ile Asp Thr Ala Ala Gly Gln Gly Leu Asp
   555 560 565 570
   gaa gtt gtc atc ggt atg cca cac cgt ggt cgc ctc aac gtg ctg ttc 2200
   Glu Val Val Ile Gly Met Pro His Arg Gly Arg Leu Asn Val Leu Phe
   575 580 585
   aac atc gtg ggc aag cca ctg gca tcc atc ttc aac gag ttt gaa ggc 2248
   Asn Ile Val Gly Lys Pro Leu Ala Ser Ile Phe Asn Glu Phe Glu Gly
   590 595 600
   caa atg gag cag ggc cag atc ggt ggc tcc ggt gac gtg aag tac cac 2296
   Gln Met Glu Gln Gly Gln Ile Gly Gly Ser Gly Asp Val Lys Tyr His
   605 610 615
   ctc ggt tcc gaa ggc cag cac ctg cag atg ttc ggc gac ggc gag atc 2344
   Leu Gly Ser Glu Gly Gln His Leu Gln Met Phe Gly Asp Gly Glu Ile
   620 625 630
   aag gtc tcc ctg act gct aac ccg tcc cac ctg gaa gct gtt aac cca 2392
   Lys Val Ser Leu Thr Ala Asn Pro Ser His Leu Glu Ala Val Asn Pro
   635 640 645 650
   gtg atg gaa ggt atc gtc cgc gca aag cag gac tac ctg gac aag ggc 2440
   Val Met Glu Gly Ile Val Arg Ala Lys Gln Asp Tyr Leu Asp Lys Gly
   655 660 665
   gta gac ggc aag act gtt gtg cca ctg ctg ctc cac ggt gac gct gca 2488
   Val Asp Gly Lys Thr Val Val Pro Leu Leu Leu His Gly Asp Ala Ala
   670 675 680
   ttc gca ggc ctg ggc atc gtg cca gaa acc atc aac ctg gct aag cgc 2536
   Phe Ala Gly Leu Gly Ile Val Pro Glu Thr Ile Asn Leu Ala Lys Arg
   685 690 695
   gtc gac gtc gga ggc acc atc cac atc gtg gtg aac aac cag atc ggc 2584
   Val Asp Val Gly Gly Thr Ile His Ile Val Val Asn Asn Gln Ile Gly
   700 705 710
   ttc acc acc acc cca gac tcc agc cgc tcc atg cac tac gca acc gac 2632
   Phe Thr Thr Thr Pro Asp Ser Ser Arg Ser Met His Tyr Ala Thr Asp
   715 720 725 730
   tac gcc aag gca ttc ggc tgc cca gtc ttc cac gtc aat ggt gat gac 2680
   Tyr Ala Lys Ala Phe Gly Cys Pro Val Phe His Val Asn Gly Asp Asp
   735 740 745
   cca gag gca gtt gtc tgg gtt ggc cag ctg gca acc gag tac cgt cgt 2728
   Pro Glu Ala Val Val Trp Val Gly Gln Leu Ala Thr Glu Tyr Arg Arg
   750 755 760
   cgc ttc ggc aag gac gtc ttc atc gac ctc gtt tgc tac cgc ctc cgc 2776
   Arg Phe Gly Lys Asp Val Phe Ile Asp Leu Val Cys Tyr Arg Leu Arg
   765 770 775
   ggc cac aac gaa gct gat gat cct tcc atg acc cag cca aag atg tat 2824
   Gly His Asn Glu Ala Asp Asp Pro Ser Met Thr Gln Pro Lys Met Tyr
   780 785 790
   gag ctc atc acc ggc cgc gag acc gtt cgt gct cag tac acc gaa gac 2872
   Glu Leu Ile Thr Gly Arg Glu Thr Val Arg Ala Gln Tyr Thr Glu Asp
   795 800 805 810
   ctg ctc gga cgt gga gac ctc tcc aac gaa gat gca gaa gca gtc gtc 2920
   Leu Leu Gly Arg Gly Asp Leu Ser Asn Glu Asp Ala Glu Ala Val Val
   815 820 825
   cgc gac ttc cac gac cag atg gaa tct gtg ttc aac gaa gtc aag gaa 2968
   Arg Asp Phe His Asp Gln Met Glu Ser Val Phe Asn Glu Val Lys Glu
   830 835 840
   ggc ggc aag aag cag gct gag gca cag acc ggc atc acc ggc tcc cag 3016
   Gly Gly Lys Lys Gln Ala Glu Ala Gln Thr Gly Ile Thr Gly Ser Gln
   845 850 855
   aag ctt cca cac ggc ctt gag acc aac atc tcc cgt gaa gag ctc ctg 3064
   Lys Leu Pro His Gly Leu Glu Thr Asn Ile Ser Arg Glu Glu Leu Leu
   860 865 870
   gaa ctg gga cag gct ttc gcc aac acc cca gaa ggc ttc aac tac cac 3112
   Glu Leu Gly Gln Ala Phe Ala Asn Thr Pro Glu Gly Phe Asn Tyr His
   875 880 885 890
   cca cgt gtg gct cca gtt gct aag aag cgc gtc tcc tct gtc acc gaa 3160
   Pro Arg Val Ala Pro Val Ala Lys Lys Arg Val Ser Ser Val Thr Glu
   895 900 905
   ggt ggc atc gac tgg gca tgg ggc gag ctc ctc gcc ttc ggt tcc ctg 3208
   Gly Gly Ile Asp Trp Ala Trp Gly Glu Leu Leu Ala Phe Gly Ser Leu
   910 915 920
   gct aac tcc ggc cgc ttg gtt cgc ctt gca ggt gaa gat tcc cgc cgc 3256
   Ala Asn Ser Gly Arg Leu Val Arg Leu Ala Gly Glu Asp Ser Arg Arg
   925 930 935
   ggt acc ttc acc cag cgc cac gca gtt gcc atc gac cca gcg acc gct 3304
   Gly Thr Phe Thr Gln Arg His Ala Val Ala Ile Asp Pro Ala Thr Ala
   940 945 950
   gaa gag ttc aac cca ctc cac gag ctt gca cag tcc aag ggc aac aac 3352
   Glu Glu Phe Asn Pro Leu His Glu Leu Ala Gln Ser Lys Gly Asn Asn
   955 960 965 970
   ggt aag ttc ctg gtc tac aac tcc gca ctg acc gag tac gca ggc atg 3400
   Gly Lys Phe Leu Val Tyr Asn Ser Ala Leu Thr Glu Tyr Ala Gly Met
   975 980 985
   ggc ttc gag tac ggc tac tcc gta gga aac gaa gac tcc gtc gtt gca 3448
   Gly Phe Glu Tyr Gly Tyr Ser Val Gly Asn Glu Asp Ser Val Val Ala
   990 995 1000
   tgg gaa gca cag ttc ggc gac ttc gcc aac ggc gct cag acc atc 3493
   Trp Glu Ala Gln Phe Gly Asp Phe Ala Asn Gly Ala Gln Thr Ile
   1005 1010 1015
   atc gat gag tac gtc tcc tca ggc gaa gct aag tgg ggc cag acc 3538
   Ile Asp Glu Tyr Val Ser Ser Gly Glu Ala Lys Trp Gly Gln Thr
   1020 1025 1030
   tcc aag ctg atc ctt ctg ctg cct cac ggc tac gaa ggc cag ggc 3583
   Ser Lys Leu Ile Leu Leu Leu Pro His Gly Tyr Glu Gly Gln Gly
   1035 1040 1045
   cca gac cac tct tcc gca cgt atc gag cgc ttc ctg cag ctg tgc 3628
   Pro Asp His Ser Ser Ala Arg Ile Glu Arg Phe Leu Gln Leu Cys
   1050 1055 1060
   gct gag ggt tcc atg act gtt gct cag cca tcc acc cca gca aac 3673
   Ala Glu Gly Ser Met Thr Val Ala Gln Pro Ser Thr Pro Ala Asn
   1065 1070 1075
   cac ttc cac ctg ctg cgt cgt cac gct ctg tcc gac ctg aag cgt 3718
   His Phe His Leu Leu Arg Arg His Ala Leu Ser Asp Leu Lys Arg
   1080 1085 1090
   cca ctg gtt atc ttc acc ccg aag tcc atg ctg cgt aac aag gct 3763
   Pro Leu Val  Ile Phe Thr Pro Lys  Ser Met Leu Arg Asn  Lys Ala
           1095                1100                1105
   gct gcc tcc  gca cca gaa gac ttc  act gag gtc acc aag  ttc caa      3808
   Ala Ala Ser  Ala Pro Glu Asp Phe  Thr Glu Val Thr Lys  Phe Gln
           1110                1115                1120
   tcc gtg atc  gac gat cca aac gtt  gca gat gca gcc aag  gtg aag      3853
   Ser Val Ile  Asp Asp Pro Asn Val  Ala Asp Ala Ala Lys  Val Lys
           1125                1130                1135
   aag gtc atg  ctg gtc tcc ggc aag  ctg tac tac gaa ttg  gca aag      3898
   Lys Val Met  Leu Val Ser Gly Lys  Leu Tyr Tyr Glu Leu  Ala Lys
           1140                1145                1150
   cgc aag gag  aag gac gga cgc gac  gac atc gcg atc gtt  cgt atc      3943
   Arg Lys Glu  Lys Asp Gly Arg Asp  Asp Ile Ala Ile Val  Arg Ile
           1155                1160                1165
   gaa atg ctc  cac cca att ccg ttc  aac cgc atc tcc gag  gct ctt      3988
   Glu Met Leu  His Pro Ile Pro Phe  Asn Arg Ile Ser Glu  Ala Leu
           1170                1175                1180
   gcc ggc tac  cct aac gct gag gaa  gtc ctc ttc gtt cag  gat gag      4033
   Ala Gly Tyr  Pro Asn Ala Glu Glu  Val Leu Phe Val Gln  Asp Glu
           1185                1190                1195
   cca gca aac  cag ggc cca tgg ccg  ttc tac cag gag cac  ctc cca      4078
   Pro Ala Asn  Gln Gly Pro Trp Pro  Phe Tyr Gln Glu His  Leu Pro
           1200                1205                1210
   gag ctg atc  ccg aac atg cca aag  atg cgc cgc gtt tcc  cgc cgc      4123
   Glu Leu Ile  Pro Asn Met Pro Lys  Met Arg Arg Val Ser  Arg Arg
           1215                1220                1225
   gct cag tcc  tcc acc gca act ggt  gtt gct aag gtg cac  cag ctg      4168
   Ala Gln Ser  Ser Thr Ala Thr Gly  Val Ala Lys Val His  Gln Leu
           1230                1235                1240
   gag gag aag  cag ctt atc gac gag  gct ttc gag gct taa gtc  ttt      4213
   Glu Glu Lys  Gln Leu Ile Asp Glu  Ala Phe Glu Ala     Val  Phe
           1245                1250                    1255
   atagtcctgc actagcctag agggccttat gcagtgtgaa tcacacagca taaggccctt   4273
   tttgctgccg tggttgccta aggtggaagg catgaaacga atctgtgcgg tcacgatctc   4333
   ttcagtactt ttgctaagtg gctgctcctc cacttccacc acgcagctcg ag           4385
<210> 16
   <211> 1254
   <212> PRT
   <213> Corynebacterium glutamicum
<400> 16
   Met Leu Gln Leu Gly Leu Arg His Asn Gln Pro Thr Thr Asn Val Thr
   1               5                   10                  15
   Val Asp Lys Ile Lys Leu Asn Lys Pro Ser Arg Ser Lys Glu Lys Arg
               20                  25                  30
   Arg Val Pro Ala Val Ser Ser Ala Ser Thr Phe Gly Gln Asn Ala Trp
   35 40 45
   Leu Val Asp Glu Met Phe Gln Gln Phe Gln Lys Asp Pro Lys Ser Val
   50 55 60
   Asp Lys Glu Trp Arg Glu Leu Phe Glu Ala Gln Gly Gly Pro Asn Ala
   65 70 75 80
   Thr Pro Ala Thr Thr Glu Ala Gln Pro Ser Ala Pro Lys Glu Ser Ala
   85 90 95
   Lys Pro Ala Pro Lys Ala Ala Pro Ala Ala Lys Ala Ala Pro Arg Val
   100 105 110
   Glu Thr Lys Pro Ala Ala Lys Thr Ala Pro Lys Ala Lys Glu Ser Ser
   115 120 125
   Val Pro Gln Gln Pro Lys Leu Pro Glu Pro Gly Gln Thr Pro Ile Arg
   130 135 140
   Gly Ile Phe Lys Ser Ile Ala Lys Asn Met Asp Ile Ser Leu Glu Ile
   145 150 155 160
   Pro Thr Ala Thr Ser Val Arg Asp Met Pro Ala Arg Leu Met Phe Glu
   165 170 175
   Asn Arg Ala Met Val Asn Asp Gln Leu Lys Arg Thr Arg Gly Gly Lys
   180 185 190
   Ile Ser Phe Thr His Ile Ile Gly Tyr Ala Met Val Lys Ala Val Met
   195 200 205
   Ala His Pro Asp Met Asn Asn Ser Tyr His Arg Arg Gln Ala Pro Thr
   210 215 220
   Leu Ile Val Pro Glu His Ile Asn Leu Gly Leu Ala Ile Asp Leu Pro
   225 230 235 240
   Gln Lys Asp Gly Ser Arg Ala Leu Val Val Ala Ala Ile Lys Glu Thr
   245 250 255
   Glu Lys Met Asn Phe Ser Glu Phe Leu Ala Ala Tyr Glu Asp Ile Val
   260 265 270
   Thr Arg Ser Arg Lys Gly Lys Leu Thr Met Asp Asp Tyr Gln Gly Val
   275 280 285
   Thr Val Ser Leu Thr Asn Pro Gly Gly Ile Gly Thr Arg His Ser Val
   290 295 300
   Pro Arg Leu Thr Lys Gly Gln Gly Thr Ile Ile Gly Val Gly Ser Met
   305 310 315 320
   Asp Tyr Pro Ala Glu Phe Gln Gly Ala Ser Glu Asp Arg Leu Ala Glu
   325 330 335
   Leu Gly Val Gly Lys Leu Val Thr Ile Thr Ser Thr Tyr Asp His Arg
   340 345 350
   Val Ile Gln Gly Ala Val Ser Gly Glu Phe Leu Arg Thr Met Ser Arg
   355 360 365
   Leu Leu Thr Asp Asp Ser Phe Trp Asp Glu Ile Phe Asp Ala Met Asn
   370 375 380
   Val Pro Tyr Thr Pro Met Arg Trp Ala Gln Asp Val Pro Asn Thr Gly
   385 390 395 400
   Val Asp Lys Asn Thr Arg Val Met Gln Leu Ile Glu Ala Tyr Arg Ser
   405 410 415
   Arg Gly His Leu Ile Ala Asp Thr Asn Pro Leu Ser Trp Val Gln Pro
   420 425 430
   Gly Met Pro Val Pro Asp His Arg Asp Leu Asp Ile Glu Thr His Ser
   435 440 445
   Leu Thr Ile Trp Asp Leu Asp Arg Thr Phe Ser Val Gly Gly Phe Gly
   450 455 460
   Gly Lys Glu Thr Met Thr Leu Arg Glu Val Leu Ser Arg Leu Arg Ala
   465 470 475 480
   Ala Tyr Thr Leu Lys Val Gly Ser Glu Tyr Thr His Ile Leu Asp Arg
   485 490 495
   Asp Glu Arg Thr Trp Leu Gln Asp Arg Leu Glu Ala Gly Met Pro Lys
   500 505 510
   Pro Thr Gln Ala Glu Gln Lys Tyr Ile Leu Gln Lys Leu Asn Ala Ala
   515 520 525
   Glu Ala Phe Glu Asn Phe Leu Gln Thr Lys Tyr Val Gly Gln Lys Arg
   530 535 540
   Phe Ser Leu Glu Gly Ala Glu Ala Leu Ile Pro Leu Met Asp Ser Ala
   545 550 555 560
   Ile Asp Thr Ala Ala Gly Gln Gly Leu Asp Glu Val Val Ile Gly Met
   565 570 575
   Pro His Arg Gly Arg Leu Asn Val Leu Phe Asn Ile Val Gly Lys Pro
   580 585 590
   Leu Ala Ser Ile Phe Asn Glu Phe Glu Gly Gln Met Glu Gln Gly Gln
   595 600 605
   Ile Gly Gly Ser Gly Asp Val Lys Tyr His Leu Gly Ser Glu Gly Gln
   610 615 620
   His Leu Gln Met Phe Gly Asp Gly Glu Ile Lys Val Ser Leu Thr Ala
   625 630 635 640
   Asn Pro Ser His Leu Glu Ala Val Asn Pro Val Met Glu Gly Ile Val
   645 650 655
   Arg Ala Lys Gln Asp Tyr Leu Asp Lys Gly Val Asp Gly Lys Thr Val
   660 665 670
   Val Pro Leu Leu Leu His Gly Asp Ala Ala Phe Ala Gly Leu Gly Ile
   675 680 685
   Val Pro Glu Thr Ile Asn Leu Ala Lys Arg Val Asp Val Gly Gly Thr
   690 695 700
   Ile His Ile Val Val Asn Asn Gln Ile Gly Phe Thr Thr Thr Pro Asp
   705 710 715 720
   Ser Ser Arg Ser Met His Tyr Ala Thr Asp Tyr Ala Lys Ala Phe Gly
   725 730 735
   Cys Pro Val Phe His Val Asn Gly Asp Asp Pro Glu Ala Val Val Trp
   740 745 750
   Val Gly Gln Leu Ala Thr Glu Tyr Arg Arg Arg Phe Gly Lys Asp Val
   755 760 765
   Phe Ile Asp Leu Val Cys Tyr Arg Leu Arg Gly His Asn Glu Ala Asp
   770 775 780
   Asp Pro Ser Met Thr Gln Pro Lys Met Tyr Glu Leu Ile Thr Gly Arg
   785 790 795 800
   Glu Thr Val Arg Ala Gln Tyr Thr Glu Asp Leu Leu Gly Arg Gly Asp
   805 810 815
   Leu Ser Asn Glu Asp Ala Glu Ala Val Val Arg Asp Phe His Asp Gln
   820 825 830
   Met Glu Ser Val Phe Asn Glu Val Lys Glu Gly Gly Lys Lys Gln Ala
   835 840 845
   Glu Ala Gln Thr Gly Ile Thr Gly Ser Gln Lys Leu Pro His Gly Leu
   850 855 860
   Glu Thr Asn Ile Ser Arg Glu Glu Leu Leu Glu Leu Gly Gln Ala Phe
   865 870 875 880
   Ala Asn Thr Pro Glu Gly Phe Asn Tyr His Pro Arg Val Ala Pro Val
   885 890 895
   Ala Lys Lys Arg Val Ser Ser Val Thr Glu Gly Gly Ile Asp Trp Ala
   900 905 910
   Trp Gly Glu Leu Leu Ala Phe Gly Ser Leu Ala Asn Ser Gly Arg Leu
   915 920 925
   Val Arg Leu Ala Gly Glu Asp Ser Arg Arg Gly Thr Phe Thr Gln Arg
   930 935 940
   His Ala Val Ala Ile Asp Pro Ala Thr Ala Glu Glu Phe Asn Pro Leu
   945 950 955 960
   His Glu Leu Ala Gln Ser Lys Gly Asn Asn Gly Lys Phe Leu Val Tyr
   965 970 975
   Asn Ser Ala Leu Thr Glu Tyr Ala Gly Met Gly Phe Glu Tyr Gly Tyr
   980 985 990
   Ser Val Gly Asn Glu Asp Ser Val Val Ala Trp Glu Ala Gln Phe Gly
   995 1000 1005
   Asp Phe Ala Asn Gly Ala Gln Thr Ile Ile Asp Glu Tyr Val Ser
   1010 1015 1020
   Ser Gly Glu Ala Lys Trp Gly Gln Thr Ser Lys Leu Ile Leu Leu
   1025 1030 1035
   Leu Pro His Gly Tyr Glu Gly Gln Gly Pro Asp His Ser Ser Ala
   1040 1045 1050
   Arg Ile Glu Arg Phe Leu Gln Leu Cys Ala Glu Gly Ser Met Thr
   1055 1060 1065
   Val Ala Gln Pro Ser Thr Pro Ala Asn His Phe His Leu Leu Arg
   1070 1075 1080
   Arg His Ala Leu Ser Asp Leu Lys Arg Pro Leu Val Ile Phe Thr
   1085 1090 1095
   Pro Lys Ser Met Leu Arg Asn Lys Ala Ala Ala Ser Ala Pro Glu
   1100 1105 1110
   Asp Phe Thr Glu Val Thr Lys Phe Gln Ser Val Ile Asp Asp Pro
   1115 1120 1125
   Asn Val Ala Asp Ala Ala Lys Val Lys Lys Val Met Leu Val Ser
   1130 1135 1140
   Gly Lys Leu Tyr Tyr Glu Leu Ala Lys Arg Lys Glu Lys Asp Gly
   1145 1150 1155
   Arg Asp Asp Ile Ala Ile Val Arg Ile Glu Met Leu His Pro Ile
   1160 1165 1170
   Pro Phe Asn Arg Ile Ser Glu Ala Leu Ala Gly Tyr Pro Asn Ala
   1175 1180 1185
   Glu Glu Val Leu Phe Val Gln Asp Glu Pro Ala Asn Gln Gly Pro
   1190 1195 1200
   Trp Pro Phe Tyr Gln Glu His Leu Pro Glu Leu Ile Pro Asn Met
   1205 1210 1215
   Pro Lys Met Arg Arg Val Ser Arg Arg Ala Gln Ser Ser Thr Ala
   1220 1225 1230
   Thr Gly Val Ala Lys Val His Gln Leu Glu Glu Lys Gln Leu Ile
   1235 1240 1245
   Asp Glu Ala Phe Glu Ala
   1250
<210> 17
   <211> 1800
   <212> DNA
   <213> Corynebacterium glutamicum
<220>
   <221> CDS
   <222> (211)..(1620)
   <223>
<400> 17
   gatcctccgc attcagtaat ctttcccaca gcatcggctt gttctaaaac agcacggtgc 60
   ttagaccgta cataaaacac ctgcatttct gtgcccattt gggcccggat gtgggtgttt 120
   ttcattttct tccactctaa aattaagtat ggaaaaccaa ccgcacccgg atgcacgaca 180
   atgacccact aaacacgtat ccttgaatgc gtg act gaa cat tat gac gta gta 234
   Met Thr Glu His Tyr Asp Val Val
   1 5
   gta ctc gga gcc ggc ccc ggt ggc tat gtc tcc gcc atc cgt gca gcg 282
   Val Leu Gly Ala Gly Pro Gly Gly Tyr Val Ser Ala Ile Arg Ala Ala
   10 15 20
   cag ctt ggc aag aag gtt gct gta att gag aag cag tac tgg ggt ggt 330
   Gln Leu Gly Lys Lys Val Ala Val Ile Glu Lys Gln Tyr Trp Gly Gly
   25 30 35 40
   gtt tgc cta aac gtg ggc tgc att cct tcc aag tct ctg atc aaa aac 378
   Val Cys Leu Asn Val Gly Cys Ile Pro Ser Lys Ser Leu Ile Lys Asn
   45 50 55
   gct gaa gtt gcc cat acc ttt acc cat gag aag aag acc ttc ggc atc 426
   Ala Glu Val Ala His Thr Phe Thr His Glu Lys Lys Thr Phe Gly Ile
   60 65 70
   aat ggc gaa gtg acc ttc aac tat gag gat gct cac aag cgt tcc cgt 474
   Asn Gly Glu Val Thr Phe Asn Tyr Glu Asp Ala His Lys Arg Ser Arg
   75 80 85
   ggc gtt tcc gac aag atc gtt gga ggc gtt cat tac ttg atg aag aag 522
   Gly Val Ser Asp Lys Ile Val Gly Gly Val His Tyr Leu Met Lys Lys
   90 95 100
   aac aag atc atc gaa att cat ggt ctt gga aac ttc aag gat gct aag 570
   Asn Lys Ile Ile Glu Ile His Gly Leu Gly Asn Phe Lys Asp Ala Lys
   105 110 115 120
   act ctt gag gtc acc gac ggt aag gat gct ggc aag acc atc acc ttt 618
   Thr Leu Glu Val Thr Asp Gly Lys Asp Ala Gly Lys Thr Ile Thr Phe
   125 130 135
   gat gac tgc atc atc gca acc ggt tcg gta gtc aac acc ctc cgt ggc 666
   Asp Asp Cys Ile Ile Ala Thr Gly Ser Val Val Asn Thr Leu Arg Gly
   140 145 150
   gtt gac ttc tca gag aac gtt gtg tct ttt gaa gag cag att ctt aac 714
   Val Asp Phe Ser Glu Asn Val Val Ser Phe Glu Glu Gln Ile Leu Asn
   155 160 165
   cct gtt gcg cca aag aag atg gtc att gtt ggt gca ggc gca att gga 762
   Pro Val Ala Pro Lys Lys Met Val Ile Val Gly Ala Gly Ala Ile Gly
   170 175 180
   atg gaa ttc gcc tac gtt ctt ggt aac tac ggt gta gat gta acc gtc 810
   Met Glu Phe Ala Tyr Val Leu Gly Asn Tyr Gly Val Asp Val Thr Val
   185 190 195 200
   atc gag ttc atg gat cgt gtg ctt cca aat gaa gat gct gaa gtc tcc 858
   Ile Glu Phe Met Asp Arg Val Leu Pro Asn Glu Asp Ala Glu Val Ser
   205 210 215
   aag gtt att gca aag gcc tac aag aag atg ggc gtt aag ctt ctt cct 906
   Lys Val Ile Ala Lys Ala Tyr Lys Lys Met Gly Val Lys Leu Leu Pro
   220 225 230
   ggc cat gca acc act gct gtt cgg gac aac ggt gac ttt gtc gag gtt 954
   Gly His Ala Thr Thr Ala Val Arg Asp Asn Gly Asp Phe Val Glu Val
   235 240 245
   gat tac cag aag aag ggc tct gac aag aca gag act ctt act gtt gat 1002
   Asp Tyr Gln Lys Lys Gly Ser Asp Lys Thr Glu Thr Leu Thr Val Asp
   250 255 260
   cga gtc atg gtt tcc gtt ggt ttc cgt cca cgc gtt gag gga ttt ggt 1050
   Arg Val Met Val Ser Val Gly Phe Arg Pro Arg Val Glu Gly Phe Gly
   265 270 275 280
   ctt gaa aac act ggc gtt aag ctc acc gag cgt ggc gca atc gag atc 1098
   Leu Glu Asn Thr Gly Val Lys Leu Thr Glu Arg Gly Ala Ile Glu Ile
   285 290 295
   gat gat tac atg cgt acc aac gtc gat ggc att tac gcc atc ggt gac 1146
   Asp Asp Tyr Met Arg Thr Asn Val Asp Gly Ile Tyr Ala Ile Gly Asp
   300 305 310
   gtg acc gcc aag ctt cag ctt gct cac gtc gca gaa gca cag ggc att 1194
   Val Thr Ala Lys Leu Gln Leu Ala His Val Ala Glu Ala Gln Gly Ile
   315 320 325
   gtt gcc gca gag act att gct ggt gca gaa act cag act ctt ggt gat 1242
   Val Ala Ala Glu Thr Ile Ala Gly Ala Glu Thr Gln Thr Leu Gly Asp
   330 335 340
   tac atg atg atg cca cgt gca acc ttc tgc aac cca cag gtt tct tcc 1290
   Tyr Met Met Met Pro Arg Ala Thr Phe Cys Asn Pro Gln Val Ser Ser
   345 350 355 360
   ttt ggt tac acc gaa gag cag gcc aag gag aag tgg cca gat cgt gag 1338
   Phe Gly Tyr Thr Glu Glu Gln Ala Lys Glu Lys Trp Pro Asp Arg Glu
                   365                 370                 375
   atc aag gtt gct tcc ctc cca ttc tct gca aac ggt aaa gca gtt ggc     1386
   Ile Lys Val Ala Ser Leu Pro Phe Ser Ala Asn Gly Lys Ala Val Gly
               380                 385                 390
   ctg gca gaa act gat ggt ttc gca aag atc gtt gct gat gca gaa ttc     1434
   Leu Ala Glu Thr Asp Gly Phe Ala Lys Ile Val Ala Asp Ala Glu Phe
           395                 400                 405
   ggt gag ctg ctc ggt gca cac ctg gtt gga gca aat gca tca gag ctc     1482
   Gly Glu Leu Leu Gly Ala His Leu Val Gly Ala Asn Ala Ser Glu Leu
       410                 415                 420
   atc aat gaa ttg gtg ctt gct cag aac tgg gat ctc acc act gaa gag     1530
   Ile Asn Glu Leu Val Leu Ala Gln Asn Trp Asp Leu Thr Thr Glu Glu
   425                 430                 435                 440
   atc tct cgt agc gtc cat att cac cca acg cta tct gag gca gtt aag     1578
   Ile Ser Arg Ser Val His Ile His Pro Thr Leu Ser Glu Ala Val Lys
                   445                 450                 455
   gaa gct gca cac ggt atc tct gga cac atg atc aac ttc tag             1620
   Glu Ala Ala His Gly Ile Ser Gly His Met Ile Asn Phe
               460                 465
   aatccacctc gttggccctg tttctgtatg gaaacagggc caaaaccgat tttcaatcca   1680
   aaccgagtgc tggttagtgc tcatactcac acaaactttt cgattatcaa agagaattat   1740
   ttctaaaatt cggtatcgtc taagaaatga gtttgccaat agctcagcat caaaatgctg   1800
<210> 18
   <211> 469
   <212> PRT
   <213> Corynebacterium glutamicum
<400> 18
   Met Thr Glu His Tyr Asp Val Val Val Leu Gly Ala Gly Pro Gly Gly
   1 5 10 15
   Tyr Val Ser Ala Ile Arg Ala Ala Gln Leu Gly Lys Lys Val Ala Val
   20 25 30
   Ile Glu Lys Gln Tyr Trp Gly Gly Val Cys Leu Asn Val Gly Cys Ile
   35 40 45
   Pro Ser Lys Ser Leu Ile Lys Asn Ala Glu Val Ala His Thr Phe Thr
   50 55 60
   His Glu Lys Lys Thr Phe Gly Ile Asn Gly Glu Val Thr Phe Asn Tyr
   65 70 75 80
   Glu Asp Ala His Lys Arg Ser Arg Gly Val Ser Asp Lys Ile Val Gly
   85 90 95
   Gly Val His Tyr Leu Met Lys Lys Asn Lys Ile Ile Glu Ile His Gly
   100 105 110
   Leu Gly Asn Phe Lys Asp Ala Lys Thr Leu Glu Val Thr Asp Gly Lys
   115 120 125
   Asp Ala Gly Lys Thr Ile Thr Phe Asp Asp Cys Ile Ile Ala Thr Gly
   130 135 140
   Ser Val Val Asn Thr Leu Arg Gly Val Asp Phe Ser Glu Asn Val Val
   145 150 155 160
   Ser Phe Glu Glu Gln Ile Leu Asn Pro Val Ala Pro Lys Lys Met Val
   165 170 175
   Ile Val Gly Ala Gly Ala Ile Gly Met Glu Phe Ala Tyr Val Leu Gly
   180 185 190
   Asn Tyr Gly Val Asp Val Thr Val Ile Glu Phe Met Asp Arg Val Leu
   195 200 205
   Pro Asn Glu Asp Ala Glu Val Ser Lys Val Ile Ala Lys Ala Tyr Lys
   210 215 220
   Lys Met Gly Val Lys Leu Leu Pro Gly His Ala Thr Thr Ala Val Arg
   225 230 235 240
   Asp Asn Gly Asp Phe Val Glu Val Asp Tyr Gln Lys Lys Gly Ser Asp
   245 250 255
   Lys Thr Glu Thr Leu Thr Val Asp Arg Val Met Val Ser Val Gly Phe
   260 265 270
   Arg Pro Arg Val Glu Gly Phe Gly Leu Glu Asn Thr Gly Val Lys Leu
   275 280 285
   Thr Glu Arg Gly Ala Ile Glu Ile Asp Asp Tyr Met Arg Thr Asn Val
   290 295 300
   Asp Gly Ile Tyr Ala Ile Gly Asp Val Thr Ala Lys Leu Gln Leu Ala
   305 310 315 320
   His Val Ala Glu Ala Gln Gly Ile Val Ala Ala Glu Thr Ile Ala Gly
   325 330 335
   Ala Glu Thr Gln Thr Leu Gly Asp Tyr Met Met Met Pro Arg Ala Thr
   340 345 350
   Phe Cys Asn Pro Gln Val Ser Ser Phe Gly Tyr Thr Glu Glu Gln Ala
   355 360 365
   Lys Glu Lys Trp Pro Asp Arg Glu Ile Lys Val Ala Ser Leu Pro Phe
   370 375 380
   Ser Ala Asn Gly Lys Ala Val Gly Leu Ala Glu Thr Asp Gly Phe Ala
   385 390 395 400
   Lys Ile Val Ala Asp Ala Glu Phe Gly Glu Leu Leu Gly Ala His Leu
   405 410 415
   Val Gly Ala Asn Ala Ser Glu Leu Ile Asn Glu Leu Val Leu Ala Gln
   420 425 430
   Asn Trp Asp Leu Thr Thr Glu Glu Ile Ser Arg Ser Val His Ile His
   435 440 445
   Pro Thr Leu Ser Glu Ala Val Lys Glu Ala Ala His Gly Ile Ser Gly
   450 455 460
   His Met Ile Asn Phe
   465
<210> 19
   <211> 2014
   <212> DNA
   <213> Bacillus subtilis
<220>
   <221> CDS
   <222> (464).. (1885)
   <223>
<400> 19
   gatccttttt aacccatcac atatacctgc cgttcactat tatttagtga aatgagatat      60
   tatgatattt tctgaattgt gattaaaaag gcaactttat gcccatgcaa cagaaactat     120
   aaaaaataca gagaatgaaa agaaacagat agatttttta gttctttagg cccgtagtct     180
   gcaaatcctt ttatgatttt ctatcaaaca aaagaggaaa atagaccagt tgcaatccaa     240
   acgagagtct aatagaatga ggtcgaaaag taaatcgcgc gggtttgtta ctgataaagc     300
   aggcaagacc taaaatgtgt aaagggcaaa gtgtatactt tggcgtcacc ccttacatat     360
   tttaggtctt tttttattgt gcgtaactaa cttgccatct tcaaacagga gggctggaag     420
   aagcagaccg ctaacacagt acataaaaaa ggagacatga acg atg aac atc aaa       475
                                                   Met Asn Ile Lys
                                                   1
   aag ttt gca aaa caa gca aca gta tta acc ttt act acc gca ctg ctg       523
   Lys Phe Ala Lys Gln Ala Thr Val Leu Thr Phe Thr Thr Ala Leu Leu
   5                   10                  15                  20
   gca gga ggc gca act caa gcg ttt gcg aaa gaa acg aac caa aag cca       571
   Ala Gly Gly Ala Thr Gln Ala Phe Ala Lys Glu Thr Asn Gln Lys Pro
                   25                  30                  35
   tat aag gaa aca tac ggc att tcc cat att aca cgc cat gat atg ctg       619
   Tyr Lys Glu Thr Tyr Gly Ile Ser His Ile Thr Arg His Asp Met Leu
               40                  45                  50
   caa atc cct gaa cag caa aaa aat gaa aaa tat caa gtt cct gaa ttc       667
   Gln Ile Pro Glu Gln Gln Lys Asn Glu Lys Tyr Gln Val Pro Glu Phe
           55                  60                  65
   gat tcg tcc aca att aaa aat atc tct tct gca aaa ggc ctg gac gtt       715
   Asp Ser Ser Thr Ile Lys Asn Ile Ser Ser Ala Lys Gly Leu Asp Val
       70                  75                  80
   tgg gac agc tgg cca tta caa aac gct gac ggc act gtc gca aac tat       763
   Trp Asp Ser Trp Pro Leu Gln Asn Ala Asp Gly Thr Val Ala Asn Tyr
   85                  90                  95                  100
   cac ggc tac cac atc gtc ttt gca tta gcc gga gat cct aaa aat gcg       811
   His Gly Tyr His Ile Val Phe Ala Leu Ala Gly Asp Pro Lys Asn Ala
                   105                 110                 115
   gat gac aca tcg att tac atg ttc tat caa aaa gtc ggc gaa act tct       859
   Asp Asp Thr Ser Ile Tyr Met Phe Tyr Gln Lys Val Gly Glu Thr Ser
               120                 125                 130
   att gac agc tgg aaa aac gct ggc cgc gtc ttt aaa gac agc gac aaa       907
   Ile Asp Ser Trp Lys Asn Ala Gly Arg Val Phe Lys Asp Ser Asp Lys
           135                 140                 145
   ttc gat gca aat gat tct atc cta aaa gac caa aca caa gaa tgg tca       955
   Phe Asp Ala Asn Asp Ser Ile Leu Lys Asp Gln Thr Gln Glu Trp Ser
       150                 155                 160
   ggt tca gcc aca ttt aca tct gac gga aaa atc cgt tta ttc tac act      1003
   Gly Ser Ala Thr Phe Thr Ser Asp Gly Lys Ile Arg Leu Phe Tyr Thr
   165 170 175 180
   gat ttc tcc ggt aaa cat tac ggc aaa caa aca ctg aca act gca caa 1051
   Asp Phe Ser Gly Lys His Tyr Gly Lys Gln Thr Leu Thr Thr Ala Gln
   185 190 195
   gtt aac gta tca gca tca gac agc tct ttg aac atc aac ggt gta gag 1099
   Val Asn Val Ser Ala Ser Asp Ser Ser Leu Asn Ile Asn Gly Val Glu
   200 205 210
   gat tat aaa tca atc ttt gac ggt gac gga aaa acg tat caa aat gta 1147
   Asp Tyr Lys Ser Ile Phe Asp Gly Asp Gly Lys Thr Tyr Gln Asn Val
   215 220 225
   cag cag ttc atc gat gaa ggc aac tac agc tca ggc gac aac cat acg 1195
   Gln Gln Phe Ile Asp Glu Gly Asn Tyr Ser Ser Gly Asp Asn His Thr
   230 235 240
   ctg aga gat cct cac tac gta gaa gat aaa ggc cac aaa tac tta gta 1243
   Leu Arg Asp Pro His Tyr Val Glu Asp Lys Gly His Lys Tyr Leu Val
   245 250 255 260
   ttt gaa gca aac act gga act gaa gat ggc tac caa ggc gaa gaa tct 1291
   Phe Glu Ala Asn Thr Gly Thr Glu Asp Gly Tyr Gln Gly Glu Glu Ser
   265 270 275
   tta ttt aac aaa gca tac tat ggc aaa agc aca tca ttc ttc cgt caa 1339
   Leu Phe Asn Lys Ala Tyr Tyr Gly Lys Ser Thr Ser Phe Phe Arg Gln
   280 285 290
   gaa agt caa aaa ctt ctg caa agc gat aaa aaa cgc acg gct gag tta 1387
   Glu Ser Gln Lys Leu Leu Gln Ser Asp Lys Lys Arg Thr Ala Glu Leu
   295 300 305
   gca aac ggc gct ctc ggt atg att gag cta aac gat gat tac aca ctg 1435
   Ala Asn Gly Ala Leu Gly Met Ile Glu Leu Asn Asp Asp Tyr Thr Leu
   310 315 320
   aaa aaa gtg atg aaa ccg ctg att gca tct aac aca gta aca gat gaa 1483
   Lys Lys Val Met Lys Pro Leu Ile Ala Ser Asn Thr Val Thr Asp Glu
   325 330 335 340
   att gaa cgc gcg aac gtc ttt aaa atg aac ggc aaa tgg tac ctg ttc 1531
   Ile Glu Arg Ala Asn Val Phe Lys Met Asn Gly Lys Trp Tyr Leu Phe
   345 350 355
   act gac tcc cgc gga tca aaa atg acg att gac ggc att acg tct aac 1579
   Thr Asp Ser Arg Gly Ser Lys Met Thr Ile Asp Gly Ile Thr Ser Asn
   360 365 370
   gat att tac atg ctt ggt tat gtt tct aat tct tta act ggc cca tac 1627
   Asp Ile Tyr Met Leu Gly Tyr Val Ser Asn Ser Leu Thr Gly Pro Tyr
   375 380 385
   aag ccg ctg aac aaa act ggc ctt gtg tta aaa atg gat ctt gat cct 1675
   Lys Pro Leu Asn Lys Thr Gly Leu Val Leu Lys Met Asp Leu Asp Pro
   390 395 400
   aac gat gta acc ttt act tac tca cac ttc gct gta cct caa gcg aaa 1723
   Asn Asp Val Thr Phe Thr Tyr Ser His Phe Ala Val Pro Gln Ala Lys
   405 410 415 420
   gga aac aat gtc gtg att aca agc tat atg aca aac aga gga ttc tac 1771
   Gly Asn Asn Val Val Ile Thr Ser Tyr Met Thr Asn Arg Gly Phe Tyr
                   425                 430                 435
   gca gac aaa caa tca acg ttt gcg cca agc ttc ctg ctg aac atc aaa      1819
   Ala Asp Lys Gln Ser Thr Phe Ala Pro Ser Phe Leu Leu Asn Ile Lys
               440                 445                 450
   ggc aag aaa aca tct gtt gtc aaa gac agc atc ctt gaa caa gga caa      1867
   Gly Lys Lys Thr Ser Val Val Lys Asp Ser Ile Leu Glu Gln Gly Gln
           455                 460                 465
   tta aca gtt aac aaa taa aaacgcaaaa gaaaatgccg atatcctatt             1915
   Leu Thr Val Asn Lys
       470
   ggcattttct tttatttctt atcaacataa aggtgaatcc catatgaact atataaaagc    1975
   aggcaaatgg ctaaccgtat tcctaacctt ttgaagatc                           2014
<210> 20
   <211> 473
   <212> PRT
   <213> Bacillus subtilis
<400> 20
   Met Asn Ile Lys Lys Phe Ala Lys Gln Ala Thr Val Leu Thr Phe Thr
   1               5                   10                  15
   Thr Ala Leu Leu Ala Gly Gly Ala Thr Gln Ala Phe Ala Lys Glu Thr
               20                  25                  30
   Asn Gln Lys Pro Tyr Lys Glu Thr Tyr Gly Ile Ser His Ile Thr Arg
           35                  40                  45
   His Asp Met Leu Gln Ile Pro Glu Gln Gln Lys Asn Glu Lys Tyr Gln
       50                  55                  60
   Val Pro Glu Phe Asp Ser Ser Thr Ile Lys Asn Ile Ser Ser Ala Lys
   65                  70                  75                  80
   Gly Leu Asp Val Trp Asp Ser Trp Pro Leu Gln Asn Ala Asp Gly Thr
                   85                  90                  95
   Val Ala Asn Tyr His Gly Tyr His Ile Val Phe Ala Leu Ala Gly Asp
               100                 105                 110
   Pro Lys Asn Ala Asp Asp Thr Ser Ile Tyr Met Phe Tyr Gln Lys Val
           115                 120                 125
   Gly Glu Thr Ser Ile Asp Ser Trp Lys Asn Ala Gly Arg Val Phe Lys
       130                 135                 140
   Asp Ser Asp Lys Phe Asp Ala Asn Asp Ser Ile Leu Lys Asp Gln Thr
   145                 150                 155                 160
   Gln Glu Trp Ser Gly Ser Ala Thr Phe Thr Ser Asp Gly Lys Ile Arg
                   165                 170                 175
   Leu Phe Tyr Thr Asp Phe Ser Gly Lys His Tyr Gly Lys Gln Thr Leu
               180                 185                 190
   Thr Thr Ala Gln Val Asn Val Ser Ala Ser Asp Ser Ser Leu Asn Ile
           195                 200                 205
   Asn Gly Val Glu Asp Tyr Lys Ser Ile Phe Asp Gly Asp Gly Lys Thr
   210 215 220
   Tyr Gln Asn Val Gln Gln Phe Ile Asp Glu Gly Asn Tyr Ser Ser Gly
   225 230 235 240
   Asp Asn His Thr Leu Arg Asp Pro His Tyr Val Glu Asp Lys Gly His
   245 250 255
   Lys Tyr Leu Val Phe Glu Ala Asn Thr Gly Thr Glu Asp Gly Tyr Gln
   260 265 270
   Gly Glu Glu Ser Leu Phe Asn Lys Ala Tyr Tyr Gly Lys Ser Thr Ser
   275 280 285
   Phe Phe Arg Gln Glu Ser Gln Lys Leu Leu Gln Ser Asp Lys Lys Arg
   290 295 300
   Thr Ala Glu Leu Ala Asn Gly Ala Leu Gly Met Ile Glu Leu Asn Asp
   305 310 315 320
   Asp Tyr Thr Leu Lys Lys Val Met Lys Pro Leu Ile Ala Ser Asn Thr
   325 330 335
   Val Thr Asp Glu Ile Glu Arg Ala Asn Val Phe Lys Met Asn Gly Lys
   340 345 350
   Trp Tyr Leu Phe Thr Asp Ser Arg Gly Ser Lys Met Thr Ile Asp Gly
   355 360 365
   Ile Thr Ser Asn Asp Ile Tyr Met Leu Gly Tyr Val Ser Asn Ser Leu
   370 375 380
   Thr Gly Pro Tyr Lys Pro Leu Asn Lys Thr Gly Leu Val Leu Lys Met
   385 390 395 400
   Asp Leu Asp Pro Asn Asp Val Thr Phe Thr Tyr Ser His Phe Ala Val
   405 410 415
   Pro Gln Ala Lys Gly Asn Asn Val Val Ile Thr Ser Tyr Met Thr Asn
   420 425 430
   Arg Gly Phe Tyr Ala Asp Lys Gln Ser Thr Phe Ala Pro Ser Phe Leu
   435 440 445
   Leu Asn Ile Lys Gly Lys Lys Thr Ser Val Val Lys Asp Ser Ile Leu
   450 455 460
   Glu Gln Gly Gln Leu Thr Val Asn Lys
   465 470
<210> 21
   <211> 24
   <212> DNA
   <213> Artificial
<220>
   <223> primer
<400> 21
   cgggatcctt tttaacccat caca 24
<210> 22
   <211> 29
   <212> DNA
   <213> Artificial
<220>
   <223> primer
<400> 22
   gaagatcttc aaaaggttag gaatacggt 29
<210> 23
   <211> 23
   <212> DNA
   <213> Artificial
<220>
   <223> primer
<400> 23
   ccttttgaag atcgaccagt tgg 23
<210> 24
   <211> 44
   <212> DNA
   <213> Artificial
<220>
   <223> primer
<400> 24
   tacctggaat gctgttttcc cagggatcgc agtggtgagt aacc 44
<210> 25
   <211> 28
   <212> DNA
   <213> Artificial
<220>
   <223> primer
<400> 25
   cctgggaaaa cagcattcca ggtattag 28
<210> 26
   <211> 23
   <212> DNA
   <213> Artificial
<220>
   <223> primer
<400> 26
   tgcaggtcga ctctagagga tcc 23
<210> 27
   <211> 2028
   <212> DNA
   <213> Corynebacterium glutamicum
<220>
   <221> CDS
   <222> (1).. (2028)
   <223>
<400> 27
   atg gcg ttc tcc gta gag atg ccc gag ctg ggc gaa tca gta acc gaa      48
   Met Ala Phe Ser Val Glu Met Pro Glu Leu Gly Glu Ser Val Thr Glu
   1               5                   10                  15
   ggc acg atc acc cag tgg ttg aag tct gtt ggt gac act gtt gag gta      96
   Gly Thr Ile Thr Gln Trp Leu Lys Ser Val Gly Asp Thr Val Glu Val
               20                  25                  30
   gat gag ccg ttg ctc gag gtc tca act gac aag gtc gac acc gag att      144
   Asp Glu Pro Leu Leu Glu Val Ser Thr Asp Lys Val Asp Thr Glu Ile
           35                  40                  45
   ccc tct cct gtc gcc ggt gtc atc cta gag att aag gct gaa gag gat      192
   Pro Ser Pro Val Ala Gly Val Ile Leu Glu Ile Lys Ala Glu Glu Asp
       50                  55                  60
   gac acc gtc gac gtc ggc ggt gtc att gca ata atc ggc gat gct gat      240
   Asp Thr Val Asp Val Gly Gly Val Ile Ala Ile Ile Gly Asp Ala Asp
   65                  70                  75                  80
   gag act cct gcc aac gaa gct cct gcc gac gag gca cca gct cct gcc      288
   Glu Thr Pro Ala Asn Glu Ala Pro Ala Asp Glu Ala Pro Ala Pro Ala
                   85                  90                  95
   gaa gag gaa gaa cca gtt aag gaa gag cca aag aag gag gca gct cct      336
   Glu Glu Glu Glu Pro Val Lys Glu Glu Pro Lys Lys Glu Ala Ala Pro
               100                 105                 110
   gaa gct cca gca gca act ggc gcc gca acc gat gtg gaa atg cca gaa      384
   Glu Ala Pro Ala Ala Thr Gly Ala Ala Thr Asp Val Glu Met Pro Glu
           115                 120                 125
   ctc ggc gaa tcc gtc acc gaa ggc acc att acc cag tgg ctc aag gct      432
   Leu Gly Glu Ser Val Thr Glu Gly Thr Ile Thr Gln Trp Leu Lys Ala
       130                 135                 140
   gtc ggc gac acc gtc gaa gta gac gaa cca ctt ctt gag gtc tcc acc     480
   Val Gly Asp Thr Val Glu Val Asp Glu Pro Leu Leu Glu Val Ser Thr
   145                 150                 155                 160
   gac aag gtc gac acc gaa atc cca tcc cca gta gca ggc acc atc gtg     528
   Asp Lys Val Asp Thr Glu Ile Pro Ser Pro Val Ala Gly Thr Ile Val
                   165                 170                 175
   gag atc ctt gca gac gaa gac gac acc gtc gac gtc ggc gca gtc atc     576
   Glu Ile Leu Ala Asp Glu Asp Asp Thr Val Asp Val Gly Ala Val Ile
               180                 185                 190
   gcc cgc atc ggt gac gca aac gca gct gca gca cct gcc gaa gag gaa     624
   Ala Arg Ile Gly Asp Ala Asn Ala Ala Ala Ala Pro Ala Glu Glu Glu
           195                 200                 205
   gca gct cct gcc gaa gag gaa gaa cca gtt aag gaa gag cca aag aag     672
   Ala Ala Pro Ala Glu Glu Glu Glu Pro Val Lys Glu Glu Pro Lys Lys
       210                 215                 220
   gag gca gct cct gaa gct cca gca gca act ggc gcc gca acc gat gtg     720
   Glu Ala Ala Pro Glu Ala Pro Ala Ala Thr Gly Ala Ala Thr Asp Val
   225                 230                 235                 240
   gaa atg cca gaa ctc ggc gaa tcc gtc acc gaa ggc acc att acc cag     768
   Glu Met Pro Glu Leu Gly Glu Ser Val Thr Glu Gly Thr Ile Thr Gln
                   245                 250                 255
   tgg ctc aag gct gtc ggc gac acc gtc gaa gta gac gaa cca ctt ctt     816
   Trp Leu Lys Ala Val Gly Asp Thr Val Glu Val Asp Glu Pro Leu Leu
               260                 265                 270
   gag gtc tcc acc gac aag gtc gac acc gaa atc cca tcc cca gta gca     864
   Glu Val Ser Thr Asp Lys Val Asp Thr Glu Ile Pro Ser Pro Val Ala
           275                 280                 285
   ggc acc atc gtg gag atc ctt gca gac gaa gac gac acc gtc gac gtc     912
   Gly Thr Ile Val Glu Ile Leu Ala Asp Glu Asp Asp Thr Val Asp Val
       290                 295                 300
   ggc gca gtc atc gcc cgc atc ggt gac gca aac gca gct gca gca cct     960
   Gly Ala Val Ile Ala Arg Ile Gly Asp Ala Asn Ala Ala Ala Ala Pro
   305                 310                 315                 320
   gcc gaa gag gaa gca gct cct gcc gaa gag gag gaa cca gtt aag gaa     1008
   Ala Glu Glu Glu Ala Ala Pro Ala Glu Glu Glu Glu Pro Val Lys Glu
                   325                 330                 335
   gag cca aag aag gaa gag ccc aag aag gaa gag ccc aag aag gaa gca     1056
   Glu Pro Lys Lys Glu Glu Pro Lys Lys Glu Glu Pro Lys Lys Glu Ala
               340                 345                 350
   gct act aca cct gct gcg gca tcc gca act gtg tcc gct tct ggc gac     1104
   Ala Thr Thr Pro Ala Ala Ala Ser Ala Thr Val Ser Ala Ser Gly Asp
           355                 360                 365
   aac gtt cca tac gtc acc cca ctg gtg cgc aag ctt gct gaa aag cac     1152
   Asn Val Pro Tyr Val Thr Pro Leu Val Arg Lys Leu Ala Glu Lys His
       370                 375                 380
   ggc gtt gac ttg aac acc gtg acc ggt acc ggt atc ggt ggc cgt atc     1200
   Gly Val Asp Leu Asn Thr Val Thr Gly Thr Gly Ile Gly Gly Arg Ile
   385                 390                 395                 400
   cgc aag cag gat gtt ttg gct gct gcg aac ggc gag gct gca cct gct      1248
   Arg Lys Gln Asp Val Leu Ala Ala Ala Asn Gly Glu Ala Ala Pro Ala
                   405                 410                 415
   gag gct gct gct cct gtt tcc gct tgg tcc act aag tct gtt gac cct      1296
   Glu Ala Ala Ala Pro Val Ser Ala Trp Ser Thr Lys Ser Val Asp Pro
               420                 425                 430
   gag aag gct aag ctc cgt ggt acc act cag aag gtc aac cgc atc cgt      1344
   Glu Lys Ala Lys Leu Arg Gly Thr Thr Gln Lys Val Asn Arg Ile Arg
           435                 440                 445
   gag atc acc gcg atg aag acc gtc gag gct ctg cag att tct gct cag      1392
   Glu Ile Thr Ala Met Lys Thr Val Glu Ala Leu Gln Ile Ser Ala Gln
       450                 455                 460
   ctc acc cag ctg cac gag gtc gat atg act cgc gtt gct gag ctg cgt      1440
   Leu Thr Gln Leu His Glu Val Asp Met Thr Arg Val Ala Glu Leu Arg
   465                 470                 475                 480
   aag aag aac aag ccc gcg ttc atc gag aag cac ggt gtg aac ctc act      1488
   Lys Lys Asn Lys Pro Ala Phe Ile Glu Lys His Gly Val Asn Leu Thr
                   485                 490                 495
   tac ctg cca ttc ttc gtg aag gca gtt gtc gag gct ttg gtt tcc cat      1536
   Tyr Leu Pro Phe Phe Val Lys Ala Val Val Glu Ala Leu Val Ser His
               500                 505                 510
   cca aac gtc aac gcg tct ttc aac gcg aag acc aag gag atg acc tac      1584
   Pro Asn Val Asn Ala Ser Phe Asn Ala Lys Thr Lys Glu Met Thr Tyr
           515                 520                 525
   cac tcc tcc gtt aac ctc tcc atc gct gtt gat acc cca gct ggt ctg      1632
   His Ser Ser Val Asn Leu Ser Ile Ala Val Asp Thr Pro Ala Gly Leu
       530                 535                 540
   ttg acc cca gtc att cac gat gct cag gat ctc tcc atc cca gag atc      1680
   Leu Thr Pro Val Ile His Asp Ala Gln Asp Leu Ser Ile Pro Glu Ile
   545                 550                 555                 560
   gca aag gca att gtt gac ctg gct gat cgt tca cgc aac aac aag ctg      1728
   Ala Lys Ala Ile Val Asp Leu Ala Asp Arg Ser Arg Asn Asn Lys Leu
                   565                 570                 575
   aag cca aac gat ctg tcc ggt ggc acc ttc acc atc acc aac att ggt      1776
   Lys Pro Asn Asp Leu Ser Gly Gly Thr Phe Thr Ile Thr Asn Ile Gly
               580                 585                 590
   tct gaa ggc gca ctg tct gat acc cca atc ctg gtt cca cca cag gct      1824
   Ser Glu Gly Ala Leu Ser Asp Thr Pro Ile Leu Val Pro Pro Gln Ala
           595                 600                 605
   ggc atc ttg ggc acc ggc gcg atc gtg aag cgt cca gtt gtc atc acc      1872
   Gly Ile Leu Gly Thr Gly Ala Ile Val Lys Arg Pro Val Val Ile Thr
       610                 615                 620
   gag gat gga att gat tcc atc gcg atc cgt cag atg gtc ttc cta cca      1920
   Glu Asp Gly Ile Asp Ser Ile Ala Ile Arg Gln Met Val Phe Leu Pro
   625                 630                 635                 640
   ctg acc tac gac cac cag gtt gta gat ggc gca gat gct ggt cgc ttc      1968
   Leu Thr Tyr Asp His Gln Val Val Asp Gly Ala Asp Ala Gly Arg Phe
                   645                 650                 655
   ctg acc acc atc aag gac cgc ctt gag acc gct aac ttc gaa ggc gat 2016
   Leu Thr Thr Ile Lys Asp Arg Leu Glu Thr Ala Asn Phe Glu Gly Asp
   660 665 670
   ctg cag ctc taa 2028
   Leu Gln Leu
   675
<210> 28
   <211> 675
   <212> PRT
   <213> Corynebacterium glutamicum
<400> 28
   Met Ala Phe Ser Val Glu Met Pro Glu Leu Gly Glu Ser Val Thr Glu
   1 5 10 15
   Gly Thr Ile Thr Gln Trp Leu Lys Ser Val Gly Asp Thr Val Glu Val
   20 25 30
   Asp Glu Pro Leu Leu Glu Val Ser Thr Asp Lys Val Asp Thr Glu Ile
   35 40 45
   Pro Ser Pro Val Ala Gly Val Ile Leu Glu Ile Lys Ala Glu Glu Asp
   50 55 60
   Asp Thr Val Asp Val Gly Gly Val Ile Ala Ile Ile Gly Asp Ala Asp
   65 70 75 80
   Glu Thr Pro Ala Asn Glu Ala Pro Ala Asp Glu Ala Pro Ala Pro Ala
   85 90 95
   Glu Glu Glu Glu Pro Val Lys Glu Glu Pro Lys Lys Glu Ala Ala Pro
   100 105 110
   Glu Ala Pro Ala Ala Thr Gly Ala Ala Thr Asp Val Glu Met Pro Glu
   115 120 125
   Leu Gly Glu Ser Val Thr Glu Gly Thr Ile Thr Gln Trp Leu Lys Ala
   130 135 140
   Val Gly Asp Thr Val Glu Val Asp Glu Pro Leu Leu Glu Val Ser Thr
   145 150 155 160
   Asp Lys Val Asp Thr Glu Ile Pro Ser Pro Val Ala Gly Thr Ile Val
   165 170 175
   Glu Ile Leu Ala Asp Glu Asp Asp Thr Val Asp Val Gly Ala Val Ile
   180 185 190
   Ala Arg Ile Gly Asp Ala Asn Ala Ala Ala Ala Pro Ala Glu Glu Glu
   195 200 205
   Ala Ala Pro Ala Glu Glu Glu Glu Pro Val Lys Glu Glu Pro Lys Lys
   210 215 220
   Glu Ala Ala Pro Glu Ala Pro Ala Ala Thr Gly Ala Ala Thr Asp Val
   225 230 235 240
   Glu Met Pro Glu Leu Gly Glu Ser Val Thr Glu Gly Thr Ile Thr Gln
   245 250 255
   Trp Leu Lys Ala Val Gly Asp Thr Val Glu Val Asp Glu Pro Leu Leu
   260 265 270
   Glu Val Ser Thr Asp Lys Val Asp Thr Glu Ile Pro Ser Pro Val Ala
   275 280 285
   Gly Thr Ile Val Glu Ile Leu Ala Asp Glu Asp Asp Thr Val Asp Val
   290 295 300
   Gly Ala Val Ile Ala Arg Ile Gly Asp Ala Asn Ala Ala Ala Ala Pro
   305 310 315 320
   Ala Glu Glu Glu Ala Ala Pro Ala Glu Glu Glu Glu Pro Val Lys Glu
   325 330 335
   Glu Pro Lys Lys Glu Glu Pro Lys Lys Glu Glu Pro Lys Lys Glu Ala
   340 345 350
   Ala Thr Thr Pro Ala Ala Ala Ser Ala Thr Val Ser Ala Ser Gly Asp
   355 360 365
   Asn Val Pro Tyr Val Thr Pro Leu Val Arg Lys Leu Ala Glu Lys His
   370 375 380
   Gly Val Asp Leu Asn Thr Val Thr Gly Thr Gly Ile Gly Gly Arg Ile
   385 390 395 400
   Arg Lys Gln Asp Val Leu Ala Ala Ala Asn Gly Glu Ala Ala Pro Ala
   405 410 415
   Glu Ala Ala Ala Pro Val Ser Ala Trp Ser Thr Lys Ser Val Asp Pro
   420 425 430
   Glu Lys Ala Lys Leu Arg Gly Thr Thr Gln Lys Val Asn Arg Ile Arg
   435 440 445
   Glu Ile Thr Ala Met Lys Thr Val Glu Ala Leu Gln Ile Ser Ala Gln
   450 455 460
   Leu Thr Gln Leu His Glu Val Asp Met Thr Arg Val Ala Glu Leu Arg
   465 470 475 480
   Lys Lys Asn Lys Pro Ala Phe Ile Glu Lys His Gly Val Asn Leu Thr
   485 490 495
   Tyr Leu Pro Phe Phe Val Lys Ala Val Val Glu Ala Leu Val Ser His
   500 505 510
   Pro Asn Val Asn Ala Ser Phe Asn Ala Lys Thr Lys Glu Met Thr Tyr
   515 520 525
   His Ser Ser Val Asn Leu Ser Ile Ala Val Asp Thr Pro Ala Gly Leu
   530 535 540
   Leu Thr Pro Val Ile His Asp Ala Gln Asp Leu Ser Ile Pro Glu Ile
   545 550 555 560
   Ala Lys Ala Ile Val Asp Leu Ala Asp Arg Ser Arg Asn Asn Lys Leu
   565 570 575
   Lys Pro Asn Asp Leu Ser Gly Gly Thr Phe Thr Ile Thr Asn Ile Gly
   580 585 590
   Ser Glu Gly Ala Leu Ser Asp Thr Pro Ile Leu Val Pro Pro Gln Ala
   595 600 605
   Gly Ile Leu Gly Thr Gly Ala Ile Val Lys Arg Pro Val Val Ile Thr
   610 615 620
   Glu Asp Gly Ile Asp Ser Ile Ala Ile Arg Gln Met Val Phe Leu Pro
   625 630 635 640
   Leu Thr Tyr Asp His Gln Val Val Asp Gly Ala Asp Ala Gly Arg Phe
   645 650 655
   Leu Thr Thr Ile Lys Asp Arg Leu Glu Thr Ala Asn Phe Glu Gly Asp
   660 665 670
   Leu Gln Leu
   675
<210> 29
   <211> 3481
   <212> DNA
   <213> Corynebacterium glutamicum
<220>
   <221> CDS
   <222> (1437).. (3035)
   <223> yggB
<400> 29
   gatcttgccg atttcggcag gatcaatgtc ggcgtgaatg atcttggcat caggtgcgaa      60
   agtgtcaacg tcaccggtga cgcggtcatc aaagcgggag ccgatagcaa tcagcaggtc     120
   gctgcgctgc agtgcaccaa cagcggacac agtgccatgc atgcctggca tacccatgtg     180
   cagctcgtgg gactctggga aggttcccag cgccatcaat gtggtgacaa ctggaatgcc     240
   ggtgtgctca gcgaacgcac gaagctcttc gtgggcatca gccttgataa cgccgccgcc     300
   aacgtaaagg acaggcttct tagactcacc gatcagtttg acagcctgct caatctgtcg     360
   agcatgcggt gttgaaactg ggcggtagcc tggcaggtcg atctttggtg gccagacgaa     420
   atccaattca gcgttctgaa catccttggg gatatccact agaacaggac cagggcgacc     480
   agtaatcgcg aggtggaatg cctcagccaa tgcctgtgga atgtcgttgg ggttggtgac     540
   catgaagttg tgcttggtca ctggcatggt gatgccgcgg atatcggctt cctggaaagc     600
   atcggtaccc agcaggctac ttccgacctg gccggtgatg gcaaccatgg gaacggagtc     660
   caagtttgca tcagcgattg gggtaaccaa gttggttgcg cctgggccag aggttgcaat     720
   gcagacgcca acgcgtccag taacctgcgc gtagccggtt gctgcgtggc ctgcgccctg     780
   ctcgtggcgc actaggacgt ggcgcacctt tgtggaggaa tagagcgggt catacaccgg     840
   tagcaccgca ccaccaggaa taccgaacac gatgtcggcg ttaagctcct cgagcgatcg     900
   aacaattgcc tgtgcacctg tcatccgctc aggggcggcg gatcgaccac ggcttgcaac     960
   cgtggcggga gtgggctgtt gagaagctgc cacattcacg actttctggc tcctttacta    1020
   aataaggatt ttcacaggac ccgtccaagc caagccgatt tcaactcagc ctaaagacaa    1080
   agccctcatt taaaattgtt ccgacgcgga tgcgtgtgca cgcagtgcga cagatgtctg    1140
   ttgcaaagtt ggctacttgg gtcataacca acaagaaagc cctcgttcca acactgtggt    1200
   gagtgttgtc gagggcgctt gacgagacga cttggaaggc cgttacggca ggcgccgcgc    1260
   ggttactact acaagtcgaa taatggtcat ggtgtgtcat gctacacaca tcgagtttcc    1320
   aattccacaa cgcacgaaaa ttcccacccc caaaactccc ccacttcggt taaggaatca    1380
   ggattctcac aaagttcagg caggctcccg ctacttttca gcgctaatct tggctc atg    1439
                                                                 Met
                                                                 1
   att tta ggc gta ccc att caa tat ttg ctc tat tca ttg tgg aat tgg      1487
   Ile Leu Gly Val Pro Ile Gln Tyr Leu Leu Tyr Ser Leu Trp Asn Trp
               5                   10                  15
   att gtc gat acc ggt ttt gat gta gca att atc ctg gtc ttg gcg ttt      1535
   Ile Val Asp Thr Gly Phe Asp Val Ala Ile Ile Leu Val Leu Ala Phe
           20                  25                  30
   ttg att cca cgt atc ggc cga ctg gcc atg cgt att atc aag cag cga 1583
   Leu Ile Pro Arg Ile Gly Arg Leu Ala Met Arg Ile Ile Lys Gln Arg
   35 40 45
   gtg gag tct gca gcc gat gcg gac acc act aag aac cag ctc gcg ttc 1631
   Val Glu Ser Ala Ala Asp Ala Asp Thr Thr Lys Asn Gln Leu Ala Phe
   50 55 60 65
   gct ggc gtt ggc gtt tat atc gcg caa att gtg gcg ttt ttc atg ctt 1679
   Ala Gly Val Gly Val Tyr Ile Ala Gln Ile Val Ala Phe Phe Met Leu
   70 75 80
   gcc gtc tcc gcg atg cag gct ttt ggt ttc tct ctc gcg ggc gct gcg 1727
   Ala Val Ser Ala Met Gln Ala Phe Gly Phe Ser Leu Ala Gly Ala Ala
   85 90 95
   att ccg gca acc att gcg tca gct gcc att ggt ctt ggt gcg cag tcg 1775
   Ile Pro Ala Thr Ile Ala Ser Ala Ala Ile Gly Leu Gly Ala Gln Ser
   100 105 110
   att gtt gcg gac ttc ttg gcc gga ttt ttc atc ctg acg gaa aag caa 1823
   Ile Val Ala Asp Phe Leu Ala Gly Phe Phe Ile Leu Thr Glu Lys Gln
   115 120 125
   ttc ggc gtg ggt gac tgg gtg cgc ttt gag ggc aac ggc atc gtt gtt 1871
   Phe Gly Val Gly Asp Trp Val Arg Phe Glu Gly Asn Gly Ile Val Val
   130 135 140 145
   gaa ggc acc gtc att gag atc acc atg cgc gcg acc aaa att cgc acg 1919
   Glu Gly Thr Val Ile Glu Ile Thr Met Arg Ala Thr Lys Ile Arg Thr
   150 155 160
   att gca caa gag acc gtg atc atc ccg aac tcc acg gcg aaa gtg tgc 1967
   Ile Ala Gln Glu Thr Val Ile Ile Pro Asn Ser Thr Ala Lys Val Cys
   165 170 175
   atc aac aat tct aat aac tgg tcg cgt gcg gtt gtc gtt att ccg atc 2015
   Ile Asn Asn Ser Asn Asn Trp Ser Arg Ala Val Val Val Ile Pro Ile
   180 185 190
   ccc atg ttg ggt tct gaa aac atc aca gat gtc atc gcg cgc tct gaa 2063
   Pro Met Leu Gly Ser Glu Asn Ile Thr Asp Val Ile Ala Arg Ser Glu
   195 200 205
   gct gcg act cgt cgc gca ctt ggc cag gag aaa atc gca ccg gaa atc 2111
   Ala Ala Thr Arg Arg Ala Leu Gly Gln Glu Lys Ile Ala Pro Glu Ile
   210 215 220 225
   ctc ggt gaa ctc gat gtg cac cca gcc acg gaa gtc aca ccg cca acg 2159
   Leu Gly Glu Leu Asp Val His Pro Ala Thr Glu Val Thr Pro Pro Thr
   230 235 240
   gtg gtc ggc atg ccg tgg atg gtc acc atg cgt ttc ctc gtg caa gtc 2207
   Val Val Gly Met Pro Trp Met Val Thr Met Arg Phe Leu Val Gln Val
   245 250 255
   acc gcc ggc aat caa tgg ctg gtc gaa cgc gcc atc cgc aca gaa atc 2255
   Thr Ala Gly Asn Gln Trp Leu Val Glu Arg Ala Ile Arg Thr Glu Ile
   260 265 270
   atc aac gaa ttc tgg gaa gaa tac ggc agc gca acc act aca tcg gga 2303
   Ile Asn Glu Phe Trp Glu Glu Tyr Gly Ser Ala Thr Thr Thr Ser Gly
   275 280 285
   acc ctc att gat tcc tta cac gtt gag cat gaa gag cca aag acc tcg      2351
   Thr Leu Ile Asp Ser Leu His Val Glu His Glu Glu Pro Lys Thr Ser
   290                 295                 300                 305
   ctt atc gac gcc tcc ccc cag gct ctt aag gaa ccg aag ccg gag gct      2399
   Leu Ile Asp Ala Ser Pro Gln Ala Leu Lys Glu Pro Lys Pro Glu Ala
                   310                 315                 320
   gcg gcg acg gtt gca tcg cta gct gca tcg tct aac gac gat gca gac      2447
   Ala Ala Thr Val Ala Ser Leu Ala Ala Ser Ser Asn Asp Asp Ala Asp
               325                 330                 335
   aat gca gac gcc tcg gcg atc aat gca ggc aat cca gag aag gaa ctt      2495
   Asn Ala Asp Ala Ser Ala Ile Asn Ala Gly Asn Pro Glu Lys Glu Leu
           340                 345                 350
   gat tcc gat gtg ctg gaa caa gaa ctc tcc agc gaa gaa ccg gaa gaa      2543
   Asp Ser Asp Val Leu Glu Gln Glu Leu Ser Ser Glu Glu Pro Glu Glu
       355                 360                 365
   aca gca aaa cca gat cac tct ctc cga ggc ttc ttc cgc act gat tac      2591
   Thr Ala Lys Pro Asp His Ser Leu Arg Gly Phe Phe Arg Thr Asp Tyr
   370                 375                 380                 385
   tac cca aat cgg tgg cag aag atc ctg tcg ttt ggc gga cgt gtc cgc      2639
   Tyr Pro Asn Arg Trp Gln Lys Ile Leu Ser Phe Gly Gly Arg Val Arg
                   390                 395                 400
   atg agc act tcc ctg ttg ttg ggt gcg ctg ctc ttg ctg tca cta ttt      2687
   Met Ser Thr Ser Leu Leu Leu Gly Ala Leu Leu Leu Leu Ser Leu Phe
               405                 410                 415
   aag gtc atg act gtg gaa cca agt gag aat tgg caa aac tcc agt gga      2735
   Lys Val Met Thr Val Glu Pro Ser Glu Asn Trp Gln Asn Ser Ser Gly
           420                 425                 430
   tgg ctg tca cca agc act gcc acc tca act gcg gtg acc acc tcc gaa      2783
   Trp Leu Ser Pro Ser Thr Ala Thr Ser Thr Ala Val Thr Thr Ser Glu
       435                 440                 445
   act tcc gcg cca gca agc acg cct tcg atg aca gtg ccc act acg gtg      2831
   Thr Ser Ala Pro Ala Ser Thr Pro Ser Met Thr Val Pro Thr Thr Val
   450                 455                 460                 465
   gag gag acc cca acg atg gaa tct agc gtc gaa acg cag cag gaa acc      2879
   Glu Glu Thr Pro Thr Met Glu Ser Ser Val Glu Thr Gln Gln Glu Thr
                   470                 475                 480
   tca acc cct gca acc gca acg ccc cag cga gcc gac acc atc gaa ccg      2927
   Ser Thr Pro Ala Thr Ala Thr Pro Gln Arg Ala Asp Thr Ile Glu Pro
               485                 490                 495
   acc gag gaa gcc acg tcg cag gag gaa acg act gca tcg cag acg cag      2975
   Thr Glu Glu Ala Thr Ser Gln Glu Glu Thr Thr Ala Ser Gln Thr Gln
           500                 505                 510
   tct cca gca gtg gaa gca cca acc gcg gtc caa gaa aca gtt gcg ccg      3023
   Ser Pro Ala Val Glu Ala Pro Thr Ala Val Gln Glu Thr Val Ala Pro
       515                 520                 525
   acg tcc acc cct taggacgctg attacagacg tgtcccattt ctttactact          3075
   Thr Ser Thr Pro
   530
   attggaaatt atgagttcag acgcagaaaa ggcatccgtg gagctttccg aaaaatttca 3135
   cccagaacgc acccatattt tgggcgccgt tgtttttggc ctgatctcat tattagtcat 3195
   cggcgcagcc cctcagtacc tgttttggct gctcgcgctc cctgtcatct tcggttactg 3255
   ggttctaaaa tcatccacga tcgttgatga acagggcatc accgcaaact acgccttcaa 3315
   gggcaaaaag gttgtggcct gggaagacct cgcaggaatc ggattcaagg gtgcccgcac 3375
   tttcgctcgc accacctccg atgcagaagt caccctcccc ggcgtcacct tcaactccct 3435
   tccccgcctt gaagctgctt cccacggccg catccccgat gcgatc 3481
<210> 30
   <211> 533
   <212> PRT
   <213> Corynebacterium glutamicum
<400> 30
   Met Ile Leu Gly Val Pro Ile Gln Tyr Leu Leu Tyr Ser Leu Trp Asn
   1 5 10 15
   Trp Ile Val Asp Thr Gly Phe Asp Val Ala Ile Ile Leu Val Leu Ala
   20 25 30
   Phe Leu Ile Pro Arg Ile Gly Arg Leu Ala Met Arg Ile Ile Lys Gln
   35 40 45
   Arg Val Glu Ser Ala Ala Asp Ala Asp Thr Thr Lys Asn Gln Leu Ala
   50 55 60
   Phe Ala Gly Val Gly Val Tyr Ile Ala Gln Ile Val Ala Phe Phe Met
   65 70 75 80
   Leu Ala Val Ser Ala Met Gln Ala Phe Gly Phe Ser Leu Ala Gly Ala
   85 90 95
   Ala Ile Pro Ala Thr Ile Ala Ser Ala Ala Ile Gly Leu Gly Ala Gln
   100 105 110
   Ser Ile Val Ala Asp Phe Leu Ala Gly Phe Phe Ile Leu Thr Glu Lys
   115 120 125
   Gln Phe Gly Val Gly Asp Trp Val Arg Phe Glu Gly Asn Gly Ile Val
   130 135 140
   Val Glu Gly Thr Val Ile Glu Ile Thr Met Arg Ala Thr Lys Ile Arg
   145 150 155 160
   Thr Ile Ala Gln Glu Thr Val Ile Ile Pro Asn Ser Thr Ala Lys Val
   165 170 175
   Cys Ile Asn Asn Ser Asn Asn Trp Ser Arg Ala Val Val Val Ile Pro
   180 185 190
   Ile Pro Met Leu Gly Ser Glu Asn Ile Thr Asp Val Ile Ala Arg Ser
   195 200 205
   Glu Ala Ala Thr Arg Arg Ala Leu Gly Gln Glu Lys Ile Ala Pro Glu
   210 215 220
   Ile Leu Gly Glu Leu Asp Val His Pro Ala Thr Glu Val Thr Pro Pro
   225 230 235 240
   Thr Val Val Gly Met Pro Trp Met Val Thr Met Arg Phe Leu Val Gln
   245 250 255
   Val Thr Ala Gly Asn Gln Trp Leu Val Glu Arg Ala Ile Arg Thr Glu
   260 265 270
   Ile Ile Asn Glu Phe Trp Glu Glu Tyr Gly Ser Ala Thr Thr Thr Ser
   275 280 285
   Gly Thr Leu Ile Asp Ser Leu His Val Glu His Glu Glu Pro Lys Thr
   290 295 300
   Ser Leu Ile Asp Ala Ser Pro Gln Ala Leu Lys Glu Pro Lys Pro Glu
   305 310 315 320
   Ala Ala Ala Thr Val Ala Ser Leu Ala Ala Ser Ser Asn Asp Asp Ala
   325 330 335
   Asp Asn Ala Asp Ala Ser Ala Ile Asn Ala Gly Asn Pro Glu Lys Glu
   340 345 350
   Leu Asp Ser Asp Val Leu Glu Gln Glu Leu Ser Ser Glu Glu Pro Glu
   355 360 365
   Glu Thr Ala Lys Pro Asp His Ser Leu Arg Gly Phe Phe Arg Thr Asp
   370 375 380
   Tyr Tyr Pro Asn Arg Trp Gln Lys Ile Leu Ser Phe Gly Gly Arg Val
   385 390 395 400
   Arg Met Ser Thr Ser Leu Leu Leu Gly Ala Leu Leu Leu Leu Ser Leu
   405 410 415
   Phe Lys Val Met Thr Val Glu Pro Ser Glu Asn Trp Gln Asn Ser Ser
   420 425 430
   Gly Trp Leu Ser Pro Ser Thr Ala Thr Ser Thr Ala Val Thr Thr Ser
   435 440 445
   Glu Thr Ser Ala Pro Ala Ser Thr Pro Ser Met Thr Val Pro Thr Thr
   450 455 460
   Val Glu Glu Thr Pro Thr Met Glu Ser Ser Val Glu Thr Gln Gln Glu
   465 470 475 480
   Thr Ser Thr Pro Ala Thr Ala Thr Pro Gln Arg Ala Asp Thr Ile Glu
   485 490 495
   Pro Thr Glu Glu Ala Thr Ser Gln Glu Glu Thr Thr Ala Ser Gln Thr
   500 505 510
   Gln Ser Pro Ala Val Glu Ala Pro Thr Ala Val Gln Glu Thr Val Ala
   515 520 525
   Pro Thr Ser Thr Pro
   530
<210> 31
   <211> 3481
   <212> DNA
   <213> Corynebacterium glutamicum
<220>
   <221> CDS
   <222> (1437).. (3035)
   <223>
<400> 31
   gatcttgccg atttcggcag gatcaatgtc ggcgtgaatg atcttggcat caggtgcgaa 60
   agtgtcaacg tcaccggtga cgcggtcatc aaagcgggag ccgatagcaa tcagcaggtc 120
   gctgcgctgc agtgcaccaa cagcggacac agtgccatgc atgcctggca tacccatgtg 180
   cagctcgtgg gactctggga aggttcccag cgccatcaat gtggtgacaa ctggaatgcc 240
   ggtgtgctca gcgaacgcac gaagctcttc gtgggcatca gccttgataa cgccgccgcc 300
   aacgtaaagg acaggcttct tagactcacc gatcagtttg acagcctgct caatctgtcg 360
   agcatgcggt gttgaaactg ggcggtagcc tggcaggtcg atctttggtg gccagacgaa 420
   atccaattca gcgttctgaa catccttggg gatatccact agaacaggac cagggcgacc 480
   agtaatcgcg aggtggaatg cctcagccaa tgcctgtgga atgtcgttgg ggttggtgac 540
   catgaagttg tgcttggtca ctggcatggt gatgccgcgg atatcggctt cctggaaagc 600
   atcggtaccc agcaggctac ttccgacctg gccggtgatg gcaaccatgg gaacggagtc 660
   caagtttgca tcagcgattg gggtaaccaa gttggttgcg cctgggccag aggttgcaat 720
   gcagacgcca acgcgtccag taacctgcgc gtagccggtt gctgcgtggc ctgcgccctg 780
   ctcgtggcgc actaggacgt ggcgcacctt tgtggaggaa tagagcgggt catacaccgg 840
   tagcaccgca ccaccaggaa taccgaacac gatgtcggcg ttaagctcct cgagcgatcg 900
   aacaattgcc tgtgcacctg tcatccgctc aggggcggcg gatcgaccac ggcttgcaac 960
   cgtggcggga gtgggctgtt gagaagctgc cacattcacg actttctggc tcctttacta 1020
   aataaggatt ttcacaggac ccgtccaagc caagccgatt tcaactcagc ctaaagacaa 1080
   agccctcatt taaaattgtt ccgacgcgga tgcgtgtgca cgcagtgcga cagatgtctg 1140
   ttgcaaagtt ggctacttgg gtcataacca acaagaaagc cctcgttcca acactgtggt 1200
   gagtgttgtc gagggcgctt gacgagacga cttggaaggc cgttacggca ggcgccgcgc 1260
   ggttactact acaagtcgaa taatggtcat ggtgtgtcat gctacacaca tcgagtttcc 1320
   aattccacaa cgcacgaaaa ttcccacccc caaaactccc ccacttcggt taaggaatca 1380
   ggattctcac aaagttcagg caggctcccg ctacttttca gcgctaatct tggctc atg 1439
   Met
   1
   att tta ggc gta ccc att caa tat ttg ctc tat tca ttg tgg aat tgg 1487
   Ile Leu Gly Val Pro Ile Gln Tyr Leu Leu Tyr Ser Leu Trp Asn Trp
   5 10 15
   att gtc gat acc ggt ttt gat gta gca att atc ctg gtc ttg gcg ttt 1535
   Ile Val Asp Thr Gly Phe Asp Val Ala Ile Ile Leu Val Leu Ala Phe
   20 25 30
   ttg att cca cgt atc ggc cga ctg gcc atg cgt att atc aag cag cga 1583
   Leu Ile Pro Arg Ile Gly Arg Leu Ala Met Arg Ile Ile Lys Gln Arg
   35 40 45
   gtg gag tct gca gcc gat gcg gac acc act aag aac cag ctc gcg ttc 1631
   Val Glu Ser Ala Ala Asp Ala Asp Thr Thr Lys Asn Gln Leu Ala Phe
   50 55 60 65
   gct ggc gtt ggc gtt tat atc gcg caa att gtg gcg ttt ttc atg ctt 1679
   Ala Gly Val Gly Val Tyr Ile Ala Gln Ile Val Ala Phe Phe Met Leu
   70 75 80
   gcc gtc tcc gcg atg cag gct ttt ggt ttc tct ctc gcg ggc gct gcg 1727
   Ala Val Ser Ala Met Gln Ala Phe Gly Phe Ser Leu Ala Gly Ala Ala
   85 90 95
   att ccg gca acc att gcg tca gct gcc att ggt ctt ggt gtg cag tcg 1775
   Ile Pro Ala Thr Ile Ala Ser Ala Ala Ile Gly Leu Gly Val Gln Ser
   100 105 110
   att gtt gcg gac ttc ttg gcc gga ttt ttc atc ctg acg gaa aag caa 1823
   Ile Val Ala Asp Phe Leu Ala Gly Phe Phe Ile Leu Thr Glu Lys Gln
   115 120 125
   ttc ggc gtg ggt gac tgg gtg cgc ttt gag ggc aac ggc atc gtt gtt 1871
   Phe Gly Val Gly Asp Trp Val Arg Phe Glu Gly Asn Gly Ile Val Val
   130 135 140 145
   gaa ggc acc gtc att gag atc acc atg cgc gcg acc aaa att cgc acg 1919
   Glu Gly Thr Val Ile Glu Ile Thr Met Arg Ala Thr Lys Ile Arg Thr
   150 155 160
   att gca caa gag acc gtg atc atc ccg aac tcc acg gcg aaa gtg tgc 1967
   Ile Ala Gln Glu Thr Val Ile Ile Pro Asn Ser Thr Ala Lys Val Cys
   165 170 175
   atc aac aat tct aat aac tgg tcg cgt gcg gtt gtc gtt att ccg atc 2015
   Ile Asn Asn Ser Asn Asn Trp Ser Arg Ala Val Val Val Ile Pro Ile
   180 185 190
   ccc atg ttg ggt tct gaa aac atc aca gat gtc atc gcg cgc tct gaa 2063
   Pro Met Leu Gly Ser Glu Asn Ile Thr Asp Val Ile Ala Arg Ser Glu
   195 200 205
   gct gcg act cgt cgc gca ctt ggc cag gag aaa atc gca ccg gaa atc 2111
   Ala Ala Thr Arg Arg Ala Leu Gly Gln Glu Lys Ile Ala Pro Glu Ile
   210 215 220 225
   ctc ggt gaa ctc gat gtg cac cca gcc acg gaa gtc aca ccg cca acg 2159
   Leu Gly Glu Leu Asp Val His Pro Ala Thr Glu Val Thr Pro Pro Thr
   230 235 240
   gtg gtc ggc atg ccg tgg atg gtc acc atg cgt ttc ctc gtg caa gtc 2207
   Val Val Gly Met Pro Trp Met Val Thr Met Arg Phe Leu Val Gln Val
   245 250 255
   acc gcc ggc aat caa tgg ctg gtc gaa cgc gcc atc cgc aca gaa atc 2255
   Thr Ala Gly Asn Gln Trp Leu Val Glu Arg Ala Ile Arg Thr Glu Ile
   260 265 270
   atc aac gaa ttc tgg gaa gaa tac ggc agc gca acc act aca tcg gga 2303
   Ile Asn Glu Phe Trp Glu Glu Tyr Gly Ser Ala Thr Thr Thr Ser Gly
   275 280 285
   acc ctc att gat tcc tta cac gtt gag cat gaa gag cca aag acc tcg 2351
   Thr Leu Ile Asp Ser Leu His Val Glu His Glu Glu Pro Lys Thr Ser
   290 295 300 305
   ctt atc gac gcc tcc ccc cag gct ctt aag gaa ccg aag ccg gag gct 2399
   Leu Ile Asp Ala Ser Pro Gln Ala Leu Lys Glu Pro Lys Pro Glu Ala
   310 315 320
   gcg gcg acg gtt gca tcg cta gct gca tcg tct aac gac gat gca gac 2447
   Ala Ala Thr Val Ala Ser Leu Ala Ala Ser Ser Asn Asp Asp Ala Asp
   325 330 335
   aat gca gac gcc tcg gcg atc aat gca ggc aat cca gag aag gaa ctt 2495
   Asn Ala Asp Ala Ser Ala Ile Asn Ala Gly Asn Pro Glu Lys Glu Leu
   340 345 350
   gat tcc gat gtg ctg gaa caa gaa ctc tcc agc gaa gaa ccg gaa gaa 2543
   Asp Ser Asp Val Leu Glu Gln Glu Leu Ser Ser Glu Glu Pro Glu Glu
   355 360 365
   aca gca aaa cca gat cac tct ctc cga ggc ttc ttc cgc act gat tac 2591
   Thr Ala Lys Pro Asp His Ser Leu Arg Gly Phe Phe Arg Thr Asp Tyr
   370                 375                 380                 385
   tac cca aat cgg tgg cag aag atc ctg tcg ttt ggc gga cgt gtc cgc      2639
   Tyr Pro Asn Arg Trp Gln Lys Ile Leu Ser Phe Gly Gly Arg Val Arg
                   390                 395                 400
   atg agc act tcc ctg ttg ttg ggt gcg ctg ctc ttg ctg tca cta ttt      2687
   Met Ser Thr Ser Leu Leu Leu Gly Ala Leu Leu Leu Leu Ser Leu Phe
               405                 410                 415
   aag gtc atg act gtg gaa cca agt gag aat tgg caa aac tcc agt gga      2735
   Lys Val Met Thr Val Glu Pro Ser Glu Asn Trp Gln Asn Ser Ser Gly
           420                 425                 430
   tgg ctg tca cca agc act gcc acc tca act gcg gtg acc acc tcc gaa      2783
   Trp Leu Ser Pro Ser Thr Ala Thr Ser Thr Ala Val Thr Thr Ser Glu
       435                 440                 445
   act tcc gcg cca gca agc acg cct tcg atg aca gtg ccc act acg gtg      2831
   Thr Ser Ala Pro Ala Ser Thr Pro Ser Met Thr Val Pro Thr Thr Val
   450                 455                 460                 465
   gag gag acc cca acg atg gaa tct agc gtc gaa acg cag cag gaa acc      2879
   Glu Glu Thr Pro Thr Met Glu Ser Ser Val Glu Thr Gln Gln Glu Thr
                   470                 475                 480
   tca acc cct gca acc gca acg ccc cag cga gcc gac acc atc gaa ccg      2927
   Ser Thr Pro Ala Thr Ala Thr Pro Gln Arg Ala Asp Thr Ile Glu Pro
               485                 490                 495
   acc gag gaa gcc acg tcg cag gag gaa acg act gca tcg cag acg cag      2975
   Thr Glu Glu Ala Thr Ser Gln Glu Glu Thr Thr Ala Ser Gln Thr Gln
           500                 505                 510
   tct cca gca gtg gaa gca cca acc gcg gtc caa gaa aca gtt gcg ccg      3023
   Ser Pro Ala Val Glu Ala Pro Thr Ala Val Gln Glu Thr Val Ala Pro
       515                 520                 525
   acg tcc acc cct taggacgctg attacagacg tgtcccattt ctttactact          3075
   Thr Ser Thr Pro
   530
   attggaaatt atgagttcag acgcagaaaa ggcatccgtg gagctttccg aaaaatttca    3135
   cccagaacgc acccatattt tgggcgccgt tgtttttggc ctgatctcat tattagtcat    3195
   cggcgcagcc cctcagtacc tgttttggct gctcgcgctc cctgtcatct tcggttactg    3255
   ggttctaaaa tcatccacga tcgttgatga acagggcatc accgcaaact acgccttcaa    3315
   gggcaaaaag gttgtggcct gggaagacct cgcaggaatc ggattcaagg gtgcccgcac    3375
   tttcgctcgc accacctccg atgcagaagt caccctcccc ggcgtcacct tcaactccct    3435
   tccccgcctt gaagctgctt cccacggccg catccccgat gcgatc                    3481
<210> 32
   <211> 533
   <212> PRT
   <213> Corynebacterium glutamicum
<400> 32
   Met Ile Leu Gly Val Pro Ile Gln Tyr Leu Leu Tyr Ser Leu Trp Asn
   1 5 10 15
   Trp Ile Val Asp Thr Gly Phe Asp Val Ala Ile Ile Leu Val Leu Ala
   20 25 30
   Phe Leu Ile Pro Arg Ile Gly Arg Leu Ala Met Arg Ile Ile Lys Gln
   35 40 45
   Arg Val Glu Ser Ala Ala Asp Ala Asp Thr Thr Lys Asn Gln Leu Ala
   50 55 60
   Phe Ala Gly Val Gly Val Tyr Ile Ala Gln Ile Val Ala Phe Phe Met
   65 70 75 80
   Leu Ala Val Ser Ala Met Gln Ala Phe Gly Phe Ser Leu Ala Gly Ala
   85 90 95
   Ala Ile Pro Ala Thr Ile Ala Ser Ala Ala Ile Gly Leu Gly Val Gln
   100 105 110
   Ser Ile Val Ala Asp Phe Leu Ala Gly Phe Phe Ile Leu Thr Glu Lys
   115 120 125
   Gln Phe Gly Val Gly Asp Trp Val Arg Phe Glu Gly Asn Gly Ile Val
   130 135 140
   Val Glu Gly Thr Val Ile Glu Ile Thr Met Arg Ala Thr Lys Ile Arg
   145 150 155 160
   Thr Ile Ala Gln Glu Thr Val Ile Ile Pro Asn Ser Thr Ala Lys Val
   165 170 175
   Cys Ile Asn Asn Ser Asn Asn Trp Ser Arg Ala Val Val Val Ile Pro
   180 185 190
   Ile Pro Met Leu Gly Ser Glu Asn Ile Thr Asp Val Ile Ala Arg Ser
   195 200 205
   Glu Ala Ala Thr Arg Arg Ala Leu Gly Gln Glu Lys Ile Ala Pro Glu
   210 215 220
   Ile Leu Gly Glu Leu Asp Val His Pro Ala Thr Glu Val Thr Pro Pro
   225 230 235 240
   Thr Val Val Gly Met Pro Trp Met Val Thr Met Arg Phe Leu Val Gln
   245 250 255
   Val Thr Ala Gly Asn Gln Trp Leu Val Glu Arg Ala Ile Arg Thr Glu
   260 265 270
   Ile Ile Asn Glu Phe Trp Glu Glu Tyr Gly Ser Ala Thr Thr Thr Ser
   275 280 285
   Gly Thr Leu Ile Asp Ser Leu His Val Glu His Glu Glu Pro Lys Thr
   290 295 300
   Ser Leu Ile Asp Ala Ser Pro Gln Ala Leu Lys Glu Pro Lys Pro Glu
   305 310 315 320
   Ala Ala Ala Thr Val Ala Ser Leu Ala Ala Ser Ser Asn Asp Asp Ala
   325 330 335
   Asp Asn Ala Asp Ala Ser Ala Ile Asn Ala Gly Asn Pro Glu Lys Glu
   340 345 350
   Leu Asp Ser Asp Val Leu Glu Gln Glu Leu Ser Ser Glu Glu Pro Glu
   355 360 365
   Glu Thr Ala Lys Pro Asp His Ser Leu Arg Gly Phe Phe Arg Thr Asp
   370 375 380
   Tyr Tyr Pro Asn Arg Trp Gln Lys Ile Leu Ser Phe Gly Gly Arg Val
   385 390 395 400
   Arg Met Ser Thr Ser Leu Leu Leu Gly Ala Leu Leu Leu Leu Ser Leu
   405 410 415
   Phe Lys Val Met Thr Val Glu Pro Ser Glu Asn Trp Gln Asn Ser Ser
   420 425 430
   Gly Trp Leu Ser Pro Ser Thr Ala Thr Ser Thr Ala Val Thr Thr Ser
   435 440 445
   Glu Thr Ser Ala Pro Ala Ser Thr Pro Ser Met Thr Val Pro Thr Thr
   450 455 460
   Val Glu Glu Thr Pro Thr Met Glu Ser Ser Val Glu Thr Gln Gln Glu
   465 470 475 480
   Thr Ser Thr Pro Ala Thr Ala Thr Pro Gln Arg Ala Asp Thr Ile Glu
   485 490 495
   Pro Thr Glu Glu Ala Thr Ser Gln Glu Glu Thr Thr Ala Ser Gln Thr
   500 505 510
   Gln Ser Pro Ala Val Glu Ala Pro Thr Ala Val Gln Glu Thr Val Ala
   515 520 525
   Pro Thr Ser Thr Pro
   530
<210> 33
   <211> 20
   <212> DNA
   <213> Artificial DNA
<220>
   <223> yggB N-terminus primer
<400> 33
   ggctgttgag aagctgccac 20
<210> 34
   <211> 29
   <212> DNA
   <213> Artificial DNA
<220>
   <223> yggB N-terminus primer2
<400> 34
   ccgcaacaat cgactgcaca ccaagacca 29
<210> 35
   <211> 28
   <212> DNA
   <213> Artificial DNA
<220>
   <223> yggB C-terminus primer1
<400> 35
   gggagctcca cggcatgccg accaccgt 28
<210> 36
   <211> 29
   <212> DNA
   <213> Artificial DNA
<220>
   <223> yggB C-terminus primer2
<400> 36
   tggtcttggt gtgcagtcga ttgttgcgg 29
<210> 37
   <211> 28
   <212> DNA
   <213> Artificial DNA
<220>
   <223> yggB 2nd primer
<400> 37
   gggagctcga ctttctggct cctttact 28
<210> 38
   <211> 28
   <212> DNA
   <213> Artificial DNA
<220>
   <223> sucA8primer
<400> 38
   ctgcgtcgac gtcggaggca ccatccac 28
<210> 39
   <211> 30
   <212> DNA
   <213> Artificial DNA
<220>
   <223> sucA801primer
<400> 39
   ctgcgtctcg acgtcggagg caccatccac 30
<210> 40
   <211> 36
   <212> DNA
   <213> Artificial DNA
<220>
   <223> sucA805primer
<400> 40
   gctaaaagct gcgtcgacgt cggaggcacc atccac 36
<210> 41
   <211> 36
   <212> DNA
   <213> Artificial DNA
<220>
   <223> sucA77
<400> 41
   gctataagct gcgtcgacgt cggaggcacc atccac 36
<210> 42
   <211> 4389
   <212> DNA
   <213> Corynebacterium glutamicum
<220>
   <221> CDS
   <222> (443).. (2542)
   <223>
<400> 42
   gtcgacaagc aaaatcgaag cggcagcacg ccgcgtcgga gccttaaacg ccatcgccgc 60
   catccctgat ggtttcaatc atcaagtcgg tgaacgcggg cgcaacctgt catccggaca 120
   gcgccaactg atcgcgctgg cgcgcgccga actcatcgag ccttccatca tgcttctcga 180
   cgaagccacc tccaccctcg accccgccac cgaagccgtt atcctcaacg cctccgatcg 240
   agtcactaag ggacgcacca gcatcatcgt cgcgcaccgc ttggcaaccg ctaaaagggc 300
   cgaccgtatt cttgttgttg aacaaggacg tatcattgag gacggatctc acgacgcgtt    360
   gttgtctgct aacggcacct acgcccgcat gtggcattta atggcctgac acgttatttt    420
   taggagaact gtcaacaaat ta atg cta caa ctg ggg ctt agg cat aat cag     472
                            Met Leu Gln Leu Gly Leu Arg His Asn Gln
                            1               5                   10
   cca acg acc aac gtt aca gtg gat aaa ata aag ctc aat aaa ccc tca      520
   Pro Thr Thr Asn Val Thr Val Asp Lys Ile Lys Leu Asn Lys Pro Ser
                   15                  20                  25
   aga agc aag gaa aag agg cga gta cct gcc gtg agc agc gct agt act      568
   Arg Ser Lys Glu Lys Arg Arg Val Pro Ala Val Ser Ser Ala Ser Thr
               30                  35                  40
   ttc ggc cag aat gcg tgg ctg gta gac gag atg ttc cag cag ttc cag      616
   Phe Gly Gln Asn Ala Trp Leu Val Asp Glu Met Phe Gln Gln Phe Gln
           45                  50                  55
   aag gac ccc aag tcc gtg gac aag gaa tgg aga gaa ctc ttt gag gcg      664
   Lys Asp Pro Lys Ser Val Asp Lys Glu Trp Arg Glu Leu Phe Glu Ala
       60                  65                  70
   cag ggg gga cca aat gct acc ccc gct aca aca gaa gca cag cct tca      712
   Gln Gly Gly Pro Asn Ala Thr Pro Ala Thr Thr Glu Ala Gln Pro Ser
   75                  80                  85                  90
   gcg ccc aag gag tct gcg aaa cca gca cca aag gct gcc cct gca gcc      760
   Ala Pro Lys Glu Ser Ala Lys Pro Ala Pro Lys Ala Ala Pro Ala Ala
                   95                  100                 105
   aag gca gca ccg cgc gta gaa acc aag ccg gcc gcc aag acc gcc cct      808
   Lys Ala Ala Pro Arg Val Glu Thr Lys Pro Ala Ala Lys Thr Ala Pro
               110                 115                 120
   aag gcc aag gag tcc tca gtg cca cag caa cct aag ctt ccg gag cca      856
   Lys Ala Lys Glu Ser Ser Val Pro Gln Gln Pro Lys Leu Pro Glu Pro
           125                 130                 135
   gga caa acc cca atc agg ggt att ttc aag tcc atc gcg aag aac atg      904
   Gly Gln Thr Pro Ile Arg Gly Ile Phe Lys Ser Ile Ala Lys Asn Met
       140                 145                 150
   gat atc tcc ctg gaa atc cca acc gca acc tcg gtt cgc gat atg cca      952
   Asp Ile Ser Leu Glu Ile Pro Thr Ala Thr Ser Val Arg Asp Met Pro
   155                 160                 165                 170
   gct cgc ctc atg ttc gaa aac cgc gcg atg gtc aac gat cag ctc aag     1000
   Ala Arg Leu Met Phe Glu Asn Arg Ala Met Val Asn Asp Gln Leu Lys
                   175                 180                 185
   cgc acc cgc ggt ggc aag atc tcc ttc acc cac atc att ggc tac gcc     1048
   Arg Thr Arg Gly Gly Lys Ile Ser Phe Thr His Ile Ile Gly Tyr Ala
               190                 195                 200
   atg gtg aag gca gtc atg gct cac ccg gac atg aac aac tcc tac gac     1096
   Met Val Lys Ala Val Met Ala His Pro Asp Met Asn Asn Ser Tyr Asp
           205                 210                 215
   gtc atc gac ggc aag cca acc ctg atc gtg cct gag cac atc aac ctg     1144
   Val Ile Asp Gly Lys Pro Thr Leu Ile Val Pro Glu His Ile Asn Leu
       220                 225                 230
   ggc ctt gcc atc gac ctt cct cag aag gac ggc tcc cgc gca ctt gtc     1192
   Gly Leu Ala Ile Asp Leu Pro Gln Lys Asp Gly Ser Arg Ala Leu Val
   235 240 245 250
   gta gca gcc atc aag gaa acc gag aag atg aac ttc tcc gag ttc ctc 1240
   Val Ala Ala Ile Lys Glu Thr Glu Lys Met Asn Phe Ser Glu Phe Leu
   255 260 265
   gca gca tac gaa gac atc gtg aca cgc tcc cgc aag ggc aag ctc acc 1288
   Ala Ala Tyr Glu Asp Ile Val Thr Arg Ser Arg Lys Gly Lys Leu Thr
   270 275 280
   atg gat gac tac cag ggc gtt acc gtt tcc ttg acc aac cca ggt ggc 1336
   Met Asp Asp Tyr Gln Gly Val Thr Val Ser Leu Thr Asn Pro Gly Gly
   285 290 295
   atc ggt acc cgc cac tct gtc cca cgt ctg acc aag ggc cag ggc acc 1384
   Ile Gly Thr Arg His Ser Val Pro Arg Leu Thr Lys Gly Gln Gly Thr
   300 305 310
   atc atc ggt gtc ggt tcc atg gat tac cca gca gag ttc cag ggc gct 1432
   Ile Ile Gly Val Gly Ser Met Asp Tyr Pro Ala Glu Phe Gln Gly Ala
   315 320 325 330
   tcc gaa gac cgc ctt gca gag ctc ggc gtt gga aag ctt gtc acc atc 1480
   Ser Glu Asp Arg Leu Ala Glu Leu Gly Val Gly Lys Leu Val Thr Ile
   335 340 345
   acc tcc acc tac gat cac cgc gtg atc cag ggt gct gtg tcc ggt gaa 1528
   Thr Ser Thr Tyr Asp His Arg Val Ile Gln Gly Ala Val Ser Gly Glu
   350 355 360
   ttc ctg cgt acc atg tct cgc ctg ctc acc gat gat tcc ttc tgg gat 1576
   Phe Leu Arg Thr Met Ser Arg Leu Leu Thr Asp Asp Ser Phe Trp Asp
   365 370 375
   gag atc ttc gac gca atg aac gtt cct tac acc cca atg cgt tgg gca 1624
   Glu Ile Phe Asp Ala Met Asn Val Pro Tyr Thr Pro Met Arg Trp Ala
   380 385 390
   cag gac gtt cca aac acc ggt gtt gat aag aac acc cgc gtc atg cag 1672
   Gln Asp Val Pro Asn Thr Gly Val Asp Lys Asn Thr Arg Val Met Gln
   395 400 405 410
   ctc att gag gca tac cgc tcc cgt gga cac ctc atc gct gac acc aac 1720
   Leu Ile Glu Ala Tyr Arg Ser Arg Gly His Leu Ile Ala Asp Thr Asn
   415 420 425
   cca ctt tca tgg gtt cag cct ggc atg cca gtt cca gac cac cgc gac 1768
   Pro Leu Ser Trp Val Gln Pro Gly Met Pro Val Pro Asp His Arg Asp
   430 435 440
   ctc gac atc gag acc cac agc ctg acc atc tgg gat ctg gac cgt acc 1816
   Leu Asp Ile Glu Thr His Ser Leu Thr Ile Trp Asp Leu Asp Arg Thr
   445 450 455
   ttc agc gtc ggt ggc ttc ggc ggc aag gag acc atg acc ctg cgc gag 1864
   Phe Ser Val Gly Gly Phe Gly Gly Lys Glu Thr Met Thr Leu Arg Glu
   460 465 470
   gta ctg tcc cgc ctg cgc gct gcc tac acc ttg aag gtc ggc tcc gaa 1912
   Val Leu Ser Arg Leu Arg Ala Ala Tyr Thr Leu Lys Val Gly Ser Glu
   475 480 485 490
   tac acc cac atc ctg gac cgc gac gag cgc acc tgg ctg cag gac cgc 1960
   Tyr Thr His Ile Leu Asp Arg Asp Glu Arg Thr Trp Leu Gln Asp Arg
   495 500 505
   ctc gaa gcc gga atg cca aag cca acc cag gca gag cag aag tac atc 2008
   Leu Glu Ala Gly Met Pro Lys Pro Thr Gln Ala Glu Gln Lys Tyr Ile
   510 515 520
   ctg cag aag ctg aac gcc gca gag gct ttc gag aac ttc ctg cag acc 2056
   Leu Gln Lys Leu Asn Ala Ala Glu Ala Phe Glu Asn Phe Leu Gln Thr
   525 530 535
   aag tac gtc ggc cag aag cgc ttc tcc ctc gaa ggt gca gaa gct ctc 2104
   Lys Tyr Val Gly Gln Lys Arg Phe Ser Leu Glu Gly Ala Glu Ala Leu
   540 545 550
   atc cca ctg atg gac tcc gcc atc gac acc gcc gca ggc cag ggc ctc 2152
   Ile Pro Leu Met Asp Ser Ala Ile Asp Thr Ala Ala Gly Gln Gly Leu
   555 560 565 570
   gac gaa gtt gtc atc ggt atg cca cac cgt ggt cgc ctc aac gtg ctg 2200
   Asp Glu Val Val Ile Gly Met Pro His Arg Gly Arg Leu Asn Val Leu
   575 580 585
   ttc aac atc gtg ggc aag cca ctg gca tcc atc ttc aac gag ttt gaa 2248
   Phe Asn Ile Val Gly Lys Pro Leu Ala Ser Ile Phe Asn Glu Phe Glu
   590 595 600
   ggc caa atg gag cag ggc cag atc ggt ggc tcc ggt gac gtg aag tac 2296
   Gly Gln Met Glu Gln Gly Gln Ile Gly Gly Ser Gly Asp Val Lys Tyr
   605 610 615
   cac ctc ggt tcc gaa ggc cag cac ctg cag atg ttc ggc gac ggc gag 2344
   His Leu Gly Ser Glu Gly Gln His Leu Gln Met Phe Gly Asp Gly Glu
   620 625 630
   atc aag gtc tcc ctg act gct aac ccg tcc cac ctg gaa gct gtt aac 2392
   Ile Lys Val Ser Leu Thr Ala Asn Pro Ser His Leu Glu Ala Val Asn
   635 640 645 650
   cca gtg atg gaa ggt atc gtc cgc gca aag cag gac tac ctg gac aag 2440
   Pro Val Met Glu Gly Ile Val Arg Ala Lys Gln Asp Tyr Leu Asp Lys
   655 660 665
   ggc gta gac ggc aag act gtt gtg cca ctg ctg ctc cac ggt gac gct 2488
   Gly Val Asp Gly Lys Thr Val Val Pro Leu Leu Leu His Gly Asp Ala
   670 675 680
   gca ttc gca ggc ctg ggc atc gtg cca gaa acc atc aac ctg gct aag 2536
   Ala Phe Ala Gly Leu Gly Ile Val Pro Glu Thr Ile Asn Leu Ala Lys
   685 690 695
   ctg cgt cgacgtcgga ggcaccatcc acatcgtggt gaacaaccag atcggcttca 2592
   Leu Arg
   700
   ccaccacccc agactccagc cgctccatgc actacgcaac cgactacgcc aaggcattcg 2652
   gctgcccagt cttccacgtc aatggtgatg acccagaggc agttgtctgg gttggccagc 2712
   tggcaaccga gtaccgtcgt cgcttcggca aggacgtctt catcgacctc gtttgctacc 2772
   gcctccgcgg ccacaacgaa gctgatgatc cttccatgac ccagccaaag atgtatgagc 2832
   tcatcaccgg ccgcgagacc gttcgtgctc agtacaccga agacctgctc ggacgtggag 2892
   acctctccaa cgaagatgca gaagcagtcg tccgcgactt ccacgaccag atggaatctg 2952
   tgttcaacga agtcaaggaa ggcggcaaga agcaggctga ggcacagacc ggcatcaccg 3012
   gctcccagaa gcttccacac ggccttgaga ccaacatctc ccgtgaagag ctcctggaac 3072
   tgggacaggc tttcgccaac accccagaag gcttcaacta ccacccacgt gtggctccag 3132
   ttgctaagaa gcgcgtctcc tctgtcaccg aaggtggcat cgactgggca tggggcgagc 3192
   tcctcgcctt cggttccctg gctaactccg gccgcttggt tcgccttgca ggtgaagatt 3252
   cccgccgcgg taccttcacc cagcgccacg cagttgccat cgacccagcg accgctgaag 3312
   agttcaaccc actccacgag cttgcacagt ccaagggcaa caacggtaag ttcctggtct 3372
   acaactccgc actgaccgag tacgcaggca tgggcttcga gtacggctac tccgtaggaa 3432
   acgaagactc cgtcgttgca tgggaagcac agttcggcga cttcgccaac ggcgctcaga 3492
   ccatcatcga tgagtacgtc tcctcaggcg aagctaagtg gggccagacc tccaagctga 3552
   tccttctgct gcctcacggc tacgaaggcc agggcccaga ccactcttcc gcacgtatcg 3612
   agcgcttcct gcagctgtgc gctgagggtt ccatgactgt tgctcagcca tccaccccag 3672
   caaaccactt ccacctgctg cgtcgtcacg ctctgtccga cctgaagcgt ccactggtta 3732
   tcttcacccc gaagtccatg ctgcgtaaca aggctgctgc ctccgcacca gaagacttca 3792
   ctgaggtcac caagttccaa tccgtgatcg acgatccaaa cgttgcagat gcagccaagg 3852
   tgaagaaggt catgctggtc tccggcaagc tgtactacga attggcaaag cgcaaggaga 3912
   aggacggacg cgacgacatc gcgatcgttc gtatcgaaat gctccaccca attccgttca 3972
   accgcatctc cgaggctctt gccggctacc ctaacgctga ggaagtcctc ttcgttcagg 4032
   atgagccagc aaaccagggc ccatggccgt tctaccagga gcacctccca gagctgatcc 4092
   cgaacatgcc aaagatgcgc cgcgtttccc gccgcgctca gtcctccacc gcaactggtg 4152
   ttgctaaggt gcaccagctg gaggagaagc agcttatcga cgaggctttc gaggcttaag 4212
   tctttatagt cctgcactag cctagagggc cttatgcagt gtgaatcaca cagcataagg 4272
   ccctttttgc tgccgtggtt gcctaaggtg gaaggcatga aacgaatctg tgcggtcacg 4332
   atctcttcag tacttttgct aagtggctgc tcctccactt ccaccacgca gctcgag 4389
<210> 43
   <211> 700
   <212> PRT
   <213> Corynebacterium glutamicum
<400> 43
   Met Leu Gln Leu Gly Leu Arg His Asn Gln Pro Thr Thr Asn Val Thr
   1 5 10 15
   Val Asp Lys Ile Lys Leu Asn Lys Pro Ser Arg Ser Lys Glu Lys Arg
   20 25 30
   Arg Val Pro Ala Val Ser Ser Ala Ser Thr Phe Gly Gln Asn Ala Trp
   35 40 45
   Leu Val Asp Glu Met Phe Gln Gln Phe Gln Lys Asp Pro Lys Ser Val
   50 55 60
   Asp Lys Glu Trp Arg Glu Leu Phe Glu Ala Gln Gly Gly Pro Asn Ala
   65 70 75 80
   Thr Pro Ala Thr Thr Glu Ala Gln Pro Ser Ala Pro Lys Glu Ser Ala
   85 90 95
   Lys Pro Ala Pro Lys Ala Ala Pro Ala Ala Lys Ala Ala Pro Arg Val
   100 105 110
   Glu Thr Lys Pro Ala Ala Lys Thr Ala Pro Lys Ala Lys Glu Ser Ser
   115 120 125
   Val Pro Gln Gln Pro Lys Leu Pro Glu Pro Gly Gln Thr Pro Ile Arg
   130 135 140
   Gly Ile Phe Lys Ser Ile Ala Lys Asn Met Asp Ile Ser Leu Glu Ile
   145 150 155 160
   Pro Thr Ala Thr Ser Val Arg Asp Met Pro Ala Arg Leu Met Phe Glu
   165 170 175
   Asn Arg Ala Met Val Asn Asp Gln Leu Lys Arg Thr Arg Gly Gly Lys
   180 185 190
   Ile Ser Phe Thr His Ile Ile Gly Tyr Ala Met Val Lys Ala Val Met
   195 200 205
   Ala His Pro Asp Met Asn Asn Ser Tyr Asp Val Ile Asp Gly Lys Pro
   210 215 220
   Thr Leu Ile Val Pro Glu His Ile Asn Leu Gly Leu Ala Ile Asp Leu
   225 230 235 240
   Pro Gln Lys Asp Gly Ser Arg Ala Leu Val Val Ala Ala Ile Lys Glu
   245 250 255
   Thr Glu Lys Met Asn Phe Ser Glu Phe Leu Ala Ala Tyr Glu Asp Ile
   260 265 270
   Val Thr Arg Ser Arg Lys Gly Lys Leu Thr Met Asp Asp Tyr Gln Gly
   275 280 285
   Val Thr Val Ser Leu Thr Asn Pro Gly Gly Ile Gly Thr Arg His Ser
   290 295 300
   Val Pro Arg Leu Thr Lys Gly Gln Gly Thr Ile Ile Gly Val Gly Ser
   305 310 315 320
   Met Asp Tyr Pro Ala Glu Phe Gln Gly Ala Ser Glu Asp Arg Leu Ala
   325 330 335
   Glu Leu Gly Val Gly Lys Leu Val Thr Ile Thr Ser Thr Tyr Asp His
   340 345 350
   Arg Val Ile Gln Gly Ala Val Ser Gly Glu Phe Leu Arg Thr Met Ser
   355 360 365
   Arg Leu Leu Thr Asp Asp Ser Phe Trp Asp Glu Ile Phe Asp Ala Met
   370 375 380
   Asn Val Pro Tyr Thr Pro Met Arg Trp Ala Gln Asp Val Pro Asn Thr
   385 390 395 400
   Gly Val Asp Lys Asn Thr Arg Val Met Gln Leu Ile Glu Ala Tyr Arg
   405 410 415
   Ser Arg Gly His Leu Ile Ala Asp Thr Asn Pro Leu Ser Trp Val Gln
   420 425 430
   Pro Gly Met Pro Val Pro Asp His Arg Asp Leu Asp Ile Glu Thr His
   435 440 445
   Ser Leu Thr Ile Trp Asp Leu Asp Arg Thr Phe Ser Val Gly Gly Phe
   450 455 460
   Gly Gly Lys Glu Thr Met Thr Leu Arg Glu Val Leu Ser Arg Leu Arg
   465 470 475 480
   Ala Ala Tyr Thr Leu Lys Val Gly Ser Glu Tyr Thr His Ile Leu Asp
   485 490 495
   Arg Asp Glu Arg Thr Trp Leu Gln Asp Arg Leu Glu Ala Gly Met Pro
   500 505 510
   Lys Pro Thr Gln Ala Glu Gln Lys Tyr Ile Leu Gln Lys Leu Asn Ala
   515 520 525
   Ala Glu Ala Phe Glu Asn Phe Leu Gln Thr Lys Tyr Val Gly Gln Lys
   530 535 540
   Arg Phe Ser Leu Glu Gly Ala Glu Ala Leu Ile Pro Leu Met Asp Ser
   545 550 555 560
   Ala Ile Asp Thr Ala Ala Gly Gln Gly Leu Asp Glu Val Val Ile Gly
   565 570 575
   Met Pro His Arg Gly Arg Leu Asn Val Leu Phe Asn Ile Val Gly Lys
   580 585 590
   Pro Leu Ala Ser Ile Phe Asn Glu Phe Glu Gly Gln Met Glu Gln Gly
   595 600 605
   Gln Ile Gly Gly Ser Gly Asp Val Lys Tyr His Leu Gly Ser Glu Gly
   610 615 620
   Gln His Leu Gln Met Phe Gly Asp Gly Glu Ile Lys Val Ser Leu Thr
   625 630 635 640
   Ala Asn Pro Ser His Leu Glu Ala Val Asn Pro Val Met Glu Gly Ile
   645 650 655
   Val Arg Ala Lys Gln Asp Tyr Leu Asp Lys Gly Val Asp Gly Lys Thr
   660 665 670
   Val Val Pro Leu Leu Leu His Gly Asp Ala Ala Phe Ala Gly Leu Gly
   675 680 685
   Ile Val Pro Glu Thr Ile Asn Leu Ala Lys Leu Arg
   690 695 700
<210> 44
   <211> 4391
   <212> DNA
   <213> Corynebacterium glutamicum
<220>
   <221> CDS
   <222> (443).. (4210)
   <223> sucA801
<400> 44
   gtcgacaagc aaaatcgaag cggcagcacg ccgcgtcgga gccttaaacg ccatcgccgc 60
   catccctgat ggtttcaatc atcaagtcgg tgaacgcggg cgcaacctgt catccggaca 120
   gcgccaactg atcgcgctgg cgcgcgccga actcatcgag ccttccatca tgcttctcga 180
   cgaagccacc tccaccctcg accccgccac cgaagccgtt atcctcaacg cctccgatcg 240
   agtcactaag ggacgcacca gcatcatcgt cgcgcaccgc ttggcaaccg ctaaaagggc 300
   cgaccgtatt cttgttgttg aacaaggacg tatcattgag gacggatctc acgacgcgtt 360
   gttgtctgct aacggcacct acgcccgcat gtggcattta atggcctgac acgttatttt 420
   taggagaact gtcaacaaat ta atg cta caa ctg ggg ctt agg cat aat cag 472
   Met Leu Gln Leu Gly Leu Arg His Asn Gln
   1 5 10
   cca acg acc aac gtt aca gtg gat aaa ata aag ctc aat aaa ccc tca 520
   Pro Thr Thr Asn Val Thr Val Asp Lys Ile Lys Leu Asn Lys Pro Ser
   15 20 25
   aga agc aag gaa aag agg cga gta cct gcc gtg agc agc gct agt act 568
   Arg Ser Lys Glu Lys Arg Arg Val Pro Ala Val Ser Ser Ala Ser Thr
   30 35 40
   ttc ggc cag aat gcg tgg ctg gta gac gag atg ttc cag cag ttc cag 616
   Phe Gly Gln Asn Ala Trp Leu Val Asp Glu Met Phe Gln Gln Phe Gln
   45 50 55
   aag gac ccc aag tcc gtg gac aag gaa tgg aga gaa ctc ttt gag gcg 664
   Lys Asp Pro Lys Ser Val Asp Lys Glu Trp Arg Glu Leu Phe Glu Ala
   60 65 70
   cag ggg gga cca aat gct acc ccc gct aca aca gaa gca cag cct tca 712
   Gln Gly Gly Pro Asn Ala Thr Pro Ala Thr Thr Glu Ala Gln Pro Ser
   75 80 85 90
   gcg ccc aag gag tct gcg aaa cca gca cca aag gct gcc cct gca gcc 760
   Ala Pro Lys Glu Ser Ala Lys Pro Ala Pro Lys Ala Ala Pro Ala Ala
   95 100 105
   aag gca gca ccg cgc gta gaa acc aag ccg gcc gcc aag acc gcc cct 808
   Lys Ala Ala Pro Arg Val Glu Thr Lys Pro Ala Ala Lys Thr Ala Pro
   110 115 120
   aag gcc aag gag tcc tca gtg cca cag caa cct aag ctt ccg gag cca 856
   Lys Ala Lys Glu Ser Ser Val Pro Gln Gln Pro Lys Leu Pro Glu Pro
   125 130 135
   gga caa acc cca atc agg ggt att ttc aag tcc atc gcg aag aac atg 904
   Gly Gln Thr Pro Ile Arg Gly Ile Phe Lys Ser Ile Ala Lys Asn Met
   140 145 150
   gat atc tcc ctg gaa atc cca acc gca acc tcg gtt cgc gat atg cca 952
   Asp Ile Ser Leu Glu Ile Pro Thr Ala Thr Ser Val Arg Asp Met Pro
   155 160 165 170
   gct cgc ctc atg ttc gaa aac cgc gcg atg gtc aac gat cag ctc aag 1000
   Ala Arg Leu Met Phe Glu Asn Arg Ala Met Val Asn Asp Gln Leu Lys
   175 180 185
   cgc acc cgc ggt ggc aag atc tcc ttc acc cac atc att ggc tac gcc 1048
   Arg Thr Arg Gly Gly Lys Ile Ser Phe Thr His Ile Ile Gly Tyr Ala
   190 195 200
   atg gtg aag gca gtc atg gct cac ccg gac atg aac aac tcc tac gac 1096
   Met Val Lys Ala Val Met Ala His Pro Asp Met Asn Asn Ser Tyr Asp
   205 210 215
   gtc atc gac ggc aag cca acc ctg atc gtg cct gag cac atc aac ctg 1144
   Val Ile Asp Gly Lys Pro Thr Leu Ile Val Pro Glu His Ile Asn Leu
   220 225 230
   ggc ctt gcc atc gac ctt cct cag aag gac ggc tcc cgc gca ctt gtc 1192
   Gly Leu Ala Ile Asp Leu Pro Gln Lys Asp Gly Ser Arg Ala Leu Val
   235 240 245 250
   gta gca gcc atc aag gaa acc gag aag atg aac ttc tcc gag ttc ctc 1240
   Val Ala Ala Ile Lys Glu Thr Glu Lys Met Asn Phe Ser Glu Phe Leu
   255 260 265
   gca gca tac gaa gac atc gtg aca cgc tcc cgc aag ggc aag ctc acc 1288
   Ala Ala Tyr Glu Asp Ile Val Thr Arg Ser Arg Lys Gly Lys Leu Thr
   270 275 280
   atg gat gac tac cag ggc gtt acc gtt tcc ttg acc aac cca ggt ggc 1336
   Met Asp Asp Tyr Gln Gly Val Thr Val Ser Leu Thr Asn Pro Gly Gly
   285 290 295
   atc ggt acc cgc cac tct gtc cca cgt ctg acc aag ggc cag ggc acc 1384
   Ile Gly Thr Arg His Ser Val Pro Arg Leu Thr Lys Gly Gln Gly Thr
   300 305 310
   atc atc ggt gtc ggt tcc atg gat tac cca gca gag ttc cag ggc gct 1432
   Ile Ile Gly Val Gly Ser Met Asp Tyr Pro Ala Glu Phe Gln Gly Ala
   315 320 325 330
   tcc gaa gac cgc ctt gca gag ctc ggc gtt gga aag ctt gtc acc atc 1480
   Ser Glu Asp Arg Leu Ala Glu Leu Gly Val Gly Lys Leu Val Thr Ile
   335 340 345
   acc tcc acc tac gat cac cgc gtg atc cag ggt gct gtg tcc ggt gaa 1528
   Thr Ser Thr Tyr Asp His Arg Val Ile Gln Gly Ala Val Ser Gly Glu
   350 355 360
   ttc ctg cgt acc atg tct cgc ctg ctc acc gat gat tcc ttc tgg gat 1576
   Phe Leu Arg Thr Met Ser Arg Leu Leu Thr Asp Asp Ser Phe Trp Asp
   365 370 375
   gag atc ttc gac gca atg aac gtt cct tac acc cca atg cgt tgg gca 1624
   Glu Ile Phe Asp Ala Met Asn Val Pro Tyr Thr Pro Met Arg Trp Ala
   380 385 390
   cag gac gtt cca aac acc ggt gtt gat aag aac acc cgc gtc atg cag 1672
   Gln Asp Val Pro Asn Thr Gly Val Asp Lys Asn Thr Arg Val Met Gln
   395 400 405 410
   ctc att gag gca tac cgc tcc cgt gga cac ctc atc gct gac acc aac 1720
   Leu Ile Glu Ala Tyr Arg Ser Arg Gly His Leu Ile Ala Asp Thr Asn
   415 420 425
   cca ctt tca tgg gtt cag cct ggc atg cca gtt cca gac cac cgc gac 1768
   Pro Leu Ser Trp Val Gln Pro Gly Met Pro Val Pro Asp His Arg Asp
   430 435 440
   ctc gac atc gag acc cac agc ctg acc atc tgg gat ctg gac cgt acc 1816
   Leu Asp Ile Glu Thr His Ser Leu Thr Ile Trp Asp Leu Asp Arg Thr
   445 450 455
   ttc agc gtc ggt ggc ttc ggc ggc aag gag acc atg acc ctg cgc gag 1864
   Phe Ser Val Gly Gly Phe Gly Gly Lys Glu Thr Met Thr Leu Arg Glu
   460 465 470
   gta ctg tcc cgc ctg cgc gct gcc tac acc ttg aag gtc ggc tcc gaa 1912
   Val Leu Ser Arg Leu Arg Ala Ala Tyr Thr Leu Lys Val Gly Ser Glu
   475 480 485 490
   tac acc cac atc ctg gac cgc gac gag cgc acc tgg ctg cag gac cgc 1960
   Tyr Thr His Ile Leu Asp Arg Asp Glu Arg Thr Trp Leu Gln Asp Arg
   495 500 505
   ctc gaa gcc gga atg cca aag cca acc cag gca gag cag aag tac atc 2008
   Leu Glu Ala Gly Met Pro Lys Pro Thr Gln Ala Glu Gln Lys Tyr Ile
   510 515 520
   ctg cag aag ctg aac gcc gca gag gct ttc gag aac ttc ctg cag acc 2056
   Leu Gln Lys Leu Asn Ala Ala Glu Ala Phe Glu Asn Phe Leu Gln Thr
   525 530 535
   aag tac gtc ggc cag aag cgc ttc tcc ctc gaa ggt gca gaa gct ctc 2104
   Lys Tyr Val Gly Gln Lys Arg Phe Ser Leu Glu Gly Ala Glu Ala Leu
   540 545 550
   atc cca ctg atg gac tcc gcc atc gac acc gcc gca ggc cag ggc ctc 2152
   Ile Pro Leu Met Asp Ser Ala Ile Asp Thr Ala Ala Gly Gln Gly Leu
   555 560 565 570
   gac gaa gtt gtc atc ggt atg cca cac cgt ggt cgc ctc aac gtg ctg 2200
   Asp Glu Val Val Ile Gly Met Pro His Arg Gly Arg Leu Asn Val Leu
   575 580 585
   ttc aac atc gtg ggc aag cca ctg gca tcc atc ttc aac gag ttt gaa 2248
   Phe Asn Ile Val Gly Lys Pro Leu Ala Ser Ile Phe Asn Glu Phe Glu
   590 595 600
   ggc caa atg gag cag ggc cag atc ggt ggc tcc ggt gac gtg aag tac 2296
   Gly Gln Met Glu Gln Gly Gln Ile Gly Gly Ser Gly Asp Val Lys Tyr
   605 610 615
   cac ctc ggt tcc gaa ggc cag cac ctg cag atg ttc ggc gac ggc gag 2344
   His Leu Gly Ser Glu Gly Gln His Leu Gln Met Phe Gly Asp Gly Glu
   620 625 630
   atc aag gtc tcc ctg act gct aac ccg tcc cac ctg gaa gct gtt aac 2392
   Ile Lys Val Ser Leu Thr Ala Asn Pro Ser His Leu Glu Ala Val Asn
   635 640 645 650
   cca gtg atg gaa ggt atc gtc cgc gca aag cag gac tac ctg gac aag 2440
   Pro Val Met Glu Gly Ile Val Arg Ala Lys Gln Asp Tyr Leu Asp Lys
   655 660 665
   ggc gta gac ggc aag act gtt gtg cca ctg ctg ctc cac ggt gac gct 2488
   Gly Val Asp Gly Lys Thr Val Val Pro Leu Leu Leu His Gly Asp Ala
   670 675 680
   gca ttc gca ggc ctg ggc atc gtg cca gaa acc atc aac ctg gct aag 2536
   Ala Phe Ala Gly Leu Gly Ile Val Pro Glu Thr Ile Asn Leu Ala Lys
   685 690 695
   ctg cgt ctc gac gtc gga ggc acc atc cac atc gtg gtg aac aac cag 2584
   Leu Arg Leu Asp Val Gly Gly Thr Ile His Ile Val Val Asn Asn Gln
   700 705 710
   atc ggc ttc acc acc acc cca gac tcc agc cgc tcc atg cac tac gca 2632
   Ile Gly Phe Thr Thr Thr Pro Asp Ser Ser Arg Ser Met His Tyr Ala
   715 720 725 730
   acc gac tac gcc aag gca ttc ggc tgc cca gtc ttc cac gtc aat ggt 2680
   Thr Asp Tyr Ala Lys Ala Phe Gly Cys Pro Val Phe His Val Asn Gly
   735 740 745
   gat gac cca gag gca gtt gtc tgg gtt ggc cag ctg gca acc gag tac 2728
   Asp Asp Pro Glu Ala Val Val Trp Val Gly Gln Leu Ala Thr Glu Tyr
   750 755 760
   cgt cgt cgc ttc ggc aag gac gtc ttc atc gac ctc gtt tgc tac cgc 2776
   Arg Arg Arg Phe Gly Lys Asp Val Phe Ile Asp Leu Val Cys Tyr Arg
   765 770 775
   ctc cgc ggc cac aac gaa gct gat gat cct tcc atg acc cag cca aag 2824
   Leu Arg Gly His Asn Glu Ala Asp Asp Pro Ser Met Thr Gln Pro Lys
   780 785 790
   atg tat gag ctc atc acc ggc cgc gag acc gtt cgt gct cag tac acc 2872
   Met Tyr Glu Leu Ile Thr Gly Arg Glu Thr Val Arg Ala Gln Tyr Thr
   795 800 805 810
   gaa gac ctg ctc gga cgt gga gac ctc tcc aac gaa gat gca gaa gca 2920
   Glu Asp Leu Leu Gly Arg Gly Asp Leu Ser Asn Glu Asp Ala Glu Ala
   815 820 825
   gtc gtc cgc gac ttc cac gac cag atg gaa tct gtg ttc aac gaa gtc 2968
   Val Val Arg Asp Phe His Asp Gln Met Glu Ser Val Phe Asn Glu Val
   830 835 840
   aag gaa ggc ggc aag aag cag gct gag gca cag acc ggc atc acc ggc 3016
   Lys Glu Gly Gly Lys Lys Gln Ala Glu Ala Gln Thr Gly Ile Thr Gly
   845 850 855
   tcc cag aag ctt cca cac ggc ctt gag acc aac atc tcc cgt gaa gag 3064
   Ser Gln Lys Leu Pro His Gly Leu Glu Thr Asn Ile Ser Arg Glu Glu
   860 865 870
   ctc ctg gaa ctg gga cag gct ttc gcc aac acc cca gaa ggc ttc aac 3112
   Leu Leu Glu Leu Gly Gln Ala Phe Ala Asn Thr Pro Glu Gly Phe Asn
   875 880 885 890
   tac cac cca cgt gtg gct cca gtt gct aag aag cgc gtc tcc tct gtc 3160
   Tyr His Pro Arg Val Ala Pro Val Ala Lys Lys Arg Val Ser Ser Val
   895 900 905
   acc gaa ggt ggc atc gac tgg gca tgg ggc gag ctc ctc gcc ttc ggt 3208
   Thr Glu Gly Gly Ile Asp Trp Ala Trp Gly Glu Leu Leu Ala Phe Gly
   910 915 920
   tcc ctg gct aac tcc ggc cgc ttg gtt cgc ctt gca ggt gaa gat tcc 3256
   Ser Leu Ala Asn Ser Gly Arg Leu Val Arg Leu Ala Gly Glu Asp Ser
   925 930 935
   cgc cgc ggt acc ttc acc cag cgc cac gca gtt gcc atc gac cca gcg 3304
   Arg Arg Gly Thr Phe Thr Gln Arg His Ala Val Ala Ile Asp Pro Ala
   940 945 950
   acc gct gaa gag ttc aac cca ctc cac gag ctt gca cag tcc aag ggc 3352
   Thr Ala Glu Glu Phe Asn Pro Leu His Glu Leu Ala Gln Ser Lys Gly
   955 960 965 970
   aac aac ggt aag ttc ctg gtc tac aac tcc gca ctg acc gag tac gca 3400
   Asn Asn Gly Lys Phe Leu Val Tyr Asn Ser Ala Leu Thr Glu Tyr Ala
   975 980 985
   ggc atg ggc ttc gag tac ggc tac tcc gta gga aac gaa gac tcc gtc 3448
   Gly Met Gly Phe Glu Tyr Gly Tyr Ser Val Gly Asn Glu Asp Ser Val
   990 995 1000
   gtt gca tgg gaa gca cag ttc ggc gac ttc gcc aac ggc gct cag 3493
   Val Ala Trp Glu Ala Gln Phe Gly Asp Phe Ala Asn Gly Ala Gln
   1005 1010 1015
   acc atc atc gat gag tac gtc tcc tca ggc gaa gct aag tgg ggc 3538
   Thr Ile Ile Asp Glu Tyr Val Ser Ser Gly Glu Ala Lys Trp Gly
   1020 1025 1030
   cag acc tcc aag ctg atc ctt ctg ctg cct cac ggc tac gaa ggc 3583
   Gln Thr Ser Lys Leu Ile Leu Leu Leu Pro His Gly Tyr Glu Gly
   1035 1040 1045
   cag ggc cca gac cac tct tcc gca cgt atc gag cgc ttc ctg cag 3628
   Gln Gly Pro  Asp His Ser Ser Ala  Arg Ile Glu Arg Phe  Leu Gln
           1050                 1055                 1060
   ctg tgc gct  gag ggt tcc atg act  gtt gct cag cca tcc  acc cca      3673
   Leu Cys Ala  Glu Gly Ser Met Thr  Val Ala Gln Pro Ser  Thr Pro
           1065                 1070                 1075
   gca aac cac  ttc cac ctg ctg cgt  cgt cac gct ctg tcc  gac ctg      3718
   Ala Asn His  Phe His Leu Leu Arg  Arg His Ala Leu Ser  Asp Leu
           1080                 1085                 1090
   aag cgt cca  ctg gtt atc ttc acc  ccg aag tcc atg ctg  cgt aac      3763
   Lys Arg Pro  Leu Val Ile Phe Thr  Pro Lys Ser Met Leu  Arg Asn
           1095                 1100                 1105
   aag gct gct  gcc tcc gca cca gaa  gac ttc act gag gtc  acc aag      3808
   Lys Ala Ala  Ala Ser Ala Pro Glu  Asp Phe Thr Glu Val  Thr Lys
           1110                 1115                 1120
   ttc caa tcc  gtg atc gac gat cca  aac gtt gca gat gca  gcc aag      3853
   Phe Gln Ser  Val Ile Asp Asp Pro  Asn Val Ala Asp Ala  Ala Lys
           1125                 1130                 1135
   gtg aag aag  gtc atg ctg gtc tcc  ggc aag ctg tac tac  gaa ttg      3898
   Val Lys Lys  Val Met Leu Val Ser  Gly Lys Leu Tyr Tyr  Glu Leu
           1140                 1145                 1150
   gca aag cgc  aag gag aag gac gga  cgc gac gac atc gcg  atc gtt      3943
   Ala Lys Arg  Lys Glu Lys Asp Gly  Arg Asp Asp Ile Ala  Ile Val
           1155                 1160                 1165
   cgt atc gaa  atg ctc cac cca att  ccg ttc aac cgc atc  tcc gag      3988
   Arg Ile Glu  Met Leu His Pro Ile  Pro Phe Asn Arg Ile  Ser Glu
           1170                 1175                 1180
   gct ctt gcc  ggc tac cct aac gct  gag gaa gtc ctc ttc  gtt cag      4033
   Ala Leu Ala  Gly Tyr Pro Asn Ala  Glu Glu Val Leu Phe  Val Gln
           1185                 1190                 1195
   gat gag cca  gca aac cag ggc cca  tgg ccg ttc tac cag  gag cac      4078
   Asp Glu Pro  Ala Asn Gln Gly Pro  Trp Pro Phe Tyr Gln  Glu His
           1200                 1205                 1210
   ctc cca gag  ctg atc ccg aac atg  cca aag atg cgc cgc  gtt tcc      4123
   Leu Pro Glu  Leu Ile Pro Asn Met  Pro Lys Met Arg Arg  Val Ser
           1215                 1220                 1225
   cgc cgc gct  cag tcc tcc acc gca  act ggt gtt gct aag  gtg cac      4168
   Arg Arg Ala  Gln Ser Ser Thr Ala  Thr Gly Val Ala Lys  Val His
           1230                 1235                 1240
   cag ctg gag  gag aag cag ctt atc  gac gag gct ttc gag  gct          4210
   Gln Leu Glu  Glu Lys Gln Leu Ile  Asp Glu Ala Phe Glu  Ala
           1245                 1250                 1255
   taagtcttta tagtcctgca ctagcctaga gggccttatg cagtgtgaat cacacagcat   4270
   aaggcccttt ttgctgccgt ggttgcctaa ggtggaaggc atgaaacgaa tctgtgcggt   4330
   cacgatctct tcagtacttt tgctaagtgg ctgctcctcc acttccacca cgcagctcga   4390
   g                                                                   4391
<210> 45
   <211> 1256
   <212> PRT
   <213> Corynebacterium glutamicum
<400> 45
   Met Leu Gln Leu Gly Leu Arg His Asn Gln Pro Thr Thr Asn Val Thr
   1               5                   10                  15
   Val Asp Lys Ile Lys Leu Asn Lys Pro Ser Arg Ser Lys Glu Lys Arg
               20                  25                  30
   Arg Val Pro Ala Val Ser Ser Ala Ser Thr Phe Gly Gln Asn Ala Trp
           35                  40                  45
   Leu Val Asp Glu Met Phe Gln Gln Phe Gln Lys Asp Pro Lys Ser Val
       50                  55                  60
   Asp Lys Glu Trp Arg Glu Leu Phe Glu Ala Gln Gly Gly Pro Asn Ala
   65                  70                  75                  80
   Thr Pro Ala Thr Thr Glu Ala Gln Pro Ser Ala Pro Lys Glu Ser Ala
                   85                  90                  95
   Lys Pro Ala Pro Lys Ala Ala Pro Ala Ala Lys Ala Ala Pro Arg Val
               100                 105                 110
   Glu Thr Lys Pro Ala Ala Lys Thr Ala Pro Lys Ala Lys Glu Ser Ser
           115                 120                 125
   Val Pro Gln Gln Pro Lys Leu Pro Glu Pro Gly Gln Thr Pro Ile Arg
       130                 135                 140
   Gly Ile Phe Lys Ser Ile Ala Lys Asn Met Asp Ile Ser Leu Glu Ile
   145                 150                 155                 160
   Pro Thr Ala Thr Ser Val Arg Asp Met Pro Ala Arg Leu Met Phe Glu
                   165                 170                 175
   Asn Arg Ala Met Val Asn Asp Gln Leu Lys Arg Thr Arg Gly Gly Lys
               180                 185                 190
   Ile Ser Phe Thr His Ile Ile Gly Tyr Ala Met Val Lys Ala Val Met
           195                 200                 205
   Ala His Pro Asp Met Asn Asn Ser Tyr Asp Val Ile Asp Gly Lys Pro
       210                 215                 220
   Thr Leu Ile Val Pro Glu His Ile Asn Leu Gly Leu Ala Ile Asp Leu
   225                 230                 235                 240
   Pro Gln Lys Asp Gly Ser Arg Ala Leu Val Val Ala Ala Ile Lys Glu
                   245                 250                 255
   Thr Glu Lys Met Asn Phe Ser Glu Phe Leu Ala Ala Tyr Glu Asp Ile
               260                 265                 270
   Val Thr Arg Ser Arg Lys Gly Lys Leu Thr Met Asp Asp Tyr Gln Gly
           275                 280                 285
   Val Thr Val Ser Leu Thr Asn Pro Gly Gly Ile Gly Thr Arg His Ser
       290                 295                 300
   Val Pro Arg Leu Thr Lys Gly Gln Gly Thr Ile Ile Gly Val Gly Ser
   305                 310                 315                 320
   Met Asp Tyr Pro Ala Glu Phe Gln Gly Ala Ser Glu Asp Arg Leu Ala
                   325                 330                 335
   Glu Leu Gly Val Gly Lys Leu Val Thr Ile Thr Ser Thr Tyr Asp His
   340 345 350
   Arg Val Ile Gln Gly Ala Val Ser Gly Glu Phe Leu Arg Thr Met Ser
   355 360 365
   Arg Leu Leu Thr Asp Asp Ser Phe Trp Asp Glu Ile Phe Asp Ala Met
   370 375 380
   Asn Val Pro Tyr Thr Pro Met Arg Trp Ala Gln Asp Val Pro Asn Thr
   385 390 395 400
   Gly Val Asp Lys Asn Thr Arg Val Met Gln Leu Ile Glu Ala Tyr Arg
   405 410 415
   Ser Arg Gly His Leu Ile Ala Asp Thr Asn Pro Leu Ser Trp Val Gln
   420 425 430
   Pro Gly Met Pro Val Pro Asp His Arg Asp Leu Asp Ile Glu Thr His
   435 440 445
   Ser Leu Thr Ile Trp Asp Leu Asp Arg Thr Phe Ser Val Gly Gly Phe
   450 455 460
   Gly Gly Lys Glu Thr Met Thr Leu Arg Glu Val Leu Ser Arg Leu Arg
   465 470 475 480
   Ala Ala Tyr Thr Leu Lys Val Gly Ser Glu Tyr Thr His Ile Leu Asp
   485 490 495
   Arg Asp Glu Arg Thr Trp Leu Gln Asp Arg Leu Glu Ala Gly Met Pro
   500 505 510
   Lys Pro Thr Gln Ala Glu Gln Lys Tyr Ile Leu Gln Lys Leu Asn Ala
   515 520 525
   Ala Glu Ala Phe Glu Asn Phe Leu Gln Thr Lys Tyr Val Gly Gln Lys
   530 535 540
   Arg Phe Ser Leu Glu Gly Ala Glu Ala Leu Ile Pro Leu Met Asp Ser
   545 550 555 560
   Ala Ile Asp Thr Ala Ala Gly Gln Gly Leu Asp Glu Val Val Ile Gly
   565 570 575
   Met Pro His Arg Gly Arg Leu Asn Val Leu Phe Asn Ile Val Gly Lys
   580 585 590
   Pro Leu Ala Ser Ile Phe Asn Glu Phe Glu Gly Gln Met Glu Gln Gly
   595 600 605
   Gln Ile Gly Gly Ser Gly Asp Val Lys Tyr His Leu Gly Ser Glu Gly
   610 615 620
   Gln His Leu Gln Met Phe Gly Asp Gly Glu Ile Lys Val Ser Leu Thr
   625 630 635 640
   Ala Asn Pro Ser His Leu Glu Ala Val Asn Pro Val Met Glu Gly Ile
   645 650 655
   Val Arg Ala Lys Gln Asp Tyr Leu Asp Lys Gly Val Asp Gly Lys Thr
   660 665 670
   Val Val Pro Leu Leu Leu His Gly Asp Ala Ala Phe Ala Gly Leu Gly
   675 680 685
   Ile Val Pro Glu Thr Ile Asn Leu Ala Lys Leu Arg Leu Asp Val Gly
   690 695 700
   Gly Thr Ile His Ile Val Val Asn Asn Gln Ile Gly Phe Thr Thr Thr
   705 710 715 720
   Pro Asp Ser Ser Arg Ser Met His Tyr Ala Thr Asp Tyr Ala Lys Ala
   725 730 735
   Phe Gly Cys Pro Val Phe His Val Asn Gly Asp Asp Pro Glu Ala Val
   740 745 750
   Val Trp Val Gly Gln Leu Ala Thr Glu Tyr Arg Arg Arg Phe Gly Lys
   755 760 765
   Asp Val Phe Ile Asp Leu Val Cys Tyr Arg Leu Arg Gly His Asn Glu
   770 775 780
   Ala Asp Asp Pro Ser Met Thr Gln Pro Lys Met Tyr Glu Leu Ile Thr
   785 790 795 800
   Gly Arg Glu Thr Val Arg Ala Gln Tyr Thr Glu Asp Leu Leu Gly Arg
   805 810 815
   Gly Asp Leu Ser Asn Glu Asp Ala Glu Ala Val Val Arg Asp Phe His
   820 825 830
   Asp Gln Met Glu Ser Val Phe Asn Glu Val Lys Glu Gly Gly Lys Lys
   835 840 845
   Gln Ala Glu Ala Gln Thr Gly Ile Thr Gly Ser Gln Lys Leu Pro His
   850 855 860
   Gly Leu Glu Thr Asn Ile Ser Arg Glu Glu Leu Leu Glu Leu Gly Gln
   865 870 875 880
   Ala Phe Ala Asn Thr Pro Glu Gly Phe Asn Tyr His Pro Arg Val Ala
   885 890 895
   Pro Val Ala Lys Lys Arg Val Ser Ser Val Thr Glu Gly Gly Ile Asp
   900 905 910
   Trp Ala Trp Gly Glu Leu Leu Ala Phe Gly Ser Leu Ala Asn Ser Gly
   915 920 925
   Arg Leu Val Arg Leu Ala Gly Glu Asp Ser Arg Arg Gly Thr Phe Thr
   930 935 940
   Gln Arg His Ala Val Ala Ile Asp Pro Ala Thr Ala Glu Glu Phe Asn
   945 950 955 960
   Pro Leu His Glu Leu Ala Gln Ser Lys Gly Asn Asn Gly Lys Phe Leu
   965 970 975
   Val Tyr Asn Ser Ala Leu Thr Glu Tyr Ala Gly Met Gly Phe Glu Tyr
   980 985 990
   Gly Tyr Ser Val Gly Asn Glu Asp Ser Val Val Ala Trp Glu Ala Gln
   995 1000 1005
   Phe Gly Asp Phe Ala Asn Gly Ala Gln Thr Ile Ile Asp Glu Tyr
   1010 1015 1020
   Val Ser Ser Gly Glu Ala Lys Trp Gly Gln Thr Ser Lys Leu Ile
   1025 1030 1035
   Leu Leu Leu Pro His Gly Tyr Glu Gly Gln Gly Pro Asp His Ser
   1040 1045 1050
   Ser Ala Arg Ile Glu Arg Phe Leu Gln Leu Cys Ala Glu Gly Ser
   1055 1060 1065
   Met Thr Val Ala Gln Pro Ser Thr Pro Ala Asn His Phe His Leu
   1070 1075 1080
   Leu Arg Arg His Ala Leu Ser Asp Leu Lys Arg Pro Leu Val Ile
   1085 1090 1095
   Phe Thr Pro Lys Ser Met Leu Arg Asn Lys Ala Ala Ala Ser Ala
   1100 1105 1110
   Pro Glu Asp Phe Thr Glu Val Thr Lys Phe Gln Ser Val Ile Asp
   1115 1120 1125
   Asp Pro Asn Val Ala Asp Ala Ala Lys Val Lys Lys Val Met Leu
   1130 1135 1140
   Val Ser Gly Lys Leu Tyr Tyr Glu Leu Ala Lys Arg Lys Glu Lys
   1145 1150 1155
   Asp Gly Arg Asp Asp Ile Ala Ile Val Arg Ile Glu Met Leu His
   1160 1165 1170
   Pro Ile Pro Phe Asn Arg Ile Ser Glu Ala Leu Ala Gly Tyr Pro
   1175 1180 1185
   Asn Ala Glu Glu Val Leu Phe Val Gln Asp Glu Pro Ala Asn Gln
   1190 1195 1200
   Gly Pro Trp Pro Phe Tyr Gln Glu His Leu Pro Glu Leu Ile Pro
   1205 1210 1215
   Asn Met Pro Lys Met Arg Arg Val Ser Arg Arg Ala Gln Ser Ser
   1220 1225 1230
   Thr Ala Thr Gly Val Ala Lys Val His Gln Leu Glu Glu Lys Gln
   1235 1240 1245
   Leu Ile Asp Glu Ala Phe Glu Ala
   1250 1255
<210> 46
   <211> 4391
   <212> DNA
   <213> Corynebacterium glutamicum
<220>
   <221> CDS
   <222> (443).. (4210)
   <223> sucA805
<400> 46
   gtcgacaagc aaaatcgaag cggcagcacg ccgcgtcgga gccttaaacg ccatcgccgc 60
   catccctgat ggtttcaatc atcaagtcgg tgaacgcggg cgcaacctgt catccggaca 120
   gcgccaactg atcgcgctgg cgcgcgccga actcatcgag ccttccatca tgcttctcga 180
   cgaagccacc tccaccctcg accccgccac cgaagccgtt atcctcaacg cctccgatcg 240
   agtcactaag ggacgcacca gcatcatcgt cgcgcaccgc ttggcaaccg ctaaaagggc 300
   cgaccgtatt cttgttgttg aacaaggacg tatcattgag gacggatctc acgacgcgtt 360
   gttgtctgct aacggcacct acgcccgcat gtggcattta atggcctgac acgttatttt 420
   taggagaact gtcaacaaat ta atg cta caa ctg ggg ctt agg cat aat cag 472
   Met Leu Gln Leu Gly Leu Arg His Asn Gln
   1 5 10
   cca acg acc aac gtt aca gtg gat aaa ata aag ctc aat aaa ccc tca 520
   Pro Thr Thr Asn Val Thr Val Asp Lys Ile Lys Leu Asn Lys Pro Ser
   15 20 25
   aga agc aag gaa aag agg cga gta cct gcc gtg agc agc gct agt act 568
   Arg Ser Lys Glu Lys Arg Arg Val Pro Ala Val Ser Ser Ala Ser Thr
           30                  35                  40
   ttc ggc cag aat gcg tgg ctg gta gac gag atg ttc cag cag ttc cag      616
   Phe Gly Gln Asn Ala Trp Leu Val Asp Glu Met Phe Gln Gln Phe Gln
       45                  50                  55
   aag gac ccc aag tcc gtg gac aag gaa tgg aga gaa ctc ttt gag gcg      664
   Lys Asp Pro Lys Ser Val Asp Lys Glu Trp Arg Glu Leu Phe Glu Ala
       60                  65                  70
   cag ggg gga cca aat gct acc ccc gct aca aca gaa gca cag cct tca      712
   Gln Gly Gly Pro Asn Ala Thr Pro Ala Thr Thr Glu Ala Gln Pro Ser
   75                  80                  85                  90
   gcg ccc aag gag tct gcg aaa cca gca cca aag gct gcc cct gca gcc      760
   Ala Pro Lys Glu Ser Ala Lys Pro Ala Pro Lys Ala Ala Pro Ala Ala
                   95                  100                 105
   aag gca gca ccg cgc gta gaa acc aag ccg gcc gcc aag acc gcc cct      808
   Lys Ala Ala Pro Arg Val Glu Thr Lys Pro Ala Ala Lys Thr Ala Pro
               110                 115                 120
   aag gcc aag gag tcc tca gtg cca cag caa cct aag ctt ccg gag cca      856
   Lys Ala Lys Glu Ser Ser Val Pro Gln Gln Pro Lys Leu Pro Glu Pro
           125                 130                 135
   gga caa acc cca atc agg ggt att ttc aag tcc atc gcg aag aac atg      904
   Gly Gln Thr Pro Ile Arg Gly Ile Phe Lys Ser Ile Ala Lys Asn Met
       140                 145                 150
   gat atc tcc ctg gaa atc cca acc gca acc tcg gtt cgc gat atg cca      952
   Asp Ile Ser Leu Glu Ile Pro Thr Ala Thr Ser Val Arg Asp Met Pro
   155                 160                 165                 170
   gct cgc ctc atg ttc gaa aac cgc gcg atg gtc aac gat cag ctc aag     1000
   Ala Arg Leu Met Phe Glu Asn Arg Ala Met Val Asn Asp Gln Leu Lys
                   175                 180                 185
   cgc acc cgc ggt ggc aag atc tcc ttc acc cac atc att ggc tac gcc     1048
   Arg Thr Arg Gly Gly Lys Ile Ser Phe Thr His Ile Ile Gly Tyr Ala
               190                 195                 200
   atg gtg aag gca gtc atg gct cac ccg gac atg aac aac tcc tac gac     1096
   Met Val Lys Ala Val Met Ala His Pro Asp Met Asn Asn Ser Tyr Asp
           205                 210                 215
   gtc atc gac ggc aag cca acc ctg atc gtg cct gag cac atc aac ctg     1144
   Val Ile Asp Gly Lys Pro Thr Leu Ile Val Pro Glu His Ile Asn Leu
       220                 225                 230
   ggc ctt gcc atc gac ctt cct cag aag gac ggc tcc cgc gca ctt gtc     1192
   Gly Leu Ala Ile Asp Leu Pro Gln Lys Asp Gly Ser Arg Ala Leu Val
   235                 240                 245                 250
   gta gca gcc atc aag gaa acc gag aag atg aac ttc tcc gag ttc ctc     1240
   Val Ala Ala Ile Lys Glu Thr Glu Lys Met Asn Phe Ser Glu Phe Leu
                   255                 260                 265
   gca gca tac gaa gac atc gtg aca cgc tcc cgc aag ggc aag ctc acc     1288
   Ala Ala Tyr Glu Asp Ile Val Thr Arg Ser Arg Lys Gly Lys Leu Thr
               270                 275                 280
   atg gat gac tac cag ggc gtt acc gtt tcc ttg acc aac cca ggt ggc     1336
   Met Asp Asp Tyr Gln Gly Val Thr Val Ser Leu Thr Asn Pro Gly Gly
   285 290 295
   atc ggt acc cgc cac tct gtc cca cgt ctg acc aag ggc cag ggc acc 1384
   Ile Gly Thr Arg His Ser Val Pro Arg Leu Thr Lys Gly Gln Gly Thr
   300 305 310
   atc atc ggt gtc ggt tcc atg gat tac cca gca gag ttc cag ggc gct 1432
   Ile Ile Gly Val Gly Ser Met Asp Tyr Pro Ala Glu Phe Gln Gly Ala
   315 320 325 330
   tcc gaa gac cgc ctt gca gag ctc ggc gtt gga aag ctt gtc acc atc 1480
   Ser Glu Asp Arg Leu Ala Glu Leu Gly Val Gly Lys Leu Val Thr Ile
   335 340 345
   acc tcc acc tac gat cac cgc gtg atc cag ggt gct gtg tcc ggt gaa 1528
   Thr Ser Thr Tyr Asp His Arg Val Ile Gln Gly Ala Val Ser Gly Glu
   350 355 360
   ttc ctg cgt acc atg tct cgc ctg ctc acc gat gat tcc ttc tgg gat 1576
   Phe Leu Arg Thr Met Ser Arg Leu Leu Thr Asp Asp Ser Phe Trp Asp
   365 370 375
   gag atc ttc gac gca atg aac gtt cct tac acc cca atg cgt tgg gca 1624
   Glu Ile Phe Asp Ala Met Asn Val Pro Tyr Thr Pro Met Arg Trp Ala
   380 385 390
   cag gac gtt cca aac acc ggt gtt gat aag aac acc cgc gtc atg cag 1672
   Gln Asp Val Pro Asn Thr Gly Val Asp Lys Asn Thr Arg Val Met Gln
   395 400 405 410
   ctc att gag gca tac cgc tcc cgt gga cac ctc atc gct gac acc aac 1720
   Leu Ile Glu Ala Tyr Arg Ser Arg Gly His Leu Ile Ala Asp Thr Asn
   415 420 425
   cca ctt tca tgg gtt cag cct ggc atg cca gtt cca gac cac cgc gac 1768
   Pro Leu Ser Trp Val Gln Pro Gly Met Pro Val Pro Asp His Arg Asp
   430 435 440
   ctc gac atc gag acc cac agc ctg acc atc tgg gat ctg gac cgt acc 1816
   Leu Asp Ile Glu Thr His Ser Leu Thr Ile Trp Asp Leu Asp Arg Thr
   445 450 455
   ttc agc gtc ggt ggc ttc ggc ggc aag gag acc atg acc ctg cgc gag 1864
   Phe Ser Val Gly Gly Phe Gly Gly Lys Glu Thr Met Thr Leu Arg Glu
   460 465 470
   gta ctg tcc cgc ctg cgc gct gcc tac acc ttg aag gtc ggc tcc gaa 1912
   Val Leu Ser Arg Leu Arg Ala Ala Tyr Thr Leu Lys Val Gly Ser Glu
   475 480 485 490
   tac acc cac atc ctg gac cgc gac gag cgc acc tgg ctg cag gac cgc 1960
   Tyr Thr His Ile Leu Asp Arg Asp Glu Arg Thr Trp Leu Gln Asp Arg
   495 500 505
   ctc gaa gcc gga atg cca aag cca acc cag gca gag cag aag tac atc 2008
   Leu Glu Ala Gly Met Pro Lys Pro Thr Gln Ala Glu Gln Lys Tyr Ile
   510 515 520
   ctg cag aag ctg aac gcc gca gag gct ttc gag aac ttc ctg cag acc 2056
   Leu Gln Lys Leu Asn Ala Ala Glu Ala Phe Glu Asn Phe Leu Gln Thr
   525 530 535
   aag tac gtc ggc cag aag cgc ttc tcc ctc gaa ggt gca gaa gct ctc 2104
   Lys Tyr Val Gly Gln Lys Arg Phe Ser Leu Glu Gly Ala Glu Ala Leu
   540 545 550
   atc cca ctg atg gac tcc gcc atc gac acc gcc gca ggc cag ggc ctc 2152
   Ile Pro Leu Met Asp Ser Ala Ile Asp Thr Ala Ala Gly Gln Gly Leu
   555 560 565 570
   gac gaa gtt gtc atc ggt atg cca cac cgt ggt cgc ctc aac gtg ctg 2200
   Asp Glu Val Val Ile Gly Met Pro His Arg Gly Arg Leu Asn Val Leu
   575 580 585
   ttc aac atc gtg ggc aag cca ctg gca tcc atc ttc aac gag ttt gaa 2248
   Phe Asn Ile Val Gly Lys Pro Leu Ala Ser Ile Phe Asn Glu Phe Glu
   590 595 600
   ggc caa atg gag cag ggc cag atc ggt ggc tcc ggt gac gtg aag tac 2296
   Gly Gln Met Glu Gln Gly Gln Ile Gly Gly Ser Gly Asp Val Lys Tyr
   605 610 615
   cac ctc ggt tcc gaa ggc cag cac ctg cag atg ttc ggc gac ggc gag 2344
   His Leu Gly Ser Glu Gly Gln His Leu Gln Met Phe Gly Asp Gly Glu
   620 625 630
   atc aag gtc tcc ctg act gct aac ccg tcc cac ctg gaa gct gtt aac 2392
   Ile Lys Val Ser Leu Thr Ala Asn Pro Ser His Leu Glu Ala Val Asn
   635 640 645 650
   cca gtg atg gaa ggt atc gtc cgc gca aag cag gac tac ctg gac aag 2440
   Pro Val Met Glu Gly Ile Val Arg Ala Lys Gln Asp Tyr Leu Asp Lys
   655 660 665
   ggc gta gac ggc aag act gtt gtg cca ctg ctg ctc cac ggt gac gct 2488
   Gly Val Asp Gly Lys Thr Val Val Pro Leu Leu Leu His Gly Asp Ala
   670 675 680
   gca ttc gca ggc ctg ggc atc gtg cca gaa acc atc aac ctg gct aaa 2536
   Ala Phe Ala Gly Leu Gly Ile Val Pro Glu Thr Ile Asn Leu Ala Lys
   685 690 695
   agc tgc gtc gac gtc gga ggc acc atc cac atc gtg gtg aac aac cag 2584
   Ser Cys Val Asp Val Gly Gly Thr Ile His Ile Val Val Asn Asn Gln
   700 705 710
   atc ggc ttc acc acc acc cca gac tcc agc cgc tcc atg cac tac gca 2632
   Ile Gly Phe Thr Thr Thr Pro Asp Ser Ser Arg Ser Met His Tyr Ala
   715 720 725 730
   acc gac tac gcc aag gca ttc ggc tgc cca gtc ttc cac gtc aat ggt 2680
   Thr Asp Tyr Ala Lys Ala Phe Gly Cys Pro Val Phe His Val Asn Gly
   735 740 745
   gat gac cca gag gca gtt gtc tgg gtt ggc cag ctg gca acc gag tac 2728
   Asp Asp Pro Glu Ala Val Val Trp Val Gly Gln Leu Ala Thr Glu Tyr
   750 755 760
   cgt cgt cgc ttc ggc aag gac gtc ttc atc gac ctc gtt tgc tac cgc 2776
   Arg Arg Arg Phe Gly Lys Asp Val Phe Ile Asp Leu Val Cys Tyr Arg
   765 770 775
   ctc cgc ggc cac aac gaa gct gat gat cct tcc atg acc cag cca aag 2824
   Leu Arg Gly His Asn Glu Ala Asp Asp Pro Ser Met Thr Gln Pro Lys
   780 785 790
   atg tat gag ctc atc acc ggc cgc gag acc gtt cgt gct cag tac acc 2872
   Met Tyr Glu Leu Ile Thr Gly Arg Glu Thr Val Arg Ala Gln Tyr Thr
   795 800 805 810
   gaa gac ctg ctc gga cgt gga gac ctc tcc aac gaa gat gca gaa gca 2920
   Glu Asp Leu Leu Gly Arg Gly Asp Leu Ser Asn Glu Asp Ala Glu Ala
   815 820 825
   gtc gtc cgc gac ttc cac gac cag atg gaa tct gtg ttc aac gaa gtc 2968
   Val Val Arg Asp Phe His Asp Gln Met Glu Ser Val Phe Asn Glu Val
   830 835 840
   aag gaa ggc ggc aag aag cag gct gag gca cag acc ggc atc acc ggc 3016
   Lys Glu Gly Gly Lys Lys Gln Ala Glu Ala Gln Thr Gly Ile Thr Gly
   845 850 855
   tcc cag aag ctt cca cac ggc ctt gag acc aac atc tcc cgt gaa gag 3064
   Ser Gln Lys Leu Pro His Gly Leu Glu Thr Asn Ile Ser Arg Glu Glu
   860 865 870
   ctc ctg gaa ctg gga cag gct ttc gcc aac acc cca gaa ggc ttc aac 3112
   Leu Leu Glu Leu Gly Gln Ala Phe Ala Asn Thr Pro Glu Gly Phe Asn
   875 880 885 890
   tac cac cca cgt gtg gct cca gtt gct aag aag cgc gtc tcc tct gtc 3160
   Tyr His Pro Arg Val Ala Pro Val Ala Lys Lys Arg Val Ser Ser Val
   895 900 905
   acc gaa ggt ggc atc gac tgg gca tgg ggc gag ctc ctc gcc ttc ggt 3208
   Thr Glu Gly Gly Ile Asp Trp Ala Trp Gly Glu Leu Leu Ala Phe Gly
   910 915 920
   tcc ctg gct aac tcc ggc cgc ttg gtt cgc ctt gca ggt gaa gat tcc 3256
   Ser Leu Ala Asn Ser Gly Arg Leu Val Arg Leu Ala Gly Glu Asp Ser
   925 930 935
   cgc cgc ggt acc ttc acc cag cgc cac gca gtt gcc atc gac cca gcg 3304
   Arg Arg Gly Thr Phe Thr Gln Arg His Ala Val Ala Ile Asp Pro Ala
   940 945 950
   acc gct gaa gag ttc aac cca ctc cac gag ctt gca cag tcc aag ggc 3352
   Thr Ala Glu Glu Phe Asn Pro Leu His Glu Leu Ala Gln Ser Lys Gly
   955 960 965 970
   aac aac ggt aag ttc ctg gtc tac aac tcc gca ctg acc gag tac gca 3400
   Asn Asn Gly Lys Phe Leu Val Tyr Asn Ser Ala Leu Thr Glu Tyr Ala
   975 980 985
   ggc atg ggc ttc gag tac ggc tac tcc gta gga aac gaa gac tcc gtc 3448
   Gly Met Gly Phe Glu Tyr Gly Tyr Ser Val Gly Asn Glu Asp Ser Val
   990 995 1000
   gtt gca tgg gaa gca cag ttc ggc gac ttc gcc aac ggc gct cag 3493
   Val Ala Trp Glu Ala Gln Phe Gly Asp Phe Ala Asn Gly Ala Gln
   1005 1010 1015
   acc atc atc gat gag tac gtc tcc tca ggc gaa gct aag tgg ggc 3538
   Thr Ile Ile Asp Glu Tyr Val Ser Ser Gly Glu Ala Lys Trp Gly
   1020 1025 1030
   cag acc tcc aag ctg atc ctt ctg ctg cct cac ggc tac gaa ggc 3583
   Gln Thr Ser Lys Leu Ile Leu Leu Leu Pro His Gly Tyr Glu Gly
   1035 1040 1045
   cag ggc cca gac cac tct tcc gca cgt atc gag cgc ttc ctg cag 3628
   Gln Gly Pro Asp His Ser Ser Ala Arg Ile Glu Arg Phe Leu Gln
   1050 1055 1060
   ctg tgc gct gag ggt tcc atg act gtt gct cag cca tcc acc cca 3673
   Leu Cys Ala Glu Gly Ser Met Thr Val Ala Gln Pro Ser Thr Pro
   1065 1070 1075
   gca aac cac ttc cac ctg ctg cgt cgt cac gct ctg tcc gac ctg 3718
   Ala Asn His Phe His Leu Leu Arg Arg His Ala Leu Ser Asp Leu
   1080 1085 1090
   aag cgt cca ctg gtt atc ttc acc ccg aag tcc atg ctg cgt aac 3763
   Lys Arg Pro Leu Val Ile Phe Thr Pro Lys Ser Met Leu Arg Asn
   1095 1100 1105
   aag gct gct gcc tcc gca cca gaa gac ttc act gag gtc acc aag 3808
   Lys Ala Ala Ala Ser Ala Pro Glu Asp Phe Thr Glu Val Thr Lys
   1110 1115 1120
   ttc caa tcc gtg atc gac gat cca aac gtt gca gat gca gcc aag 3853
   Phe Gln Ser Val Ile Asp Asp Pro Asn Val Ala Asp Ala Ala Lys
   1125 1130 1135
   gtg aag aag gtc atg ctg gtc tcc ggc aag ctg tac tac gaa ttg 3898
   Val Lys Lys Val Met Leu Val Ser Gly Lys Leu Tyr Tyr Glu Leu
   1140 1145 1150
   gca aag cgc aag gag aag gac gga cgc gac gac atc gcg atc gtt 3943
   Ala Lys Arg Lys Glu Lys Asp Gly Arg Asp Asp Ile Ala Ile Val
   1155 1160 1165
   cgt atc gaa atg ctc cac cca att ccg ttc aac cgc atc tcc gag 3988
   Arg Ile Glu Met Leu His Pro Ile Pro Phe Asn Arg Ile Ser Glu
   1170 1175 1180
   gct ctt gcc ggc tac cct aac gct gag gaa gtc ctc ttc gtt cag 4033
   Ala Leu Ala Gly Tyr Pro Asn Ala Glu Glu Val Leu Phe Val Gln
   1185 1190 1195
   gat gag cca gca aac cag ggc cca tgg ccg ttc tac cag gag cac 4078
   Asp Glu Pro Ala Asn Gln Gly Pro Trp Pro Phe Tyr Gln Glu His
   1200 1205 1210
   ctc cca gag ctg atc ccg aac atg cca aag atg cgc cgc gtt tcc 4123
   Leu Pro Glu Leu Ile Pro Asn Met Pro Lys Met Arg Arg Val Ser
   1215 1220 1225
   cgc cgc gct cag tcc tcc acc gca act ggt gtt gct aag gtg cac 4168
   Arg Arg Ala Gln Ser Ser Thr Ala Thr Gly Val Ala Lys Val His
   1230 1235 1240
   cag ctg gag gag aag cag ctt atc gac gag gct ttc gag gct 4210
   Gln Leu Glu Glu Lys Gln Leu Ile Asp Glu Ala Phe Glu Ala
   1245 1250 1255
   taagtcttta tagtcctgca ctagcctaga gggccttatg cagtgtgaat cacacagcat 4270
   aaggcccttt ttgctgccgt ggttgcctaa ggtggaaggc atgaaacgaa tctgtgcggt 4330
   cacgatctct tcagtacttt tgctaagtgg ctgctcctcc acttccacca cgcagctcga 4390
   g 4391
<210> 47
   <211> 1256
   <212> PRT
   <213> Corynebacterium glutamicum
<400> 47
   Met Leu Gln Leu Gly Leu Arg His Asn Gln Pro Thr Thr Asn Val Thr
   1 5 10 15
   Val Asp Lys Ile Lys Leu Asn Lys Pro Ser Arg Ser Lys Glu Lys Arg
   20 25 30
   Arg Val Pro Ala Val Ser Ser Ala Ser Thr Phe Gly Gln Asn Ala Trp
   35 40 45
   Leu Val Asp Glu Met Phe Gln Gln Phe Gln Lys Asp Pro Lys Ser Val
   50 55 60
   Asp Lys Glu Trp Arg Glu Leu Phe Glu Ala Gln Gly Gly Pro Asn Ala
   65 70 75 80
   Thr Pro Ala Thr Thr Glu Ala Gln Pro Ser Ala Pro Lys Glu Ser Ala
   85 90 95
   Lys Pro Ala Pro Lys Ala Ala Pro Ala Ala Lys Ala Ala Pro Arg Val
   100 105 110
   Glu Thr Lys Pro Ala Ala Lys Thr Ala Pro Lys Ala Lys Glu Ser Ser
   115 120 125
   Val Pro Gln Gln Pro Lys Leu Pro Glu Pro Gly Gln Thr Pro Ile Arg
   130 135 140
   Gly Ile Phe Lys Ser Ile Ala Lys Asn Met Asp Ile Ser Leu Glu Ile
   145 150 155 160
   Pro Thr Ala Thr Ser Val Arg Asp Met Pro Ala Arg Leu Met Phe Glu
   165 170 175
   Asn Arg Ala Met Val Asn Asp Gln Leu Lys Arg Thr Arg Gly Gly Lys
   180 185 190
   Ile Ser Phe Thr His Ile Ile Gly Tyr Ala Met Val Lys Ala Val Met
   195 200 205
   Ala His Pro Asp Met Asn Asn Ser Tyr Asp Val Ile Asp Gly Lys Pro
   210 215 220
   Thr Leu Ile Val Pro Glu His Ile Asn Leu Gly Leu Ala Ile Asp Leu
   225 230 235 240
   Pro Gln Lys Asp Gly Ser Arg Ala Leu Val Val Ala Ala Ile Lys Glu
   245 250 255
   Thr Glu Lys Met Asn Phe Ser Glu Phe Leu Ala Ala Tyr Glu Asp Ile
   260 265 270
   Val Thr Arg Ser Arg Lys Gly Lys Leu Thr Met Asp Asp Tyr Gln Gly
   275 280 285
   Val Thr Val Ser Leu Thr Asn Pro Gly Gly Ile Gly Thr Arg His Ser
   290 295 300
   Val Pro Arg Leu Thr Lys Gly Gln Gly Thr Ile Ile Gly Val Gly Ser
   305 310 315 320
   Met Asp Tyr Pro Ala Glu Phe Gln Gly Ala Ser Glu Asp Arg Leu Ala
   325 330 335
   Glu Leu Gly Val Gly Lys Leu Val Thr Ile Thr Ser Thr Tyr Asp His
   340 345 350
   Arg Val Ile Gln Gly Ala Val Ser Gly Glu Phe Leu Arg Thr Met Ser
   355 360 365
   Arg Leu Leu Thr Asp Asp Ser Phe Trp Asp Glu Ile Phe Asp Ala Met
   370 375 380
   Asn Val Pro Tyr Thr Pro Met Arg Trp Ala Gln Asp Val Pro Asn Thr
   385 390 395 400
   Gly Val Asp Lys Asn Thr Arg Val Met Gln Leu Ile Glu Ala Tyr Arg
   405 410 415
   Ser Arg Gly His Leu Ile Ala Asp Thr Asn Pro Leu Ser Trp Val Gln
   420 425 430
   Pro Gly Met Pro Val Pro Asp His Arg Asp Leu Asp Ile Glu Thr His
   435 440 445
   Ser Leu Thr Ile Trp Asp Leu Asp Arg Thr Phe Ser Val Gly Gly Phe
   450 455 460
   Gly Gly Lys Glu Thr Met Thr Leu Arg Glu Val Leu Ser Arg Leu Arg
   465 470 475 480
   Ala Ala Tyr Thr Leu Lys Val Gly Ser Glu Tyr Thr His Ile Leu Asp
   485 490 495
   Arg Asp Glu Arg Thr Trp Leu Gln Asp Arg Leu Glu Ala Gly Met Pro
   500 505 510
   Lys Pro Thr Gln Ala Glu Gln Lys Tyr Ile Leu Gln Lys Leu Asn Ala
   515 520 525
   Ala Glu Ala Phe Glu Asn Phe Leu Gln Thr Lys Tyr Val Gly Gln Lys
   530 535 540
   Arg Phe Ser Leu Glu Gly Ala Glu Ala Leu Ile Pro Leu Met Asp Ser
   545 550 555 560
   Ala Ile Asp Thr Ala Ala Gly Gln Gly Leu Asp Glu Val Val Ile Gly
   565 570 575
   Met Pro His Arg Gly Arg Leu Asn Val Leu Phe Asn Ile Val Gly Lys
   580 585 590
   Pro Leu Ala Ser Ile Phe Asn Glu Phe Glu Gly Gln Met Glu Gln Gly
   595 600 605
   Gln Ile Gly Gly Ser Gly Asp Val Lys Tyr His Leu Gly Ser Glu Gly
   610 615 620
   Gln His Leu Gln Met Phe Gly Asp Gly Glu Ile Lys Val Ser Leu Thr
   625 630 635 640
   Ala Asn Pro Ser His Leu Glu Ala Val Asn Pro Val Met Glu Gly Ile
   645 650 655
   Val Arg Ala Lys Gln Asp Tyr Leu Asp Lys Gly Val Asp Gly Lys Thr
   660 665 670
   Val Val Pro Leu Leu Leu His Gly Asp Ala Ala Phe Ala Gly Leu Gly
   675 680 685
   Ile Val Pro Glu Thr Ile Asn Leu Ala Lys Ser Cys Val Asp Val Gly
   690 695 700
   Gly Thr Ile His Ile Val Val Asn Asn Gln Ile Gly Phe Thr Thr Thr
   705 710 715 720
   Pro Asp Ser Ser Arg Ser Met His Tyr Ala Thr Asp Tyr Ala Lys Ala
   725 730 735
   Phe Gly Cys Pro Val Phe His Val Asn Gly Asp Asp Pro Glu Ala Val
   740 745 750
   Val Trp Val Gly Gln Leu Ala Thr Glu Tyr Arg Arg Arg Phe Gly Lys
   755 760 765
   Asp Val Phe Ile Asp Leu Val Cys Tyr Arg Leu Arg Gly His Asn Glu
   770 775 780
   Ala Asp Asp Pro Ser Met Thr Gln Pro Lys Met Tyr Glu Leu Ile Thr
   785 790 795 800
   Gly Arg Glu Thr Val Arg Ala Gln Tyr Thr Glu Asp Leu Leu Gly Arg
   805 810 815
   Gly Asp Leu Ser Asn Glu Asp Ala Glu Ala Val Val Arg Asp Phe His
   820 825 830
   Asp Gln Met Glu Ser Val Phe Asn Glu Val Lys Glu Gly Gly Lys Lys
   835 840 845
   Gln Ala Glu Ala Gln Thr Gly Ile Thr Gly Ser Gln Lys Leu Pro His
   850 855 860
   Gly Leu Glu Thr Asn Ile Ser Arg Glu Glu Leu Leu Glu Leu Gly Gln
   865 870 875 880
   Ala Phe Ala Asn Thr Pro Glu Gly Phe Asn Tyr His Pro Arg Val Ala
   885 890 895
   Pro Val Ala Lys Lys Arg Val Ser Ser Val Thr Glu Gly Gly Ile Asp
   900 905 910
   Trp Ala Trp Gly Glu Leu Leu Ala Phe Gly Ser Leu Ala Asn Ser Gly
   915 920 925
   Arg Leu Val Arg Leu Ala Gly Glu Asp Ser Arg Arg Gly Thr Phe Thr
   930 935 940
   Gln Arg His Ala Val Ala Ile Asp Pro Ala Thr Ala Glu Glu Phe Asn
   945 950 955 960
   Pro Leu His Glu Leu Ala Gln Ser Lys Gly Asn Asn Gly Lys Phe Leu
   965 970 975
   Val Tyr Asn Ser Ala Leu Thr Glu Tyr Ala Gly Met Gly Phe Glu Tyr
   980 985 990
   Gly Tyr Ser Val Gly Asn Glu Asp Ser Val Val Ala Trp Glu Ala Gln
   995 1000 1005
   Phe Gly Asp Phe Ala Asn Gly Ala Gln Thr Ile Ile Asp Glu Tyr
   1010 1015 1020
   Val Ser Ser Gly Glu Ala Lys Trp Gly Gln Thr Ser Lys Leu Ile
   1025 1030 1035
   Leu Leu Leu Pro His Gly Tyr Glu Gly Gln Gly Pro Asp His Ser
   1040 1045 1050
   Ser Ala Arg Ile Glu Arg Phe Leu Gln Leu Cys Ala Glu Gly Ser
   1055 1060 1065
   Met Thr Val Ala Gln Pro Ser Thr Pro Ala Asn His Phe His Leu
   1070 1075 1080
   Leu Arg Arg His Ala Leu Ser Asp Leu Lys Arg Pro Leu Val Ile
   1085 1090 1095
   Phe Thr Pro Lys Ser Met Leu Arg Asn Lys Ala Ala Ala Ser Ala
   1100 1105 1110
   Pro Glu Asp Phe Thr Glu Val Thr Lys Phe Gln Ser Val Ile Asp
   1115 1120 1125
   Asp Pro Asn Val Ala Asp Ala Ala Lys Val Lys Lys Val Met Leu
   1130 1135 1140
   Val Ser Gly Lys Leu Tyr Tyr Glu Leu Ala Lys Arg Lys Glu Lys
   1145 1150 1155
   Asp Gly Arg Asp Asp Ile Ala Ile Val Arg Ile Glu Met Leu His
   1160 1165 1170
   Pro Ile Pro Phe Asn Arg Ile Ser Glu Ala Leu Ala Gly Tyr Pro
   1175 1180 1185
   Asn Ala Glu Glu Val Leu Phe Val Gln Asp Glu Pro Ala Asn Gln
   1190 1195 1200
   Gly Pro Trp Pro Phe Tyr Gln Glu His Leu Pro Glu Leu Ile Pro
   1205 1210 1215
   Asn Met Pro Lys Met Arg Arg Val Ser Arg Arg Ala Gln Ser Ser
   1220 1225 1230
   Thr Ala Thr Gly Val Ala Lys Val His Gln Leu Glu Glu Lys Gln
   1235 1240 1245
   Leu Ile Asp Glu Ala Phe Glu Ala
   1250 1255
<210> 48
   <211> 4391
   <212> DNA
   <213> Corynebacterium glutamicum
<220>
   <221> CDS
   <222> (443)..(4210)
   <223>
<400> 48
   gtcgacaagc aaaatcgaag cggcagcacg ccgcgtcgga gccttaaacg ccatcgccgc 60
   catccctgat ggtttcaatc atcaagtcgg tgaacgcggg cgcaacctgt catccggaca 120
   gcgccaactg atcgcgctgg cgcgcgccga actcatcgag ccttccatca tgcttctcga 180
   cgaagccacc tccaccctcg accccgccac cgaagccgtt atcctcaacg cctccgatcg 240
   agtcactaag ggacgcacca gcatcatcgt cgcgcaccgc ttggcaaccg ctaaaagggc 300
   cgaccgtatt cttgttgttg aacaaggacg tatcattgag gacggatctc acgacgcgtt 360
   gttgtctgct aacggcacct acgcccgcat gtggcattta atggcctgac acgttatttt 420
   taggagaact gtcaacaaat ta atg cta caa ctg ggg ctt agg cat aat cag 472
   Met Leu Gln Leu Gly Leu Arg His Asn Gln
   1 5 10
   cca acg acc aac gtt aca gtg gat aaa ata aag ctc aat aaa ccc tca 520
   Pro Thr Thr Asn Val Thr Val Asp Lys Ile Lys Leu Asn Lys Pro Ser
   15 20 25
   aga agc aag gaa aag agg cga gta cct gcc gtg agc agc gct agt act 568
   Arg Ser Lys Glu Lys Arg Arg Val Pro Ala Val Ser Ser Ala Ser Thr
               30                  35                  40
   ttc ggc cag aat gcg tgg ctg gta gac gag atg ttc cag cag ttc cag       616
   Phe Gly Gln Asn Ala Trp Leu Val Asp Glu Met Phe Gln Gln Phe Gln
           45                  50                  55
   aag gac ccc aag tcc gtg gac aag gaa tgg aga gaa ctc ttt gag gcg       664
   Lys Asp Pro Lys Ser Val Asp Lys Glu Trp Arg Glu Leu Phe Glu Ala
       60                  65                  70
   cag ggg gga cca aat gct acc ccc gct aca aca gaa gca cag cct tca       712
   Gln Gly Gly Pro Asn Ala Thr Pro Ala Thr Thr Glu Ala Gln Pro Ser
   75                  80                  85                  90
   gcg ccc aag gag tct gcg aaa cca gca cca aag gct gcc cct gca gcc       760
   Ala Pro Lys Glu Ser Ala Lys Pro Ala Pro Lys Ala Ala Pro Ala Ala
                   95                  100                 105
   aag gca gca ccg cgc gta gaa acc aag ccg gcc gcc aag acc gcc cct       808
   Lys Ala Ala Pro Arg Val Glu Thr Lys Pro Ala Ala Lys Thr Ala Pro
               110                 115                 120
   aag gcc aag gag tcc tca gtg cca cag caa cct aag ctt ccg gag cca       856
   Lys Ala Lys Glu Ser Ser Val Pro Gln Gln Pro Lys Leu Pro Glu Pro
           125                 130                 135
   gga caa acc cca atc agg ggt att ttc aag tcc atc gcg aag aac atg       904
   Gly Gln Thr Pro Ile Arg Gly Ile Phe Lys Ser Ile Ala Lys Asn Met
       140                 145                 150
   gat atc tcc ctg gaa atc cca acc gca acc tcg gtt cgc gat atg cca       952
   Asp Ile Ser Leu Glu Ile Pro Thr Ala Thr Ser Val Arg Asp Met Pro
   155                 160                 165                 170
   gct cgc ctc atg ttc gaa aac cgc gcg atg gtc aac gat cag ctc aag      1000
   Ala Arg Leu Met Phe Glu Asn Arg Ala Met Val Asn Asp Gln Leu Lys
                   175                 180                 185
   cgc acc cgc ggt ggc aag atc tcc ttc acc cac atc att ggc tac gcc      1048
   Arg Thr Arg Gly Gly Lys Ile Ser Phe Thr His Ile Ile Gly Tyr Ala
               190                 195                 200
   atg gtg aag gca gtc atg gct cac ccg gac atg aac aac tcc tac gac      1096
   Met Val Lys Ala Val Met Ala His Pro Asp Met Asn Asn Ser Tyr Asp
           205                 210                 215
   gtc atc gac ggc aag cca acc ctg atc gtg cct gag cac atc aac ctg      1144
   Val Ile Asp Gly Lys Pro Thr Leu Ile Val Pro Glu His Ile Asn Leu
       220                 225                 230
   ggc ctt gcc atc gac ctt cct cag aag gac ggc tcc cgc gca ctt gtc      1192
   Gly Leu Ala Ile Asp Leu Pro Gln Lys Asp Gly Ser Arg Ala Leu Val
   235                 240                 245                 250
   gta gca gcc atc aag gaa acc gag aag atg aac ttc tcc gag ttc ctc      1240
   Val Ala Ala Ile Lys Glu Thr Glu Lys Met Asn Phe Ser Glu Phe Leu
                   255                 260                 265
   gca gca tac gaa gac atc gtg aca cgc tcc cgc aag ggc aag ctc acc      1288
   Ala Ala Tyr Glu Asp Ile Val Thr Arg Ser Arg Lys Gly Lys Leu Thr
               270                 275                 280
   atg gat gac tac cag ggc gtt acc gtt tcc ttg acc aac cca ggt ggc      1336
   Met Asp Asp Tyr Gln Gly Val Thr Val Ser Leu Thr Asn Pro Gly Gly
   285 290 295
   atc ggt acc cgc cac tct gtc cca cgt ctg acc aag ggc cag ggc acc 1384
   Ile Gly Thr Arg His Ser Val Pro Arg Leu Thr Lys Gly Gln Gly Thr
   300 305 310
   atc atc ggt gtc ggt tcc atg gat tac cca gca gag ttc cag ggc gct 1432
   Ile Ile Gly Val Gly Ser Met Asp Tyr Pro Ala Glu Phe Gln Gly Ala
   315 320 325 330
   tcc gaa gac cgc ctt gca gag ctc ggc gtt gga aag ctt gtc acc atc 1480
   Ser Glu Asp Arg Leu Ala Glu Leu Gly Val Gly Lys Leu Val Thr Ile
   335 340 345
   acc tcc acc tac gat cac cgc gtg atc cag ggt gct gtg tcc ggt gaa 1528
   Thr Ser Thr Tyr Asp His Arg Val Ile Gln Gly Ala Val Ser Gly Glu
   350 355 360
   ttc ctg cgt acc atg tct cgc ctg ctc acc gat gat tcc ttc tgg gat 1576
   Phe Leu Arg Thr Met Ser Arg Leu Leu Thr Asp Asp Ser Phe Trp Asp
   365 370 375
   gag atc ttc gac gca atg aac gtt cct tac acc cca atg cgt tgg gca 1624
   Glu Ile Phe Asp Ala Met Asn Val Pro Tyr Thr Pro Met Arg Trp Ala
   380 385 390
   cag gac gtt cca aac acc ggt gtt gat aag aac acc cgc gtc atg cag 1672
   Gln Asp Val Pro Asn Thr Gly Val Asp Lys Asn Thr Arg Val Met Gln
   395 400 405 410
   ctc att gag gca tac cgc tcc cgt gga cac ctc atc gct gac acc aac 1720
   Leu Ile Glu Ala Tyr Arg Ser Arg Gly His Leu Ile Ala Asp Thr Asn
   415 420 425
   cca ctt tca tgg gtt cag cct ggc atg cca gtt cca gac cac cgc gac 1768
   Pro Leu Ser Trp Val Gln Pro Gly Met Pro Val Pro Asp His Arg Asp
   430 435 440
   ctc gac atc gag acc cac agc ctg acc atc tgg gat ctg gac cgt acc 1816
   Leu Asp Ile Glu Thr His Ser Leu Thr Ile Trp Asp Leu Asp Arg Thr
   445 450 455
   ttc agc gtc ggt ggc ttc ggc ggc aag gag acc atg acc ctg cgc gag 1864
   Phe Ser Val Gly Gly Phe Gly Gly Lys Glu Thr Met Thr Leu Arg Glu
   460 465 470
   gta ctg tcc cgc ctg cgc gct gcc tac acc ttg aag gtc ggc tcc gaa 1912
   Val Leu Ser Arg Leu Arg Ala Ala Tyr Thr Leu Lys Val Gly Ser Glu
   475 480 485 490
   tac acc cac atc ctg gac cgc gac gag cgc acc tgg ctg cag gac cgc 1960
   Tyr Thr His Ile Leu Asp Arg Asp Glu Arg Thr Trp Leu Gln Asp Arg
   495 500 505
   ctc gaa gcc gga atg cca aag cca acc cag gca gag cag aag tac atc 2008
   Leu Glu Ala Gly Met Pro Lys Pro Thr Gln Ala Glu Gln Lys Tyr Ile
   510 515 520
   ctg cag aag ctg aac gcc gca gag gct ttc gag aac ttc ctg cag acc 2056
   Leu Gln Lys Leu Asn Ala Ala Glu Ala Phe Glu Asn Phe Leu Gln Thr
   525 530 535
   aag tac gtc ggc cag aag cgc ttc tcc ctc gaa ggt gca gaa gct ctc 2104
   Lys Tyr Val Gly Gln Lys Arg Phe Ser Leu Glu Gly Ala Glu Ala Leu
   540 545 550
   atc cca ctg atg gac tcc gcc atc gac acc gcc gca ggc cag ggc ctc 2152
   Ile Pro Leu Met Asp Ser Ala Ile Asp Thr Ala Ala Gly Gln Gly Leu
   555 560 565 570
   gac gaa gtt gtc atc ggt atg cca cac cgt ggt cgc ctc aac gtg ctg 2200
   Asp Glu Val Val Ile Gly Met Pro His Arg Gly Arg Leu Asn Val Leu
   575 580 585
   ttc aac atc gtg ggc aag cca ctg gca tcc atc ttc aac gag ttt gaa 2248
   Phe Asn Ile Val Gly Lys Pro Leu Ala Ser Ile Phe Asn Glu Phe Glu
   590 595 600
   ggc caa atg gag cag ggc cag atc ggt ggc tcc ggt gac gtg aag tac 2296
   Gly Gln Met Glu Gln Gly Gln Ile Gly Gly Ser Gly Asp Val Lys Tyr
   605 610 615
   cac ctc ggt tcc gaa ggc cag cac ctg cag atg ttc ggc gac ggc gag 2344
   His Leu Gly Ser Glu Gly Gln His Leu Gln Met Phe Gly Asp Gly Glu
   620 625 630
   atc aag gtc tcc ctg act gct aac ccg tcc cac ctg gaa gct gtt aac 2392
   Ile Lys Val Ser Leu Thr Ala Asn Pro Ser His Leu Glu Ala Val Asn
   635 640 645 650
   cca gtg atg gaa ggt atc gtc cgc gca aag cag gac tac ctg gac aag 2440
   Pro Val Met Glu Gly Ile Val Arg Ala Lys Gln Asp Tyr Leu Asp Lys
   655 660 665
   ggc gta gac ggc aag act gtt gtg cca ctg ctg ctc cac ggt gac gct 2488
   Gly Val Asp Gly Lys Thr Val Val Pro Leu Leu Leu His Gly Asp Ala
   670 675 680
   gca ttc gca ggc ctg ggc atc gtg cca gaa acc atc aac ctg gct ata 2536
   Ala Phe Ala Gly Leu Gly Ile Val Pro Glu Thr Ile Asn Leu Ala Ile
   685 690 695
   agc tgc gtc gac gtc gga ggc acc atc cac atc gtg gtg aac aac cag 2584
   Ser Cys Val Asp Val Gly Gly Thr Ile His Ile Val Val Asn Asn Gln
   700 705 710
   atc ggc ttc acc acc acc cca gac tcc agc cgc tcc atg cac tac gca 2632
   Ile Gly Phe Thr Thr Thr Pro Asp Ser Ser Arg Ser Met His Tyr Ala
   715 720 725 730
   acc gac tac gcc aag gca ttc ggc tgc cca gtc ttc cac gtc aat ggt 2680
   Thr Asp Tyr Ala Lys Ala Phe Gly Cys Pro Val Phe His Val Asn Gly
   735 740 745
   gat gac cca gag gca gtt gtc tgg gtt ggc cag ctg gca acc gag tac 2728
   Asp Asp Pro Glu Ala Val Val Trp Val Gly Gln Leu Ala Thr Glu Tyr
   750 755 760
   cgt cgt cgc ttc ggc aag gac gtc ttc atc gac ctc gtt tgc tac cgc 2776
   Arg Arg Arg Phe Gly Lys Asp Val Phe Ile Asp Leu Val Cys Tyr Arg
   765 770 775
   ctc cgc ggc cac aac gaa gct gat gat cct tcc atg acc cag cca aag 2824
   Leu Arg Gly His Asn Glu Ala Asp Asp Pro Ser Met Thr Gln Pro Lys
   780 785 790
   atg tat gag ctc atc acc ggc cgc gag acc gtt cgt gct cag tac acc 2872
   Met Tyr Glu Leu Ile Thr Gly Arg Glu Thr Val Arg Ala Gln Tyr Thr
   795 800 805 810
   gaa gac ctg ctc gga cgt gga gac ctc tcc aac gaa gat gca gaa gca 2920
   Glu Asp Leu Leu Gly Arg Gly Asp Leu Ser Asn Glu Asp Ala Glu Ala
   815 820 825
   gtc gtc cgc gac ttc cac gac cag atg gaa tct gtg ttc aac gaa gtc 2968
   Val Val Arg Asp Phe His Asp Gln Met Glu Ser Val Phe Asn Glu Val
   830 835 840
   aag gaa ggc ggc aag aag cag gct gag gca cag acc ggc atc acc ggc 3016
   Lys Glu Gly Gly Lys Lys Gln Ala Glu Ala Gln Thr Gly Ile Thr Gly
   845 850 855
   tcc cag aag ctt cca cac ggc ctt gag acc aac atc tcc cgt gaa gag 3064
   Ser Gln Lys Leu Pro His Gly Leu Glu Thr Asn Ile Ser Arg Glu Glu
   860 865 870
   ctc ctg gaa ctg gga cag gct ttc gcc aac acc cca gaa ggc ttc aac 3112
   Leu Leu Glu Leu Gly Gln Ala Phe Ala Asn Thr Pro Glu Gly Phe Asn
   875 880 885 890
   tac cac cca cgt gtg gct cca gtt gct aag aag cgc gtc tcc tct gtc 3160
   Tyr His Pro Arg Val Ala Pro Val Ala Lys Lys Arg Val Ser Ser Val
   895 900 905
   acc gaa ggt ggc atc gac tgg gca tgg ggc gag ctc ctc gcc ttc ggt 3208
   Thr Glu Gly Gly Ile Asp Trp Ala Trp Gly Glu Leu Leu Ala Phe Gly
   910 915 920
   tcc ctg gct aac tcc ggc cgc ttg gtt cgc ctt gca ggt gaa gat tcc 3256
   Ser Leu Ala Asn Ser Gly Arg Leu Val Arg Leu Ala Gly Glu Asp Ser
   925 930 935
   cgc cgc ggt acc ttc acc cag cgc cac gca gtt gcc atc gac cca gcg 3304
   Arg Arg Gly Thr Phe Thr Gln Arg His Ala Val Ala Ile Asp Pro Ala
   940 945 950
   acc gct gaa gag ttc aac cca ctc cac gag ctt gca cag tcc aag ggc 3352
   Thr Ala Glu Glu Phe Asn Pro Leu His Glu Leu Ala Gln Ser Lys Gly
   955 960 965 970
   aac aac ggt aag ttc ctg gtc tac aac tcc gca ctg acc gag tac gca 3400
   Asn Asn Gly Lys Phe Leu Val Tyr Asn Ser Ala Leu Thr Glu Tyr Ala
   975 980 985
   ggc atg ggc ttc gag tac ggc tac tcc gta gga aac gaa gac tcc gtc 3448
   Gly Met Gly Phe Glu Tyr Gly Tyr Ser Val Gly Asn Glu Asp Ser Val
   990 995 1000
   gtt gca tgg gaa gca cag ttc ggc gac ttc gcc aac ggc gct cag 3493
   Val Ala Trp Glu Ala Gln Phe Gly Asp Phe Ala Asn Gly Ala Gln
   1005 1010 1015
   acc atc atc gat gag tac gtc tcc tca ggc gaa gct aag tgg ggc 3538
   Thr Ile Ile Asp Glu Tyr Val Ser Ser Gly Glu Ala Lys Trp Gly
   1020 1025 1030
   cag acc tcc aag ctg atc ctt ctg ctg cct cac ggc tac gaa ggc 3583
   Gln Thr Ser Lys Leu Ile Leu Leu Leu Pro His Gly Tyr Glu Gly
   1035 1040 1045
   cag ggc cca gac cac tct tcc gca cgt atc gag cgc ttc ctg cag 3628
   Gln Gly Pro Asp His Ser Ser Ala Arg Ile Glu Arg Phe Leu Gln
           1050                1055                1060
   ctg tgc gct gag ggt tcc atg act gtt gct cag cca tcc acc cca      3673
   Leu Cys Ala Glu Gly Ser Met Thr Val Ala Gln Pro Ser Thr Pro
           1065                1070                1075
   gca aac cac ttc cac ctg ctg cgt cgt cac gct ctg tcc gac ctg      3718
   Ala Asn His Phe His Leu Leu Arg Arg His Ala Leu Ser Asp Leu
           1080                1085                1090
   aag cgt cca ctg gtt atc ttc acc ccg aag tcc atg ctg cgt aac      3763
   Lys Arg Pro Leu Val Ile Phe Thr Pro Lys Ser Met Leu Arg Asn
           1095                1100                1105
   aag gct gct gcc tcc gca cca gaa gac ttc act gag gtc acc aag      3808
   Lys Ala Ala Ala Ser Ala Pro Glu Asp Phe Thr Glu Val Thr Lys
           1110                1115                1120
   ttc caa tcc gtg atc gac gat cca aac gtt gca gat gca gcc aag      3853
   Phe Gln Ser Val Ile Asp Asp Pro Asn Val Ala Asp Ala Ala Lys
           1125                1130                1135
   gtg aag aag gtc atg ctg gtc tcc ggc aag ctg tac tac gaa ttg      3898
   Val Lys Lys Val Met Leu Val Ser Gly Lys Leu Tyr Tyr Glu Leu
           1140                1145                1150
   gca aag cgc aag gag aag gac gga cgc gac gac atc gcg atc gtt      3943
   Ala Lys Arg Lys Glu Lys Asp Gly Arg Asp Asp Ile Ala Ile Val
           1155                1160                1165
   cgt atc gaa atg ctc cac cca att ccg ttc aac cgc atc tcc gag      3988
   Arg Ile Glu Met Leu His Pro Ile Pro Phe Asn Arg Ile Ser Glu
           1170                1175                1180
   gct ctt gcc ggc tac cct aac gct gag gaa gtc ctc ttc gtt cag      4033
   Ala Leu Ala Gly Tyr Pro Asn Ala Glu Glu Val Leu Phe Val Gln
           1185                1190                1195
   gat gag cca gca aac cag ggc cca tgg ccg ttc tac cag gag cac      4078
   Asp Glu Pro Ala Asn Gln Gly Pro Trp Pro Phe Tyr Gln Glu His
           1200                1205                1210
   ctc cca gag ctg atc ccg aac atg cca aag atg cgc cgc gtt tcc      4123
   Leu Pro Glu Leu Ile Pro Asn Met Pro Lys Met Arg Arg Val Ser
           1215                1220                1225
   cgc cgc gct cag tcc tcc acc gca act ggt gtt gct aag gtg cac      4168
   Arg Arg Ala Gln Ser Ser Thr Ala Thr Gly Val Ala Lys Val His
           1230                1235                1240
   cag ctg gag gag aag cag ctt atc gac gag gct ttc gag gct          4210
   Gln Leu Glu Glu Lys Gln Leu Ile Asp Glu Ala Phe Glu Ala
           1245                1250                1255
   taagtcttta tagtcctgca ctagcctaga gggccttatg cagtgtgaat cacacagcat 4270
   aaggcccttt ttgctgccgt ggttgcctaa ggtggaaggc atgaaacgaa tctgtgcggt 4330
   cacgatctct tcagtacttt tgctaagtgg ctgctcctcc acttccacca cgcagctcga 4390
   g                                                                  4391
<210> 49
   <211> 1256
   <212> PRT
   <213> Corynebacterium glutamicum
<400> 49
   Met Leu Gln Leu Gly Leu Arg His Asn Gln Pro Thr Thr Asn Val Thr
   1               5                   10                  15
   Val Asp Lys Ile Lys Leu Asn Lys Pro Ser Arg Ser Lys Glu Lys Arg
               20                  25                  30
   Arg Val Pro Ala Val Ser Ser Ala Ser Thr Phe Gly Gln Asn Ala Trp
           35                  40                  45
   Leu Val Asp Glu Met Phe Gln Gln Phe Gln Lys Asp Pro Lys Ser Val
       50                  55                  60
   Asp Lys Glu Trp Arg Glu Leu Phe Glu Ala Gln Gly Gly Pro Asn Ala
   65                  70                  75                  80
   Thr Pro Ala Thr Thr Glu Ala Gln Pro Ser Ala Pro Lys Glu Ser Ala
                   85                  90                  95
   Lys Pro Ala Pro Lys Ala Ala Pro Ala Ala Lys Ala Ala Pro Arg Val
               100                 105                 110
   Glu Thr Lys Pro Ala Ala Lys Thr Ala Pro Lys Ala Lys Glu Ser Ser
           115                 120                 125
   Val Pro Gln Gln Pro Lys Leu Pro Glu Pro Gly Gln Thr Pro Ile Arg
       130                 135                 140
   Gly Ile Phe Lys Ser Ile Ala Lys Asn Met Asp Ile Ser Leu Glu Ile
   145                 150                 155                 160
   Pro Thr Ala Thr Ser Val Arg Asp Met Pro Ala Arg Leu Met Phe Glu
                   165                 170                 175
   Asn Arg Ala Met Val Asn Asp Gln Leu Lys Arg Thr Arg Gly Gly Lys
               180                 185                 190
   Ile Ser Phe Thr His Ile Ile Gly Tyr Ala Met Val Lys Ala Val Met
           195                 200                 205
   Ala His Pro Asp Met Asn Asn Ser Tyr Asp Val Ile Asp Gly Lys Pro
       210                 215                 220
   Thr Leu Ile Val Pro Glu His Ile Asn Leu Gly Leu Ala Ile Asp Leu
   225                 230                 235                 240
   Pro Gln Lys Asp Gly Ser Arg Ala Leu Val Val Ala Ala Ile Lys Glu
                   245                 250                 255
   Thr Glu Lys Met Asn Phe Ser Glu Phe Leu Ala Ala Tyr Glu Asp Ile
               260                 265                 270
   Val Thr Arg Ser Arg Lys Gly Lys Leu Thr Met Asp Asp Tyr Gln Gly
           275                 280                 285
   Val Thr Val Ser Leu Thr Asn Pro Gly Gly Ile Gly Thr Arg His Ser
       290                 295                 300
   Val Pro Arg Leu Thr Lys Gly Gln Gly Thr Ile Ile Gly Val Gly Ser
   305                 310                 315                 320
   Met Asp Tyr Pro Ala Glu Phe Gln Gly Ala Ser Glu Asp Arg Leu Ala
                   325                 330                 335
   Glu Leu Gly Val Gly Lys Leu Val Thr Ile Thr Ser Thr Tyr Asp His
               340                 345                 350
   Arg Val Ile Gln Gly Ala Val Ser Gly Glu Phe Leu Arg Thr Met Ser
   355 360 365
   Arg Leu Leu Thr Asp Asp Ser Phe Trp Asp Glu Ile Phe Asp Ala Met
   370 375 380
   Asn Val Pro Tyr Thr Pro Met Arg Trp Ala Gln Asp Val Pro Asn Thr
   385 390 395 400
   Gly Val Asp Lys Asn Thr Arg Val Met Gln Leu Ile Glu Ala Tyr Arg
   405 410 415
   Ser Arg Gly His Leu Ile Ala Asp Thr Asn Pro Leu Ser Trp Val Gln
   420 425 430
   Pro Gly Met Pro Val Pro Asp His Arg Asp Leu Asp Ile Glu Thr His
   435 440 445
   Ser Leu Thr Ile Trp Asp Leu Asp Arg Thr Phe Ser Val Gly Gly Phe
   450 455 460
   Gly Gly Lys Glu Thr Met Thr Leu Arg Glu Val Leu Ser Arg Leu Arg
   465 470 475 480
   Ala Ala Tyr Thr Leu Lys Val Gly Ser Glu Tyr Thr His Ile Leu Asp
   485 490 495
   Arg Asp Glu Arg Thr Trp Leu Gln Asp Arg Leu Glu Ala Gly Met Pro
   500 505 510
   Lys Pro Thr Gln Ala Glu Gln Lys Tyr Ile Leu Gln Lys Leu Asn Ala
   515 520 525
   Ala Glu Ala Phe Glu Asn Phe Leu Gln Thr Lys Tyr Val Gly Gln Lys
   530 535 540
   Arg Phe Ser Leu Glu Gly Ala Glu Ala Leu Ile Pro Leu Met Asp Ser
   545 550 555 560
   Ala Ile Asp Thr Ala Ala Gly Gln Gly Leu Asp Glu Val Val Ile Gly
   565 570 575
   Met Pro His Arg Gly Arg Leu Asn Val Leu Phe Asn Ile Val Gly Lys
   580 585 590
   Pro Leu Ala Ser Ile Phe Asn Glu Phe Glu Gly Gln Met Glu Gln Gly
   595 600 605
   Gln Ile Gly Gly Ser Gly Asp Val Lys Tyr His Leu Gly Ser Glu Gly
   610 615 620
   Gln His Leu Gln Met Phe Gly Asp Gly Glu Ile Lys Val Ser Leu Thr
   625 630 635 640
   Ala Asn Pro Ser His Leu Glu Ala Val Asn Pro Val Met Glu Gly Ile
   645 650 655
   Val Arg Ala Lys Gln Asp Tyr Leu Asp Lys Gly Val Asp Gly Lys Thr
   660 665 670
   Val Val Pro Leu Leu Leu His Gly Asp Ala Ala Phe Ala Gly Leu Gly
   675 680 685
   Ile Val Pro Glu Thr Ile Asn Leu Ala Ile Ser Cys Val Asp Val Gly
   690 695 700
   Gly Thr Ile His Ile Val Val Asn Asn Gln Ile Gly Phe Thr Thr Thr
   705 710 715 720
   Pro Asp Ser Ser Arg Ser Met His Tyr Ala Thr Asp Tyr Ala Lys Ala
   725 730 735
   Phe Gly Cys Pro Val Phe His Val Asn Gly Asp Asp Pro Glu Ala Val
   740 745 750
   Val Trp Val Gly Gln Leu Ala Thr Glu Tyr Arg Arg Arg Phe Gly Lys
   755 760 765
   Asp Val Phe Ile Asp Leu Val Cys Tyr Arg Leu Arg Gly His Asn Glu
   770 775 780
   Ala Asp Asp Pro Ser Met Thr Gln Pro Lys Met Tyr Glu Leu Ile Thr
   785 790 795 800
   Gly Arg Glu Thr Val Arg Ala Gln Tyr Thr Glu Asp Leu Leu Gly Arg
   805 810 815
   Gly Asp Leu Ser Asn Glu Asp Ala Glu Ala Val Val Arg Asp Phe His
   820 825 830
   Asp Gln Met Glu Ser Val Phe Asn Glu Val Lys Glu Gly Gly Lys Lys
   835 840 845
   Gln Ala Glu Ala Gln Thr Gly Ile Thr Gly Ser Gln Lys Leu Pro His
   850 855 860
   Gly Leu Glu Thr Asn Ile Ser Arg Glu Glu Leu Leu Glu Leu Gly Gln
   865 870 875 880
   Ala Phe Ala Asn Thr Pro Glu Gly Phe Asn Tyr His Pro Arg Val Ala
   885 890 895
   Pro Val Ala Lys Lys Arg Val Ser Ser Val Thr Glu Gly Gly Ile Asp
   900 905 910
   Trp Ala Trp Gly Glu Leu Leu Ala Phe Gly Ser Leu Ala Asn Ser Gly
   915 920 925
   Arg Leu Val Arg Leu Ala Gly Glu Asp Ser Arg Arg Gly Thr Phe Thr
   930 935 940
   Gln Arg His Ala Val Ala Ile Asp Pro Ala Thr Ala Glu Glu Phe Asn
   945 950 955 960
   Pro Leu His Glu Leu Ala Gln Ser Lys Gly Asn Asn Gly Lys Phe Leu
   965 970 975
   Val Tyr Asn Ser Ala Leu Thr Glu Tyr Ala Gly Met Gly Phe Glu Tyr
   980 985 990
   Gly Tyr Ser Val Gly Asn Glu Asp Ser Val Val Ala Trp Glu Ala Gln
   995 1000 1005
   Phe Gly Asp Phe Ala Asn Gly Ala Gln Thr Ile Ile Asp Glu Tyr
   1010 1015 1020
   Val Ser Ser Gly Glu Ala Lys Trp Gly Gln Thr Ser Lys Leu Ile
   1025 1030 1035
   Leu Leu Leu Pro His Gly Tyr Glu Gly Gln Gly Pro Asp His Ser
   1040 1045 1050
   Ser Ala Arg Ile Glu Arg Phe Leu Gln Leu Cys Ala Glu Gly Ser
   1055 1060 1065
   Met Thr Val Ala Gln Pro Ser Thr Pro Ala Asn His Phe His Leu
   1070 1075 1080
   Leu Arg Arg His Ala Leu Ser Asp Leu Lys Arg Pro Leu Val Ile
   1085 1090 1095
   Phe Thr Pro Lys Ser Met Leu Arg Asn Lys Ala Ala Ala Ser Ala
   1100 1105 1110
   Pro Glu Asp Phe Thr Glu Val Thr Lys Phe Gln Ser Val Ile Asp
   1115 1120 1125
   Asp Pro Asn Val Ala Asp Ala Ala Lys Val Lys Lys Val Met Leu
   1130 1135 1140
   Val Ser Gly Lys Leu Tyr Tyr Glu Leu Ala Lys Arg Lys Glu Lys
   1145 1150 1155
   Asp Gly Arg Asp Asp Ile Ala Ile Val Arg Ile Glu Met Leu His
   1160 1165 1170
   Pro Ile Pro Phe Asn Arg Ile Ser Glu Ala Leu Ala Gly Tyr Pro
   1175 1180 1185
   Asn Ala Glu Glu Val Leu Phe Val Gln Asp Glu Pro Ala Asn Gln
   1190 1195 1200
   Gly Pro Trp Pro Phe Tyr Gln Glu His Leu Pro Glu Leu Ile Pro
   1205 1210 1215
   Asn Met Pro Lys Met Arg Arg Val Ser Arg Arg Ala Gln Ser Ser
   1220 1225 1230
   Thr Ala Thr Gly Val Ala Lys Val His Gln Leu Glu Glu Lys Gln
   1235 1240 1245
   Leu Ile Asp Glu Ala Phe Glu Ala
   1250 1255
<210> 50
   <211> 3774
   <212> DNA
   <213> Corynebacterium glutamicum
<220>
   <221> CDS
   <222> (1)..(3774)
   <223>
<400> 50
   atg cta caa ctg ggg ctt agg cat aat cag cca acg acc aac gtt aca 48
   Met Leu Gln Leu Gly Leu Arg His Asn Gln Pro Thr Thr Asn Val Thr
   1 5 10 15
   gtg gat aaa aca aag ctc aat aaa ccc tca aga agc aag gaa aag agg 96
   Val Asp Lys Thr Lys Leu Asn Lys Pro Ser Arg Ser Lys Glu Lys Arg
   20 25 30
   cga gta cct gcc gtg agc agc gct agt act ttc ggc cag aat gcg tgg 144
   Arg Val Pro Ala Val Ser Ser Ala Ser Thr Phe Gly Gln Asn Ala Trp
   35 40 45
   ctg gta gac gag atg ttc cag cag ttc cag aag gac ccc aag tcc gtg 192
   Leu Val Asp Glu Met Phe Gln Gln Phe Gln Lys Asp Pro Lys Ser Val
   50 55 60
   gac aag gaa tgg aga gaa ctc ttt gag gcg cag ggg gga cca aat act 240
   Asp Lys Glu Trp Arg Glu Leu Phe Glu Ala Gln Gly Gly Pro Asn Thr
   65 70 75 80
   acc ccc gct aca aca gaa gca cag cct tca gcg ccc aag gag tct gcg 288
   Thr Pro Ala Thr Thr Glu Ala Gln Pro Ser Ala Pro Lys Glu Ser Ala
                       85                  90                  95
   aaa cca gca cca aag gct gcc cct gca gcc aag gca gca ccg cgc gta      336
   Lys Pro Ala Pro Lys Ala Ala Pro Ala Ala Lys Ala Ala Pro Arg Val
                   100                 105                 110
   gaa acc aag ccg gcc gac aag acc gcc cct aag gcc aag gag tcc tca      384
   Glu Thr Lys Pro Ala Asp Lys Thr Ala Pro Lys Ala Lys Glu Ser Ser
               115                 120                 125
   gtg cca cag caa cct aag ctt ccg gag cca gga caa acc cca atc agg      432
   Val Pro Gln Gln Pro Lys Leu Pro Glu Pro Gly Gln Thr Pro Ile Arg
           130                 135                 140
   ggt att ttc aag tcc atc gcg aag aac atg gat atc tcc ctg gaa atc      480
   Gly Ile Phe Lys Ser Ile Ala Lys Asn Met Asp Ile Ser Leu Glu Ile
   145                 150                 155                 160
   cca acc gca acc tcg gtt cgc gat atg cca gct cgc ctc atg ttc gaa      528
   Pro Thr Ala Thr Ser Val Arg Asp Met Pro Ala Arg Leu Met Phe Glu
                   165                 170                 175
   aac cgc gcg atg gtc aac gat cag ctc aag cgc acc cgc ggt ggc aag      576
   Asn Arg Ala Met Val Asn Asp Gln Leu Lys Arg Thr Arg Gly Gly Lys
               180                 185                 190
   atc tcc ttc acc cac atc att ggc tac gcc atg gtg aag gca gtc atg      624
   Ile Ser Phe Thr His Ile Ile Gly Tyr Ala Met Val Lys Ala Val Met
           195                 200                 205
   gct cac ccg gac atg aac aac tcc tac gac gtc atc gac ggc aag cca      672
   Ala His Pro Asp Met Asn Asn Ser Tyr Asp Val Ile Asp Gly Lys Pro
       210                 215                 220
   acc ctg atc gtg cct gag cac atc aac ctg ggc ctt gct atc gac ctt      720
   Thr Leu Ile Val Pro Glu His Ile Asn Leu Gly Leu Ala Ile Asp Leu
   225                 230                 235                 240
   cct cag aag gac ggc tcc cgc gca ctt gtc gta gca gcc atc aag gaa      768
   Pro Gln Lys Asp Gly Ser Arg Ala Leu Val Val Ala Ala Ile Lys Glu
                   245                 250                 255
   acc gag aag atg aac ttc tcc gag ttc ctc gca gcc tac gaa gac atc      816
   Thr Glu Lys Met Asn Phe Ser Glu Phe Leu Ala Ala Tyr Glu Asp Ile
               260                 265                 270
   gtg gca cgc tcc cgc aag ggc aag ctc acc atg gat gac tac cag ggc      864
   Val Ala Arg Ser Arg Lys Gly Lys Leu Thr Met Asp Asp Tyr Gln Gly
           275                 280                 285
   gtt acc gtt tcc ttg acc aac cca ggt ggc atc ggt acc cgc cac tct      912
   Val Thr Val Ser Leu Thr Asn Pro Gly Gly Ile Gly Thr Arg His Ser
       290                 295                 300
   gtt cca cgt cta acc aag ggc cag ggc acc atc atc ggt gtc ggt tcc      960
   Val Pro Arg Leu Thr Lys Gly Gln Gly Thr Ile Ile Gly Val Gly Ser
   305                 310                 315                 320
   atg gat tac cca gca gag ttc cag ggc gct tca gaa gac cgc ctt gca     1008
   Met Asp Tyr Pro Ala Glu Phe Gln Gly Ala Ser Glu Asp Arg Leu Ala
                   325                 330                 335
   gag ctc ggc gtt ggc aaa ctt gtc acc atc acc tcc acc tac gat cac     1056
   Glu Leu Gly Val Gly Lys Leu Val Thr Ile Thr Ser Thr Tyr Asp His
   340 345 350
   cgc gtg atc cag ggt gct gtg tcc ggt gaa ttc ctg cgc acc atg tct 1104
   Arg Val Ile Gln Gly Ala Val Ser Gly Glu Phe Leu Arg Thr Met Ser
   355 360 365
   cgc ctg ctc acc gat gat tcc ttc tgg gat gag atc ttc gac gca atg 1152
   Arg Leu Leu Thr Asp Asp Ser Phe Trp Asp Glu Ile Phe Asp Ala Met
   370 375 380
   aac gtt cct tac acc cca atg cgt tgg gca cag gac gtt cca aac acc 1200
   Asn Val Pro Tyr Thr Pro Met Arg Trp Ala Gln Asp Val Pro Asn Thr
   385 390 395 400
   ggt gtt gat aag aac acc cgc gtc atg cag ctc att gag gca tac cgc 1248
   Gly Val Asp Lys Asn Thr Arg Val Met Gln Leu Ile Glu Ala Tyr Arg
   405 410 415
   tcc cgt gga cac ctc atc gct gac acc aac cca ctt tca tgg gtt cag 1296
   Ser Arg Gly His Leu Ile Ala Asp Thr Asn Pro Leu Ser Trp Val Gln
   420 425 430
   cct ggc atg cca gtt cca gac cac cgc gac ctc gac atc gag acc cac 1344
   Pro Gly Met Pro Val Pro Asp His Arg Asp Leu Asp Ile Glu Thr His
   435 440 445
   aac ctg acc atc tgg gat ctg gac cgt acc ttc aac gtc ggt ggc ttc 1392
   Asn Leu Thr Ile Trp Asp Leu Asp Arg Thr Phe Asn Val Gly Gly Phe
   450 455 460
   ggc ggc aag gag acc atg acc ctg cgc gag gta ctg tcc cgc ctc cgc 1440
   Gly Gly Lys Glu Thr Met Thr Leu Arg Glu Val Leu Ser Arg Leu Arg
   465 470 475 480
   gct gcg tac acc ctc aag gtc ggc tcc gaa tac acc cac atc ctg gac 1488
   Ala Ala Tyr Thr Leu Lys Val Gly Ser Glu Tyr Thr His Ile Leu Asp
   485 490 495
   cgc gac gag cgc acc tgg ctg cag gac cgc ctc gag gcc gga atg cca 1536
   Arg Asp Glu Arg Thr Trp Leu Gln Asp Arg Leu Glu Ala Gly Met Pro
   500 505 510
   aag cca acc cag gca gag cag aag tac atc ctg cag aag ctg aac gcc 1584
   Lys Pro Thr Gln Ala Glu Gln Lys Tyr Ile Leu Gln Lys Leu Asn Ala
   515 520 525
   gcg gag gct ttc gag aac ttc ctg cag acc aag tac gtc ggc cag aag 1632
   Ala Glu Ala Phe Glu Asn Phe Leu Gln Thr Lys Tyr Val Gly Gln Lys
   530 535 540
   cgc ttc tcc ctc gaa ggt gca gaa gca ctt atc cca ctg atg gac tcc 1680
   Arg Phe Ser Leu Glu Gly Ala Glu Ala Leu Ile Pro Leu Met Asp Ser
   545 550 555 560
   gcc atc gac acc gcc gca ggc caa ggc ctc gac gaa gtt gtc atc ggt 1728
   Ala Ile Asp Thr Ala Ala Gly Gln Gly Leu Asp Glu Val Val Ile Gly
   565 570 575
   atg cca cac cgt ggt cgc ctc aac gtg ctg ttc aac atc gtg ggc aag 1776
   Met Pro His Arg Gly Arg Leu Asn Val Leu Phe Asn Ile Val Gly Lys
   580 585 590
   cca ctg gca tcc atc ttc aac gag ttt gaa ggc caa atg gag cag ggc 1824
   Pro Leu Ala Ser Ile Phe Asn Glu Phe Glu Gly Gln Met Glu Gln Gly
   595 600 605
   cag atc ggt ggc tcc ggt gac gtg aag tac cac ctc ggt tcc gaa ggc 1872
   Gln Ile Gly Gly Ser Gly Asp Val Lys Tyr His Leu Gly Ser Glu Gly
   610 615 620
   cag cac ctg cag atg ttc ggc gac ggc gag atc aag gtc tcc ctg act 1920
   Gln His Leu Gln Met Phe Gly Asp Gly Glu Ile Lys Val Ser Leu Thr
   625 630 635 640
   gct aac ccg tcc cac ctg gaa gct gtt aac cca gtg atg gaa ggt atc 1968
   Ala Asn Pro Ser His Leu Glu Ala Val Asn Pro Val Met Glu Gly Ile
   645 650 655
   gtc cgc gca aag cag gac tac ctg gac aag ggc gta gac ggc aag act 2016
   Val Arg Ala Lys Gln Asp Tyr Leu Asp Lys Gly Val Asp Gly Lys Thr
   660 665 670
   gtt gtg cca ctg ctg ctc cac ggt gac gct gca ttc gca ggc ctg ggc 2064
   Val Val Pro Leu Leu Leu His Gly Asp Ala Ala Phe Ala Gly Leu Gly
   675 680 685
   atc gtg cca gaa acc atc aac ctg gct aag ctg cgt ggc tac gac gtc 2112
   Ile Val Pro Glu Thr Ile Asn Leu Ala Lys Leu Arg Gly Tyr Asp Val
   690 695 700
   ggc ggc acc atc cac atc gtg gtg aac aac cag atc ggc ttc acc acc 2160
   Gly Gly Thr Ile His Ile Val Val Asn Asn Gln Ile Gly Phe Thr Thr
   705 710 715 720
   acc cca gac tcc agc cgc tcc atg cac tac gca acc gac tac gcc aag 2208
   Thr Pro Asp Ser Ser Arg Ser Met His Tyr Ala Thr Asp Tyr Ala Lys
   725 730 735
   gca ttc ggc tgc cca gtc ttc cac gtc aac ggc gac gac cca gag gca 2256
   Ala Phe Gly Cys Pro Val Phe His Val Asn Gly Asp Asp Pro Glu Ala
   740 745 750
   gtt gtc tgg gtt ggc cag ctg gcc acc gag tac cgt cgt cgc ttc ggc 2304
   Val Val Trp Val Gly Gln Leu Ala Thr Glu Tyr Arg Arg Arg Phe Gly
   755 760 765
   aag gac gtc ttc atc gac ctc gtc tgc tac cgc ctc cgc ggc cac aac 2352
   Lys Asp Val Phe Ile Asp Leu Val Cys Tyr Arg Leu Arg Gly His Asn
   770 775 780
   gaa gct gat gat cct tcc atg acc cag cca aag atg tat gag ctc atc 2400
   Glu Ala Asp Asp Pro Ser Met Thr Gln Pro Lys Met Tyr Glu Leu Ile
   785 790 795 800
   acc ggc cgc gag acc gtt cgt gct cag tac acc gaa gac ctg ctc gga 2448
   Thr Gly Arg Glu Thr Val Arg Ala Gln Tyr Thr Glu Asp Leu Leu Gly
   805 810 815
   cgt gga gac ctc tcc aac gaa gat gca gaa gca gtc gtc cgc gac ttc 2496
   Arg Gly Asp Leu Ser Asn Glu Asp Ala Glu Ala Val Val Arg Asp Phe
   820 825 830
   cac gac cag atg gaa tct gtg ttc aac gaa gtc aag gaa ggc ggc aag 2544
   His Asp Gln Met Glu Ser Val Phe Asn Glu Val Lys Glu Gly Gly Lys
   835 840 845
   aag cag gct gag gca cag acc ggc atc acc ggc tcc cag aag ctt cca 2592
   Lys Gln Ala Glu Ala Gln Thr Gly Ile Thr Gly Ser Gln Lys Leu Pro
   850 855 860
   cac ggc ctt gag acc aac atc tcc cgt gaa gag ctc ctg gaa ctg gga 2640
   His Gly Leu Glu Thr Asn Ile Ser Arg Glu Glu Leu Leu Glu Leu Gly
   865 870 875 880
   cag gct ttc gcc aac acc cca gaa ggc ttc aac tac cac cca cgt gtg 2688
   Gln Ala Phe Ala Asn Thr Pro Glu Gly Phe Asn Tyr His Pro Arg Val
   885 890 895
   gct ccc gtt gct aag aag cgc gtc tcc tct gtc acc gaa ggt ggc atc 2736
   Ala Pro Val Ala Lys Lys Arg Val Ser Ser Val Thr Glu Gly Gly Ile
   900 905 910
   gac tgg gca tgg ggc gag ctc ctc gcc ttc ggt tcc ctg gct aac tcc 2784
   Asp Trp Ala Trp Gly Glu Leu Leu Ala Phe Gly Ser Leu Ala Asn Ser
   915 920 925
   ggc cgc ttg gtt cgc ctt gca ggt gaa gat tcc cgc cgc ggt acc ttc 2832
   Gly Arg Leu Val Arg Leu Ala Gly Glu Asp Ser Arg Arg Gly Thr Phe
   930 935 940
   acc cag cgc cac gca gtt gcc atc gac cca gcg acc gct gaa gag ttc 2880
   Thr Gln Arg His Ala Val Ala Ile Asp Pro Ala Thr Ala Glu Glu Phe
   945 950 955 960
   aac cca ctc cac gag ctt gca cag tcc aag ggc aac aac ggt aag ttc 2928
   Asn Pro Leu His Glu Leu Ala Gln Ser Lys Gly Asn Asn Gly Lys Phe
   965 970 975
   ctg gtc tac aac tcc gca ctg acc gag tac gca ggc atg ggc ttc gag 2976
   Leu Val Tyr Asn Ser Ala Leu Thr Glu Tyr Ala Gly Met Gly Phe Glu
   980 985 990
   tac ggc tac tcc gta gga aac gaa gac tcc atc gtt gca tgg gaa gca 3024
   Tyr Gly Tyr Ser Val Gly Asn Glu Asp Ser Ile Val Ala Trp Glu Ala
   995 1000 1005
   cag ttc ggc gac ttc gcc aac ggc gct cag acc atc atc gat gag 3069
   Gln Phe Gly Asp Phe Ala Asn Gly Ala Gln Thr Ile Ile Asp Glu
   1010 1015 1020
   tac gtc tcc tca ggc gaa gct aag tgg ggc cag acc tcc aag ctg 3114
   Tyr Val Ser Ser Gly Glu Ala Lys Trp Gly Gln Thr Ser Lys Leu
   1025 1030 1035
   atc ctt ctg ctg cct cac ggc tac gaa ggc cag ggc cca gac cac 3159
   Ile Leu Leu Leu Pro His Gly Tyr Glu Gly Gln Gly Pro Asp His
   1040 1045 1050
   tct tcc gca cgt atc gag cgc ttc ctg cag ctg tgc gct gag ggt 3204
   Ser Ser Ala Arg Ile Glu Arg Phe Leu Gln Leu Cys Ala Glu Gly
   1055 1060 1065
   tcc atg act gtt gct cag cca tcc acc cca gca aac cac ttc cac 3249
   Ser Met Thr Val Ala Gln Pro Ser Thr Pro Ala Asn His Phe His
   1070 1075 1080
   cta ctg cgt cgt cac gct ctg tcc gac ctg aag cgt cca ctg gtt 3294
   Leu Leu Arg Arg His Ala Leu Ser Asp Leu Lys Arg Pro Leu Val
   1085 1090 1095
   atc ttc acc ccg aag tcc atg ctg cgt aac aag gct gct gcc tcc 3339
   Ile Phe Thr Pro Lys Ser Met Leu Arg Asn Lys Ala Ala Ala Ser
   1100 1105 1110
   gca cca gaa gac ttc act gag gtc acc aag ttc cag tcc gtg atc 3384
   Ala Pro Glu Asp Phe Thr Glu Val Thr Lys Phe Gln Ser Val Ile
   1115 1120 1125
   aac gat cca aac gtt gca gat gca gcc aag gtg aag aag gtc atg 3429
   Asn Asp Pro Asn Val Ala Asp Ala Ala Lys Val Lys Lys Val Met
   1130 1135 1140
   ctg gtc tcc ggc aag ctg tac tac gaa ttg gca aag cgc aag gag 3474
   Leu Val Ser Gly Lys Leu Tyr Tyr Glu Leu Ala Lys Arg Lys Glu
   1145 1150 1155
   aag gac gga cgc gac gac atc gcg atc gtt cgt atc gaa atg ctc 3519
   Lys Asp Gly Arg Asp Asp Ile Ala Ile Val Arg Ile Glu Met Leu
   1160 1165 1170
   cac cca att ccg ttc aac cgc atc tcc gag gct ctt gcc ggc tac 3564
   His Pro Ile Pro Phe Asn Arg Ile Ser Glu Ala Leu Ala Gly Tyr
   1175 1180 1185
   cct aac gct gag gaa gtc ctc ttc gtt cag gat gag cca gca aac 3609
   Pro Asn Ala Glu Glu Val Leu Phe Val Gln Asp Glu Pro Ala Asn
   1190 1195 1200
   cag ggc cca tgg ccg ttc tac cag gag cac ctc cca gag ctg atc 3654
   Gln Gly Pro Trp Pro Phe Tyr Gln Glu His Leu Pro Glu Leu Ile
   1205 1210 1215
   ccg aac atg cca aag atg cgc cgc gtt tcc cgc cgc gct cag tcc 3699
   Pro Asn Met Pro Lys Met Arg Arg Val Ser Arg Arg Ala Gln Ser
   1220 1225 1230
   tcc acc gca act ggt gtt gcc aag gtg cac cag ctg gag gag aag 3744
   Ser Thr Ala Thr Gly Val Ala Lys Val His Gln Leu Glu Glu Lys
   1235 1240 1245
   cag ctt atc gac gag gct ttc gag gct taa 3774
   Gln Leu Ile Asp Glu Ala Phe Glu Ala
   1250 1255
<210> 51
   <211> 1257
   <212> PRT
   <213> Corynebacterium glutamicum
<400> 51
   Met Leu Gln Leu Gly Leu Arg His Asn Gln Pro Thr Thr Asn Val Thr
   1 5 10 15
   Val Asp Lys Thr Lys Leu Asn Lys Pro Ser Arg Ser Lys Glu Lys Arg
   20 25 30
   Arg Val Pro Ala Val Ser Ser Ala Ser Thr Phe Gly Gln Asn Ala Trp
   35 40 45
   Leu Val Asp Glu Met Phe Gln Gln Phe Gln Lys Asp Pro Lys Ser Val
   50 55 60
   Asp Lys Glu Trp Arg Glu Leu Phe Glu Ala Gln Gly Gly Pro Asn Thr
   65 70 75 80
   Thr Pro Ala Thr Thr Glu Ala Gln Pro Ser Ala Pro Lys Glu Ser Ala
   85 90 95
   Lys Pro Ala Pro Lys Ala Ala Pro Ala Ala Lys Ala Ala Pro Arg Val
   100 105 110
   Glu Thr Lys Pro Ala Asp Lys Thr Ala Pro Lys Ala Lys Glu Ser Ser
   115 120 125
   Val Pro Gln Gln Pro Lys Leu Pro Glu Pro Gly Gln Thr Pro Ile Arg
   130 135 140
   Gly Ile Phe Lys Ser Ile Ala Lys Asn Met Asp Ile Ser Leu Glu Ile
   145 150 155 160
   Pro Thr Ala Thr Ser Val Arg Asp Met Pro Ala Arg Leu Met Phe Glu
   165 170 175
   Asn Arg Ala Met Val Asn Asp Gln Leu Lys Arg Thr Arg Gly Gly Lys
   180 185 190
   Ile Ser Phe Thr His Ile Ile Gly Tyr Ala Met Val Lys Ala Val Met
   195 200 205
   Ala His Pro Asp Met Asn Asn Ser Tyr Asp Val Ile Asp Gly Lys Pro
   210 215 220
   Thr Leu Ile Val Pro Glu His Ile Asn Leu Gly Leu Ala Ile Asp Leu
   225 230 235 240
   Pro Gln Lys Asp Gly Ser Arg Ala Leu Val Val Ala Ala Ile Lys Glu
   245 250 255
   Thr Glu Lys Met Asn Phe Ser Glu Phe Leu Ala Ala Tyr Glu Asp Ile
   260 265 270
   Val Ala Arg Ser Arg Lys Gly Lys Leu Thr Met Asp Asp Tyr Gln Gly
   275 280 285
   Val Thr Val Ser Leu Thr Asn Pro Gly Gly Ile Gly Thr Arg His Ser
   290 295 300
   Val Pro Arg Leu Thr Lys Gly Gln Gly Thr Ile Ile Gly Val Gly Ser
   305 310 315 320
   Met Asp Tyr Pro Ala Glu Phe Gln Gly Ala Ser Glu Asp Arg Leu Ala
   325 330 335
   Glu Leu Gly Val Gly Lys Leu Val Thr Ile Thr Ser Thr Tyr Asp His
   340 345 350
   Arg Val Ile Gln Gly Ala Val Ser Gly Glu Phe Leu Arg Thr Met Ser
   355 360 365
   Arg Leu Leu Thr Asp Asp Ser Phe Trp Asp Glu Ile Phe Asp Ala Met
   370 375 380
   Asn Val Pro Tyr Thr Pro Met Arg Trp Ala Gln Asp Val Pro Asn Thr
   385 390 395 400
   Gly Val Asp Lys Asn Thr Arg Val Met Gln Leu Ile Glu Ala Tyr Arg
   405 410 415
   Ser Arg Gly His Leu Ile Ala Asp Thr Asn Pro Leu Ser Trp Val Gln
   420 425 430
   Pro Gly Met Pro Val Pro Asp His Arg Asp Leu Asp Ile Glu Thr His
   435 440 445
   Asn Leu Thr Ile Trp Asp Leu Asp Arg Thr Phe Asn Val Gly Gly Phe
   450 455 460
   Gly Gly Lys Glu Thr Met Thr Leu Arg Glu Val Leu Ser Arg Leu Arg
   465 470 475 480
   Ala Ala Tyr Thr Leu Lys Val Gly Ser Glu Tyr Thr His Ile Leu Asp
   485 490 495
   Arg Asp Glu Arg Thr Trp Leu Gln Asp Arg Leu Glu Ala Gly Met Pro
   500 505 510
   Lys Pro Thr Gln Ala Glu Gln Lys Tyr Ile Leu Gln Lys Leu Asn Ala
   515 520 525
   Ala Glu Ala Phe Glu Asn Phe Leu Gln Thr Lys Tyr Val Gly Gln Lys
   530 535 540
   Arg Phe Ser Leu Glu Gly Ala Glu Ala Leu Ile Pro Leu Met Asp Ser
   545 550 555 560
   Ala Ile Asp Thr Ala Ala Gly Gln Gly Leu Asp Glu Val Val Ile Gly
   565 570 575
   Met Pro His Arg Gly Arg Leu Asn Val Leu Phe Asn Ile Val Gly Lys
   580 585 590
   Pro Leu Ala Ser Ile Phe Asn Glu Phe Glu Gly Gln Met Glu Gln Gly
   595 600 605
   Gln Ile Gly Gly Ser Gly Asp Val Lys Tyr His Leu Gly Ser Glu Gly
   610 615 620
   Gln His Leu Gln Met Phe Gly Asp Gly Glu Ile Lys Val Ser Leu Thr
   625 630 635 640
   Ala Asn Pro Ser His Leu Glu Ala Val Asn Pro Val Met Glu Gly Ile
   645 650 655
   Val Arg Ala Lys Gln Asp Tyr Leu Asp Lys Gly Val Asp Gly Lys Thr
   660 665 670
   Val Val Pro Leu Leu Leu His Gly Asp Ala Ala Phe Ala Gly Leu Gly
   675 680 685
   Ile Val Pro Glu Thr Ile Asn Leu Ala Lys Leu Arg Gly Tyr Asp Val
   690 695 700
   Gly Gly Thr Ile His Ile Val Val Asn Asn Gln Ile Gly Phe Thr Thr
   705 710 715 720
   Thr Pro Asp Ser Ser Arg Ser Met His Tyr Ala Thr Asp Tyr Ala Lys
   725 730 735
   Ala Phe Gly Cys Pro Val Phe His Val Asn Gly Asp Asp Pro Glu Ala
   740 745 750
   Val Val Trp Val Gly Gln Leu Ala Thr Glu Tyr Arg Arg Arg Phe Gly
   755 760 765
   Lys Asp Val Phe Ile Asp Leu Val Cys Tyr Arg Leu Arg Gly His Asn
   770 775 780
   Glu Ala Asp Asp Pro Ser Met Thr Gln Pro Lys Met Tyr Glu Leu Ile
   785 790 795 800
   Thr Gly Arg Glu Thr Val Arg Ala Gln Tyr Thr Glu Asp Leu Leu Gly
   805 810 815
   Arg Gly Asp Leu Ser Asn Glu Asp Ala Glu Ala Val Val Arg Asp Phe
   820 825 830
   His Asp Gln Met Glu Ser Val Phe Asn Glu Val Lys Glu Gly Gly Lys
                   835                 840                 845
   Lys Gln Ala Glu Ala Gln Thr Gly Ile Thr Gly Ser Gln Lys Leu Pro
               850                 855                 860
   His Gly Leu Glu Thr Asn Ile Ser Arg Glu Glu Leu Leu Glu Leu Gly
   865                 870                 875                 880
   Gln Ala Phe Ala Asn Thr Pro Glu Gly Phe Asn Tyr His Pro Arg Val
                   885                 890                 895
   Ala Pro Val Ala Lys Lys Arg Val Ser Ser Val Thr Glu Gly Gly Ile
               900                 905                 910
   Asp Trp Ala Trp Gly Glu Leu Leu Ala Phe Gly Ser Leu Ala Asn Ser
           915                 920                 925
   Gly Arg Leu Val Arg Leu Ala Gly Glu Asp Ser Arg Arg Gly Thr Phe
       930                 935                 940
   Thr Gln Arg His Ala Val Ala Ile Asp Pro Ala Thr Ala Glu Glu Phe
   945                 950                 955                 960
   Asn Pro Leu His Glu Leu Ala Gln Ser Lys Gly Asn Asn Gly Lys Phe
                   965                 970                 975
   Leu Val Tyr Asn Ser Ala Leu Thr Glu Tyr Ala Gly Met Gly Phe Glu
               980                 985                 990
   Tyr Gly Tyr Ser Val Gly Asn Glu  Asp Ser Ile Val Ala  Trp Glu Ala
           995                 1000                1005
   Gln Phe  Gly Asp Phe Ala Asn  Gly Ala Gln Thr Ile  Ile Asp Glu
       1010                1015                1020
   Tyr Val  Ser Ser Gly Glu Ala  Lys Trp Gly Gln Thr  Ser Lys Leu
       1025                1030                1035
   Ile Leu  Leu Leu Pro His Gly  Tyr Glu Gly Gln Gly  Pro Asp His
       1040                1045                1050
   Ser Ser  Ala Arg Ile Glu Arg  Phe Leu Gln Leu Cys  Ala Glu Gly
       1055                1060                1065
   Ser Met  Thr Val Ala Gln Pro  Ser Thr Pro Ala Asn  His Phe His
       1070                1075                1080
   Leu Leu  Arg Arg His Ala Leu  Ser Asp Leu Lys Arg  Pro Leu Val
       1085                1090                1095
   Ile Phe  Thr Pro Lys Ser Met  Leu Arg Asn Lys Ala  Ala Ala Ser
       1100                1105                1110
   Ala Pro  Glu Asp Phe Thr Glu  Val Thr Lys Phe Gln  Ser Val Ile
       1115                1120                1125
   Asn Asp  Pro Asn Val Ala Asp  Ala Ala Lys Val Lys  Lys Val Met
       1130                1135                1140
   Leu Val  Ser Gly Lys Leu Tyr  Tyr Glu Leu Ala Lys  Arg Lys Glu
       1145                1150                1155
   Lys Asp  Gly Arg Asp Asp Ile  Ala Ile Val Arg Ile  Glu Met Leu
       1160                1165                1170
   His Pro  Ile Pro Phe Asn Arg  Ile Ser Glu Ala Leu  Ala Gly Tyr
       1175                1180                1185
   Pro Asn  Ala Glu Glu Val Leu  Phe Val Gln Asp Glu  Pro Ala Asn
       1190                1195                1200
   Gln Gly  Pro Trp Pro Phe Tyr  Gln Glu His Leu Pro  Glu Leu Ile
       1205                1210                1215
   Pro Asn  Met Pro Lys Met Arg  Arg Val Ser Arg Arg  Ala Gln Ser
       1220                1225                1230
   Ser Thr  Ala Thr Gly Val Ala  Lys Val His Gln Leu  Glu Glu Lys
       1235                1240                1245
   Gln Leu  Ile Asp Glu Ala Phe  Glu Ala
       1250                1255

## Description

### Field of the Invention

The present invention relates to an L-glutamic acid-producing microorganism and a method for producing L-glutamic acid using the microorganism. L-glutamic acid is widely used in food industry, for example, as a raw material for production of seasonings.

### Brief Description of the Related Art

L-glutamic acid has been conventionally produced on an industrial scale by fermentative methods using L-glutamic acid-producing coryneform bacteria such as those belonging to the genus *Brevibacterium* and *Corynebacterium.* To improve the L-glutamic acid-producing ability of the coryneform bacteria, strains isolated from nature or artificial mutants of such strains are used as the L-glutamic acid-producing coryneform bacteria.

Various technologies for improving the L-glutamic acid-producing ability by enhancing L-glutamic acid biosynthetic enzyme activity using recombinant DNA techniques have been reported. For example, coryneform bacteria with an amplified citrate synthase gene (JP07-121228B), and coryneform bacteria with an amplified glutamate dehydrogenase gene (EP 955368) have been reported.

In the production of substances by fermentation, it is necessary to maintain sufficient growth of bacterial cells to sufficiently produce a target substance. Accordingly, it is necessary to breed a strain with enhanced biosynthesis of a target substance without causing a decrease in bacterial growth. In the production of L-glutamic acid, it has been reported that it is advantageous to decrease α-ketoglutarate dehydrogenase activity (JP06-237779A, and WO95/34672). However, there have been no reports of decreasing the α-ketoglutarate dehydrogenase activity while maintaining a sufficient level of L-glutamic acid production.

### SUMMARY OF THE INVENTION

An object of the present invention is to provide a technology to improve the L-glutamic acid-producing ability of coryneform bacteria for fermentative production of L-glutamic acid. When the α-ketoglutarate dehydrogenase gene is disrupted, energy generated through the TCA cycle is decreased, leading to retardation of the bacterial growth. As a result, sufficient yield of L-glutamic acid may not be obtained.

Therefore, a mutant strain having a decreased ability to degrade L-glutamic acid while maintaining an ordinary growth rate would be useful for efficient production of L-glutamic acid. Mutant strains were obtained that have a decreased α-ketoglutarate dehydrogenase activity and maintain almost the same growth rate as a wild-type strain, by introducing mutations into a chromosomal odhA gene that encodes the E1o subunits of the α-ketoglutarate dehydrogenase complex of coryneform bacterium. L-glutamic acid was found to be efficiently produced by using such mutant strains.

It is an object of the present invention to provide an L-glutamic acid-producing coryneform bacterium, comprising
a) intracellular α-ketoglutarate dehydrogenase activity which is less than half that of a non-mutated or wild-type strain, and
b) a mutation in a coding region or an expression control region of a chromosomal odhA gene encoding the Elo subunit of the α-ketoglutarate dehydrogenase complex,
wherein said bacterium grows almost at the same growth rate as a non-mutated or wild-type strain.

It is a further object of the present invention to provide the coryneform bacterium as described above, wherein said odhA gene encodes a protein selected from the group consisting of:
(A) a protein comprising an amino acid sequence of SEQ ID NO: 10,
(B) a protein comprising an amino acid sequence of SEQ ID NO: 10, whereby one or several amino acids in said protein are substituted, deleted, inserted, or added, and wherein said protein has an activity of the E1o subunit of the α-ketoglutarate dehydrogenase complex.
(C) a protein comprising amino acids 37 to 1257 of SEQ ID NO: 10, and
(D) a protein comprising amino acids 37 to 1257 of SEQ ID NO: 10, whereby one or several amino acids in said protein are substituted, deleted, inserted, or added, and wherein said protein has an activity of the E1o subunit of the α-ketoglutarate dehydrogenase complex.

It is a further object of the present invention to provide the coryneform bacterium as described above, wherein said several amino acids are 2 to 20 amino acids.

It is a further object of the present invention to provide the coryneform bacterium as described above, wherein said odhA gene is selected from the group consisting of:
(a) a gene comprising nucleotides 443 to 4213 of SEQ ID NO: 9,
(b) a gene that is able to hybridize under stringent conditions to a polynucleotide comprising nucleotides 443 to 4213 of SEQ ID NO: 9 or a probe prepared from a polynucleotide comprising nucleotides 443 to 4213 of SEQ ID NO: 9, and wherein said gene encodes a protein which has the activity of the E1o subunit of the α-ketoglutarate dehydrogenase complex,
(c) a gene comprising nucleotides 551 to 4213 of SEQ ID NO: 9, and
(d) a gene that is able to hybridize under stringent conditions to a polynucleotide comprising nucleotides 551 to 4213 of SEQ ID NO: 9 or a probe prepared from a polynucleotide comprising nucleotides 551 to 4213 of SEQ ID NO: 9, and wherein said gene encodes a protein which has the activity of the E1o subunit of the α-ketoglutarate dehydrogenase complex.

It is a further object of the present invention to provide the coryneform bacterium as described above, wherein said mutation is introduced into a region encoding a thiamine pyrophosphate binding region.

It is a further object of the present invention to provide the coryneform bacterium as described above, wherein said mutation comprises deletion of an amino acid selected from the group consisting of Gly at position 686, Leu at position 687, Gly at position 688, Asn at position 713, Asn at position 714, and combinations thereof in the amino acid sequence shown in SEQ ID NO: 10.

It is a further object of the present invention to provide the coryneform bacterium as described above, wherein said mutation is introduced into the region comprising nucleotides 2534 to 2548 of SEQ ID NO: 9.

It is a further object of the present invention to provide the coryneform bacterium as described above, wherein said mutation comprises deletion of one or more amino acids in the region comprising amino acids 698 to 702 of SEQ ID NO: 10.

It is a further object of the present invention to provide the coryneform bacterium as described above, wherein said mutation comprises replacement of an amino acid selected from the group consisting of Lys at position 698, Leu at position 699, Arg at position 700, Tyr at position 702, and combinations thereof in the amino acid sequence shown in SEQ ID NO: 10.

It is a further object of the present invention to provide the coryneform bacterium as described above, wherein said mutation is introduced into the region comprising nucleotides 1094 to 1114 of SEQ ID NO: 9.

It is a further object of the present invention to provide the coryneform bacterium as described above, wherein said mutation comprises deletion of one or more amino acids in the region comprising amino acids 218 to 224 of SEQ ID NO: 10.

It is a further object of the present invention to provide a method for producing L-glutamic acid comprising:
a) culturing the coryneform bacterium according to claim 1 in a culture medium and
b) collecting L-glutamic acid from the culture medium and/or the bacterium.

It is a further object of the present invention to provide a gene encoding mutant α-ketoglutarate dehydrogenase selected from the group consisting:
(a) a gene comprising nucleotides 443 to 4213 of a polynucleotide selected from the group consisting of SEQ ID NO: 11, 13, and 15, or a gene comprising nucleotides 443 to 4210 of a polynucleotide selected from the group consisting of SEQ ID NO: 44, 46, and 48,
(b) a gene that is able to hybridize under stringent conditions to a polynucleotide comprising nucleotides 443 to 4213 of a polynucleotide selected from the group consisting of SEQ ID NO: 11, 13, and 15, a polynucleotide comprising nucleotides 443 to 4210 of a polynucleotide selected from the group consisting of SEQ ID NO: 44, 46, and 48, a probe prepared from nucleotides 443 to 4213 of a polynucleotide selected from the group consisting of SEQ ID NO: 11, 13, and 15, or a probe prepared from nucleotides 443 to 4210 of a polynucleotide selected from the group consisting of SEQ ID NO: 44, 46, and 48, and wherein said gene encodes a protein which has α-ketoglutarate dehydrogenase activity which is less than half that of a wild-type or npn-mutated strain by forming a complex with E2o and E3 subunits,
(c) a gene comprising nucleotides 551 to 4213 of a polynucleotide selected from the group consisting of SEQ ID NO: 11, 13, and 15, or a gene comprising nucleotides 551 to 4210 of a polynucleotide selected from the group consisting of SEQ ID NO: 44, 46, and 48, and
(d) a gene that is able to hybridize under stringent conditions to a polynucleotide comprising nucleotides 551 to 4213 of a polynucleotide selected from the group consisting of SEQ ID NO: 11, 13, and 15, a polynucleotide comprising nucleotides 551 to 4210 of a polynucleotide selected from the group consisting of SEQ ID NO: 44, 46, and 48, a probe prepared from nucleotides S51 to 4213 of a polynucleotide selected from the group consisting of SEQ ID NO: 11, 13, and 15, or a probe prepared from nucleotides 551 to 4210 of a polynucleotide selected from the group consisting of SEQ ID NO: 44, 46, and 48, and wherein said gene encodes a protein which has α-ketoglutarate dehydrogenase activity which is less than half that of a wild-type or non-mutated strain by forming a complex with E2o and E3 subunits.

It is a further object of the present invention to provide a mutant α-ketoglutarate dehydrogenase selected from the group consisting of:
(a) a protein selected from the group consisting of SEQ ID NO: 12, 14, 16, 45, 47, and 49,
(b) a protein selected from the group consisting of SEQ ID NO: 12, 14, 16, 45, 47, and 49, whereby one or several amino acids in said protein are substituted, deleted, or added, and wherein said protein exhibits α-ketoglutarate dehydrogenase activity which is less than half that of a wild-type or non-mutated strain by forming a complex with E2o and E3 subunits,
(c) a protein comprising amino acids 37 to 1256 of an amino acid sequence selected from the group consisting of SEQ ID NO: 12, 45, 47, and 49, a protein comprising amino acids 37 to 1255 of an amino acid sequence of SEQ ID NO: 14, or a protein comprising amino acids 37 to 1254 of an amino acid sequence of SEQ ID NO: 16, and
(d) a protein comprising amino acids 37 to 1257 of an amino acid sequence selected from the group consisting of SEQ ID NO: 12, 45, 47, and 49, a protein comprising amino acids 37 to 1255 of an amino acid sequence of SEQ ID NO: 14, or a protein comprising amino acids 37 to 1254 of an amino acid sequence of SEQ ID NO: 16, whereby one or several amino acids in said protein are substituted, deleted, or added, and wherein said protein exhibits α-ketoglutarate dehydrogenase activity which is less than half that of a wild-type or non-mutated strain by forming a complex with E2o and E3 subunits.

### BRIEF DESCRIPTION OF DRAWINGS

Fig. 1 shows the construction of plasmid pBS3.
Fig. 2 is shows the construction of plasmid pBS4S.
Fig. 3 shows the construction of plasmid pBSOAGN.
Fig. 4 shows the construction of plasmid pBSOA2-2.
Fig. 5 shows the change in L-glutamic acid concentration when each strain is cultured.
Fig. 6 shows the growth rate of each strain.
Fig. 7 shows the change in the amount of residual sugar when each strain is cultured.

### DESCRIPTION OF THE PREFERRED EMBODIMENTS

Hereinafter, the present invention will be described in detail.

### <1> Coryneform bacterium of the present invention

The coryneform bacterium of the present invention has an L-glutamic acid-producing ability, and grows almost at the same growth rate as a non-mutated strain or a wild-type strain. The corynebacterium of the present invention has intracellular α-ketoglutarate dehydrogenase (hereinafter, also referred to as "α-KGDH") activity which is less than half that of a non-mutated strain or the wild-type strain, by introduction of mutations into a coding region or an expression control region of the odhA gene, which encodes the E1o subunit of α-KGDH complex.

α-KGDH is also called oxoglutarate dehydrogenase or 2-oxoglutarate dehydrogenase.

In the present invention, examples of coryneform bacterium include conventional coryneform bacterium, and also include bacteria that had been classified into the genus *Brevibacterium* but are currently is classified into the genus *Corynebacterium* (Int. J. Syst. Bacteriol., 41, 255(1991)), as well as the *Brevibacterium* bacteria that are very close to *Corynebacterium* bacteria. Examples of such coryneform bacterium include the following.
*Corynebacterium acetoacidophilum*
*Corynebacterium acetoglutamicum*
*Corynebacterium alkanolyticum*
*Corynebacterium callunae*
*Corynebacterium glutamicum*
*Corynebacterium lilium*
*Corynebacterium melassecola*
*Corynebacterium thermoaminogenes (Corynebacterium efficiens)*
*Corynebacterium herculis*
*Brevibacterium divaricatum*
*Brevibacterium flavum*
*Brevibacterium immariophilum*
*Brevibacterium lactofermentum (Corynebacterium glutamicum)*
*Brevibacterium roseum*
*Brevibacterium saccharolyticum*
*Brevibacterium thiogenitalis*
*Brevibacterium ammoniagenes*
*Brevibacterium album*
*Brevibacterium cerinum*
*Microbacterium ammoniaphilum*
Specific examples of the coryneform bacteria are as follows.
*Corynebacterium acetoacidophilum* ATCC13870
*Corynebacterium acetoglutamicum* ATCC15806
*Corynebacterium alkanolyticum* ATCC21511
*Corynebacterium callunae* ATCC15991
*Corynebacterium glutamicum* ATCC13020, ATCC13032, ATCC13060, ATCC13869
*Corynebacterium lilium* ATCC15990
*Corynebacterium melassecola* ATCC17965
*Corynebacterium thermoaminogenes* AJ12340 (FERM BP-1539)
*Corynebacterium herculis* ATCC13868
*Brevibacterium divaricatum* ATCC14020
*Brevibacterium flavum* ATCC13826, ATCC14067, AJ12418 (FERM BP-2205)
*Brevibacterium immariophilum* ATCC14068
*Brevibacterium lactofermentum (Corynebacterium glutamicum)* ATCC13869
*Brevibacterium roseum* ATCC13825
*Brevibacterium saccharolyticum* ATCC14066
*Brevibacterium thiogenitalis* ATCC19240
*Brevibacterium ammoniagenes* ATCC6871, ATCC6872
*Brevibacterium album* ATCC15111
*Brevibacterium cerinum* ATCC15112
*Microbacterium ammoniaphilum* ATCC15354

These strains are available from the American Type Culture Collection (ATCC, Address: P.O. Box 1549, Manassas, VA 20108, United States of America). That is, each strain is given a unique registration number which is listed in the catalogue of the ATCC. Strains can be ordered using this registration number. The AJ12340 strain was deposited at National Institute of Bioscience and Human Technology, Agency of Industrial Science and Technology, Ministry of International Trade and Industry (currently International Patent Organism Depositary, National Institute of Advanced Industrial Science and Technology at Tsukuba Central 6, 1-1, Higashi 1-chome, Tsukuba-shi, Ibaraki-ken 305-5466, Japan) on October 27, 1989 under the provisions of the Budapest Treaty and given an accession number of FERM BP-1539. The AJ12418 strain was deposited at National Institute of Bioscience and Human Technology, Agency of Industrial Science and Technology, Ministry of International Trade and Industry on January 5, 1989 under the provisions of the Budapest Treaty and given an accession number of FERM BP-2205.

In the present invention, "L-glutamic acid-producing ability" means an ability to cause accumulation of a sufficient amount of L-glutamic acid in a medium when the coryneform bacterium of the present invention is cultured in the medium. The L-glutamic acid-producing ability may be either a property of a parent strain from which the coryneform bacterium of the present invention is bred or a property imparted or enhanced by a mutation, gene recombination, etc. Furthermore, the L-glutamic acid-producing ability may be imparted by introducing a mutation into the odhA gene which encodes the E1o subunit of α-ketoglutarate dehydrogenase complex.

Examples of the methods for imparting the L-glutamic acid-producing ability include enhancing expression of a gene encoding an L-glutamic acid biosynthetic enzyme. Examples of the enzymes involved in L-glutamic acid biosynthesis include glutamate dehydrogenase, glutamine synthetase, glutamate synthetase, isocitrate dehydrogenase, aconitate hydratase, citrate synthase, phosphoenolpyruvate carboxylase, pyruvate carboxylase, pyruvate dehydrogenase, pyruvate kinase, phosphoenolpyruvate synthase, enolase, phosphoglyceromutase, phosphoglycerate kinase, glyceraldehyde-3-phophate dehydrogenase, triose phosphate isomerase, fructose bisphosphate aldolase, phosphofructokinase, and glucose phosphate isomerase.

Enhancement of expression of these genes may be achieved by incorporating a DNA fragment containing such a gene into an appropriate plasmid which is able to autonomously replicate in coryneform bacterium, and transforming bacterial cells with the resultant plasmid; integrating such a gene into a chromosome by homologous recombination, conjugation, transposition, etc.; or introducing a mutation into a promoter region of such a gene (WO/0018935).

When the above-mentioned genes are introduced by a plasmid or integrated on a chromosome, a promoter for expression of the genes may be any promoter so long as it is able to function in coryneform bacteria. Examples of such promoters include lac promoter, trp promoter, trc promoter, PS2 promoter, and pL promoter. A native promoter of the gene may also be used.

Examples of microorganisms modified so that expression of citrate synthetase gene, phosphoenolpyruvate carboxylase gene, and/or glutamate dehydrogenase gene is enhanced include those microorganisms disclosed in JP2001-333769A (EP1078989A), JP2000-106869A (EP955368A), JP2000-189169A (EP952221A), and JP2001-333769A (EP1078989A).

The modification for imparting the L-glutamic acid-producing ability includes decreasing or eliminating an activity of an enzyme that catalyzes a reaction for synthesizing a compound other than L-glutamic acid, and branching from an L-glutamic acid biosynthesis pathway. Examples of such enzymes include isocitrate lyase, acetyl phosphate transferase, acetate kinase, acetohydroxy acid synthase, acetolactate synthase, acetyl formate transferase, lactate dehydrogenase, glutamate decarboxylase, and 1-pyrophosphate dehydrogenase.

To decrease or eliminate the activity of the enzymes as described above, a mutation or deletion which causes a decrease or loss of the activity of the enzymes may be introduced into the genes of the enzymes on the chromosome. This may be achieved by, for example, disrupting the gene encoding the enzyme on the chromosome, or by modifying an expression control sequence such as a promoter and/or Shine Dargarno (SD) sequence of the gene. In addition, the activities of such enzymes may be decreased or eliminated by introducing a missense mutation which causes an amino acid substitution, a nonsense mutation which generates a stop codon, or a frame shift mutation which adds or deletes one or two nucleotides into a coding region, or by deleting a portion of the gene (Journal of biological Chemistry 272:8611-8617 (1997)). Furthermore, the activities of such enzymes may be decreased or eliminated by constructing a gene encoding a mutant enzyme in which its coding region is deleted and replacing a chromosomal gene with the resulting gene by homologous recombination.

The L-glutamic acid-producing ability may also be imparted by screening a strain resistant to organic acid analogues or respiratory inhibitors, or by screening a strain sensitive to inhibitors of cell wall synthesis. Examples of such methods include imparting resistance to benzopirone or naphtoquinone (JP56-1889A), imparting resistance to HOQNO (JP56-140895A), imparting resistance to α-ketomalonic acid (JP57-2689A), imparting resistance to guanidine (JP56-35981A), and imparting sensitivity to penicillin (JP04-88994A).

Specific examples of such bacteria include the following strains.
*Brevibacterium flavum* AJ11355 (FERM P-5007; JP56-1889A)
*Corynebacterium glutamicum* AJ11355 (FERM P-5020; JP56-1889A)
*Brevibacterium flavum* AJ11217 (FERM P-4318; JP57-2689A)
*Corynebacterium glutamicum* AJ11218 (FERM P-4319; JP57-2689A)
*Brevibacterium flavum* AJ11564 (FERM P-5472; JP56-140895A)
*Brevibacterium flavum* AJ11439 (FERM P-5136; JP56-35981A)
*Corynebacterium glutamicum* H7684 (FERM BP-3004; JP04-88994A)

The coryneform bacterium of the present invention can be obtained from coryneform bacterium having the L-glutamic acid-producing ability as described above by introducing a mutation into a chromosomal odhA gene which encodes the E1o subunit of the α-KGDH complex, and selecting those strains that grow almost at the same growth rate as a non-mutated strain or a wild-type strain, and exhibits α-KGDH activity less than half that of the non-mutated strain or wild-type strain. Alternatively, such a mutation may be introduced into the odhA gene first, followed by imparting an L-amino acid-producing ability.

In the present invention, α-KDGH activity means an activity catalyzing a reaction of oxidative decarboxylation of α-ketoglutarate (2-oxoglutarate) to generate succinyl-CoA. The above-mentioned reaction is catalyzed by the α-KDGH complex, which includes three kinds of subunits, i.e., α-ketoglutarate dehydrogenase (E1o, EC1.2.4.2), dihydrolipoamide-S-succinyltransferase (E2o, EC:2.3.1.61), and dihydrolipoamide dehydrogenase (E3, EC:1.8.1.4). That is, these three subunits catalyze each of the following reactions:
E1o subunit:
   2-oxoglutarate + [dihydrolipoyllysine-residue succinyltransferase]lipoyllysine = [dihydrolipoyllysine-residue succinyltransferase]S-succinyldihydrolipoyllysine + CO₂
E2o subunit:
   CoA + enzymeN6-(S-succinyldihydrolipoyl)lysine=succinyl-CoA + enzyme N6-(dihydrolipoyl)lysine
E3 subunit:
   protein N6-(dihydrolipoyl)lysine + NAD⁺ = protein N6-(lipoyl)lysine+ NADH + H⁺

The activity of the "α-KDGH complex" means an activity catalyzing the total reaction of the above three reactions.

In *Escherichia coli,* these three subunits form a complex.

In the case of coryneform bacterium, the E1o subunit is encoded by the odhA gene and the E3 subunit is encoded by the lpd gene (GenBank Accession NO. Y16642; SEQ ID NO: 17). It is controversial whether the E2o subunit is encoded by the odhA gene as a bifunctional protein together with the E1o subunit (Usuda et al., Microbiology 1996 142, 3347-3354), or encoded by a separate gene registered as an accession No. NCg12126 of NC_003450 (SEQ ID NO: 27) in the GenBank database. Thus, the odhA gene may encode the E2o subunit in addition to the E1o subunit

The coryneform bacteria of the present invention have a mutation in the chromosomal odhA gene. The odhA gene includes, for example, a gene having a nucleotide sequence encoding the amino acid sequence shown in SEQ ID NO: 10. The odhA gene also includes a gene having a nucleotide sequence encoding the amino acid sequence shown in SEQ ID NO: 51. In addition, it is possible that the "gtg" at positions 551 to 553 in the nucleotide sequence of SEQ ID NO: 9 may be translated as Met and translation may be initiated at this codon, even though it is a codon for Val. Therefore, the odhA gene also includes a gene having a nucleotide sequence encoding the amino acid sequence of amino acids 37 to 1257 of SEQ ID NO: 10. Furthermore, the odhA gene may also be a gene encoding a protein that has an amino acid sequence shown in SEQ ID NO: 10 or 51, or amino acid sequence of amino acids 37 to 1257 of SEQ ID NO: 10, including substitution, deletion, insertion or addition of one or several amino acids so long as it exhibits an activity of the E1o subunit of the α-KGDH complex. The activity of the E1o subunit itself can be determined by the method of Massey et al. (Biochim. Biophys. Acta 38,447-460). "Several" as used herein is preferably 2 to 20, more preferably 2 to 10, and particularly preferably 2 to 5.

More specifically, the odhA gene preferably has a nucleotide sequence of nucleotides 443 to 4213 of SEQ ID NO: 9, or a nucleotide sequence of nucleotides 551 to 4213 of SEQ ID NO: 9. The odhA gene may have a nucleotide sequence shown in SEQ ID NO: 50. Furthermore, since the nucleotide sequences of the odhA genes may differ between the species or strains of the coryneform bacteria, the odhA gene may be a gene that hybridizes with a polynucleotide having a nucleotide sequence of nucleotides 443 to 4213 of SEQ ID NO: 9, a polynucleotide having a nucleotide sequence shown in SEQ ID NO: 50, or a nucleotide sequence of nucleotides 551 to 4213 of SEQ ID NO: 9, or a probe prepared from the nucleotide sequences under stringent conditions so long as the gene encodes a protein that exhibits the activity of the E1o subunit of the α-KGDH complex. The "stringent conditions" as used herein are conditions under which a so-called specific hybrid is formed, and a non-specific hybrid is not formed. It is difficult to clearly express this condition by using any numerical value. However, examples of stringent conditions include those under which DNAs hybridize to each other at a salt concentration with washing typical of Southern hybridization, i.e., washing once or preferably 2-3 times under 1 x SSC, 0.1% SDS at 60°C, preferably 0.1 x SSC, 0.1% SDS at 60°C, more preferably 0.1 x SSC, 0.1% SDS at 68°C.

In the present invention, "a mutation is introduced into a chromosomal odhA gene" includes the case where the odhA gene on a chromosome is replaced by a mutant odhA gene to generate substantially the same condition in which a mutation is directly introduced into the chromosomal odhA gene.

The above-mentioned mutation may be introduced not only in the coding region but also in the expression controlling region of the odhA gene. The expression controlling region includes, for example, a promoter, SD sequence, and operator. The expression controlling region of the odhA gene includes a nucleotide sequence of nucleotides 1 to 442 of SEQ ID NO: 9. Expression controlling sequence of the odhA gene may also be identified by genetic analysis software such as GENETYX or by the method disclosed in Goldstein et al. (Prokaryotic promoters in biotechnology. Biotechnol. Annu. Rev., 1995, 1, 105-128).

The "α-KGDH activity less than half that of a non-mutated or wild-type strain" preferably means that the α-KGDH activity per unit cell (per unit weight of protein) of the coryneform bacterium of the present invention cultured for 4 hours is less than half the activity of a non-mutated or wild-type strain of coryneform bacteria cultured under the same conditions. Examples of coryneform bacterium having such decreased α-KGDH activity include a bacterium in which the number of α-KGDH molecules per cell is decreased and a bacterium in which the activity per α-KGDH molecule is decreased. Examples of a wild-type coryneform bacterium used as a control include *Brevibacterium lactofermentum* ATCC13869 strain. The intracellular α-KGDH activity of the coryneform bacterium of the present invention is preferably less than 40%, more preferably less than 30% that of the activity of a non-mutated or wild-type strain. Although α-KGDH activity may be decreased to an undetectable level, it is preferable that the activity is not completely eliminated The α-KGDH activity (activity of α-KGDH complex) can be measured by the method of Shiio et al. (Isamu Shiio and Kyoko Ujigawa-Takeda, Agric. Biol. Chem., 44(8), 1897-1904, 1980).

As a result of the decrease of intracellular α-KGDH activity, the L-glutamic acid-producing ability of the coryneform bacterium is improved. In the present invention, "L-glutamic acid-producing ability is improved" preferably means that when the strain of coryneform bacterium into which a mutation has been introduced into odhA gene is cultured in a medium, the amount of L-glutamic acid which accumulates in the medium by the mutant coryneform bacterium is larger than that which accumulates by the wild-type or non-mutated strain, or the rate of L-glutamic acid production by the mutant coryneform bacterium is higher than that of the wild-type or non-mutated strain.

The phrase "the bacterium grows almost at the same growth rate compared to a wild-type strain or non-mutated strain" preferably means the bacterium of the present invention grows almost at the same growth rate as a wild-type strain such as *Brevibacterium lactofermentum* ATCC13869. Here, "almost at the same growth rate" means 80% or more, more preferably 90%, or more preferably 95% or more of the growth rate of a wild-type or non-mutated strain. It is not necessary that the coryneform bacterium of the present invention grows almost at the same growth rate as a wild-type or non-mutated strain at any culture temperature. That is, coryneform bacterium which exhibits a sufficient growth rate at low temperatures but exhibits a decreased growth rate at high temperatures such as GN-2-2 strain as shown in the Examples is also included in the coryneform bacterium of the present invention.

The growth rates of the strain of the present invention and the wild-type or non-mutated strain can be compared, for example, by inoculating the same number of cells of each strain in a medium and comparing viable cell numbers after a predetermined time. When a liquid medium is used, the viable cell number can be calculated based on, for example, optical density (OD) at a wavelength of 600 nm or weight of bacterial cells after a predetermined time. When a solid medium such as a plate medium is used, the viable cell numbers can be calculated based on the number of colonies that appeared after a predetermined time.

Examples of the method of introducing a mutation into the odhA gene on the chromosome include using X-rays or ultraviolet rays to irradiate the coryneform bacterium, and treating the coryneform bacterium with a mutagen such as N-methyl-N'-nitro-N-nitrosoguanidine. Mutation may also be introduced into the odhA gene on the chromosome by preparing a mutant odhA gene, and replacing the chromosomal odhA gene with such a mutant odhA gene by homologous recombination technique. Such a mutant odhA gene may be prepared by error-prone PCR, DNA shuffling, or StEP-PCR (Firth AE, Patrick WM; Bioinformatics. 2005 Jun 2; Statistics of protein library construction.), or overlap-extension PCR (Urban, A., Neukirchen, S. and Jaeger, K. E., A rapid and efficient method for site-directed mutagenesis using one-step overlap extension PCR. Nucleic Acids Res, 25, 2227-8. (1997)).

Homologous recombination may be performed by a method called "Red-driven integration" in which a linear DNA is used (Datsenko, K.A., PNAS, 97(12), 6640-6645, 2000) or by a method using a plasmid having temperature-sensitive replication origin (USP6303383, and JP05-007491A). Introduction of a mutation into a chromosomal odhA gene may also be performed by using a plasmid which is not replicable in cells of coryneform bacteria or a plasmid capable of transferring to coryneform bacteria by conjugation.

Examples of such a temperature-sensitive plasmid for Coryneform bacteria include p48K and pSFKT2 (JP2000-262288A), pHSC4 (France Patent Laid-open Publication No. 2667875, 1992 and JP5-7491A), pBS5Tand so forth. These plasmids can autonomously replicate at least at a temperature of 25°C, but cannot autonomously replicate at a temperature of 37°C in Coryneform bacteria. The AJ12571 strain harboring pHSC4 was deposited at National Institute of Bioscience and Human Technology, Agency of Industrial Science and Technology, Ministry of International Trade and Industry (currently International Patent Organism Depositary, National Institute of Advanced Industrial Science and Technology at Tsukuba Central 6, 1-1, Higashi 1-chome, Tsukuba-shi, Ibaraki-ken 305-5466, Japan) on August 26, 1991 under the provisions of the Budapest Treaty and given an accession number of FERM BP-3524.

A plasmid which is not replicable in cells of coryneform bacteria is preferably one replicable in cells of *Escherichia* bacteria, and examples thereof include pHSG299 (Takara Bio) and pHSG399 (Takara Bio). Examples of a plasmid capable of transferring to coryneform bacteria by conjugation include pK19mobsacB (J. Bacteriology 174:5462-65(1992)).

Mutant odhA gene is inserted into a temperature-sensitive plasmid or a plasmid which is not replicable in coryneform bacteria, and the obtained recombinant plasmids are used to transform coryneform bacteria. Transformation can be performed by conventional methods. For example, a method of treating recipient cells with calcium chloride so as to increase the permeability of DNA, which has been reported for *Escherichia coli* (Mandel, M. and Higa, A., J. Mol. Biol., 53, 159 (1970)), and a method of using competent cells prepared from growing cells to introduce a DNA, which has been reported for *Bacillus subtilis* (Duncan, C.H., Wilson, GA. and Young, F.E., Gene, 1, 153 (1977)) can be employed. In addition to these methods, a method of introducing a recombinant DNA into protoplast- or spheroplast-like recipient cells, which have been reported to be applicable to *Bacillus subtilis,* actinomycetes, and yeasts (Chang, S. and Choen, S.N., Molec. Gen. Genet, 168, 111 (1979); Bibb, M.J., Ward, J.M. and Hopwood, O.A., Nature, 274, 398 (1978); Hinnen, A., Hicks, J.B. and Fink, G.R., Proc. Natl. Sci., USA, 75, 1929 (1978)), can be employed. In addition, transformation of *Coryneform* bacteria can also be performed by the electric pulse method (Sugimoto et al., JP2-207791A).

In the case of using a plasmid which is not capable in cells of coryneform bacteria, a wild-type odhA gene on a chromosome is replaced with the mutant odhA gene on the plasmid.

In the case of using a temperature-sensitive plasmid, the transformant strain is cultured at a temperature at which the temperature sensitive replication origin does not function (e.g. 25°C) to obtain a strain into which the plasmid is introduced. Then, the transformant is cultured at a high temperature to eliminate the temperature-sensitive plasmid; and spread over a plate medium containing an antibiotic drug such as kanamycin. Although strains from which the plasmid is eliminated cannot grow on a plate containing such an antibiotic drug, a few strains in which the chromosomal odhA gene is replaced with the mutant odhA gene can grow and appear as colonies.

In such a strain in which the recombinant DNA containing the mutant odhA gene is integrated into the chromosomal DNA as described above, the recombinant DNA causes recombination with the odhA gene that originally exists on the chromosome, and the fusion genes of the chromosomal odhA gene and the mutant odhA gene are inserted into the chromosome so that the other portions of the recombinant DNA (vector segment, temperature sensitive replication origin and drug resistance marker) is present between the fusion genes. Then, in order to leave only the mutant odhA gene on the chromosomal DNA, one copy of the odhA gene is eliminated together with the vector segment (including the temperature sensitive replication origin and the drug resistance marker) from the chromosomal DNA. In this case, the normal odhA gene is left on the chromosomal DNA and the mutant odhA gene is excised from the chromosomal DNA, or to the contrary, the mutant odhA gene is left on the chromosomal DNA and the normal odhA gene is excised from the chromosome DNA. Then, a strain in which only the mutant odhA gene is left on the chromosome can be selected using PCR, Southern hybridization or the like.

Homologous recombination may be performed using a sacB gene encoding levan sucrase as a marker for recombination. The sacB gene encoding levan sucrase is used to efficiently select strains in which a chromosomal odhA gene is replaced by the mutant odhA gene and a vector portion is cured from a chromosome (Schafer, A. et al., Gene 145 (1994) 69-73). That is, in the case of the coryneform bacteria, when levan sucrase is expressed, levan generated by assimilation of sucrose becomes lethal for the bacteria and hence the bacteria cannot grow. Therefore, by culturing on a sucrose-containing plate, strains in which substitution occurs between the mutant odhA gene in the vector and a chromosomal odhA gene and from which the other portions of the vector are cured can be selected.

Examples of the sacB gene include the followings.
*Bacillus subillus :* sacB GenBank Accession Number X02730 (SEQ ID NO: 19)
*Bacillus amyloliqufaciens :* sacB GenBank Accession Number X52988
*Zymomonas mobilis :* sacB GenBank Accession Number L33402
*Bacillus stearothermophilus :* surB GenBank Accession Number U34874
*Lactobacillus sanfranciscensis :* frfA GenBank Accession Number AJ508391
*Acetobacter xylinus :* 1sxA GenBank Accession Number AB034152
*Gluconacetobacter diazotrophicus :* 1sdA GenBank Accession Number L41732

### <2> The mutant odhA gene of the present invention

The mutation to be introduced into the chromosomal odhA gene is not particularly limited so long as it is a mutation which decreases the α-KGDH activity to be less than half of the α-KGDH activity of a non-mutated strain or wild-type strain, but does not cause retardation of the growth of the bacterium. Specific examples of the mutation include the followings.

### (1) Mutation in the thiamine pyrophosphate binding region

This mutation is introduced into the binding region of thiamine pyrophosphate, which is a coenzyme. Thiamine pyrophosphate binding region means a region corresponding to that encoded by nucleotides 2498 to 2584 of the odhA gene (SEQ ID NO: 9). The amino acid sequence of the region is shown as amino acids 686 to 714 in SEQ ID NO: 10. Examples of a mutation in this region include one that causes a deletion of one or more amino acid residues selected from Gly at position 686, Leu at position 687, Gly at position 688, Asn at position 713, and Asn at position 714.

The mutation in the thiamine pyrophosphate binding region may also be introduced in the region of nucleotides 2534 to 2548 of the odhA gene (SEQ ID NO: 9). This mutation is named a GN type mutation. The GN type mutation is preferably a mutation that causes deletion and/or substitution of one or more amino acid residues selected from Lys at position 698, Leu at position 699, Arg at position 700, Gly at position 701, and Tyr at position 702 in SEQ ID NO: 10. An example of such a mutation is that which deletes Gly at position 701 in SEQ ID NO: 10. Examples of the mutant odhA gene having this mutation include a gene having nucleotide sequence of nucleotides 443 to 4213 of SEQ ID NO: 13, and genes having nucleotide sequence of nucleotides 443 to 4210 of SEQ ID NOS: 44, 46 or 48, and the mutant α-KGDH proteins encoded by these genes are shown in SEQ ID NOS: 14, 45, 47, and 49. Examples of the mutant odhA gene having this mutation also include a gene having nucleotide sequence of nucleotides 551 to 4213 of SEQ ID NO: 13 and genes having nucleotide sequence of nucleotides 551 to 4210 of SEQ ID NOS: 44, 46, or 48, and the mutant α-KGDH proteins encoded by these genes are shown in amino acids 37 to 1255 of SEQ ID NO: 14, and amino acids 37 to 1256 of SEQ ID NOS: 45, 47, and 49. In the nucleotide sequence of SEQ ID NO: 13, a mutation which deletes "t" at position 253 8 and "ggcta" at positions 2543 to 2547 of SEQ ID NO: 9 is introduced.

Furthermore, mutant odhA genes having a mutation which results in replacement of one or more amino acid residues selected from Lys at position 698, Leu at position 699, Arg at position 700, and Tyr at position 702 with another amino acid in SEQ ID NO: 10 are also preferable. The "another amino acid" is not particularly limited so long as it is different from the original amino acid, and selected from natural amino acids such as Lys, Glu, Thr, Val, Leu, Ile, Ser, Asp, Asn, Gln, Arg, Cys, Met, Phe, Trp, Tyr, Gly, Ala, Pro, and His. However, it is preferable that Lys at position 698 is replaced by amino acids other than basic amino acids such as Arg and His, Leu at position 699 is replaced by amino acids other than hydrophobic aliphatic amino acids such as Ile and Val, Arg at position 700 is replaced by amino acids other than basic amino acids such as Lys and His, Tyr at position 702 is replaced by amino acids other than hydroxy amino acids such as Ser and Thr. It is particularly preferable that Lys at position 698 is replaced by hydrophobic aliphatic amino acid such as Ile, Leu, or Val, Leu at position 699 is replaced by hydroxyl amino acid such as Ser, Thr or Tyr, Arg at position 700 is replaced by sulfur-containing amino acid such as Cys or Met, Tyr at position 702 is replaced by hydrophobic aliphatic amino acid such as Ile, Leu, or Val.

A GN-type mutation may cause both the deletion of Gly at position 701 and replacement of one or more amino acid residues selected from Lys at position 698, Leu at position 699, Arg at position 700, and Tyr at position 702 in SEQ ID NO: 10. Examples of such mutant odhA gene include genes having the nucleotide sequence of nucleotides 443 to 4210 of SEQ ID NOS: 44,46, and 48. Amino acid sequences of mutant α-KGDH proteins encoded by these mutant odhA genes are shown in SEQ ID NOS: 45, 47, and 49. Examples of such a mutant odhA gene also include genes having nucleotide sequence of nucleotides 551 to 4210 of SEQ ID NOS: 44, 46, and 48. Amino acid sequences of mutant α-KGDH proteins encoded by these mutant odhA genes are shown in amino acids 37 to 1256 of SEQ ID NOS: 45, 47, and 49. However, the mutant odhA gene having a mutation in the thiamine pyrophosphate binding region is not limited to these examples.

### (2) 2-2 type mutation

This type of mutation is preferably a mutation introduced into the region of nucleotides 1094 to 1114 of the odhA gene (SEQ ID NO: 9) which causes deletion and/or substitution of one or more amino acid residues selected from Asp at position 218, Val at position 219, Ile at position 220, Asp at position 221, Gly at position 222, Lys at position 223, and Pro at position 224 in SEQ ID NO: 10. The 2-2 type mutation is preferably a mutation which deletes Asp at position 218 in SEQ ID NO: 10. Examples of the odhA gene having this mutation include an odhA gene having a nucleotide sequence of nucleotides 443 to 4213 of SEQ ID NO: 11, or an odhA gene having a nucleotide sequence of nucleotides 55 to 4213 of SEQ ID NO: 11. The amino acid sequences of the mutant α-KGDH encoded by these genes are shown in SEQ ID NO: 12 or amino acid numbers 37 to 1256 of SEQ ID NO: 12, respectively. The nucleotide sequence of SEQ ID NO: 11 has the mutations to delete "gacgt" at 1094 to 1098 and replace "ag" at 1110 to 1111 with "ggcc" in the nucleotide sequence shown in SEQ ID NO: 9.

The 2-2 type mutation is also preferably a mutation which replaces one or more amino acids selected from Val at position 219, Ile at position 220, Asp at position 221, Gly at position 222, and Lys at position 223. Although amino acid which replaces these amino acids are not particularly limited, Val at position 219 is preferably replaced with an amino acid other than an aliphatic hydrophobic amino acid such as Ile and Leu, Ile at position 220 is preferably replaced with an amino acid other than an aliphatic hydrophobic amino acid such as Leu, Asp at position 221 is preferably replaced with an amino acid other than an acidic amino acid such as Glu, and Gly at position 222. is preferably replaced with an amino acid other than a simple amino acid such as Ala. More preferably, Val at position 219, Ile at position 220, and Asp at position 221 are replaced with basic amino acids such as His, Arg and Lys, Gly at 222 is replaced with an amino acid having amide-containing side chain such as Asp and Gln, and Lys at position 223 is replaced with an amino acid such as Gly and Ala.

However, mutant odhA gene having 2-2 type mutation is not limited to these examples.

### (3) GN2-2 Type mutation

This mutation includes both the GN type mutation and the 2-2 type mutation. Examples of the odhA gene having this mutation include an odhA gene having a nucleotide sequence ofnucleotides 443 to 4213 of SEQ ID NO: 15, or an odhA gene having a nucleotide sequence of nucleotides 551 to 4213 of SEQ ID NO: 15. The amino acid sequences of the mutant α-KGDH encoded by these genes are shown in SEQ ID NO: 16 or amino acids 37 to 1254 of SEQ ID NO: 16, respectively. However, the mutant odhA gene having a GN2-2 type mutation is not limited to these examples.

Other kinds of mutant odhA gene used in the present invention may be screened by using a ygg mutant strain, such as ATCC13869-L as described in Example 5 shown below That is, a random mutation is introduced into the odhA gene and used to transform the ygg mutant strain. A strain which grows almost at the same growth rate as a wild-type strain or a non-mutated strain and exhibits α-KGDH activity which is less than half that of the wild-type strain or non-mutated strain is selected from the transformants, followed by sequence determination. Thereby, mutant odhA genes can be obtained.

A mutant E1o subunit of α-KGDH complex encoded by the above-described mutant odhA gene is a protein which has α-KGDH activity which is less than half that of a wild-type or non-mutated strain by forming a complex with E2o and E3 subunits of α-KGDH complex, and does not cause severe growth retardation of a coryneform bacterium when it is expressed in the coryneform bacterium.

Such properties of the mutant E1o subunit are considered to be maintained even if one or several amino acids other than the specific amino acids replaced in the above-described GN type or 2-2 type mutants, for example, amino acids that do not influence the enzymatic activity, are replaced by other amino acids. Therefore, in the above-mentioned amino acid sequences of the mutant E1o subunit protein (e.g., SEQ ID NOS: 12, 14, 16, 45, 47 and 49, amino acids 37 to 1256 of SEQ ID NOS: 12, 45, 47 and 49, amino acids 37 to 1255 of SEQ ID NO: 14, or amino acids 37 to 1254 of SEQ ID NO: 16), one or several amino acids other than the specific amino acids substituted in the GN type or 2-2 type mutants may be replaced, so long as the encoded protein exhibits α-KGDH activity less than half of a wild-type α-KGDH complex by forming a complex together with α-KGDH E2o subunit and E3 subunit proteins. Here, "several" means preferably 2 to 20, more preferably 2 to 10, and particularly preferably 2 to 5. Such amino acid substitution may be one caused by a naturally occurring mutation arising from individual difference and difference in species of bacterium from which odhA gene is derived. For example, mutant odhA gene having a nucleotide sequence encoding amino acid sequence of SEQ ID NO: 51 whereby amino acids corresponding to the above-mentioned GN-type mutation or 2-2 type mutation are deleted and/or substituted can also be used in the present invention. Such amino acids corresponding to the GN-type mutation or 2-2 type mutation in the amino acid sequence of SEQ ID NO: 51 can be easily identified by aligning the amino acid sequences of SEQ ID NOS: 10 and 51. The above-mentioned substitution is preferably a conservative substitution. In the case of aromatic amino acids, conservative substitutions are referred to substitutions between phe, trp, and tyr for each other. In the case of hydrophobic amino acids, conservative substitutions are referred to substitutions between leu, ile, and val for each other. In the case of polar amino acids, conservative substitutions are referred to substitutions between gln and asn for each other. In the case of basic amino acids, conservative substitutions are referred to substitutions between arg, lys, and his for each other. In the case of acidic amino acids, conservative substitutions are substitutions between asp and glu for each other. In the case of hydroxyl group-containig amino acids, conservative substitutions are referred to substitutions between ser and thr for each other. The conservative substitutions also include: substitution of ser or thr for ala, substitution of gln, his, or lys for arg; substitution of glu, gln, lys, his, or asp for asn; substitution of asn, glu, or gln for asp; substitution of ser or ala for cys; substitution of asn, glu, lys, his, asp, or arg for gln; substitution of asn, gln, lys, or asp for glu; substitution of vfor gly; substitution of asn, lys, gln, arg, or tyr for his; substitution of leu, met, val, or phe for ile; substitution of ile, met, val, or phe for leu; substitution of asn, glu, gln, his, or arg for lys; substitution of ile, leu, val or phe for met; substitution of trp, tyr, met, ile, or leu for phe; substitution of thr or ala for ser; substitution of ser or ala for thr; substitution of phe or tyr for trp; substitution of his, phe, or trp for tyr; and substitution of met, ile, or leu for val.

When the E1o subunit and E2o subunit are encoded by a single odhA gene, the α-KGDH complex may include the protein of SEQ ID NO: 10 (or amino acids 37 to 1257 of SEQ ID NO: 10) and the protein of SEQ ID NO: 18. When the E1o subunit is encoded by the odhA gene and the E2o subunit is encoded by the gene of SEQ ID NO: 27, the α-KGDH complex may include the protein of SEQ ID NO: 10, the protein of SEQ ID NO: 18, and the protein of SEQ ID NO: 28.

A mutant odhA gene of the present invention is a gene encoding the above-mentioned mutant α-KGDH E1o subunit. The mutant odhA gene of the present invention may be genes that hybridize with a polynucleotide each having the nucleotide sequence of nucleotides 443 to 4213 of SEQ ID NO: 11, 13, or 15, with a polynucleotide each having the nucleotide sequence ofnucleotides 443 to 4210 of SEQ ID NO: 44, 46 or 48, with the nucleotide sequence of nucleotide numbers of 551 to 4213 of SEQ ID NO: 11, 13, or 15, or with the nucleotide sequence of nucleotide numbers 443 to 4210 of SEQ ID NO: 44, 46, or 48, or with a probe prepared from the sequences under stringent conditions so long as the gene encodes a protein that exhibits α-KGDH activity less than half that of a wild-type α-KGDH complex by forming a complex together with the E2o subunit and E3 subunit proteins. Examples of stringent conditions include those under which DNAs hybridize to each other at a salt concentration with washing typical of Southern hybridization, i.e., washing once or preferably 2-3 times under 1 x SSC, 0.1% SDS at 60°C, preferably 0.1 x SSC, 0.1% SDS at 60°C, more preferably 0.1 x SSC, 0.1% SDS at 68°C.

### <3> Method of producing L-glutamic acid

L-glutamic acid can be produced by culturing the coryneform bacterium of the present invention in a medium to cause accumulation of L-glutamic acid in the medium and/or in the bacterial cells, and collecting the L-glutamic acid from the medium and/or the bacterial cells. In the production method of the present invention, L-glutamic acid is produced preferably by culturing the coryneform bacterium of the present invention, for example, at 25 to 40°C for 8 to 120 hours. If a strain which exhibits a sufficient growth rate at low temperatures but exhibits decreased growth rate at high temperatures such as the GN-2-2 strain shown in the Examples is used for L-glutamic acid production, it is preferable to culture such a strain at a low temperature such as 25 to 30°C for 8 to 30 hours so that the strain can grow; and then incubate the obtained bacterial cells at high temperature such as 34 to 40°C for 16 to 48 hours so that the strain can produce L-glutamic acid.

The culture medium may be an ordinary medium that contains a carbon source, a nitrogen source, an inorganic salt, and optionally organic micronutrients such as amino acids and vitamins. Either a synthetic medium or a natural medium may be used. Any kinds of the carbon source and nitrogen source may be used so long as they can be utilized by the strain to be cultured.

Saccharides such as glucose, glycerol, fructose, sucrose, maltose, mannose, galactose, starch hydrolysate, and molasses may be used as the carbon source. In addition, organic acids such as acetic acid and citric acid, and alcohols such as ethanol may also be used alone or in combination as a carbon source. Ammonia, ammonium salts such as ammonium sulfate, ammonium carbonate, ammonium chloride, ammonium phosphate, and ammonium acetate, nitrates, and the like may be used as the nitrogen source. Amino acids, vitamins, fatty acids, nucleic acids, substances containing peptone, casamino acid, yeast extract, and soybean protein decomposition products may be used in a slight amount as the organic nutrients. When an auxotrophic mutant strain that requires an amino acid etc. for growth is used, such a required nutrient is preferably added. Phosphates, magnesium salts, calcium salts, iron salts, manganese salts, and the like can be used as inorganic salts.

Preferably, aerobic culturing is performed by controlling the fermentation temperature and adjusting the pH of the culture medium to 3 to 9. When the pH decreases during the culture, the medium is neutralized by adding alkali such as calcium carbonate or ammonia gas. Culture for about 10 to about 120 hours results in accumulation of a considerable amount of L-glutamic acid in the medium.

Furthermore, the culture may be performed by using a liquid medium adjusted to conditions under which the produced L-glutamic acid crystallizes and precipitates. The conditions under which L-glutamic acid crystallizes include pH 5.0 to 4.0, preferably pH 4.5 to 4.0, more preferably pH 4.3 to 4.0, particularly preferably pH 4.0 (EP1233069, EP1233070).

Collection of L-glutamic acid from the medium after completion of the culture may be performed by conventional methods. L-glutamic acid may be collected, for example, by removing bacterial cells from the medium and concentrating L-glutamic acid or by using ion exchange chromatography. When the culture is performed under conditions under which L-glutamic acid crystallizes and precipitates, the crystallized L-glutamic acid can be collected, for example, by centrifugation or filtration. In this case, L-glutamic acid dissolved in the medium may also be collected after crystallization of the dissolved L-glutamic acid.

### EXAMPLES

Hereinafter, the present invention will be more specifically explained by referring to the following non-limiting examples.

### Example 1

### <1> Construction of a vector carrying the sacB gene

### (A) Construction of pBS3

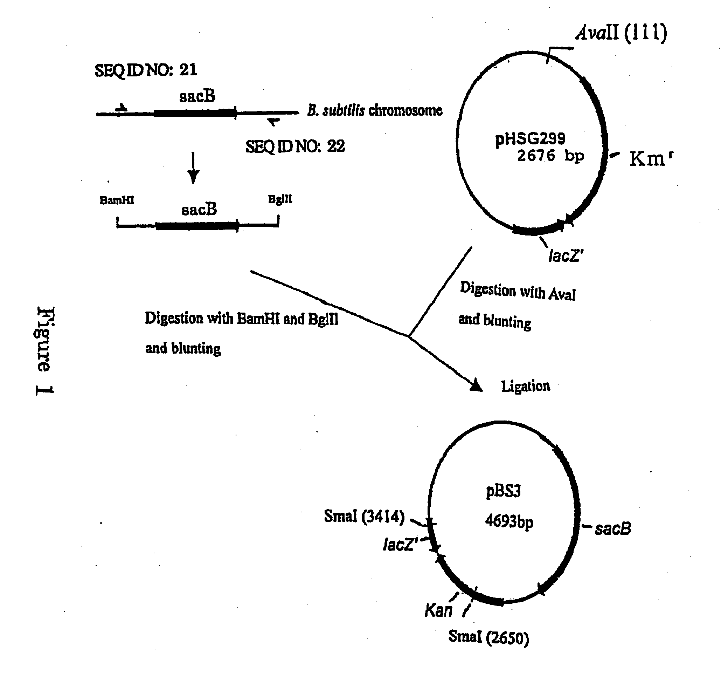

A sacB gene (SEQ ID NO: 19) was obtained by PCR using a chromosomal DNA *of-Bacillus subtilis* as a template and the oligonucleotides of SEQ ID NOS: 21 and 22 as primers. The PCR was performed using LAtaq (available from TaKaRa) according to the program of one cycle of pre-denaturation at 94°C for 5 minutes; and 25 cycles of denaturation at 94°C for 30 seconds, annealing at 49°C for 30 seconds, and elongation at 72°C for 2 minutes. The obtained PCR product was purified by a conventional method, and then digested with Bg1II and BamHI and blunt-ended. The fragment was inserted into pHSG299 which had been digested with AvaII and blunt-ended. The resulting DNA was used to transform competent cells of *Escherichia coli* JM109 (available from Takara Bio). Then, the transformed bacterial cells were applied onto an LB agar plate containing 25 µg/ml of kanamycin (hereinafter, abbreviated as "Km"), and incubated for one night Thereafter, colonies that appeared were selected as transformants. Plasmids were isolated from the obtained transformants and the plasmid having an insert of the object PCR product was named pBS3. Fig. 1 shows the procedure for constructing pBS3.

### (B) Construction of pBS4S

The SmaI recognition site in the kanamycin-resistant gene on pBS3 was destroyed by nucleotide substitution using cross-over PCR without causing amino acid substitution. First, PCR was performed using pBS3 as a template and synthetic DNAs of SEQ ID NOS: 23 and 24 as primers to obtain an N-terminal fragment of the kanamycin-resistant gene. On the other hand, to obtain a C-terminal fragment of kanamycin-resistant gene, PCR was performed using pBS3 as a template and synthetic DNAs of SEQ ID NOS: 25 and 26 as primers. The PCR was performed using Pyrobest DNA Polymerase (available from Takara Bio) according to the program of pre-denaturation at 98°C for 5 minutes; and 25 cycles of denaturation at 98°C for 10 seconds, annealing at 57°C for 30 seconds, and elongation at 72°C for 1 minute. SEQ ID NOS: 24 and 25 are partially complementary to each other and do not contain the SmaI recognition site. Then, to obtain a full-length fragment of the mutant kanamycin-resistant gene without the SmaI recognition site, the above-mentioned N-terminal and C-terminal gene products were mixed with each other in substantially equimolar amounts. PCR was performed using the gene products as a template and synthetic DNAs of SEQ ID NOS: 23 and 26 as primers to obtain a mutation-introduced Km resistant gene. The PCR was performed using Pyrobest DNA Polymerase (available from Takara Bio) according to the program of pre-denaturation at 98°C for 5 minutes; and 25 cycles of denaturation at 98°C for 10 seconds, annealing at 57°C for 30 seconds, and elongation at 72°C for 1.5 minutes.

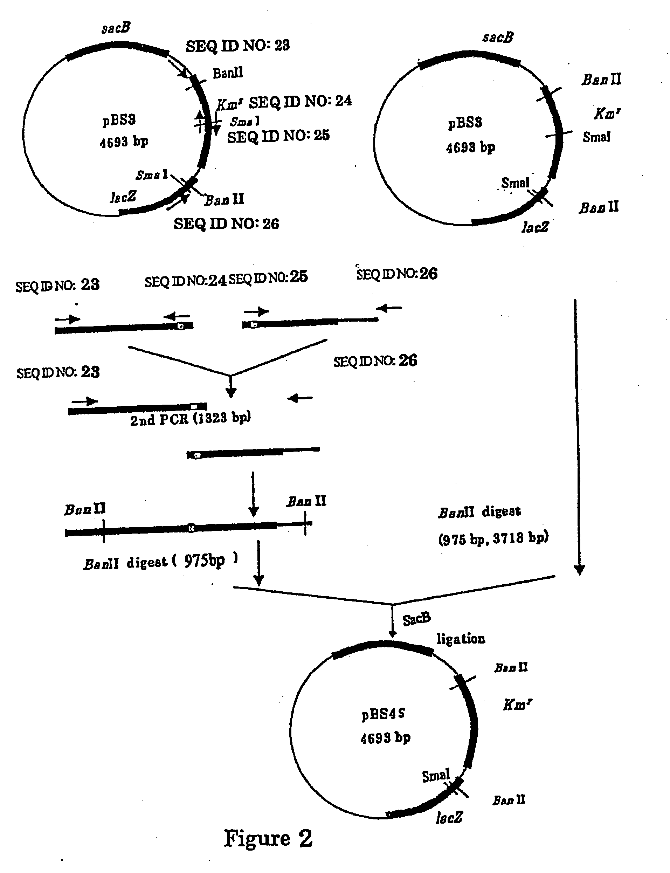

The PCR product was purified by a conventional method, and then digested with BanII and then inserted into the above-described BanII recognition site of pBS3. The resulting plasmid was used to transform competent cells of *Escherichia coli* JM109 (available from Takara Bio). That is, the transformed bacterial cells were applied onto LB agar medium containing 25 µg/ml of kanamycin, and incubated for one night. Thereafter, colonies that appeared were selected as transformants. Plasmids were isolated from the obtained transformants and the plasmid having an insert of the object PCR product was named pBS4S. Fig. 2 shows the procedure for constructing pBS4S.

### <2> Introduction of odhA mutation (GN-type) into C. glutamicum ATCC13969 strain

The sequence of odhA that encodes α-ketoglutarate dehydrogenase of coryneform bacteria has already been reported (Microbiology 142, 3347-3354 (1996), GenBank accession No. D84102).

Analysis of the nucleotide sequence of the L-glutamic acid-producing bacterium strain GN which the inventors of the present invention had succeeded in breeding revealed that this strain has deletions of the nucleotides 2538 and 2543 to 2547 in the nucleotide sequence of the odhA gene (SEQ ID NO: 9) as shown in Table 1. In Table 1, amino acid sequence of mutant E1o subunit encoded by the mutant odhA gene is also shown.

**Table 1**

| Strain | Nucleotide sequence of odhA gene | | | | | | | | | | | |
|---|---|---|---|---|---|---|---|---|---|---|---|---|
| ATCC13869 | GCT | AAG | CTG | CGT | GGC | TAC | GAC | GTC | GGA | GGC | ACC | ATC |
| OAGN | GCT | AAG | C-G | CGT | --- | --C | GAC | GTC | GGA | GGC | ACC | ATC |

| Strain | Amino acid sequence of E1o subunit | | | | | | | | | | | |
|---|---|---|---|---|---|---|---|---|---|---|---|---|
| ATCC13869 | Ala | Lys | Leu | Arg | Gly | Tyr | Asp | Val | Gly | Gly | Thr | Ile |
| OAGN | Ala | Lys | Arg | Val | --- | --- | Asp | Val | Gly | Gly | Thr | Ile |

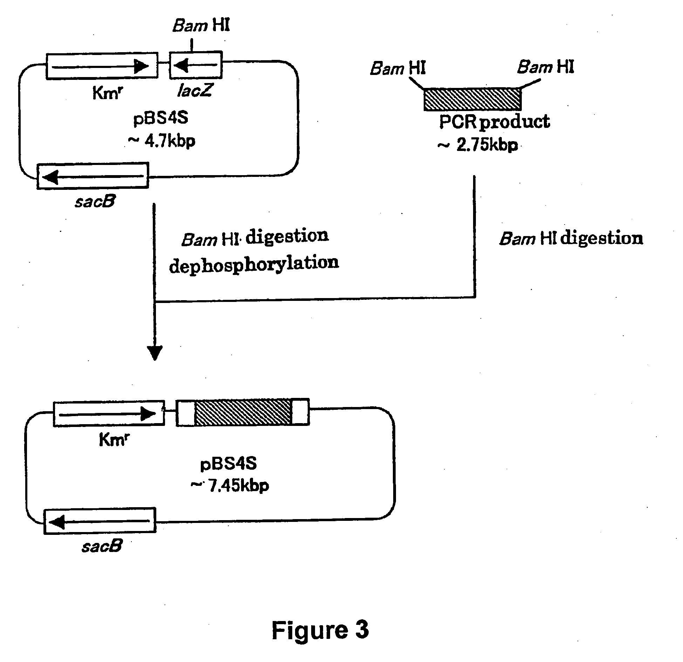

Then, this mutant odhA gene was introduced into *C. glutamicum* 2256 strain (ATCC13869) and evaluated. At first, a plasmid for introducing these mutations into *C. glutamicum* 2256 strain (ATCC13869) was prepared. A chromosomal DNA was extracted from the above-mentioned GN strain using Bacterial Genomic DNA Purif. Kit (manufactured by MS Technosystems Co., Ltd.) and PCR was performed using the obtained chromosomal DNA as a template and oligonucleotides of SEQ ID NOS: 1 and 2 as primers. PCR was performed using Pyrobest polymerase (available from Takara Bio) according to the program of 30 cycles of denaturation at 98°C for 10 seconds, annealing at 50°C for 30 seconds, and elongation at 72°C for 3 minutes to amplify a fragment of about 2.75 kb. The primers contain a BamHI recognition sequence at the 5'-end and are designed to amplify the region of nucleotide numbers 1521 to 4270 of the sequence of GenBank accession No. D84102. The amplified fragment was digested with BamHI and ligated to pBS4S vector digested with BamHI (Ligation kit Ver. 2, using a product available from Takara Bio), and thereby plasmid pBSOAGN was obtained (Fig. 3 shows the construction procedure).

pBSOAGN was introduced into *C. glutamicum* ATCC13869 strain by an electric pulse method (JP02-207791A) and the obtained bacterial cells were applied to CM-Dex agar medium (5g/l glucose, 10g/l polypeptone, 10g/l yeast extract, 1g/l KH₂PO₄, 0.4 g/l MgSO₄·7H₂O, 0.01 g/l FeSO₄·7H₂O, 0.01 g/l MnSO₄·4-5H₂O, 3 g/l urea, 1.2 g/l soybean protein hydrolysate, and 20 g/l agar, pH adjusted to 7.5) containing 25 µg/ml of kanamycin. After culturing at 25°C, it was confirmed by using PCR that the colonies which appeared were once recombinant strains in which pBSOAGN was incorporated by homologous recombination on the chromosome. The PCR was performed using the chromosomal DNA of a candidate strain as template and an oligonucleotide having a nucleotide sequence specific to the sequence of pBS4S (SEQ ID NO: 3) and an oligonucleotide having a nucleotide sequence specific to the sequence on the chromosome (SEQ ID NO: 4) as primers. That is, since the sequence of pBS4S is absent on the chromosome of a non-recombinant strain, no fragment is amplified by PCR if the candidate strain is not a recombinant strain. The obtained once recombinant strains were cultured at 25°C for one day in CM-Dex liquid medium containing 25 µg/ml kanamycin and the obtained culture was diluted appropriately and applied onto S10 plate which has a composition of the above-mentioned CM-Dex medium whereby 5g/l glucose is replaced by 10 g/l of sucrose. Several strains that grew on the S10 plate and showed kanamycin sensitivity were selected, and then the nucleotide sequence of the odhA sequence was confirmed by the method of Sanger (J. Mol. Biol., 143, 161 (1980)). The nucleotide sequence was analyzed by genetic Analyzer ABI310 (manufactured by Applied Biosystems) using BigDye terminator sequencing kit (manufactured by Applied Biyosystems). The thus obtained strain carrying the GN-type mutant odhA gene was named ATCC13869 OAGN.

### <3> Introduction of the 2-2 type mutant odhA gene into C. glutamicum ATCC13869 strain

Analysis of the nucleotide sequence of the L-glutamic acid-producing "2-2" strain which the inventors of the present invention had succeeded in breeding revealed that the strain contains deletions of the nucleotides 1094 to 1098 and replacement of "ag" at 1110 to 1111 with "ggcc" in the odhA gene (SEQ ID NO: 9) as shown in Table 2. In Table 2, amino acid sequence of mutant E1o subunit encoded by the mutant odhA gene is also shown.

**Table 2**

| Strain | Nucleotide sequence of odhA gene | | | | | | | | | | | |
|---|---|---|---|---|---|---|---|---|---|---|---|---|
| ATCC13869 | AAC | TCC | TAC | GAC | GTC | ATC | GAC | GGC | AAG | CCA | ACC | CTG |
| OA2-2 | AAC | TCC | TAC | --- | CAT | CGA | CGG | CAG | GCC | CCA | ACC | CTG |

| Strain | Amino acid sequence of E1o subunit | | | | | | | | | | | |
|---|---|---|---|---|---|---|---|---|---|---|---|---|
| ATCC13869 | Asn | Ser | Tyr | Asp | Val | Ile | Asp | Gly | Lys | Pro | Thr | Leu |
| OA2-2 | Asn | Ser | Tyr | --- | His | Arg | Arg | Gln | Ala | Pro | Thr | Leu |

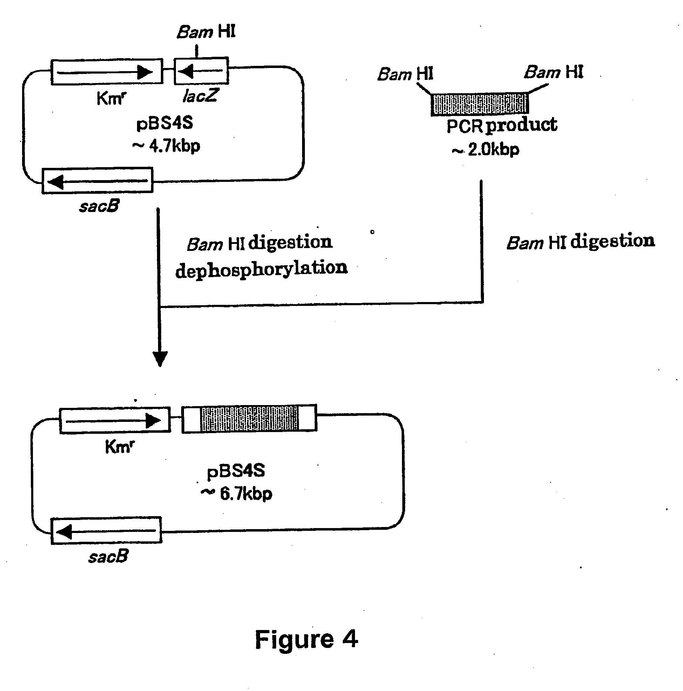

Then, this mutant odhA gene was introduced into *C. glutamicum* 2256 strain (ATCC 13 869) and evaluated. At first, a plasmid for introducing these mutations into *C. glutamicum* 2256 was prepared. A chromosomal DNA was isolated from the 2-2 strain using Bacterial Genomic DNA Purif. Kit (manufactured by MS Technosystems Co., Ltd.) and PCR was performed using this chromosomal DNA as a template and oligonucleotides of SEQ ID NOS: 5 and 6 as primers. PCR was performed using TaKaRa Ex Taq (available from Takara Bio) according to the program of 25 cycles of denaturation at 94°C for 30 seconds, annealing at 55°C for 10 seconds, and elongation at 72°C for 2 minutes to amplify a fragment of about 2.0 kb. The primers contain a BamHI recognition sequence at the 5'-end and designed to amplify the region of nucleotides 51 to 2150 of the nucleotide sequence of GenBank accession No. D84102. The amplified fragment was digested with BamHI and ligated to pBS4S vector digested with BamHI using Ligation kit Ver. 2 of Takara Bio, and thereby the plasmid pBSOA2-2 was obtained (Fig. 4 shows the construction diagram). The same procedure described in Example 1<2> was performed to obtain a strain carrying the 2-2 type mutant odhA gene (ATCC13869 OA2-2 strain).

### <4> Introduction of 2-2 type mutation into GN type odhA gene of ATCC13869 OAGN strain

According to the same procedures as described in Example 1<2>, the plasmid pBSOA2-2 constructed in Example 1<3> was introduced into ATCC13869 OAGN strain prepared in Example 1<2> to obtain ATCC13869 strain carrying both the GN type and 2-2 type mutations in odhA gene. Hereinafter, a double mutation consisting of 2-2 type mutation and GN type mutation is also called GN2-2 mutation.

Although the mutant strains were prepared using mutant odhA genes amplified by PCR using a chromosomal DNA from the GN strain or 2-2 strain as a template in Example 1<2> to <4>, they can also be prepared by using mutant odhA genes obtained by site-directed mutagenesis technique such as those using Mutan-Super Express Km kit (Takara Bio). For example, a plasmid having a similar structure to pBSOAGN prepared in Example 1<2> can be obtained as follows. That is, an odhA gene fragment is prepared by PCR using synthetic DNAs of SEQ ID NOS: 2 and 5 as primers and a chromosomal DNA of a wild-type odhA gene as a template, and the resultant PCR fragment is cloned into the BamHI recognition site of plasmid pKF19k attached to Mutan-Super Express Km kit. Next, another PCR is performed using a template of the obtained plasmid and primers of a synthetic DNA of SEQ ID NO: 7 having phosphorylated 5'-end and the selection primer attached to Mutan-Super Express Km kit. Transformation of sup⁰ *E. coli*, for example, MV1184 strain (available from Takara Bio) with the obtained PCR product results in construction of a plasmid containing the mutant odhA gene fragment. Finally, the fragment is digested with BamHI and inserted into the pBS4S vector, and thereby a plasmid similar to pBSOAGN can be constructed.

Similarly, to obtain odhA gene containing the 2-2 type mutation, a plasmid similar to pBSOA2-2 can be constructed by using the synthetic DNA of SEQ ID NO: 8 having phosphorylated 5'-end instead of the synthetic DNA of SEQ ID NO: 7 having phosphorylated 5'-end in the procedure as described above.

Furthermore, to obtain odhA gene containing GN2-2 double mutations, a fragment containing the GN-type mutation is excised or PCR-amplified from the above-described plasmid similar to pBSOAGN and used to replace the corresponding fragment on the above-described plasmid similar to pBSOA2-2.

### Example 2

### Comparison of the ability of strains ATCC13869 OAGN, OA2-2, and OAGN2-2 to degrade glutamic acid

While strains carrying a wild-type odhA gene can assimilate L-glutamic acid in a medium, the strains in which the activity of α-KGDH is weakened or eliminated due to the odhA mutation is presumed to also have a decreased ability to degrade glutamic acid. Then, the ability to degrade glutamic acid was determined using each of the odhA mutant strains ATCC13869 OAGN, OA2-2, and OAGN2-2 prepared in Example 1<2> to <4>. Each strain was cultured on a CM-Dex plate for one day at 25°C and then inoculated into a liquid medium composed of 20 g/l sodium glutamate, 2.64 g/l (NH₄)₂SO₄, 0.5 g/l KH₂PO₄, 0.5 g/l K₂HPO₄, 0.25 g/l MgSO₄·7H₂O, 0.01 g/l FeSO₄·7H₂O, 0.01 g/l MnSO₄·4-5H₂O, 0.01 g/l CaCl₂, 0.02 mg/l CuSO₄, 40g/l MOPS, 0.03 g/l protocatechinic acid, 200 µg/l vitamin B1, and 300 µg/l biotin (adjusted to pH 6.7 with NaOH), followed by culturing at 25°C and 34°C for 50 hours. The amounts of glutamic acid at the starting point, and after 25 hours and 50 hours of the culture were measured, respectively and the amounts of degraded glutamic acid were compared between the strains cultured at 25°C and 34°C. Table 3 shows the results. In particular, the GN2-2 mutant strain exhibited a significant decrease in the amount of degraded glutamic acid, especially when the culture temperature was 34°C. These results suggest that introduction of the GN2-2 mutation can efficiently reduce the α-KGDH activity and a strain having this mutation is preferably used in L-glutamic acid production.

**Table 3: Amount of degraded glutamic acid**

| Strains | 25 Hours | | 50 Hours | |
|---|---|---|---|---|
| | 25°C | 34°C | 25°C | 34°C |
| ATCC13869 | 15 | 15 | 15.4 | 15.4 |
| OAGN | 3.8 | 5.8 | 9.4 | 10.0 |
| OA2-2 | 11.7 | 15.1 | 15.4 | 15.4 |
| OAGN2-2 | 0.7 | 0.5 | 4.9 | 1.0 |

| | | | | |
|---|---|---|---|---|
| (Unit:g/l) | | | | |

### Example 3

### Comparison of the α-KGDH activity of ATCC13869 OAGN, OA2-2, and OAGN2-2 strains

The α-KGDH activity of the strains ATCC13869 OAGN, OA2-2, and OAGN2-2 was measured using the culture broth collected after 4 hours from the start of the culture in Example 4 as described below The activity was measured according to the method described in Agric. Biol. Chem., 44(8), p1897 (1980). Specifically, after bacterial cells were washed with 0.2% sodium chloride, they were suspended in a buffer solution of 100 mM TES-NaOH (pH 7.5) containing 30% glycerol. The bacterial cells were sonicated using Bioruptor (Olympus) and then centrifuged to remove non-ruptured bacterial cells, followed by gel filtration with the same buffer using Sephadex-G25 (Amersham Pharmacia). The thus obtained preparation was used as a crude enzyme solution. The crude enzyme solution was added to a reaction system containing 100 mM TES-NaOH (pH 7.7), 5 mM MgCl₂, 0.2 mM CoA, 0.3 mM cocarboxylase; 1 mM α-ketoglutaric acid, 3 mM L-cysteine, and 1 mM acetylpyridine-adenine-dinucleotide and absorption at 365 nm at 31.5°C was measured using Hitachi spectrophotometer U-2001. In the measurement of the protein concentration in the crude enzyme solution, protein Assay (Bio-Rad) was used. Bovine serum albumin was used as a standard protein.

Table 4 shows the results of the measurement of the α-KGDH activity. Introduction of the GN type mutation, 2-2 type mutation, and GN2-2 type mutation leads to a decrease in the α-KGDH as compared to ATCC13869. In particular, in the case of GN2-2 type mutation-introduced strain, a considerable decrease in the activity was observed. This indicates that the GN type mutation, 2-2 type mutation, and GN2-2 type mutation are mutations that reduce the α-KGDH activity.

**Table 4: α-KGDH activity**

| strains | Culture temperature 25°C | Culture temperature 34°C |
|---|---|---|
| ATCC13869 | 0.036 | 0.021 |
| OAGN | 0.001 | 0.002 |
| OA2-2 | 0.007 | 0.009 |
| OAGN2-2 | 0.001> | 0.001> |

| | | |
|---|---|---|
| (Unit: ΔAbs/min/mg protein) | | |

### Example 4

### Comparison of L-glutamic acid-producing ability of the strains ATCC13869 OAGN, OA2-2, and OAGN2-2

The L-glutamic acid-producing ability of the strains ATCC13869 OAGN, OA2-2, and OAGN2-2 were examined in a jar fermenter culture. First, the above-mentioned four strains were cultured on a CM-Dex agar medium at 25°C for one day and the obtained bacterial cells were inoculated in 300 ml of a sterilized seed medium containing 60 g/l glucose,1.54 g/l H₃PO₄, 1.45 g/l KOH, 0.9 g/l MgSO₄·7H₂O, 0.01 g/l FeSO₄·7H₂O, 670 µg/l vitamin B1, 3,200 µg/l biotin, 0.28 g/l DL-Met, 1.54 g/l soybean protein hydrolysate, and 0.1 ml/l defoaming agent AZ-20R, and cultured until the sugar was completely consumed. During the culture, the medium was stirred with aeration of 1/1 VVM so that the concentration of dissolved oxygen was maintained not less than 5%. The pH during the culture was controlled to pH 7.2 with ammonia gas. Then, 30 ml of the obtained seed culture was inoculated in 270 ml of a,main culture medium containing 80 g/l glucose, 3.46 g/l KH₂PO₄, 1.0 g/l MgSO₄·7H₂O, 0.01 g/l FeSO₄·7H₂O, 0.01 g/l MnSO₄·4-5H₂O, 230 µg/l vitamin B1, 525 µg/l biotin, 0.35 g/l soybean protein hydrolysate, and 0.2 ml/l defoaming agent AZ-20R, and cultured at 25°C or 34°C. During the culture, aeration was performed as described above. The pH during the culturing was controlled at pH 7.3 with ammonia gas.

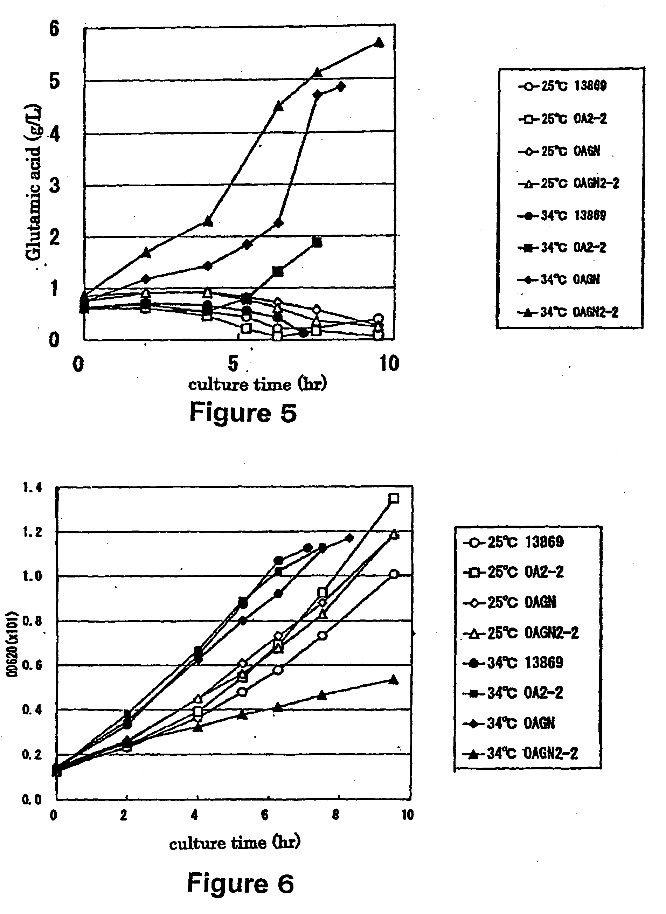

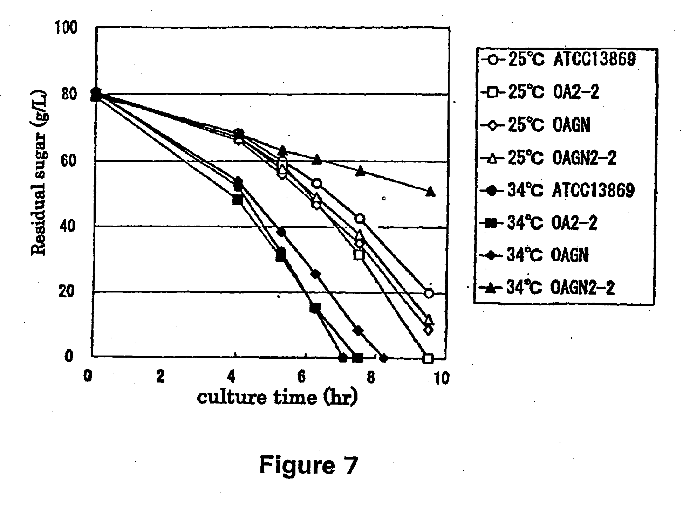

Table 5 shows amount of accumulated L-glutamic acid after about 7.5 hours from the start of the culture. Figures 5, 6, and 7 show time courses of L-glutamic acid accumulation, bacterial cell amount, and residual sugar, respectively. ATCC13869 strain did not accumulate L-glutamic acid, whereas OAGN and OAGN2-2 mutant strains accumulated L-glutamic acid. In particular, when these strains were cultured at 34°C, the amounts of accumulated L-glutamic acid significantly increased. Each mutation-introduced strain showed almost the same growth rate as the wild-type strain.

From these results, it was confirmed that the GN type mutation, 2-2 type mutation, and GN2-2 type mutation are effective to increase L-glutamic acid production. Among these mutations, GN2-2 type mutation was found to have the most significant effect on the L-glutamic acid production.

**Table 5: Amount of produced L-glutamic acid (Glu) of odhA mutation-introduced strains**

| Strains | 25°C | 34°C |
|---|---|---|
| | Glu (g/L) | Glu (g/L) |
| ATCC13869 | 0.24 | 0.13 |
| OAGN | 0.18 | 1.88 |
| OA2-2 | 0.59 | 4.70 |
| OAGN2-2 | 0.38 | 5.13 |

### Example 5

### Screening of odhA mutant strains using a yggB mutant strain

### <Construction of L30 type yggB mutant strain>

At first, a mutant strain having a mutation in yggB gene was constructed from ATCC13869 strain. The L30 type mutation is a mutation which replaces "C" at position 1768 with "T" in the yggB gene (SEQ ID NO: 29). The mutant yggB gene having the L30 type mutation is shown in SEQ ID NO: 31 and the amino acid sequence encoded by the gene is shown in SEQ ID NO: 32. The mutant yggB gene was constructed by the same method as in Example 1. That is, a fragment encoding the N-terminus portion of the ygg gene was prepared by PCR using primers of SEQ ID NOS: 33 and 34 and a template of chromosomal DNA of the ATCC13869 strain. In a similar way, a fragment encoding the C-terminus portion of the ygg gene was prepared by PCR using primers of SEQ ID NOS: 35 and 36 and a template of chromosomal DNA of the ATCC13869 strain. Subsequently, a fragment of the yggB gene including the L30 type mutation was obtained by PCR using primers of SEQ ID NOS: 37 and 35 and a template of a mixture of equal amounts of the N-terminus fragment and C-terminus fragment. The obtained PCR product is digested by SacI and ligated to SacI-digested pBS4S, and thereby the plasmid for introducing the mutation is obtained (pBS4 yggB-L). The obtained pBS4 yggB-L was integrated into the chromosome of the ATCC13869 strain and then cured from the strain according to a similar method as in Example 1. The nucleotide sequence of yggB gene of the obtained kanamycin-resistant strain is determined and the strain in which yggB gene is replaced with L30 type was selected. The strain having yggB gene of SEQ ID NO: 31 was named ATCC13869-L strain. This strain can be used in the screening of odhA mutant genes.

### <Construction of the ygg, odhA double mutant strain>

Then, each of the mutations shown in Table 6 was introduced into the chromosomal odhA gene of ATCC13869-L strain. In Table 6, nucleotide sequences of the region corresponding to nucleotides 2528 to 2562 of SEQ ID NO: 9 of each strain are shown. In Table 7, amino acid sequences of the region corresponding to amino acids 696 to 707 of SEQ ID NO: 10 of each strain are shown.

The L30sucA8 strain in which odhA gene having nucleotide sequence of SEQ ID NO: 42 is introduced can be obtained as follows. The mutant odhA gene fragment is prepared by PCR using primers of SEQ ID NOS: 2 and 5. The obtained fragment is digested with BamHI and ligated to the BamHI site of plasmid pKF19m which is attached to Mutan-Super Express Km (Takara Bio). Then, PCR is performed using a primer of SEQ ID NO: 38 having a phosphorylated 5'-end and the selection primer of Mutan-Super Express Km, and the obtained PCR product is used to transform sup0-*E. coli* strain such as MV 1184 strain to obtain a plasmid containing the mutant odhA fragment. This fragment is inserted into the pBS4S plasmid and the obtained plasmid is used to transform ATCC13869-L strain according to a similar method as in Example 1 to thereby obtain a strain in which the plasmid is integrated into its chromosome. Then, a strain which is resistant to sucrose and sensitive to kanamycin is selected from these strains. The nucleotide sequence of odhA gene of the selected strains is determined and the strain in which function of α-KGDH is deficient by frameshift mutation in odhA gene is selected as ATCC13869-L30sucA8 (odhA8) strain. The other odhA mutant strains can be obtained by the similar procedures using the yggB mutant strain.

sucA801 strain in which a mutant odhA gene having a nucleotide sequence of SEQ ID NO: 44 is introduced can be obtained by a similar method as described above in which a primer of SEQ ID NO: 39 having a phosphorylated 5'-end is used instead of a primer of SEQ ID NO: 38.

sucA805 strain in which a mutant odhA gene having a nucleotide sequence of SEQ ID NO: 46 is introduced can be obtained by a similar method as described above in which a primer of SEQ ID NO: 40 having a phosphorylated 5'-end is used instead of a primer of SEQ ID NO: 38.

sucA77 strain in which a mutant odhA gene having a nucleotide sequence of SEQ ID NO: 48 is introduced can be obtained by a similar method as described above in which a primer of SEQ ID NO: 41 having a phosphorylated 5'-end is used instead of a primer of SEQ ID NO: 38.

The L30sucA8 strain does not have intracellular α-KGDH because the sucA8 mutation is a frame-shift mutation which causes immature truncation of α-KGDH protein. On the other hand, sucA801 strain, sucA805 strain, and sucA77 strain have decreased but some α-KGDH activity because these mutations are not frame-shift mutations and do not cause immature truncation of α-KGDH protein.

**Table 6: partial nucleotide acid sequence of odhA mutant genes**

| Strains | Nucleotide sequence of odhA gene | | | | | | | | | | | |
|---|---|---|---|---|---|---|---|---|---|---|---|---|
| ATCC13869-L | CTG | GCT | AAG | CTG | CGT | GGC | TAC | GAC | GTC | GGA | GGC | ACC |
| L30sucA8 | CTG | GCT | AAG | CTG | CGT | | C | GAC | GTC | GGA | GGC | ACC |
| L30sucA801 | CTG | GCT | AAG | CTG | CGT | | CTC | GAC | GTC | GGA | GGC | ACC |
| L30sucA805 | CTG | GCT | AAA | AGC | TGC | | GTC | GAC | GTC | GGA | GGC | ACC |
| L30sucA77 | CTG | GCT | ATA | AGC | TGC | | GTC | GAC | GTC | GGA | GGC | ACC |

**Table 7: amino acid sequence of odhA mutants**

| Strains | Amino acid sequence of E1o subunit | | | | | | | | | | | |
|---|---|---|---|---|---|---|---|---|---|---|---|---|
| wild | Leu | Ala | Lys | Leu | Arg | Gly | Tyr | Asp | Val | Gly | Gly | Thr |
| L30sucA8 ( 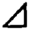 sucA) | Leu | Ala | Lys | Leu | Arg | | | Arg | Arg | Arg | Arg | His |
| L30sucA801 | Leu | Ala | Lys | Leu | Arg | --- | Leu | Asp | Val | Gly | Gly | Thr |
| L30sucA805 | Leu | Ala | Lys | Ser | Cys | --- | Val | Asp | Val | Gly | Gly | Thr |
| L30sucA77 | Leu | Ala | Ile | Ser | Cys | --- | Val | Asp | Val | Gly | Gly | Thr |

### <L-glutamic acid production using strains carrying each of the mutant odhA genes>

L-glutamic acid productivity of the obtained odhA mutant strains was evaluated by culturing these strains in Sakaguchi flask. Each of the strains listed in Table 6 was cultured at 31.5°C overnight on CM-Dex agar medium, and then 1/6 of the culture was transferred to 20 ml of a medium containing 60 g/l glucose, 22.5 g/l (NH₄)₂SO₄, 1 g/l KH₂PO₄, 0.4 g/l MgSO₄·7H₂O, 0.01 g/l FeSO₄·7H₂O, 0.01 g/l MnSO₄·4-5H₂O, 200 µg/l vitamin B1, 0.48 g/l soybean protein hydrolysate, and 300 µg/l biotin (adjusted to pH 8.0 with KOH), added with CaCO₃ and cultured with stirring at 115rpm at 31.5°C. The amount of accumulated L-glutamic acid after 19 hours of culture was shown in Table 8. The sucA801, sucA805, and sucA77 strains exhibited higher L-glutamic acid productivity than the ATCC13869-L strain carrying a wild-type odhA gene and the sucA8 strain carrying odhA gene with a frame-shift mutation. These results showed that L-glutamic acid is efficiently produced by regulating α-KGDH activity by introducing mutations into the proximate of thiamine pyrophosphate binding region of the odhA gene.

**Table 8: L-glutamic acid production by odhA mutant strains**

| Strain | L-glutamic acid (g/L) |
|---|---|
| ATCC13869-L | 4.9 |
| L30sucA8 | 19.8 |
| L30sucA801 | 22.1 |
| L30sucA805 | 23.8 |
| L30sucA77 | 21.6 |

## Claims

1. An L-glutamic acid-producing coryneform bacterium comprising
a) intracellular α-ketoglutarate dehydrogenase activity which is less than half that of a non-mutated or wild-type strain, and
b) a mutation in a region selected from the group consisting of (i) a region of nucleotides 2534 to 2548 of SEQ ID NO:9, and (ii) a region of nucleotides 1094 to 1114 of SEQ ID NO: 9 of a chromosomal odhA gene encoding the E1o subunit of the α-ketoglutarate dehydrogenase complex, wherein said bacterium grows at 80 % or more of the growth rate of a non-mutated or wild-type strain, and wherein said chromosomal odhA gene encodes a protein selected from the group consisting of:
(A) a protein comprising an amino acid sequence of SEQ ID NO: 10,
(B) a protein comprising an amino acid sequence of SEQ ID NO: 10, whereby one or 2 to 20 amino acids in said protein are substituted, deleted, inserted, or added, and wherein said protein has an activity of the E1o subunit of the α-ketoglutarate dehydrogenase complex,
(C) a protein comprising amino acids 37 to 1257 of SEQ ID NO: 10, and
(D) a protein comprising amino acids 37 to 1257 of SEQ ID NO: 10, whereby one or 2 to 20 amino acids in said protein are substituted, deleted, inserted, or added, and wherein said protein has an activity of the E1o subunit of the α-ketoglutarate dehydrogenase complex.

2. The coryneform bacterium according to claim 1, wherein said chromosomal odhA gene is selected from the group consisting of:
(a) a gene comprising nucleotides 443 to 4213 of SEQ ID NO: 9,
(b) a gene that is able to hybridize under stringent conditions to a polynucleotide comprising nucleotides 443 to 4213 of SEQ ID NO: 9 or a probe prepared from a polynucleotide comprising nucleotides 443 to 4213 of SEQ ID NO: 9, and wherein said gene encodes a protein which has the activity of the E1o subunit of the α-ketoglutarate dehydrogenase complex,
(c) a gene comprising nucleotides 551 to 4213 of SEQ ID NO: 9, and
(d) a gene that is able to hybridize under stringent conditions to a polynucleotide comprising nucleotides 551 to 4213 of SEQ ID NO: 9 or a probe prepared from a polynucleotide comprising nucleotides 551 to 4213 of SEQ ID NO: 9, and wherein said gene encodes a protein which has the activity of the E1o subunit of the α-ketoglutarate dehydrogenase complex.

3. The coryneform bacterium according to claim 1, wherein said mutation is deletion of one or more amino acids in the region comprising amino acids 698 to 702 of SEQ ID NO: 10.

4. The coryneform bacterium according to claim 1, wherein said mutation is replacement of an amino acid selected from the group consisting of Lys at position 698, Leu at position 699, Arg at position 700, Tyr at position 702, and combinations thereof in the amino acid sequence shown in SEQ ID NO: 10.

5. The coryneform bacterium according to claim 1, wherein said mutation is deletion of one or more amino acids in the region comprising amino acids 218 to 224 of SEQ ID NO: 10.

6. A method for producing L-glutamic acid comprising:
a) culturing the coryneform bacterium according to any one of claims 1 to 5 in a culture medium, and
b) collecting L-glutamic acid from the culture medium and/or the bacterium.

7. A gene encoding mutant α-ketoglutarate dehydrogenase selected from the group consisting of:
(a) a gene comprising nucleotides 443 to 4213 of a polynucleotide selected from the group consisting of SEQ ID NO: 11, 13, and 15, or a gene comprising nucleotides 443 to 4210 of a polynucleotide selected from the group consisting of SEQ ID NO: 44, 46, and 48,
(b) a gene that is able to hybridize under stringent conditions to a polynucleotide comprising nucleotides 443 to 4213 of a polynucleotide selected from the group consisting of SEQ ID NO: 11, 13, and 15, a polynucleotide comprising nucleotides 443 to 4210 of a polynucleotide selected from the group consisting of SEQ ID NO: 44, 46, and 48, a probe prepared from nucleotides 443 to 4213 of a polynucleotide selected from the group consisting of SEQ ID NO: 11, 13, and 15, or a probe prepared from nucleotides 443 to 4210 of a polynucleotide selected from the group consisting of SEQ ID NO: 44, 46, and 48, and wherein said gene encodes a protein which has α-ketoglutarate dehydrogenase activity which is less than half that of a wild-type or non-mutated strain by forming a complex with E2o and E3 subunits,
(c) a gene comprising nucleotides 551 to 4213 of a polynucleotide selected from the group consisting of SEQ ID NO: 11, 13, and 15, or a gene comprising nucleotides 551 to 4210 of a polynucleotide selected from the group consisting of SEQ ID NO: 44, 46, and 48,
(d) a gene that is able to hybridize under stringent conditions to a polynucleotide comprising nucleotides 551 to 4213 of a polynucleotide selected from the group consisting of SEQ ID NO: 11, 13, and 15, a polynucleotide comprising nucleotides 551 to 4210 of a polynucleotide selected from the group consisting of SEQ ID NO: 44, 46, and 48, or a probe prepared from nucleotides 551 to 4213 of a polynucleotide selected from the group consisting of SEQ ID NO: 11, 13, and 15, or a probe prepared from nucleotides 551 to 4210 of a polynucleotide selected from the group consisting of SEQ ID NO: 44, 46, and 48, and wherein said gene encodes a protein which has α-ketoglutarate dehydrogenase activity which is less than half that of a wild-type or non-mutated strain by forming a complex with E2o and E3 subunits.

8. A mutant α-ketoglutarate dehydrogenase selected from the group consisting of:
(a) a protein selected from the group consisting of SEQ ID NO: 12, 14, 16, 45, 47, and 49,
(b) a protein selected from the group consisting of SEQ ID NO: 12, 14, 16, 45, 47, and 49, whereby one or several amino acids in said protein are substituted, deleted, or added, and wherein said protein exhibits α-ketoglutarate dehydrogenase activity which is less than half that of a wild-type or non-mutated strain by forming a complex with E2o and E3 subunits,
(c) a protein comprising amino acids 37 to 1256 of an amino acid sequence selected from the group consisting of SEQ ID NO: 12, 45, 47, and 49, a protein comprising amino acids 37 to 1255 of an amino acid sequence of SEQ ID NO: 14, or a protein comprising amino acids 37 to 1254 of an amino acid sequence of SEQ ID NO: 16, and
(d) a protein comprising amino acids 37 to 1256 of an amino acid sequence selected from the group consisting of SEQ ID NO: 12, 45, 47, and 49, a protein comprising amino acids 37 to 1255 of an amino acid sequence of SEQ ID NO: 14, or a protein comprising amino acids 37 to 1254 of an amino acid sequence of SEQ ID NO: 16, whereby one or several amino acids in said protein are substituted, deleted, or added, and wherein said protein exhibits α-ketoglutarate dehydrogenase activity which is less than half that of a wild-type or non-mutated strain by forming a complex with E2o and E3 subunits.

## Patentansprüche

1. L-Glutaminsäure-herstellendes coryneformes Bakterium umfassend
a) intrazelluläre α-Ketoglutaratdehydrogenase-Aktivität, die weniger als die Hälfte derjenigen eines nicht-mutierten oder Wildtypstamms ist, und
b) eine Mutation in einem Bereich, ausgewählt aus der Gruppe bestehend aus (i) einem Nukleotidbreich 2534 bis 2548 der SEQ ID NO: 9 und (ii) eine Nukleotidbereich 1094 bis 1114 der SEQ ID NO: 9 eines chromosomalen odhA-Gens, das die Elo-Untereinheit des α-Ketoglutaratdehydrogenase-Komplexes kodiert, wobei das Bakterium bei 80 % oder mehr der Wachstumsgeschwindigkeit eines nicht-mutierten oder Wildtypstamms wächst, und wobei das chromosomale odhA-Gen ein Protein kodiert, das ausgewählt wird aus der Gruppe bestehend aus:
(A) einem Protein, das eine Aminosäuresequenz von SEQ ID NO: 10 umfasst,
(B) einem Protein, das eine Aminosäuresequenz von SEQ ID NO: 10 umfasst, wobei eine oder 2 bis 20 Aminosäuren im Protein substituiert, entfernt, eingefügt oder hinzugefügt sind, und wobei das Protein eine Aktivität der Elo-Untereinheit des α-Ketoglutaratdehydrogenase-Komplexes hat,
(C) einem Protein, das Aminosäuren 37 bis 1257 von SEQ ID NO: 10 umfasst, und
(D) einem Protein, das Aminosäuren 37 bis 1257 von SEQ ID NO: 10 umfasst, wobei eine oder 2 bis 20 Aminosäuren im Protein substituiert, entfernt, eingefügt oder hinzugefügt sind, und wobei das Protein eine Aktivität der Elo-Untereinheit des α-Ketoglutaratdehydrogenase-Komplexes hat.

2. Coryneformes Bakterium gemäß Anspruch 1, wobei das chromosomale odhA-Gen ausgewählt wird aus der Gruppe bestehend aus:
(a) einem Gen, das die Nukleotide 443 bis 4213 von SEQ ID NO: 9 umfasst,
(b) einem Gen, das unter stringenten Bedingungen mit einem Polynukleotid, das Nukeotide 443 bis 4213 von SEQ ID NO: 9 umfasst, oder einer Sonde, die hergerichtet ist, ausgehend von einem Polynukleotid, das Nukleotide 443 bis 4213 von SEQ ID NO: 9 umfasst, hybridisieren kann, und wobei das Gen ein Protein kodiert, das die Aktivität der Elo-Untereinheit des α-Ketoglutaratdehydrogenase-Komplexes hat,
(c) einem Gen, umfassend Nukleotide 551 bis 4213 von SEQ ID NO: 9, und
(d) einem Gen, das unter stringenten Bedingungen mit einem Polynukleotid, das Nukeotide 551 bis 4213 von SEQ ID NO: 9 umfasst, oder einer Sonde, die hergerichtet ist, ausgehend von einem Polynukleotid, das Nukleotide 551 bis 4213 von SEQ ID NO: 9 umfasst, hybridisieren kann, und wobei das Gen ein Protein kodiert, das die Aktivität der Elo-Untereinheit des α-Ketoglutaratdehydrogenase-Komplexes hat.

3. Coryneformes Bakterium gemäß Anspruch 1, wobei die Mutation eine Deletion von einer oder mehreren Aminosäuren in dem Bereich, der Aminosäuren 698 bis 702 von SEQ ID NO: 10 umfasst, ist.

4. Coryneformes Bakterium gemäß Anspruch 1, wobei die Mutation aus dem Ersetzen einer Aminosäure, ausgewählt aus der Gruppe bestehend aus Lys an der Position 698, Leu an der Position 699, Arg an der Position 700, Tyr an der Position 702 und Kombinationen davon in der Aminosäuresequenz, die in SEQ ID NO: 10 gezeigt wird, besteht.

5. Coryneformes Bakterium gemäß Anspruch 1, wobei die Mutation aus der Deletion von einer oder mehreren der Aminosäuren im Bereich, der Aminosäuren 218 bis 224 von SEQ ID NO: 10 umfasst, besteht.

6. Verfahren zum Herstellen von L-Glutaminsäure, das umfasst:
a) Kultivieren des coryneformen Bakteriums gemäß einem der Ansprüche 1 bis 5 in einem Kulturmedium und
b) Gewinnen der L-Glutaminsäure aus dem Kulturmedium und/oder dem Bakterium.

7. Gen, das für eine mutante α-Ketoglutaratdehydrogenase kodiert, das ausgewählt wird aus der Gruppe bestehend aus:
(a) einem Gen, das Nukleotide 443 bis 4213 eines Polynukleotids, ausgewählt aus der Gruppe bestehend aus SEQ ID NO: 11, 13 und 15, oder einem Gen, umfassend Nukleotide 443 bis 4210 eines Polynukleotids, ausgewählt aus der Gruppe bestehend aus SEQ ID NO: 44, 46 und 48, umfasst,
(b) einem Gen, das unter stringenten Bedingungen mit einem Polynukleotid, das Nukleotide 443 bis 4213 eines Polynukleotids, ausgewählt aus der Gruppe bestehend aus SEQ ID NO: 11, 13 und 15, umfasst, einem Polynukleotid, das Nukleotide 443 bis 4213 eines Polynukleotids, ausgewählt aus der Gruppe bestehend aus SEQ ID NO: 44, 46 und 48,umfasst, einer Sonde, die hergerichtet ist, ausgehend von Nukleotiden 443 bis 4213 eines Polynukleotids, ausgewählt aus der Gruppe bestehend aus SEQ ID NO: 11, 13 und 15, oder einer Sonde, die hergerichtet ist, ausgehend von Nukleotiden 443 bis 4210 eines Polynukleotids, ausgewählt aus der Gruppe bestehend aus SEQ ID NO: 44, 46 und 4,8 hybridisieren kann, und wobei das Gen ein Protein kodiert, das eine α-Ketoglutaratdehydrogenase-Aktivität aufweist, die weniger als die Hälfte derjenigen eines Wildtyp-oder nicht-mutierten Stamms ist, wenn ein Komplex mit den E2o- und E3-Untereinheiten gebildet wird,
(c) einem Gen, das Nukleotide 551 bis 4213 eines Polynukleotids, ausgewählt aus der Gruppe bestehend aus SEQ ID NO: 11, 13 und 15, oder einem Gen, umfassend Nukleotide 551 bis 4210 eines Polynukleotids, ausgewählt aus der Gruppe bestehend aus SEQ ID NO: 44, 46 und 48, umfasst,
(d) einem Gen, das unter stringenten Bedingungen mit einem Polynukleotid, das Nukleotide 551 bis 4213 eines Polynukleotids, ausgewählt aus der Gruppe bestehend aus SEQ ID NO: 11, 13 und 15, umfasst, einem Polynukleotid, das Nukleotide 551 bis 4210 eines Polynukleotids, ausgewählt aus der Gruppe bestehend aus SEQ ID NO: 44, 46 und 48, umfasst, oder einer Sonde, die hergerichtet ist, ausgehend von Nukleotiden 551 bis 4213 eines Polynukleotids, ausgewählt aus der Gruppe bestehend aus SEQ ID NO: 11, 13 und 15, oder einer Sonde, die hergerichtet ist, ausgehend von Nukleotiden 551 bis 4213 eines Polynukleotids, ausgewählt aus der Gruppe bestehend aus SEQ ID NO: 44, 46 und 48, hybridisieren kann und wobei das Gen ein Protein kodiert, das eine α-Ketoglutaratdehydrogenase-Aktivität aufweist, die weniger als die Hälfte derjenigen eines Wildtyp-oder nicht-mutierten Stamms ist, wenn ein Komplex mit den E2o- und E3-Untereinheiten gebildet wird.

8. Mutante α-Ketoglutaratdehydrogenase, die ausgewählt wird aus der Gruppe bestehend aus:
(a) einem Protein, ausgewählt aus der Gruppe bestehend aus SEQ ID NO: 12, 14, 16, 45, 47 und 49,
(b) einem Protein, ausgewählt aus der Gruppe bestehend aus SEQ ID NO: 12, 14, 16, 45, 47 und 49, wobei eine oder mehrere Aminosäuren im Protein substituiert, entfernt oder hinzugefügt sind, und wobei das Protein eine α-Ketoglutaratdehydrogenase-Aktivität aufweist, die weniger als die Hälfte derjenigen eines Wildtyp- oder nicht-mutierten Stamms ist, wenn ein Komplex mit E2o- und E3-Untereinheiten gebildet wird,
(c) einem Protein, das Aminosäuren 37 bis 1256 einer Aminosäuresequenz, ausgewählt aus der Gruppe bestehend aus SEQ ID NO: 12, 45, 47 und 49, umfasst, einem Protein, das Aminosäure 37 bis 1255 einer Aminosäuresequenz von SEQ ID NO: 14 umfasst, oder einem Protein, das Aminosäuren 37 bis 1254 einer Aminosäuresequenz von SEQ ID NO: 16 umfasst, und
(d) einem Protein, das Aminosäuren 37 bis 1256 einer Aminosäuresequenz, ausgewählt aus der Gruppe bestehend aus SEQ ID NO: 12, 45, 47 und 49, umfasst, einem Protein, das Aminosäuren 37 bis 1255 einer Aminosäuresequenz von SEQ ID NO: 14 umfasst, oder einem Protein, das Aminosäuren 37 bis 1254 einer Aminosäuresequenz von SEQ ID NO: 16 umfasst, wobei eine oder mehrere Aminosäuren im Protein substituiert, entfernt oder hinzugefügt sind und wobei das Protein eine α-Ketoglutaratdehydrogenase-Aktivität aufweist, die weniger als die Hälfte derjenigen eines Wildtyp- oder nicht-mutierten Stamms ist, wenn ein Komplex mit E2o- und E3-Untereinheiten gebildet wird.

## Revendications

1. Bactérie corynéforme productrice d'acide L-glutamique comprenant
a) une activité α-cétoglutarate déshydrogénase intracellulaire qui est inférieure à la moitié de celle d'une souche non mutée ou de type sauvage, et
b) une mutation dans une région choisie dans le groupe constitué de (i) une région des nucléotides 2534 à 2548 de SEQ ID No : 9, et (ii) une région des nucléotides 1094 à 1114 de SEQ ID No : 9 d'un gène odhA chromosomique codant pour la sous-unité E1o du complexe de l'α-cétoglutarate déshydrogénase, dans laquelle ladite bactérie croît à 80% ou plus du taux de croissance d'une souche non mutée ou de type sauvage, et dans laquelle ledit gène odhA chromosomique code pour une protéine choisie dans le groupe constitué de :
(A) une protéine comprenant une séquence d'acides aminés de SEQ ID No : 10,
(B) une protéine comprenant une séquence d'acides aminés de SEQ ID No : 10, par quoi un ou 2 à 20 acides aminés dans ladite protéine sont substitués, délétés, insérés, ou ajoutés, et dans laquelle ladite protéine possède une activité de la sous-unité E1o du complexe de l'α-cétoglutarate déshydrogénase,
(C) une protéine comprenant les acides aminés 37 à 1257 de SEQ ID No : 10, et
(D) une protéine comprenant les acides aminés 37 à 1257 de SEQ ID No : 10, par quoi un ou 2 à 20 acides aminés dans ladite protéine sont substitués, délétés, insérés, ou ajoutés, et dans laquelle ladite protéine possède une activité de la sous-unité E1o du complexe de l'α-cétoglutarate déshydrogénase.

2. Bactérie corynéforme selon la revendication 1, dans laquelle ledit gène odhA chromosomique est choisi dans le groupe constitué de :
(a) un gène comprenant les nucléotides 443 à 4213 de SEQ ID No : 9,
(b) un gène qui est capable de s'hybrider dans des conditions stringentes à un polynucléotide comprenant les nucléotides 443 à 4213 de SEQ ID No : 9 ou une sonde préparée à partir d'un polynucléotide comprenant les nucléotides 443 à 4213 de SEQ ID No : 9, et dans lequel ledit gène code pour une protéine qui possède l'activité de la sous-unité E1o du complexe de l'α-cétoglutarate déshydrogénase,
(c) un gène comprenant les nucléotides 551 à 4213 de SEQ ID No : 9, et
(d) un gène qui est capable de s'hybrider dans des conditions stringentes à un polynucléotide comprenant les nucléotides 551 à 4213 de SEQ ID No : 9 ou une sonde préparée à partir d'un polynucléotide comprenant les nucléotides 551 à 4213 de SEQ ID No : 9, et dans lequel ledit gène code pour une protéine qui possède l'activité de la sous-unité E1o du complexe de l'α-cétoglutarate déshydrogénase.

3. Bactérie corynéforme selon la revendication 1, dans laquelle ladite mutation est une délétion d'un ou plusieurs acides aminés dans la région comprenant les acides aminés 698 à 702 de SEQ ID No : 10.

4. Bactérie corynéforme selon la revendication 1, dans laquelle ladite mutation est un remplacement d'un acide aminé choisi dans le groupe constitué de la Lys en position 698, la Leu en position 699, l'Arg en position 700, la Tyr en position 702, et les combinaisons de ceux-ci dans la séquence d'acides aminés présentée dans SEQ ID No : 10.

5. Bactérie corynéforme selon la revendication 1, dans laquelle ladite mutation est une délétion d'un ou plusieurs acides aminés dans la région comprenant les acides aminés 218 à 224 de SEQ ID No : 10.

6. Procédé de production d'acide L-glutamique comprenant :
a) la culture de la bactérie corynéforme selon l'une quelconque des revendications 1 à 5 dans un milieu de culture, et
b) la collecte de l'acide L-glutamique à partir du milieu de culture et/ou de la bactérie.

7. Gène codant pour une α-cétoglutarate déshydrogénase mutante choisie dans le groupe constitué de :
(a) un gène comprenant les nucléotides 443 à 4213 d'un polynucléotide choisi dans le groupe constitué de SEQ ID No : 11, 13, et 15, ou un gène comprenant les nucléotides 443 à 4210 d'un polynucléotide choisi dans le groupe constitué de SEQ ID No : 44, 46, et 48,
(b) un gène qui est capable de s'hybrider dans des conditions stringentes à un polynucléotide comprenant les nucléotides 443 à 4213 d'un polynucléotide choisi dans le groupe constitué de SEQ ID No : 11, 13, et 15, un polynucléotide comprenant les nucléotides 443 à 4210 d'un polynucléotide choisi dans le groupe constitué de SEQ ID No : 44, 46, et 48, une sonde préparée à partir des nucléotides 443 à 4213 d'un polynucléotide choisi dans le groupe constitué de SEQ ID No : 11, 13, et 15, ou une sonde préparée à partir des nucléotides 443 à 4210 d'un polynucléotide choisi dans le groupe constitué de SEQ ID No : 44, 46, et 48, et dans lequel ledit gène code pour une protéine qui possède une activité α-cétoglutarate déshydrogénase qui est inférieure à la moitié de celle d'une souche non mutée ou de type sauvage par la formation d'un complexe avec les sous-unités E2o et E3,
(c) un gène comprenant les nucléotides 551 à 4213 d'un polynucléotide choisi dans le groupe constitué de SEQ ID No : 11, 13, et 15, ou un gène comprenant les nucléotides 551 à 4210 d'un polynucléotide choisi dans le groupe constitué de SEQ ID No : 44, 46, et 48,
(d) un gène qui est capable de s'hybrider dans des conditions stringentes à un polynucléotide comprenant les nucléotides 551 à 4213 d'un polynucléotide choisi dans le groupe constitué de SEQ ID No : 11, 13, et 15, un polynucléotide comprenant les nucléotides 551 à 4210 d'un polynucléotide choisi dans le groupe constitué de SEQ ID No : 44, 46, et 48, ou une sonde préparée à partir des nucléotides 551 à 4213 d'un polynucléotide choisi dans le groupe constitué de SEQ ID No : 11, 13, et 15, ou une sonde préparée à partir des nucléotides 551 à 4210 d'un polynucléotide choisi dans le groupe constitué de SEQ ID No : 44, 46, et 48, et dans lequel ledit gène code pour une protéine qui possède une activité α-cétoglutarate déshydrogénase qui est inférieure à la moitié de celle d'une souche de type sauvage ou non mutée par la formation d'un complexe avec les sous-unités E2o et E3.

8. α-cétoglutarate déshydrogénase mutante choisie dans le groupe constitué de :
(a) une protéine choisie dans le groupe constitué de SEQ ID No : 12, 14, 16, 45, 47, et 49,
(b) une protéine choisie dans le groupe constitué de SEQ ID No : 12, 14, 16, 45, 47, et 49, par quoi un ou plusieurs acides aminés dans ladite protéine sont substitués, délétés, ou ajoutés, et dans laquelle ladite protéine présente une activité α-cétoglutarate déshydrogénase qui est inférieure à la moitié de celle d'une souche de type sauvage ou non mutée par la formation d'un complexe avec les sous-unités E2o et E3,
(c) une protéine comprenant les acides aminés 37 à 1256 d'une séquence d'acides aminés choisie dans le groupe constitué de SEQ ID No : 12, 45, 47, et 49, une protéine comprenant les acides aminés 37 à 1255 d'une séquence d'acides aminés de SEQ ID No : 14, ou une protéine comprenant les acides aminés 37 à 1254 d'une séquence d'acides aminés de SEQ ID No : 16, et
(d) une protéine comprenant les acides aminés 37 à 1256 d'une séquence d'acides aminés choisie dans le groupe constitué de SEQ ID No : 12, 45, 47, et 49, une protéine comprenant les acides aminés 37 à 1255 d'une séquence d'acides aminés de SEQ ID No : 14, ou une protéine comprenant les acides aminés 37 à 1254 d'une séquence d'acides aminés de SEQ ID No : 16, par quoi un ou plusieurs acides aminés dans ladite protéine sont substitués, délétés, ou ajoutés, et dans laquelle ladite protéine présente une activité α-cétoglutarate déshydrogénase qui est inférieure à la moitié de celle d'une souche de type sauvage ou non mutée par la formation d'un complexe avec les sous-unités E2o et E3.
